# EUROPÄISCHE PATENTSCHRIFT

(11) **EP 2 029 573 B1**
(45) Veröffentlichungstag und Bekanntmachung des Hinweises auf die Patenterteilung: **26.10.2011**
(21) Anmeldenummer: 07729616.8
(22) Anmeldetag: 29.05.2007
(51) Int. Cl.: C07D 401/10, C07D 471/06, C09B 5/62, B23K 26/00

(54) **DIBENZORYLENTETRACARBONSÄUREDIIMIDE ALS INFRAROTABSORBER**
DIBENZORYLENETETRACARBOXIMIDES AS INFRARED ABSORBERS
DIBENZORYLÈNE TÉTRACARBOXYDIIMIDE EN TANT QU'ABSORBEURS D'INFRAROUGE

(30) Priorität: 30.05.2006 EP 06114685
(43) Veröffentlichungstag der Anmeldung: 04.03.2009
(73) Patentinhaber: BASF SE, 67056 Ludwigshafen (DE); Max-Planck-Gesellschaft zur Förderung der Wissenschaften e.V., 80539 München (DE)
(72) Erfinder: KÖNEMANN, Martin, 68163 Mannheim (DE); BÖHM, Arno, 68305 Mannheim (DE); AVLASEVIC, Juri, 55122 Mainz (DE); MÜLLEN, Klaus, 50939 Köln (DE)
(86) Internationale Anmeldenummer: PCT/EP2007/055194
(87) Internationale Veröffentlichungsnummer: WO 2007/138051

(56) Entgegenhaltungen:
- WO-A-2005/102672
- DESILETS, DENIS; KAZMAIER, PETER M.; BURT, RICHARD A.: "Design and synthesis of near-infrared absorbing pigments. I. Use of Pariser-Parr-Pople molecular orbital calculations for the identification of near-infrared absorbing pigment candidates" CANADIAN JOURNAL OF CHEMISTRY, Bd. 73, Nr. 3, 1995, Seiten 319-324, XP002447201
- ADACHI M; NAGAO Y: "Design of Near-Infrared Dyes Based on .pi.-Conjugation System Extension 2. Theoretical Elucidation of Framework Extended Derivatives of Perylene Chromophore" CHEMISTRY OF MATERIALS, Bd. 13, Nr. 2, 2001, Seiten 662-669, XP002447202
- MUELLER, SIBYLLE; MUELLEN, KLAUS: "Facile synthetic approach to novel core-extended perylene carboximide dyes" CHEMICAL COMMUNICATIONS, Bd. 32, 2005, Seiten 4045-4046, XP002447203
- DESELETS, DENIS; KAZMAIER, PETER M.; BURT, RICHARD A.; HAMER, GORDON K.: "Design and synthesis of near-infrared absorbing pigments. II. Structure determination of acenanthrene green and derivatives" CANADIAN JOURNAL OF CHEMISTRY, Bd. 73, Nr. 3, 1995, Seiten 325-335, XP002447204
- AVLASEVICH, YURI; MUELLEN, KLAUS: "Dibenzopentarylenebis(dicarboximide)s: Novel near-infrared absorbing dyes" CHEMICAL COMMUNICATIONS, Bd. 42, 2006, Seiten 4440-4442, XP002447205

## Beschreibung

Die vorliegende Erfindung betrifft neue Dibenzorylentetracarbonsäurediimide der allgemeinen Formel I 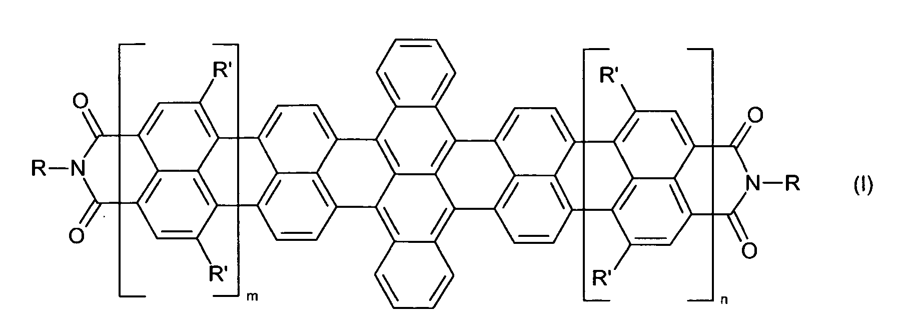 in der die Variablen folgende Bedeutung haben:
- R': gleiche oder verschiedene Reste:
Wasserstoff;
Aryloxy, Arylthio, Hetaryloxy oder Hetarylthio, an das jeweils weitere gesättigte oder ungesättigte 5- bis 7-gliedrige Ringe, deren Kohlenstoffgerüst durch eine oder mehrere Gruppierungen -O-, -S-, -NR¹-, -N=CR¹-, -CO-, -SO- und/oder -SO₂- unterbrochen sein kann, anelliert sein können, wobei das gesamte Ringsystem ein- oder mehrfach durch die Reste (i), (ii), (iii), (iv) und/oder (v) substituiert sein kann:
(i) C₁-C₃₀-Alkyl, dessen Kohlenstoffkette durch eine oder mehrere Gruppierungen -O-, -S-, -NR¹-, -N=CR¹-, -C≡C-, -CR¹=CR¹-, -CO-, -SO- und/oder -SO₂- unterbrochen sein kann und das ein- oder mehrfach substituiert sein kann durch: C₁-C₁₂-Alkoxy, C₁-C₆-Alkylthio, -C≡CR¹, -CR¹=CR¹₂, Hydroxy, Mercapto, Halogen, Cyano, Nitro, -NR²R³, -NR²COR³, -CONR²R³, -SO₂NR²R³, -COOR², -SO₃R², Aryl und/oder gesättigtes oder ungesättigtes C₄-C₇-Cycloalkyl, dessen Kohlenstoffgerüst durch eine oder mehrere Gruppierungen -O-, -S-, -NR¹-, -N=CR¹-, -CR¹=CR¹-, -CO-, -SO- und/oder -SO₂- unterbrochen sein kann, wobei die Aryl- und Cycloalkylreste jeweils ein- oder mehrfach durch C₁-C₁₈-Alkyl und/oder die vorstehenden, als Substituenten für Alkyl genannten Reste substituiert sein können;
(ii) C₃-C₈-Cycloalkyl, dessen Kohlenstoffgerüst durch eine oder mehrere Gruppierungen -O-, -S-, -NR¹-, -N=CR¹-, -CR¹=CR¹-, -CO-, -SO- und/oder -SO₂- unterbrochen sein kann und an das weitere gesättigte oder ungesättigte 5- bis 7-gliedrige Ringe, deren Kohlenstoffgerüst durch eine oder mehrere Gruppie- rungen -O-, -S-, -NR¹-, -N=CR¹-, -CR¹=CR¹-, -CO-, -SO- und/oder -SO₂- unterbrochen sein kann, anelliert sein können, wobei das gesamte Ringsystem ein- oder mehrfach substituiert sein kann durch: C₁-C₁₈-Alkyl, C₁-C₁₂-Alkoxy, C₁-C₆-Alkylthio, -C≡CR¹, -CR¹=CR¹₂, Hydroxy, Mercapto, Halogen, Cyano, Nitro, -NR²R³, -NR²COR³, -CONR²R³, -SO₂NR²R³, -COOR² und/oder-SO₃R²;
(iii) Aryl oder Hetaryl, an das weitere gesättigte oder ungesättigte 5- bis 7-gliedrige Ringe, deren Kohlenstoffgerüst durch eine oder mehrere Gruppierungen -O-, -S-, -NR¹-, -N=CR¹, -CR¹=CR¹-, -CO-, -SO- und/oder -SO₂- unterbrochen sein kann, anelliert sein können, wobei das gesamte Ringsystem ein- oder mehrfach substituiert sein kann durch: C₁-C₁₈-Alkyl, C₁-C₁₂-Alkoxy, C₁-C₆-Alkylthio, -C≡CR¹, -CR¹=CR¹₂, Hydroxy, Mercapto, Halogen, Cyano, Nitro, -NR²R³, -NR²COR³, -CONR²R³, -SO₂NR²R³, -COOR², -SO₃R², Aryl und/oder Hetaryl, das jeweils ein- oder mehrfach durch C₁-C₁₈-Alkyl, C₁-C₁₂-Alkoxy, Hydroxy, Mercapto, Halogen, Cyano, Nitro, -NR²R³, -NR²COR³, -CONR²R³, -SO₂NR²R³, -COOR² und/oder -SO₃R² substituiert sein kann;
(iv) ein Rest -U-Aryl, der ein- oder mehrfach durch die vorstehenden, als Substituenten für die Arylreste (iii) genannten Reste substituiert sein kann, wobei U eine Gruppierung -O-, -S-, -NR¹-, -CO-, -SO- oder -SO₂- bedeutet;
(v) C₁-C₁₂-Alkoxy, C₁-C₆-Alkylthio, -C≡CR¹, -CR¹=CR¹₂, Hydroxy, Mercapto, Halogen, Cyano, Nitro, -NR²R³, -NR²COR³, -CONR²R³, -SO₂NR²R³, -COOR² oder -SO₃R²;
- R: gleiche oder verschiedene Reste:
Wasserstoff;
C₁-C₃₀-Alkyl, dessen Kohlenstoffkette durch eine oder mehrere Gruppierungen -O-, -S-, -NR¹-, -N=CR¹-, -C≡C-, -CR¹=CR¹-, -CO-, -SO- und/oder -SO₂- unterbrochen sein kann und das ein- oder mehrfach durch die als Substituenten für die Reste R' genannten Reste (ii), (iii), (iv) und/oder (v) substituiert sein kann; C₃-C₈-Cycloalkyl, an das weitere gesättigte oder ungesättigte 5- bis 7-gliedrige Ringe, deren Kohlenstoffgerüst durch eine oder mehrere Gruppierungen -O-, -S-, -NR¹-, -N=CR¹-, -CR¹=CR¹-, -CO-, -SO- und/oder-SO₂- unterbrochen sein kann, anelliert sein können, wobei das gesamte Ringsystem ein- oder mehrfach durch die als Substituenten für die Reste R' genannten Reste (i), (ii), (iii), (iv) und/oder (v) substituiert sein kann;
Aryl oder Hetaryl, an das jeweils weitere gesättigte oder ungesättigte 5- bis 7-gliedrige Ringe, deren Kohlenstoffgerüst durch eine oder mehrere Gruppierungen -O-, -S-, -NR¹-, -N=CR¹-, -CR¹=CR¹-, -CO-, -SO- und/oder-SO₂₋ unterbrochen sein kann, anelliert sein können, wobei das gesamte Ringsystem ein- oder mehrfach substituiert sein kann durch die als Substituenten für die Reste R' genannten Reste (i), (ii), (iii), (iv), (v) und/oder Aryl- und/oder Hetarylazo, das jeweils ein- oder mehrfach durch C₁-C₁₀-Alkyl, C₁-C₆-Alkoxy und/oder Cyano substituiert sein kann;

- R¹: Wasserstoff oder C₁-C₁₈-Alkyl, wobei die Reste R¹ gleich oder verschieden sein können, wenn sie mehrfach auftreten;
- R², R³: unabhängig voneinander:
Wasserstoff;
C₁-C₁₈-Alkyl, dessen Kohlenstoffkette durch eine oder mehrere Gruppierungen -O-, -S-, -CO-, -SO- und/oder -SO₂- unterbrochen sein kann und das ein- oder mehrfach durch C₁-C₁₂-Alkoxy, C₁-C₆-Alkylthio, Hydroxy, Mercapto, Halogen, Cyano, Nitro und/oder -COOR¹ substituiert sein kann;
Aryl oder Hetaryl, an das jeweils weitere gesättigte oder ungesättigte 5- bis 7-gliedrige Ringe, deren Kohlenstoffgerüst durch eine oder mehrere Gruppierungen -O-, -S-, -CO- und/oder -SO₂- unterbrochen sein kann, anelliert sein können, wobei das gesamte Ringsystem ein- oder mehrfach durch C₁-C₁₂-Alkyl und/oder die vorstehenden, als Substituenten für Alkyl genannten Reste substituiert sein kann;
- m, n: unabhängig voneinander 0 oder 1.

Außerdem betrifft die vorliegende Erfindung die Herstellung der Verbindungen der Formel I. Des Weiteren betrifft die vorliegende Erfindung die Verwendung der Verbindungen der Formel I zur Einfärbung von hochmolekularen organischen und anorganischen Materialien, als IR-Laserstrahlen absorbierende Materialien beim Schweißbehandeln von Kunststoffteilen, zur Herstellung im nahinfraroten Bereich des elektromagnetischen Spektrums absorbierender wässriger Polymerisatdispersionen, zur Erzeugung für das menschliche Auge nicht sichtbarer, Infrarotlicht absorbierender Markierungen und Beschriftungen, als Infrarotabsorber für das Wärmemanagement sowie als Aktivkomponenten in der Photovoltaik.

Außerdem betrifft die Erfindung die neue Tetracen-5,11-bis(rylendicarbonsäureimide) der allgemeinen Formel IV 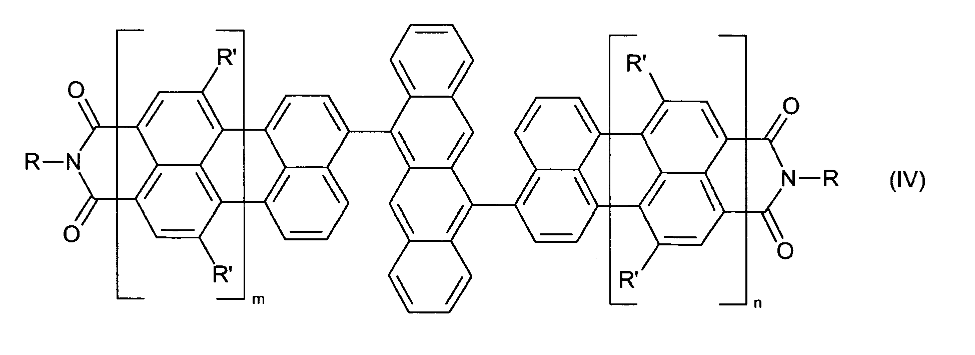 und neue Bisrylenderivate der allgemeinen Formel V 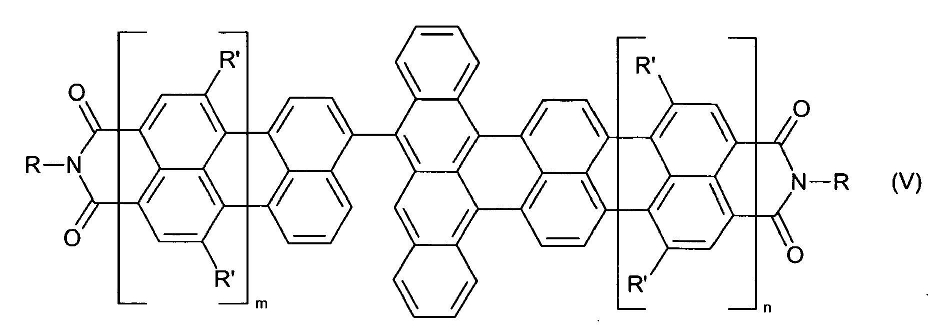 worin R und R' die zuvor genannten Bedeutungen aufweisen, als Zwischenprodukte für die Dibenzorylentetracarbonsäurediimide I.

Das Verschweißen oder Schneiden von Kunststoffen mit Laserstrahlen ist eine vorteilhafte Methode, um Kunststoffartikel mit komplexer Geometrie zu fertigen. Voraussetzung hierfür ist, dass der zu behandelnde Kunststoff die Laserstrahlung, die bei den kommerziellen Lasern bei 808 nm, 940 bis 980 nm und 1064 nm liegt, absorbiert. Er muss daher mit Substanzen, die bei diesen Wellenlängen absorbieren, additiviert oder beschichtet werden.

In der WO-A-2005/102 672 werden für diesen Zweck IR-Absorber auf Basis von Rylenverbindungen, u. a. Quaterrylentetracarbonsäurediimide, sowie dotierte Zinnoxide, wie ATO und ITO (Antimon- bzw. Indium-Zinnoxid) und Metallhexaboride, wie Lanthanhexaborid, beschrieben. Die Quaterrylentetracarbonsäurediimide können dabei vorteilhaft in Kombination mit den bei 808 nm emittierenden Lasern eingesetzt werden, die längeren Wellenlängen sind mit den anorganischen Absorbern zugänglich.

Für eine Reihe von Anwendungen, z. B. für Anwendungen im Außenbereich sowie im medizinischen oder Lebensmittelbereich, weisen die anorganischen Absorber jedoch nicht die erforderlichen Stabilitäten auf.

In der älteren deutschen Patentanmeldung 102005018241.0 werden Pentarylen- und Hexarylentetracarbonsäurediimide beschrieben, deren Absorption gegenüber den Quaterrylentetracarbonsäurediimiden bathochrom verschoben ist und sich bis in den Bereich um 975 nm erstreckt.

Die WO 2007/ 014902 beschreibt Mehrfachchromophore auf Rylenbasis.

Weiterhin besteht ein großer Bedarf an organischen Verbindungen, die sich als organische Halbleiter, insbesondere Halbleiter vom n-Typ und speziell für einen Einsatz in organischen Feldeffekttransistoren und Solarzellen eignen.

Die unveröffentlichte PCT/EP2006/070143 beschreibt Verbindungen der allgemeinen Formel 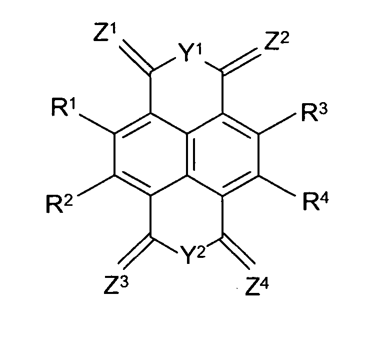 wobei
wenigstens einer der Reste R¹, R², R³ und R⁴ für Br, F oder CN steht,
Y¹ und Y² für O oder NRⁱ stehen, wobei Rⁱ für Wasserstoff oder einen Organylrest
steht,
Z¹, Z², Z³ und Z⁴ unabhängig voneinander für O oder NRⁱⁱ stehen, wobei Rⁱⁱ für einen Organylrest steht,
wobei für den Fall, dass Y¹ und/oder Y² für NRⁱ steht und wenigstens einer der Reste Z¹ bis Z⁴ für NRⁱⁱ steht, Rⁱ mit einem Rest Rⁱⁱ auch gemeinsam für eine verbrückende Gruppe mit 2 bis 5 Atomen zwischen den flankierenden Bindungen stehen kann,
und deren Verwendung als n-Halbleiter in organischen Feldeffekttransistoren.

Die unveröffentlichte PCT/EP2007/051532 beschreibt die Verwendung von Verbindungen der allgemeinen Formel 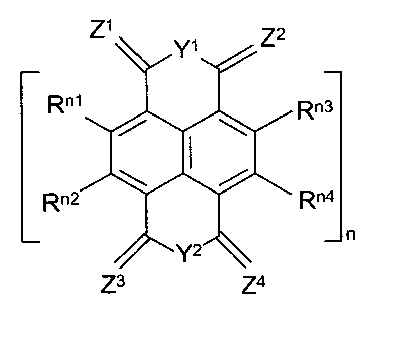 wobei
n für 2, 3 oder 4 steht,
wenigstens einer der Reste Rⁿ¹, Rⁿ², Rⁿ³ und Rⁿ⁴ für Fluor steht,
gegebenenfalls wenigstens ein weiterer Rest Rⁿ¹, Rⁿ², Rⁿ³ und Rⁿ⁴ für einen Substituenten steht, der unabhängig ausgewählt ist unter Cl und Br, und die übrigen
Reste für Wasserstoff stehen,
Y¹ und Y² für O oder NRⁱ stehen, wobei Rⁱ für Wasserstoff oder einen Organylrest
steht,
Z¹, Z², Z³ und Z⁴ für O stehen,
wobei für den Fall, dass Y¹ und/oder Y² für NRⁱ steht, auch einer der Reste Z¹ bis Z⁴ für NRⁱⁱ stehen kann, wobei die Reste Rⁱ und Rⁱⁱ gemeinsam für eine verbrückende Gruppe mit 2 bis 5 Atomen zwischen den flankierenden Bindungen stehen,
als Halbleiter, insbesondere n-Halbleiter, in der organischen Elektronik, insbesondere für organische Feldeffekttransistoren, Solarzellen und organische Leuchtdioden.

Die unveröffentlichte PCT/EP2007/053330 beschreibt die Verwendung von Verbindungen der allgemeinen Formeln I und II 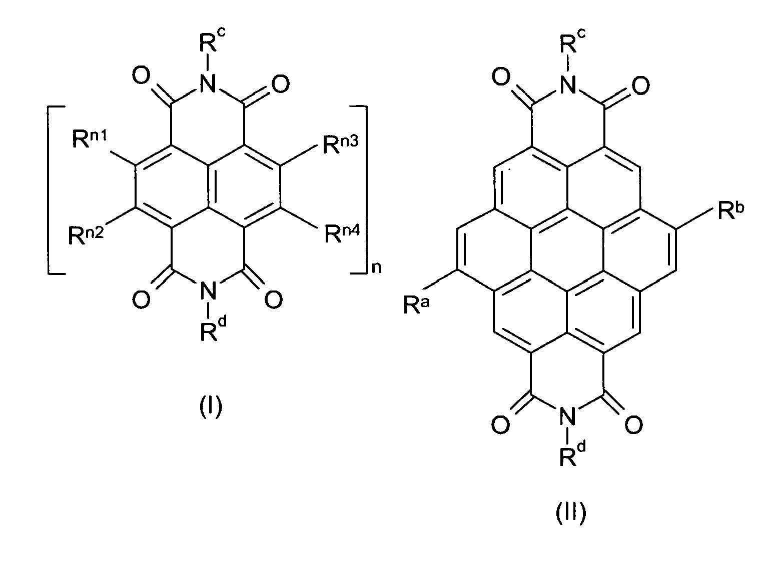 wobei
n für 1, 2, 3 oder 4 steht,
die Reste Rⁿ¹, Rⁿ², Rⁿ³ und Rⁿ⁴ für n = 1 oder 2 unabhängig voneinander ausgewählt
sind unter Wasserstoff, F, Cl, Br und CN, für n = 3 oder 4 unabhängig voneinander ausgewählt sind unter Wasserstoff, F, Cl und Br,
die Reste R^{a} und R^{b} unabhängig voneinander ausgewählt sind unter Wasserstoff und Alkyl,
die Reste R^{c} und R^{d} unabhängig voneinander ausgewählt sind unter Gruppen der Formeln II.1 bis II.5:

#-(A)ₚ-C(Rⁱ)ₓ (II.1)

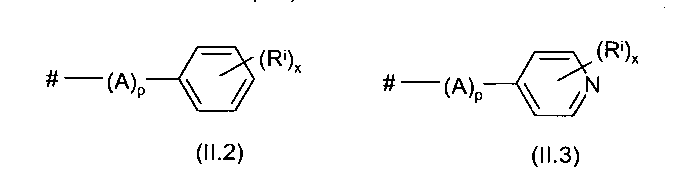

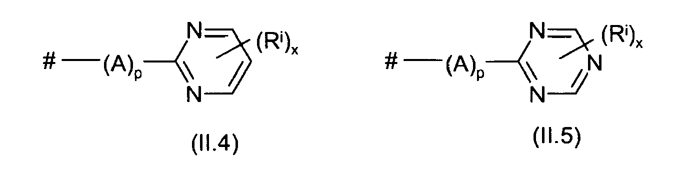

worin
- #: für die Verknüpfungsstelle zum Imidstickstoffatom steht,
- p: für 0 oder 1 steht,
- x: für 2 oder 3 steht,
- A: soweit vorhanden, für eine C₁-C₁₀-Alkylengruppe steht, die durch eine oder mehrere nicht benachbarte Gruppen, die ausgewählt sind unter -O- und -S-, unterbrochen sein kann,
die Reste Rⁱ jeweils unabhängig voneinander ausgewählt sind unter C₄-C₃₀-Alkyl, welche durch ein oder mehrere nicht benachbarte Sauerstoffatom(e) unterbrochen sein können, wobei in den Verbindungen der Formel II.1 wenigstens einer der Reste Rⁱ auch für C₄-C₃₀-Alkyloxy oder C₄-C₃₀-Alkylthio stehen kann,
als n-Halbleiter für organische Feldeffekttransistoren oder Solarzellen.

K. Müllen et al. beschreiben in einem Artikel in Chem. Mater. 2006, 18, 3715 - 3725 die Verwendung von Rylentetracarbonsäurediimiden und Coronentetracarbonsäurediimiden mit verzweigten Alkylresten an den Imidstickstoffen als n-Halbleiter für organische Feldeffekttransistoren und in photovoltaischen Zellen.

K. Müllen und Y. Avlasevich beschreiben in einem nicht vorveröffentlichten Artikel in Chem. Commun., 2006,4440-4442 Dibenzopentarylentetracarbonsäurediimiden als kernerweiterte Rylenchromophore und deren Verwendung als im NIR absorbierende Farbstoffe.D. Désilets et al. beschreiben in ihrem Artikel in Can. J. Chem. 73, 319-324 (1994) Pigmente aus N,N'-Dialkyl-3,4,9,10-perylentetracarbonsäureimiden als Infrarotstrahlung absorbierende Pigmente.
D. Désilets et al. erläutern weiterhin in ihrem Artikel in Can. J. Chem. 73, 325-335 (1995), dass die Struktur des Pigmentes "aceanthrene green" durch das 7,8,15,16-Dibenzo[a,j]perylentetracarbonsäurediimid beschrieben wird.
M. Adachi and Y. Nagao beschreiben in ihrem Artikel in Chem. Mater. 2001, 13, 662 Derivate des 3,4,9,10-Perylentetracarbonsäuredianhydriddiimids als polyaromatische Farbstoffe, die Strahlung im Nahinfrarotbereich absorbieren.
S. Müller und K. Müllen beschreiben in ihrem Artikel in Chem. Commun., 2005, 4045-4046 die Synthese der beiden kernerweiterten Perylen-Chromophoren Dibenzocoronentetracarbonsäurediimid und Indenoperylendicarbonsäuremonoimiden, die aufgrund ihres vergrößerten aromatischen π-Systems eine bathochrome bzw. hypsochrome Verschiebung aufweisen.

Der vorliegenden Erfindung liegt die Aufgabe zugrunde, organische Verbindungen bereitzustellen, die besonders langwellig absorbieren und daher auch vorteilhaft in Kombination mit den bei hohen Wellenlängen (940 bis 980 bzw. vor allem 1064 nm) emittierenden Lasern eingesetzt werden können. Der vorliegenden Erfindung liegt weiterhin die Aufgabe zu Grunde, solche Verbindungen für die Verwendung in der organischen Elektronik und Photovoltaik, insbesondere als Halbleiter in excitonischen Solarzellen, zur Verfügung zu stellen.

Demgemäß wurden die Dibenzorylentetracarbonsäurediimide der eingangs definierten Formel I gefunden.

Ein erster Gegenstand der vorliegenden Erfindung sind daher Verbindungen der Formel I, 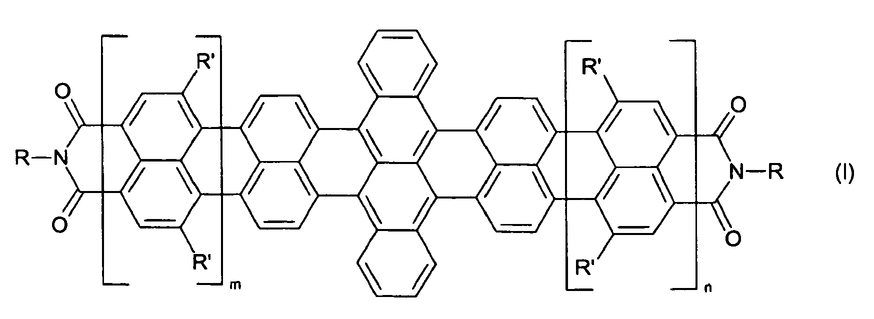 worin die Variablen folgende Bedeutung haben:
- R': gleiche oder verschiedene Reste:
Wasserstoff;
Aryloxy, Arylthio, Hetaryloxy oder Hetarylthio, an das jeweils weitere gesättigte oder ungesättigte 5- bis 7-gliedrige Ringe, deren Kohlenstoffgerüst durch eine oder mehrere Gruppierungen -O-, -S-, -NR¹-, -N=CR¹-, -CO-, -SO- und/oder - SO₂- unterbrochen sein kann, anelliert sein können, wobei das gesamte Ringsystem ein- oder mehrfach durch die Reste (i), (ii), (iii), (iv) und/oder (v) substituiert sein kann:
(i) C₁-C₃₀-Alkyl, dessen Kohlenstoffkette durch eine oder mehrere Gruppierungen -O-, -S-, -NR¹-, -N=CR¹-, -C≡C-, -CR¹=CR¹-, -CO-, -SO- und/oder -SO₂- unterbrochen sein kann und das ein- oder mehrfach substituiert sein kann durch: C₁-C₁₂-Alkoxy, C₁-C₆-Alkylthio, -C≡CR¹, -CR¹=CR¹₂, Hydroxy, Mercapto, Halogen, Cyano, Nitro, -NR²R³, -NR²COR³, -CONR²R³, -SO₂NR²R³, -COOR², -SO₃R², Aryl und/oder gesättigtes oder ungesättigtes C₄-C₇-Cycloalkyl, dessen Kohlenstoffgerüst durch eine oder mehrere Gruppierungen -O-, -S-, -NR¹-, -N=CR¹-, -CR¹=CR¹-, -CO-, -SO- und/oder -SO₂- unterbrochen sein kann, wobei die Aryl-und Cycloalkylreste jeweils ein- oder mehrfach durch C₁-C₁₈-Alkyl, C₁-C₁₂-Alkoxy, C₁-C₆-Alkylthio, -C≡CR¹, -CR¹=CR¹₂, und/oder die vorstehenden, als Substituenten für Alkyl genannten Reste substituiert sein können;
(ii) C₃-C₈-Cycloalkyl, dessen Kohlenstoffgerüst durch eine oder mehrere Gruppierungen -O-, -S-, -NR¹-, -N=CR¹-, -CR¹=CR¹-, -CO-, -SO- und/oder -SO₂- unterbrochen sein kann und an das weitere gesättigte oder ungesättigte 5- bis 7-gliedrige Ringe, deren Kohlenstoffgerüst durch eine oder mehrere Gruppierungen -O-, -S-, -NR¹-, -N=CR¹-, -CR¹=CR¹-, -CO-, -SO- und/oder -SO₂- unterbrochen sein kann, anelliert sein können, wobei das gesamte Ringsystem ein- oder mehrfach substituiert sein kann durch: C₁-C₁₈-Alkyl, C₁-C₁₂-Alkoxy, C₁-C₆-Alkylthio, -C≡CR¹, -CR¹=CR¹₂, Hydroxy, Mercapto, Halogen, Cyano, Nitro, -NR²R³, -NR²COR³, -CONR²R³, -SO₂NR²R³, -COOR² und/oder -SO₃R²;
(iii) Aryl oder Hetaryl, an das weitere gesättigte oder ungesättigte 5- bis 7-gliedrige Ringe, deren Kohlenstoffgerüst durch eine oder mehrere Gruppierungen -O-, -S-, -NR¹-, -N=CR¹-, -CR¹=CR¹-, -CO-, -SO- und/oder -SO₂- unterbrochen sein kann, anelliert sein können, wobei das gesamte Ringsystem ein- oder mehrfach substituiert sein kann durch: C₁-C₁₈-Alkyl, C₁-C₁₂-Alkoxy, C₁-C₆-Alkylthio, -C≡CR¹, -CR¹=CR¹₂, Hydroxy, Mercapto, Halogen, Cyano, Nitro, -NR²R³, -NR²COR³, -CONR²R³, -SO₂NR²R³, -COOR², -SO₃R², Aryl und/oder Het-aryl, das jeweils ein- oder mehrfach durch C₁-C₁₈-Alkyl, C₁-C₁₂-Alkoxy, Hydroxy, Mercapto, Halogen, Cyano, Nitro, -NR²R³, -NR²COR³, -CONR²R³, -SO₂NR²R³, -COOR² und/oder -SO₃R² substituiert sein kann;
(iv) ein Rest -U-Aryl, der ein- oder mehrfach durch die vorstehenden, als Substituenten für die Arylreste (iii) genannten Reste substituiert sein kann, wobei U eine Gruppierung -O-, -S-, -NR¹-, -CO-, -SO- oder -SO₂₋ bedeutet;
(v) C₁-C₁₂-Alkoxy, C₁-C₆-Alkylthio, -C≡CR¹, -CR¹=CR¹₂, Hydroxy, Mercapto, Halogen, Cyano, Nitro, -NR²R³, -NR²COR³, -CONR²R³, -SO₂NR²R³, -COOR² oder -SO₃R²;
- R: gleiche oder verschiedene Reste:
Wasserstoff; C₁-C₃₀-Alkyl, dessen Kohlenstoffkette durch eine oder mehrere Gruppierungen -O-, -S-, -NR¹-, -N=CR¹-, -C≡C-, -CR¹=CR¹-, -CO-, -SO- und/oder -SO₂- unterbrochen sein kann und das ein- oder mehrfach durch die als Substituenten für die Reste R' genannten Reste (ii), (iii), (iv), (v) - Hydroxy, Mercapto, Halogen, Cyano, Nitro, -NR²R³, -NR²COR³, -CONR²R³, -SO₂NR²R³, -COOR² und/oder -SO₃R² substituiert sein kann;
C₃-C₈-Cycloalkyl, an das weitere gesättigte oder ungesättigte 5- bis 7-gliedrige Ringe, deren Kohlenstoffgerüst durch eine oder mehrere Gruppierungen -O-, -S-, -NR¹-, -N=CR¹-, -CR¹=CR¹-, -CO-, -SO- und/oder -SO₂- unterbrochen sein kann, anelliert sein können, wobei das gesamte Ringsystem ein- oder mehrfach durch die als Substituenten für die Reste R' genannten Reste (i), (ii), (iii), (iv) und/oder (v) substituiert sein kann;
Aryl oder Hetaryl, an das jeweils weitere gesättigte oder ungesättigte 5- bis 7-gliedrige Ringe, deren Kohlenstoffgerüst durch eine oder mehrere Gruppierungen -O-, -S-, -NR¹-, -N=CR¹-, -CR¹=CR¹-, -CO-, -SO- und/oder -SO₂- unterbrochen sein kann, anelliert sein können, wobei das gesamte Ringsystem ein- oder mehrfach substituiert sein kann durch die als Substituenten für die Reste R' genannten Reste (i), (ii), (iii), (iv), (v) und/oder Aryl- und/oder Hetarylazo, das jeweils ein- oder mehrfach durch C₁-C₁₀-Alkyl, C₁-C₆-Alkoxy und/oder Cyano substituiert sein kann;
- R¹: Wasserstoff oder C₁-C₁₈-Alkyl, wobei die Reste R¹ gleich oder verschieden sein können, wenn sie mehrfach auftreten;
- R², R³: unabhängig voneinander:
Wasserstoff;
C₁-C₁₈-Alkyl, dessen Kohlenstoffkette durch eine oder mehrere Gruppierungen -O-, -S-, -CO-, -SO- und/oder -SO₂- unterbrochen sein kann und das ein- oder mehrfach durch C₁-C₁₂-Alkoxy, C₁-C₆-Alkylthio, Hydroxy, Mercapto, Halogen, Cyano, Nitro, Aryl und/oder -COOR¹ substituiert sein kann; Aryl oder Hetaryl, an das jeweils weitere gesättigte oder ungesättigte 5- bis 7-gliedrige Ringe, deren Kohlenstoffgerüst durch eine oder mehrere Gruppierungen -O-, -S-, -CO- und/oder -SO₂- unterbrochen sein kann, anelliert sein können, wobei das gesamte Ringsystem ein- oder mehrfach durch C₁-C₁₂-Alkyl und/oder die vorstehenden, als Substituenten für Alkyl genannten Reste substituiert sein kann;
C₃-C₈-Cycloalkyl, an das weitere gesättigte oder ungesättigte 5- bis 7-gliedrige Ringe, deren Kohlenstoffgerüst durch eine oder mehrere Gruppierungen -O-, -S-, -NR¹-, -N=CR¹-, -CR¹=CR¹-, -CO-, -SO- und/oder -SO₂- unterbrochen sein kann, anelliert sein können, wobei das gesamte Ringsystem ein- oder mehrfach durch C₁-C₁₂-Alkyl und/oder die vorstehenden, als Substituenten für Alkyl genannten Reste substituiert sein kann;
- m, n: unabhängig voneinander 0 oder 1.

Weiterhin wurde ein Verfahren zur Herstellung von Dibenzorylentetracarbonsäurediimiden der allgemeinen Formel la 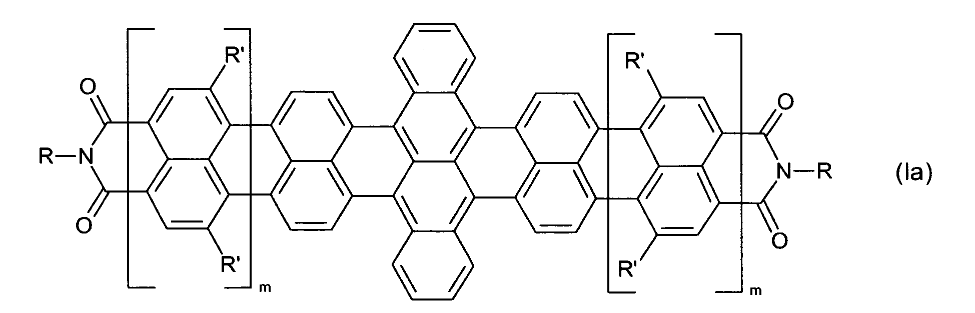 in der R, R' und m die eingangs angegebene Bedeutung haben, gefunden, welches **dadurch gekennzeichnet ist, dass** man
a) ein peri-(Dioxaborolan-2-yl)rylendicarbonsäureimid der allgemeinen Formel IIa 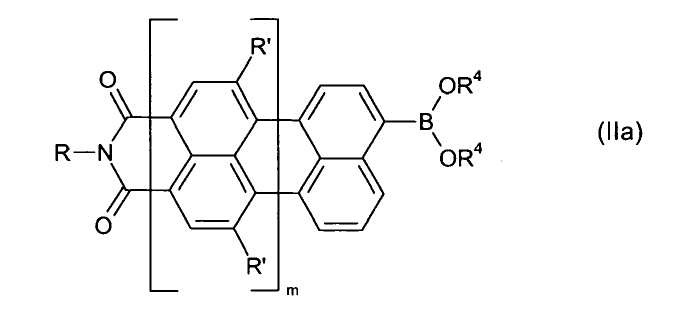 worin R⁴ gleich oder verschieden sind und unabhängig voneinander für Wasserstoff, C₁-C₃₀-Alkyl, C₅-C₈-Cycloalkyl, Aryl oder Heteroaryl stehen, und wobei zwei am selben Boratom gebundene Reste OR⁴ gemeinsam auch für -OCH₂CH₂O-stehen können, worin 1, 2, 3 oder 4 Wasserstoffatome auch durch C₁-C₃₀-Alkyl-, C₅-C₈-Cycloalkyl-, Aryl- oder Heteroarylgruppen ersetzt sein können,
   in Gegenwart eines organischen Lösungsmittels, gewünschtenfalls im Gemisch mit Wasser, sowie eines Übergangsmetallkatalysators und einer Base einer Suzuki-Kupplungsreaktion mit 5,11-Dibromtetracen der Formel III 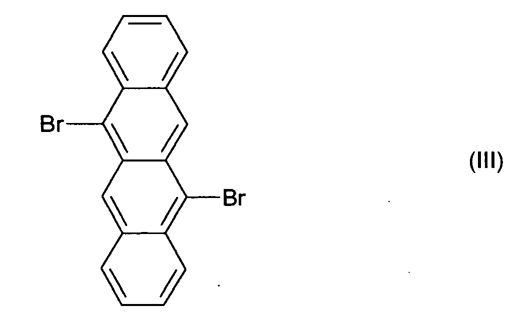 unterzieht,
b) das in Schritt a) gebildete Tetracen-5,11-bis(rylendicarbonsäureimid) der allgemeinen Formel IVa 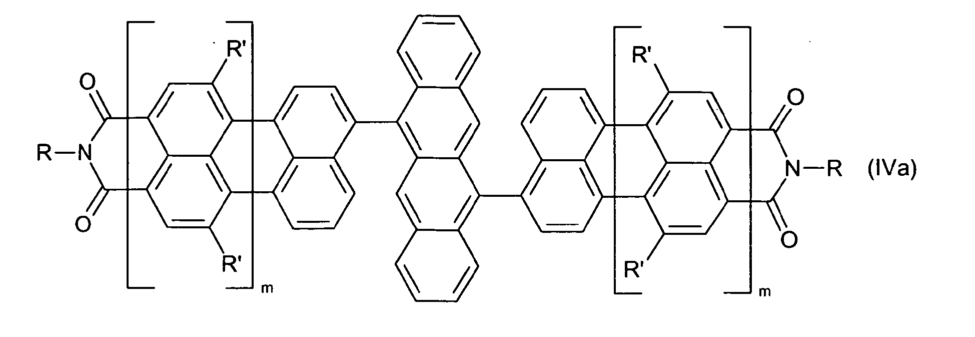 einer ersten Cyclodehydrierung in Gegenwart eines inerten organischen Lösungsmittels und einer Lewis-Säure unterzieht und
c) das in Schritt b) gebildete Bisrylenderivat der allgemeinen Formel Va 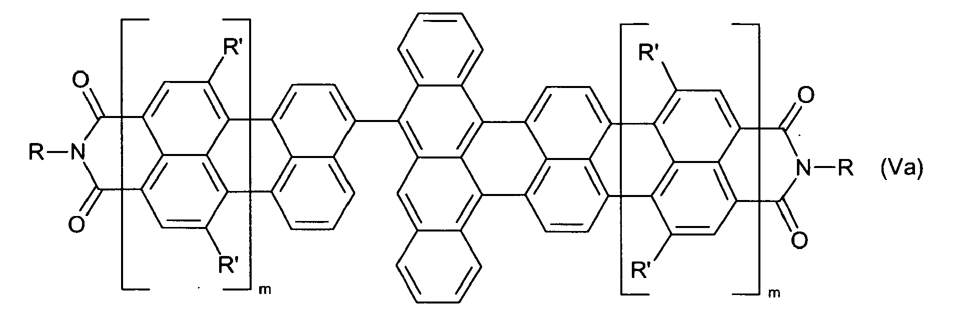 in einem Hydroxy- und Aminofunktionen aufweisenden und eine im Wesentlichen ungelöste Base enthaltenden organischen Reaktionsmedium weiter zum Dibenzorylentetracarbonsäurediimid la cyclodehydriert.

Außerdem wurde ein Verfahren zur Herstellung von Dibenzorylentetracarbonsäurediimiden der allgemeinen Formel Ib 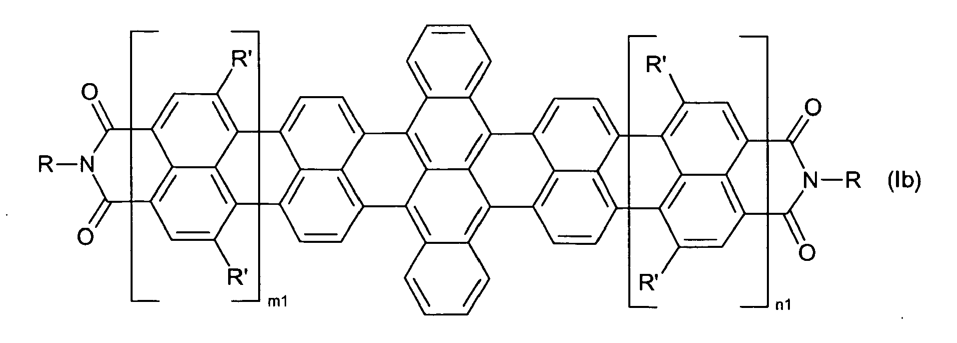 in der R und R' die eingangs angegebene Bedeutung haben, m1 und n1 voneinander verschieden sind und 0 oder 1 bedeuten, gefunden, welches **dadurch gekennzeichnet ist, dass** man
a1) ein peri-(Dioxaborolan-2-yl)rylendicarbonsäureimid der allgemeinen Formel IIb1 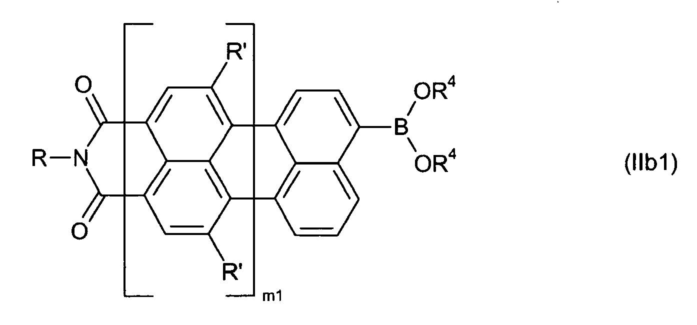 worin R⁴ gleich oder verschieden sind und unabhängig voneinander für Wasserstoff, C₁-C₃₀-Alkyl, C₅-C₈-Cycloalkyl, Aryl oder Heteroaryl stehen, und wobei zwei am selben Boratom gebundene Reste OR⁴ gemeinsam auch für -OCH₂CH₂O-stehen können, worin 1, 2, 3 oder 4 Wasserstoffatome auch durch C₁-C₃₀-Alkyl-, C₅-C₈-Cycloalkyl-, Aryl- oder Heteroarylgruppen ersetzt sein können,
   einer ersten Suzuki-Kupplungsreaktion mit 5,11-Dibromtetracen (III) unterzieht und
a2) das in Schritt a1) gebildete 5-Bromtetracen-11-rylendicarbonsäureimid der allgemeinen Formal III' 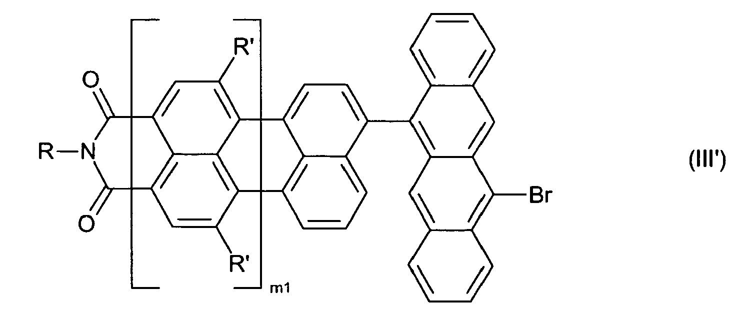 einer zweiten Suzuki-Kupplungsreaktion mit einem peri-(Dioxaborolan-2-yl)-rylendicarbonsäureimid der allgemeinen Formel IIb2 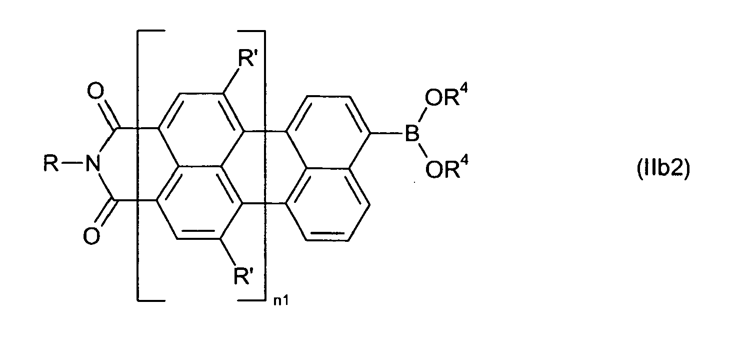 in Gegenwart eines organischen Lösungsmittels, gewünschtenfalls im Gemisch mit Wasser, sowie eines Übergangsmetallkatalysators und einer Base unterzieht,
b) das in Schritt a2) gebildete Tetracen-5,11-bis(rylendicarbonsäureimid) der allgemeinen Formel IVb 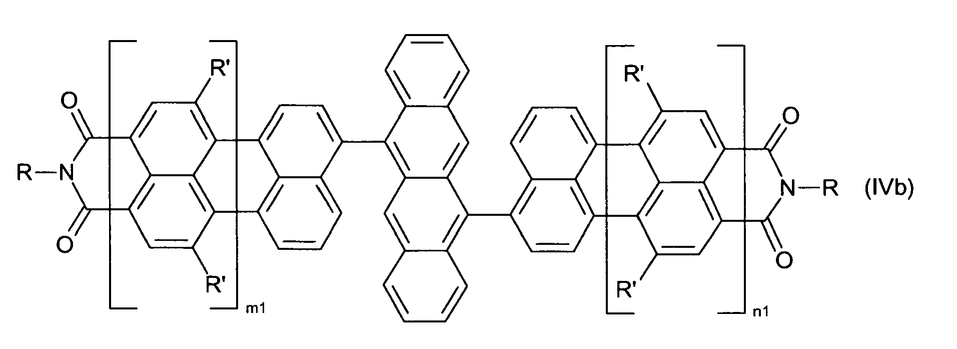 einer ersten Cyclodehydrierung in Gegenwart eines inerten organischen Lösungsmittels und einer Lewis-Säure unterzieht und
c) das in Schritt b) gebildete Bisrylenderivat der allgemeinen Formel Vb 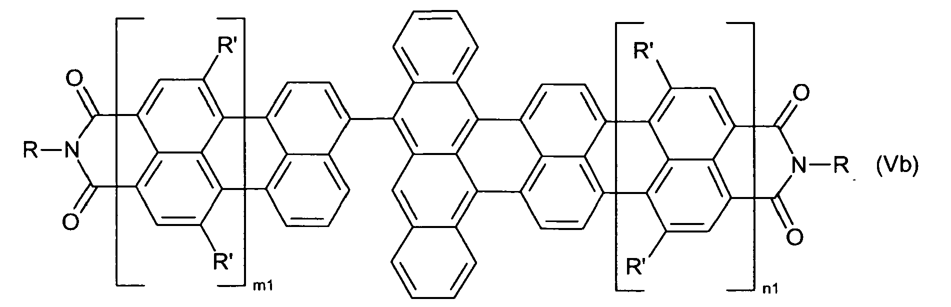 in einem Hydroxy- und Aminofunktionen aufweisenden und eine im Wesentlichen ungelöste Base enthaltenden organischen Reaktionsmedium weiter zum Dibenzorylentetracarbonsäurediimid Ib cyclodehydriert.

Nicht zuletzt wurden die bei diesen Verfahren als Zwischenprodukte auftretenden Tetracen-5,11-bis(rylendicarbonsäureimide) der allgemeinen Formel IV 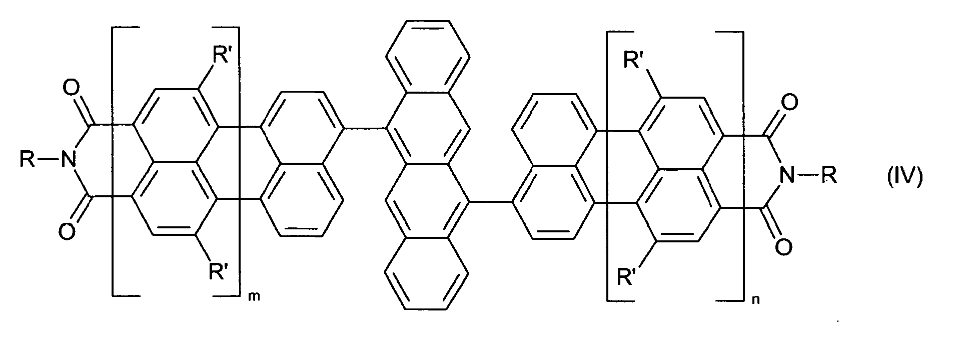 und Bisrylenderivate der allgemeinen Formel V 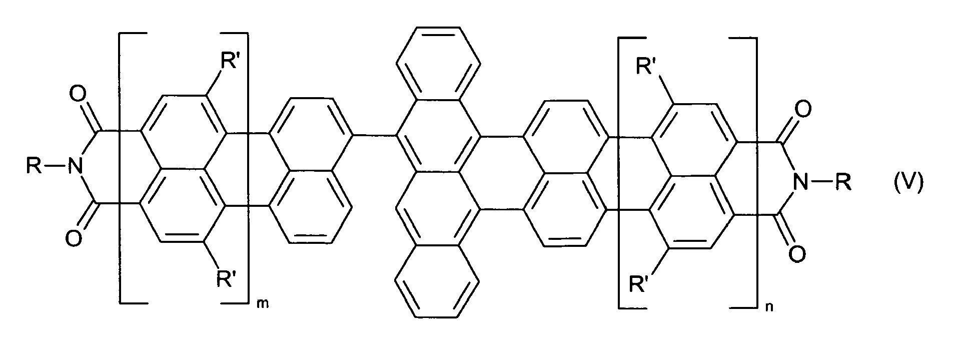 gefunden.

Im Rahmen der vorliegenden Erfindung umfasst der Ausdruck "Alkyl" geradkettiges oder verzweigtes Alkyl mit 1 bis 30 Kohlenstoffatomen (C₁-C₃₀-Alkyl), insbesondere C₁-C₂₀-Alkyl. Beispiele für Alkylgruppen sind insbesondere Methyl, Ethyl, Propyl, Isopropyl, n-Butyl, Isobutyl, sec.-Butyl, tert.-Butyl, n-Pentyl, 2-Methylbutyl, 3-Methylbutyl, 1,2-Dimethylpropyl, 1,1-Dimethylpropyl, 2,2-Dimethylpropyl, 1-Ethylpropyl, n-Hexyl, 2-Methylpentyl, 3-Methylpentyl, 4-Methylpentyl, 1,2-Dimethylbutyl, 1,3-Dimethylbutyl, 2,3-Dimethylbutyl, 1,1-Dimethylbutyl, 2,2-Dimethylbutyl, 3,3-Dimethylbutyl, 1,1,2-Trimethylpropyl, 1,2,2-Trimethylpropyl, 1-Ethylbutyl, 2-Ethylbutyl, 1-Ethyl- 2-methylpropyl, n-Heptyl, 2-Heptyl, 3-Heptyl, 2-Ethylpentyl, 1-Propylbutyl, n-Octyl, 2-Ethylhexyl, 2-Propylheptyl, 1,1,3,3-Tetramethylbutyl, Nonyl, Decyl, n-Undecyl, n-Dodecyl, n-Tridecyl, iso-Tridecyl, n-Tetradecyl, n-Hexadecyl, n-Octadecyl, 1-Hexylheptyl, 1-Heptyloctyl, 1-Octylnonyl, 1-Nonyldecyl und n-Eicosyl.

Der Ausdruck "Alkyl" umfasst auch Alkylreste, deren Kohlenstoffkette durch eine oder mehrere Gruppierungen, z. B. 1, 2, 3, 4 oder mehr als 4 nicht benachbarte Gruppierungen, O-, -S-, -NR¹-, -N=CR¹-, -C≡C-, -CR¹=CR¹-, -C(=O)-, -SO- und/oder -SO₂- unterbrochen sein kann, d.h. die Termini der Alkylgruppe werden durch Kohlenstoffatome gebildet. R¹ steht für Wasserstoff oder C₁-C₁₈-Alkyl. Geeignete Alkylreste, die durch eine oder mehrere Gruppierungen -C≡C- unterbrochen sind, werden im Folgenden auch als Alkinyl bezeichnet. Geeignete Alkylreste, die durch eine oder mehrere Gruppierungen -CR¹=CR¹- unterbrochen sind, werden im Folgenden auch als Alkenyl bezeichnet.

Alkylreste, deren Kohlenstoffgerüst durch eine oder mehrere Gruppierungen -O- unterbrochen sind, weisen formal eine Alkylgruppierung mit einem oder mehreren C₁-C₁₂-Alkoxy-Substitutuenten auf.

Beispiele für Alkyl, dessen Kohlenstoffkette durch eine oder mehrere Gruppierungen, z. B. eine, zwei, drei, vier oder mehr als vier nicht benachbarte Gruppierungen, -O-unterbrochen sind, sind die Folgenden:
Methoxymethyl, Diethoxymethyl, 2-Methoxyethyl, 2-Ethoxyethyl, 2-Propoxyethyl, Diethoxyethyl, 2-Butoxyethyl, 2-Octyloxyethyl, 2-Methoxypropyl, 3-Methoxypropyl, 3-Ethoxypropyl, 3-Propoxypropyl, 2-Isopropoxyethyl, 2-Butoxypropyl, 3-Butoxypropyl, 4-Methoxybutyl, 4-Ethoxybutyl, 4-Propoxybutyl, 6-Methoxyhexyl, 3,6-Dioxa-heptyl (5-Methoxy-3-oxa-pentyl), 3,6-Dioxa-octyl (7-Methoxy-4-oxa-heptyl), 4,8-Dioxa-nonyl (7-Methoxy-4-oxa-heptyl), 3,7-Dioxa-octyl, 3,7-Dioxa-nonyl, 4,7-Dioxa-octyl, 4,7-Dioxanonyl, 2- und 4-Butoxybutyl, 4,8-Dioxadecyl, 9-Ethoxy-5-oxa-nonyl, 3,6,9-Trioxadecyl, 3,6,9-Trioxaundecyl, 3,6,9-Trioxadodecyl, 4,8,12-Trioxatridecyl (11-Methoxy-4,8-dioxaundecyl), 4,8,12-Trioxatetradecyl, 14-Methoxy-5,10-dioxa-tetradecyl, 5,10,15-Trioxaheptadecyl, 3,6,9,12-Tetraoxatridecyl, 3,6,9,12-Tetraoxatetradecyl, 4,8,12,16-Tetraoxaheptadecyl (15-Methoxy-4,8,12-trioxa-pentadecyl), 4,8,12,16-Tetraoxa-octadecyl und dergleichen.

Alkylreste, deren Kohlenstoffgerüst durch eine oder mehrere Gruppierungen -S- unterbrochen sind, weisen formal eine Alkylgruppierung mit einem oder mehreren C₁-C₆-Alkylthio-Substitutuenten auf.

Beispiele für Alkyl, dessen Kohlenstoffkette durch eine oder mehrere Gruppierungen, z. B. 1, 2, 3, 4 oder mehr als 4, nicht benachbarte Gruppierungen -S- unterbrochen sind, sind die Folgenden:
Butylthiomethyl, 2-Methylthioethyl, 2-Ethylthioethyl, 2-Propylthioethyl, 2-Butylthioethyl, 2-Dodecylthioethyl, 3-Methylthiopropyl, 3-Ethylthiopropyl, 3-Propylthiopropyl, 3-Butylthiopropyl, 4-Methylthiobutyl, 4-Ethylthiobutyl, 4-Propylthiobutyl, 3,6-Dithiaheptyl, 3,6-Dithia-octyl, 4,8-Dithia-nonyl, 3,7-Dithia-octyl, 3,7-Di-thia-nonyl, 2- und 4-Butylthiobutyl, 4,8-Dithia-decyl, 3,6,9-Trithia-decyl, 3,6,9-Trithia-undecyl, 3,6,9-Trithia-dodecyl, 3,6,9,12-Tetrathia-tridecyl und 3,6,9,12-Tetrathia-tetradecyl.

Beispiele für Alkyl, dessen Kohlenstoffkette durch eine oder mehrere Gruppierungen, z. B. eine, zwei, drei, vier oder mehr als vier nicht benachbarte Gruppierungen, -NR¹-unterbrochen ist, wobei R¹ die vorgenannten Bedeutungen aufweist, sind die Folgenden:
2-Monomethyl- und 2-Monoethylaminoethyl, 2-Dimethylaminoethyl, 3-Methylaminopropyl, 2- und 3-Dimethylaminopropyl, 3-Monoisopropylaminopropyl, 2- und 4-Monopropylaminobutyl, 2- und 4-Dimethylaminobutyl, 6-Methylaminohexyl, 6-Dimethylaminohexyl, 6-Methyl-3,6-diazaheptyl, 3,6-Dimethyl-3,6-diazaheptyl, 3,6-Diazaoctyl, 3,6-Dimethyl-3,6-diazaoctyl, 9-Methyl-3,6,9-triazadecyl, 3,6,9-Trimethyl-3,6,9-triazadecyl, 3,6,9-Triazaundecyl, 3,6,9-Trimethyl-3,6,9-triazaundecyl, 12-Methyl-3,6,9,12-tetraazatridecyl, 3,6,9,12-Tetramethyl-3,6,9,12-tetraazatridecyl und dergleichen.

Beispiele für Alkyl, dessen Kohlenstoffkette durch eine oder mehrere Gruppierungen, z. B. eine, zwei, drei, vier oder mehr als vier nicht benachbarte Gruppierungen, -N=CR¹- unterbrochen ist, wobei R¹ die vorgenannten Bedeutungen aufweist, sind die Folgenden:
(1-Ethylethyliden)aminoethylen, (1-Ethylethyliden)aminopropylen, (1-Ethylethyliden)aminobutylen, (1-Ethylethyliden)aminodecylen und (1-Ethylethyliden)aminododecylen.

Beispiele für Alkyl, dessen Kohlenstoffkette durch -C(=O)- unterbrochen ist, sind die Folgenden:
2-Oxopropyl, 2-Oxobutyl, 3-Oxobutyl, 1-Methyl-2-oxopropyl, 2-Oxopentyl, 3-Oxopentyl, 1-Methyl-2-oxobutyl, 1-Methyl-3-oxobutyl, 2-Oxohexyl, 3-Oxohexyl, 4-Oxohexyl, 2-Ethyl-3-oxopentyl, 2-Oxoheptyl, 3-Oxoheptyl, 4-Oxoheptyl, 4-Oxoheptyl und dergleichen.

Beispiele für Alkyl, dessen Kohlenstoffkette durch -S(=O)- (Sulfinylgruppe, die im Folgenden auch als Sulfoxidogruppe bezeichnet wird) unterbrochen ist, sind die Folgenden:
2-Methylsulfinylethyl, 2-Ethylsulfinylethyl, 2-Propylsulfinylethyl, 2-lsopropylsulfoxidoethyl, 2-Butylsulfinylethyl, 2- und 3-Methylsulfinylpropyl, 2- und 3-Ethylsulfoxidopropyl, 2- und 3-Propylsulfinylpropyl, 2- und 3-Butylsulfinylpropyl, 2- und 4-Methylsulfinylbutyl, 2- und 4-Ethylsulfinylbutyl, 2- und 4-Propylsulfinylbutyl und 4-Butylsulfinylbutyl.

Beispiele für Alkyl, dessen Kohlenstoffkette durch -SO₂- (Sulfonylgruppe) unterbrochen ist, sind die Folgenden:
2-Methylsulfonylethyl, 2-Ethylsulfonylethyl, 2-Propylsulfonylethyl, 2-Isopropylsulfonylethyl, 2-Buthylsulfonylethyl, 2-Methylsulfonylpropyl, 3-Methylsulfonylpropyl, 2-Ethylsulfonylpropyl, 3-Ethylsulfonylpropyl, 2-Propylsulfonylpropyl, 3-Propylsulfonylpropyl, 2-Butylsutfonylpropyl, 3-Butylsulfonylpropyl, 2-Methylsulfonylbutyl, 4-Methylsulfonylbutyl, 2-Ethylsulfonylbutyl, 4-Ethylsulfonylbutyl, 2-Propylsulfonylbutyl, 4-Propylsulfonylbutyl und 4-Butylsulfonylbutyl.

Der Ausdruck Alkyl umfasst auch substituierte Alkylreste. Substituierte Alkylgruppen können in Abhängigkeit von der Länge der Alkylkette ein- oder mehrfach, z. B. 1-, 2-, 3-, 4-, 5- oder mehr als 5-fach substituiert sein, wie voranstehend definiert.

Beispiele für substituierte Alkylreste sind die Folgenden:
Alkyl, das durch Hydroxy substituiert ist, wie z. B. 2-Hydroxyethyl, 2-Hydroxypropyl, 3-Hydroxypropyl, 3-Hydroxybutyl, 4-Hydroxybutyl, 2-Hydroxy-2-methylpropy, 5-Hydroxy-3-oxa-pentyl, 6-Hydroxyhexyl, 7-Hydroxy-4-oxa-heptyl, 8-Hydroxy-4-oxa-octyl, 8-Hydroxy-3,6-dioxa-octyl, 9-Hydroxy-5-oxa-nonyl, 11-Hydroxy-4,8-dioxa-undecyl, 11-Hydroxy-3,6,9-trioxa-undecyl, 14-Hydroxy-5,10-dioxa-tetradecyl, 15-Hydroxy-4,8,12-trioxa-pentadecyl und dergleichen.
Alkyl, das durch Mercapto substituiert ist, wie z. B. 2-Mercaptoethyl, 2-Mercaptopropyl, 3-Mercaptopropyl, 3-Mercaptobutyl, 4-Mercaptobutyl, 2-Hydroxy-2-methylpropyl.
Alkyl, das durch Halogen, wie nachfolgend definiert, substituiert ist, wobei in der Alkylgruppe die Wasserstoffatome teilweise oder vollständig durch Halogenatome ersetzt sein können, wie C₁-C₃₀-Fluoralkyl, z. B. Trifluormethyl, Difluormethyl, Fluormethyl, Pentafluorethyl, Heptafluorpropyl, Heptafluorisopropyl, Nonafluorbutyl, Nonafluorisobutyl, Undecylfluorpentyl, Undecylfluorisopentyl und dergleichen, C₁-C₃₀-Chloralkyl, z. B. Chlormethyl, Dichlormethyl, Trichlormethyl, 2-Chlorethyl, 2- und 3-Chlorpropyl, 2-, 3-und 4-Chlorbutyl, 1,1-Dimethyl-2-chlorethyl und dergleichen, C₁-C₃₀-Bromalkyl, z. B. Bromethyl, 2-Bromethyl, 2- und 3-Brompropyl und 2-, 3- und 4-Brombutyl und dergleichen.
Alkyl, das durch Cyano substituiert ist, wie z. B. 2-Cyanoethyl, 3-Cyanopropyl, 3-Cyanobutyl und 4-Cyanobutyl und dergleichen.
Alkyl, das durch Nitro substituiert ist, wie z. B. 2-Nitroethyl, 2- und 3-Nitropropyl und 2-, 3- und 4-Nitrobutyl und dergleichen.
Alkyl, das durch Amino substituiert ist, wie z. B. 2-Aminoethyl, 2-Aminopropyl, 3-Aminopropyl, 4-Aminobutyl, 6-Aminohexyl und dergleichen.
Alkyl, das durch Carboxy substituiert ist, wie z. B. Carboxymethyl, 2-Carboxyethyl, 3-Carboxypropyl, 4-Carboxybutyl, 5-Carboxypentyl, 6-Carboxyhexyl, 7-Carboxyheptyl, 8-Carboxyoctyl, 9-Carboxynonyl, 10-Carboxydecyl, 12-Carboxydodecyl und 14-Carboxytetradecyl;
Alkyl, das durch Alkoxycarbonyl substituiert ist, wie z. B. Methoxycarbonylmethyl, Ethoxycarbonylmethyl, n-Butoxycarbonylmethyl, 2-Methoxycarbonylethyl, 2-Ethoxycarbonylethyl, 2-Methoxycarbonylpropyl, 2-Ethoxycarbonylpropyl, 2-(n-Butoxycarbonyl)propyl, 2-(4-n-Butoxycarbonyl)propyl, 3-Methoxycarbonylpropyl, 3-Ethoxycarbonylpropyl, 3-(n-Butoxycarbonyl)propyl, 3-(4-n-Butoxycarbonyl)propyl und dergleichen.
Alkyl, das durch Aminocarbonyl substituiert ist, wie z. B. Aminocarbonylmethyl, Aminocarbonylethyl, Aminocarbonylpropyl und dergleichen.
Alkyl, das durch Alkylaminocarbonyl substituiert ist, wie Methylaminocarbonylmethyl, Methylaminocarbonylethyl, Ethylcarbonylmethyl, Ethylcarbonylethyl und dergleichen.
Alkyl, das durch Dialkylaminocarbonyl substituiert ist, wie z. B. Dimethylaminocarbonylmethyl, Dimethylaminocarbonylethyl, Dimethylcarbonylpropyl, Diethylaminocarbonylmethyl, Diethylaminocarbonylethyl, Diethylcarbonylpropyl und dergleichen.
Alkyl, das durch C₄-C₇-Cycloalkyl substituiert ist, wie z. B. Cyclobutylmethyl, Cyclobutylethyl, Cyclopentylmethyl, 2-Cyclopentylethyl, 3-Cyclopentylpropyl, Cyclohexylmethyl, 2-Cyclohexylethyl, 3-Cyclohexylpropyl, 2-Cyclopentyl-1,1-dimethylethyl, 2-Cyclohexyl-1,1-dimethylethyl, 2-Cycloheptyl-1,1-dimethyl-ethyl, und dergleichen.
Alkyl, das durch C₄-C₇-Cycloalkyl, dessen Kohlenstoffgerüst durch eine oder mehrere Gruppierungen -O-, -S-, -NR¹-, -N=CR¹-, -CR¹=CR¹-, -CO-, -SO- und/oder -SO₂- unterbrochen ist, wie 2-(Cyclopentenyl)ethyl, 2-(Cyclohexenyl)ethyl, 2-(Cycloheptenyl)ethyl, 2-(4-Moprholinyl)ethyl, 1,1-Dimethyl-2-(4-morpholinyl)ethyl, 2-(4-Thiomoprholinyl)ethyl, 1,1-Dimethyl-2-(4-thiomorpholinyl)ethyl, 2-(Tetrahydrofuran-2-yl)ethyl, 2-(Tetrahydrofuran-3-yl)ethyl, 2-(Tetrahydrothiophen-2-yl)ethyl, 2-(Tetrahydrothiophen-3-yl)ethyl, 2-(Piperidin-1-yl)ethyl, 2-(Piperidin-2-yl)ethyl, 2-(Piperidin-3-yl)ethyl, 2-(Piperidin-4-yl)ethyl, 2-(Piperazin-1-yl)ethyl, 2-(Piperazin-2-yl)ethyl, 2-(Pyrrolidin-1-yl)ethyl, 2-(Pyrrolidin-2-yl)ethyl, 2-(Pyrrolidin-3-yl)ethyl,2-(Pyrrazolidin-3-yl)ethyl, 2-(Pyrrazolidin-4-yl)ethyl und dergleichen. Wenn Alkyl als Substituenten C₄-C₇-Cycloalkyl trägt, dessen Kohlenstoffgerüst durch eine oder mehrere Gruppierungen-NR¹- unterbrochen ist, so kann R¹ auch für die Bindung zu dem Alkylrest stehen.
Alkyl, das durch Aryl substituiert ist, wie z. B. Phenyl-C₁-C₁₀-alkyl, beispielsweise Benzyl, 1-Phenethyl, 2-Phenethyl, 1-Phenprop-1-yl, 2-Phenprop-1-yl, 3-Phenprop-1-yl, 1-Phenbut-1-yl, 2-Phenbut-1-yl, 3-Phenbut-1-yl, 4-Phenbut-1-yl, 1-Phenbut-2-yl, 2-Phenbut-2-yl, 3-Phenbut-2-yl, 4-Phenbut-2-yl, 1-(Phenmeth)-eth-1-yl, 1-(Phenmethyl)-1-(methyl)-eth-1-yl oder -(Phenmethyl)-1-(methyl)-prop-1-yl), 2-(Naphthalin-1-yl)-ethyl, 2-(Naphthalin-2-yl)-ethyl und dergleichen.

Der Ausdruck "Alkenyl" umfasst im Rahmen der vorliegenden Erfindung C₁-C₃₀-Alkylreste, deren Kohlenstoffkette durch eine oder mehrere Gruppierungen -CR¹=CR¹-unterbrochen sein kann. In Abhängigkeit von der Kettenlänge kann die Kohlenstoffkette dann durch 1, 2, 3, 4 oder mehr als 4 Gruppierungen -CR¹=CR¹- unterbrochen sein. Der Ausdruck Alkenyl umfasst auch C₁-C₃₀-Alkylreste, deren Kohlenstoffkette durch eine oder mehrere Gruppierungen -CR¹=CR¹- unterbrochen ist und ein- oder mehrfach, z. B. 1-, 2-, 3-, 4- oder mehr als 4-fach substituiert sein kann. Geeignete Substituenten sind die vorstehend für Alkyl genannten. Der Ausdruck Alkenyl umfasst weiterhin Reste der Formel -CR¹=CR¹₂, worin R¹ die vorgenannten Bedeutungen aufweist.

Geeignete Alkenylreste sind Ethenyl (Vinyl), 1-Propenyl, 2-Propenyl, 1-Methylethenyl, 1-Butenyl, 2-Butenyl, 3-Butenyl, 1-Pentenyl, 2-Pentenyl, 3-Pentenyl, 4-Pentenyl, 1-Hexenyl, 2-Hexenyl, 3-Hexenyl, 4-Hexenyl, 5-Hexenyl, Penta-1,3-dien-1-yl, Hexa-1,4-dien-1-yl, Hexa-1,4-dien-3-yl, Hexa-1,4-dien-6-yl, Hexa-1,5-dien-1-yl, Hexa-1,5-dien-3-yl, Hexa-1,5-dien-4-yl, Hepta-1,4-dien-1-yl, Hepta-1,4-dien-3-yl, Hepta-1,4-dien-6-yl, Hepta-1,4-dien-7-yl, Hepta-1,5-dien-1-yl, Hepta-1,5-dien-3-yl, Hepta-1,5-dien-4-yl, Hepta-1,5-dien-7-yl, Hepta-1,6-dien-1-yl, Hepta-1,6-dien-3-yl, Hepta-1,6-dien-4-yl, Hepta-1,6-dien-5-yl, Hepta-1,6-dien-2-yl, Octa-1,4-dien-1-yl, Octa-1,4-dien-2-yl, Octa-1,4-dien-3-yl, Octa-1,4-dien-6-yl, Octa-1,4-dien-7-yl, Octa-1,5-dien-1-yl, Octa-1,5-dien-3-yl, Octa-1,5-dien-4-yl, Octa-1,5-dien-7-yl, Octa-1,6-dien-1-yl, Octa-1,6-dien-3-yl, Octa-1,6-dien-4-yl, Octa-1,6-dien-5-yl, Octa-1,6-dien-2-yl, Deca-1,4-dienyl, Deca-1,5-dienyl, Deca-1,6-dienyl, Deca-1,7-dienyl, Deca-1,8-dienyl, Deca-2,5-dienyl, Deca-2,6-dienyl, Deca-2,7-dienyl, Deca-2,8-dienyl und dergleichen.

Der Ausdruck Alkinyl umfasst im Rahmen der vorliegenden Erfindung C₁-C₃₀-Alkylreste, deren Kohlenstoffkette durch eine oder mehrere Gruppierungen --C≡C- unterbrochen sein kann. In Abhängigkeit von der Kettenlänge kann die Kohlenstoffkette dann durch 1, 2, 3, 4 oder mehr als 4 Gruppierungen -CΞC- unterbrochen sein. Der Ausdruck Alkinyl umfasst auch C₁-C₃₀-Alkylreste, deren Kohlenstoffkette durch eine oder mehrere Gruppierungen -CR¹=CR¹- unterbrochen ist und ein- oder mehrfach, z. B. 1-, 2-, 3-, 4- oder mehr als 4-fach substituiert sein kann. Geeignete Substituenten sind die vorstehend für Alkyl genannten. Der Ausdruck Alkinyl umfasst weiterhin Reste der Formel -C≡CR¹, worin R¹ die vorgenannten Bedeutungen aufweist.

Geeignete Alkinylreste sind Ethinyl, 1- und 2-Propinyl, 1-, 2- und 3-Butinyl, 1-, 2-, 3-und 4-Pentinyl, 1-, 2-, 3-, 4- und 5-Hexinyl, 1-, 2-, 3-, 4-, 5-, 6-, 7-, 8- und 9-Decinyl, 1-, 2-, 3-, 4-, 5-, 6-, 7-, 8-, 9-, 10- und 11-Dodecinyl und 1-, 2-, 3-, 4-, 5-, 6-, 7-, 8-, 9-, 10-, 11-, 12-, 13-, 14-, 15-, 16- und 17-Octadecinyl.

Der Ausdruck "Cycloalkyl" umfasst im Rahmen der vorliegenden Erfindung unsubstituierte als auch substituierte Cycloalkylgruppen mit im Allgemeinen 3 bis 8 Kohlenstoffringgliedern. Das Kohlenstoffringgerüst kann durch eine oder mehrere, z. B. eine oder zwei, Gruppierungen -C(=O)- unterbrochen sein. Beispiele für C₃-C₈-Cycloalkyl sind Cyclopropyl, Cyclobutyl, Cyclopentyl, Cyclohexyl, Cycloheptyl oder Cyclooctyl. Geeignete Substituenten sind in der Regel ausgewählt unter C₁-C₁₈-Alkyl, C₁-C₁₂-Alkoxy, C₁-C₆-Alkylthio, -CΞCR¹, -CR¹=CR¹2, Hydroxy, Mercapto, Halogen, Cyano, Nitro, -NR²R³, -NR²COR³, -CONR²R³, -SO₂NR²R³, -COOR² und/oder -SO₃R², worin R¹, R² und R³ die zuvor genannten Bedeutungen aufweisen. Substituierte Cycloalkylgruppen können einen oder mehrere (z. B. 1, 2, 3, 4, 5 oder mehr als 5) Substituenten aufweisen, wobei im Falle von Halogen der Cycloalkylrest partiell oder vollständig durch Halogen substituiert ist. Des Weiteren kann an Cycloalkyl ein oder mehrere, z. B ein oder zwei, weitere gesättigte oder ungesättigte 5- bis 7-gliedrige Ringe, deren Kohlenstoffgerüst durch eine oder mehrere Gruppierungen -O-, -S-, -NR¹-, -N=CR¹-, -CR¹=CR¹-, -CO-, -SO- und/oder -SO₂- unterbrochen sein kann, anelliert sein, so dass ein bicyclisches oder tricyclisches Ringsystem entsteht. Beispiele für anellierte Ringe sind Cyclopentan, Cyclohexan, Cycloheptan, Piperidin, Piperazin, Morpholin, Thiomorpholin, 1,3-Dioxolan, 1,3-Dioxolan-2-on oder 4H-1,3-Oxazin.

Beispiele für Cycloalkyl sind Cyclopentyl, 2- und 3-Methylcyclopentyl, 2- und 3-Ethylcyclopentyl, Chlorpentyl, Dichlorpentyl, Dimethylcyclopentyl, Cyclohexyl, 2-, 3-und 4-Methylcyclohexyl, 2-, 3- und 4-Ethylcyclohexyl, 3- und 4-Propylcyclohexyl, 3- und 4-lsopropylcyclohexyl, 3- und 4-Butylcyclohexyl, 3- und 4-sec.-Butylcyclohexyl, 3- und 4-tert.-Butylcyclohexyl, Chlorhexyl, Dimethylcyclohexyl, Diethylcyclohexyl, Methoxycyclohexyl, Dimethoxycyclohexyl, Diethoxycyclohexyl, Butoxycyclohexyl, Methylthiocyclohexyl, Chlorcyclohexyl, Dichlorcyclohexyl, Cycloheptyl, 2-, 3- und 4-Methylcycloheptyl, 2-, 3- und 4-Ethylcycloheptyl, 3- und 4-Propylcycloheptyl, 3- und 4-lsopropylcycloheptyl, 3- und 4-Butylcycloheptyl, 3- und 4-sec.-Butylcycloheptyl, 3-und 4-tert.-Butylcycloheptyl, Cyclooctyl, 2-, 3-, 4- und 5-Methylcyclooctyl, 2-, 3-, 4- und 5-Ethylcyclooctyl, 3-, 4- und 5-Propytcyclooctyl.

Der Ausdruck Cycloalkenyl umfasst im Rahmen der vorliegenden Erfindung substituierte und unsubstituierte C₃-C₈-Cycloalkylreste, deren Kohlenstoffringgerüst durch eine oder mehrere, z. B. 1, 2, 3 oder 4, Gruppierungen -CR¹=CR¹- unterbrochen ist. Geeignete Beispiele umfassen Cyclopent-1-en-1-yl, Cyclopent-2-en-1-yl, Cyclopent-3-en-1-yl, Cyclohex-1-en-1-yl, Cyclohex-2-en-1-yl, Cyclohex-3-en-1-yl, Cyclohexa-2,5-dien-1-yl, 1-, 2- und 3-Cycloheptenyl und 1-, 2-, 3- und 4-Cyclooctenyl und dergleichen. Geeignete Substituenten sind die zuvor für Cycloalkyl genannten.

Der Ausdruck "Heterocyclyl (Heterocycloalkyl)" umfasst im Rahmen der vorliegenden Erfindung gesättigtes, partiell gesättigtes oder ungesättigtes C₃-C₈-Cycloalkyl, dessen Kohlenstoffgerüst durch eine oder mehrere Gruppierungen, z. B. 1, 2, 3 oder 4 gleiche oder verschiedene Gruppierungen-O-, -S-, -NR¹-, -N=CR¹-, -SO- und/oder -SO₂- unterbrochen ist und gegebenenfalls eine oder mehrere, beispielsweise 1 oder 2, Gruppierung(en) -C(=O)- aufweist. Stickstoffhaltiges Heterocycloalkyl kann prinzipiell sowohl über ein Kohlenstoffatom als auch über ein Stickstoffatom gebunden sein. In diesem Fall steht R¹ in der Gruppierung -NR¹- für eine Bindung. Beispiele für heterocyclische Gruppen sind Pyrrolidinyl, Piperidinyl, Imidazolidinyl, Pyrazolidinyl, Oxazolidinyl, Morpholidinyl, Thiazolidinyl, Isothiazolidinyl, Isoxazolidinyl, Piperazinyl-, Tetrahydrothi-ophenyl, Dihydrothien-2-yl, Tetrahydrofuranyl, Dihydrofuran-2-yl, Tetrahydropyranyl, 1,2-Oxazolin-5-yl, 1,3-Oxazolin-2-yl, Dioxanyl, 1,2-Dihydropyrazin-1-yl, 1,4-Dihydropyrimidin-1-yl und 3,4-Dihydropyrimidin-3-yl.

Der Ausdruck "Aryl" umfasst im Rahmen der vorliegenden Erfindung ein- oder mehr-kernige aromatische Kohlenwasserstoffreste, die unsubstituiert oder substituiert sein können. Aryl steht in der Regel für Kohlenwasserstoffreste mit 6 bis 10, bis 14, bis 18, vorzugsweise 6 bis 10 Kohlenstoffringgliedern. Aryl steht vorzugsweise für unsubstituiertes oder substituiertes Phenyl, Naphthyl, Anthracenyl, Phenanthrenyl, Naphthacenyl, Chrysenyl, Pyrenyl, etc., und besonders bevorzugt für Phenyl oder Naphthyl. Substituierte Aryle können in Abhängigkeit von der Anzahl und Größe ihrer Ringsysteme einen oder mehrere (z. B. 1, 2, 3, 4, 5 oder mehr als 5) Substituenten aufweisen. Des Weiteren kann an Aryl weitere Ringe, deren Kohlenstoffgerüst durch eine oder mehrere Gruppierungen -O-, -S-, -NR¹-, -N=CR¹-, -CR¹=CR¹-, -CO-, -SO- und/oder-SO₂- unterbrochen sein kann, anelliert sein. Beispielsweise können an Aryl ein, zwei oder drei gesättigte oder ungesättigte 5- bis 7-gliedrige Ringe, deren Kohlenstoffgerüst durch eine oder mehrere Gruppierungen -O-, -S-, -NR¹-, -N=CR¹-, -CR¹=CR¹-, -CO-, -SO-und/oder -SO₂- unterbrochen sein kann, anelliert sein. Beispiele für anellierte Ringe sind Cyclopentan, Cyclohexan, Cycloheptan, Piperidin, Piperazin, Morpholin, Thiomorpholin, 1,3-Dioxolan, 1,3-Dioxolan-2-on oder 4H-1,3-Oxazin.

Aryl, das einen oder mehrere Reste trägt, steht beispielsweise für 2-, 3- und 4-Methylphenyl, 2,4-, 2,5-, 3,5- und 2,6-Dimethylphenyl, 2,4,6-Trimethylphenyl, 2-, 3-und 4-Ethylphenyl, 2,4-, 2,5-, 3,5- und 2,6-Diethylphenyl, 2,4,6-Triethylphenyl, 2-, 3-und 4-Propylphenyl, 2,4-, 2,5-, 3,5- und 2,6-Dipropylphenyl, 2,4,6-Tripropylphenyl, 2-, 3- und 4-Isopropylphenyl, 2,4-, 2,5-, 3,5- und 2,6-Diisopropylphenyl, 2,4,6-Triisopropylphenyl, 2-, 3- und 4-Butylphenyl, 2,4-, 2,5-, 3,5- und 2,6-Dibutylphenyl, 2,4,6-Tributylphenyl, 2-, 3- und 4-Isobutylphenyl, 2,4-, 2,5-, 3,5- und 2,6-Diisobutylphenyl, 2,4,6-Triisobutylphenyl, 2-, 3- und 4-sec-Butylphenyl, 2,4-, 2,5-, 3,5- und 2,6-Di-sec-butylphenyl, 2,4,6-Tri-sec-butylphenyl, 2-, 3- und 4-tert.-Butylphenyl, 2,4-, 2,5-, 3,5- und 2,6-Di-tert.-butylphenyl, 2,4,6-Tri-tert.-butylphenyl und 2-, 3-, 4-Dodecylphenyl; 2-, 3- und 4-Methoxyphenyl, 2,4-, 2,5-, 3,5- und 2,6-Dimethoxyphenyl, 2,4,6-Trimethoxyphenyl, 2-, 3- und 4-Ethoxyphenyl, 2,4-, 2,5-, 3,5- und 2,6-Diethoxyphenyl, 2,4,6-Triethoxyphenyl, 2-, 3-und 4-Propoxyphenyl, 2,4-, 2,5-, 3,5- und 2,6-Dipropoxyphenyl, 2-, 3- und 4-lsopropoxyphenyl, 2,4-, 2,5-, 3,5- und 2,6-Diisopropoxyphenyl, 2-, 3- und 4-Butoxyphenyl, 2-, 3-, 4-Hexyloxyphenyl; 2-, 3-, 4-Chlorphenyl, 2,4-, 2,5-, 3,5- und 2,6-Dichlorphenyl, Trichlorphenyl, 2-, 3-, 4-Fluorphenyl, 2,4-, 2,5-, 3,5- und 2,6-Difluorphenyl, Trifluorphenyl, wie z. B. 2,4,6-Trifluorphenyl, Tetrafluorphenyl, Pentafluorphenyl, 2-, 3- und 4-Cyanophenyl; 2-Nitrophenyl, 4-Nitrophenyl, 2,4-Dinitrophenyl, 2,6-Dinitrophenyl; 4-Dimethylaminophenyl; 4-Acetylphenyl; Methoxyethylphenyl, Ethoxymethylphenyl; Methylthiophenyl, Isopropylthiophenyl oder tert.-Butylthiophenyl; Methylnaphthyl; Isopropylnaphthyl oder Ethoxynaphthyl.

Der Ausdruck "Aryloxy" steht im Rahmen der vorliegenden Erfindung für über ein Sauerstoffatom gebundenes Aryl wie vorstehend definiert.

Der Ausdruck " Hetaryl (Heteroaryl)" umfasst im Rahmen der vorliegenden Erfindung unsubstituierte oder substituierte, heteroaromatische, ein- oder mehrkernige Gruppen, mit im Allgemeine 5 bis 14 Ringatomen, vorzugsweise 5 oder 6 Ringatomen, in denen 1, 2, 3 oder 4 der Ringkohlenstoffatome durch ein, zwei, drei oder vier Heteroatome, ausgewählt unter O, N und S ersetzt sind, wie Furyl, Thienyl, Oxazolyl, Isoxazolyl, Thiazolyl, Isothiazolyl, Benzofuranyl, Benzthiazolyl, Benzimidazolyl, Pyridyl, Chinolinyl, Acridinyl, Pyridazinyl, Pyrimidinyl, Pyrazinyl, Pyrrolyl, Imidazolyl, Pyrazolyl, Indolyl, Purinyl, Indazolyl, Benzotriazolyl, 1,2,3-Triazolyl, 1,3,4-Triazolyl und Carbazolyl, wobei diese heterocycloaromatischen Gruppen im Falle einer Substitution im Allgemeinen 1, 2, 3, 4 oder mehr als 4 Substituenten, tragen können. Geeignete Substituenten sind die vorstehend genannten.

Halogen steht für Fluor, Chlor, Brom oder lod.

C₁-C₁₂-Alkoxy steht für eine über ein Sauerstoffatom gebundene C₁-C₁₂-Alkylgruppe. Beispiele für Alkoxy sind: Methoxy, Ethoxy, n-Propoxy, 1-Methylethoxy, Butoxy, 1-Methylpropoxy, 2-Methylpropoxy, 1,1-Dimethylethoxy, n-Pentoxy, 1-Methylbutoxy, 2-Methylbutoxy, 3-Methylbutoxy, 1,1-Dimethylpropoxy, 1,2-Dimethylpropoxy, 2,2-Dimethylpropoxy, 1-Ethylpropoxy, Hexoxy, 1-Methylpentoxy, 2-Methylpentoxy, 3-Methylpentoxy, 4-Methylpentoxy, 1,1-Dimethylbutoxy, 1,2-Dimethylbutoxy, 1,3-Dimethylbutoxy, 2,2-Dimethylbutoxy, 2,3-Dimethylbutoxy, 3,3-Dimethylbutoxy, 1-Ethylbutoxy, 2-Ethylbutoxy, 1,1,2-Trimethylpropoxy, 1,2,2-Trimethylpropoxy, 1-Ethyl-1-methylpropoxy oder 1-Ethyl-2-methylpropoxy.

C₁-C₆-Alkylthio steht für eine über ein Schwefelatom gebundene C₁-C₆-Alkylgruppe. Beispiele für Alkylthio sind Methylthio, Ethylthio, Propylthio, Butylthio, Pentylthio und Hexylthio.

Die erfindungsgemäßen Dibenzorylentetracarbonsäurediimide I können ein symmetrisch aufgebautes Ringsystem aufweisen, das einem durch 2 anellierte Benzolringe erweiterten Pentarylen- oder Terrylengerüst (m = n = 1 oder 0) entspricht (Dibenzorylentetracarbonsäurediimide la).

Die Dibenzorylentetracarbonsäurediimide 1 können auch ein unsymmetrisch aufgebautes Ringsystem aufweisen, das einem durch 2 anellierte Benzolringe erweiterten Quaterrylengerüst (m ≠ n) entspricht (Dibenzorylentetracarbonsäurediimide Ib).

Bevorzugt sind die Dibenzorylentetracarbonsäurediimide la, wobei die Dibenzopentarylentetracarbonsäurediimide la (m = n = 1) besonders bevorzugt sind.

Die Substituenten R an den Imidstickstoffatomen der Dibenzorylentetracarbonsäurediimide I können gleich oder verschieden sein. Dibenzorylentetracarbonsäurediimide la mit zwei verschiedenen Substituenten R können dabei analog zu den Dibenzorylentetracarbonsäurediimiden Ib durch stufenweise Suzuki-Kupplung von 5,11-Dibromtetracen mit zwei den jeweiligen Rest R aufweisenden peri-(Dioxaborolan-2-yl)rylendicarbonsäureimiden II erhalten werden. Vorzugsweise sind die Substituenten R jedoch gleich.

Vorzugsweise sind die beiden Reste R unabhängig voneinander ausgewählt sind unter Gruppen der Formeln II.1 bis II.5:

#-(A)ₚ-C(Rⁱ)ₓ (II.1)

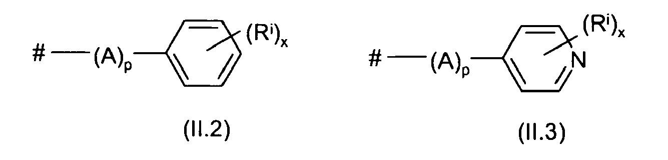

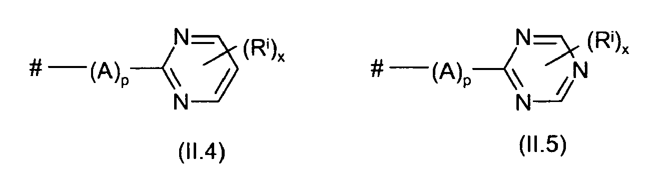

worin
- #: für die Verknüpfungsstelle zum Imidstickstoffatom steht,
- p: für 0 oder 1 steht,
- x: in den Verbindungen der Formel II.1 für 2 oder 3 steht, in den Verbindungen der Formeln II.2, II.3 und II.4 für 1, 2 oder 3 steht und in den Verbindungen der Formel II.5 für 1 oder 2 steht,
- A: soweit vorhanden, für eine C₁-C₁₀-Alkylengruppe steht, die durch eine oder mehrere nicht benachbarte Gruppen, die ausgewählt sind unter -O- und -S-, unterbrochen sein kann,
wobei für den Fall, dass in den Verbindungen der Formel II.1 x für 2 steht, das Kohlenstoffatom, das die Reste Rⁱ trägt, zusätzlich ein H-Atom trägt,
die Reste Rⁱ jeweils unabhängig voneinander ausgewählt sind unter C₁-C₃₀-Alkyl, welche durch ein oder mehrere nicht benachbarte Sauerstoffatom(e) unterbrochen sein können, wobei in den Verbindungen der Formel II.1 wenigstens einer der Reste Rⁱ auch für C₁-C₃₀-Alkyloxy oder C₁-C₃₀-Alkylthio stehen kann.

In einer bevorzugten Ausführung steht x in den Verbindungen der Formeln II.1 bis II.4 für 2 oder 3, insbesondere für 2, und in den Verbindungen der Formel II.5 für 2.

Eine weitere Ausführungsform der Erfindung sind Verbindungen der Formel (1), wobei die Reste Rⁱ jeweils unabhängig voneinander ausgewählt sind unter C₃-C₃₀-Alkyl, welche durch ein oder mehrere nicht benachbarte Sauerstoffatom(e) unterbrochen sein können, wobei in den Verbindungen der Formel II.1 wenigstens einer der Reste Rⁱ auch für C₃-C₃₀-Alkyloxy oder C₃-C₃₀-Alkylthio stehen kann.

In einer Verbindung der Formel I können die Gruppen R gleiche oder verschiedene Bedeutungen besitzen. Bevorzugt besitzen die Gruppen R in einer Verbindung der Formel I die gleiche Bedeutung.

Eine Ausführungsform der Erfindung sind Verbindungen der Formel (1), wobei die Gruppen die Gruppen R für Gruppen der Formel (II.1) stehen (sogenannte Schwalbenschwanzreste). Bevorzugt sind in den Gruppen der Formel (II.1) die Reste Rⁱ ausgewählt sind unter C₁-C₁₂-Alkyl, besonders bevorzugt C₄-C₈-Alkyl. Bevorzugt stehen die Gruppen R dann beide für eine Gruppe der Formel 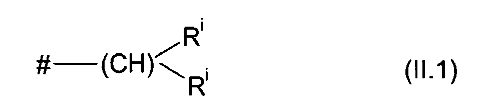 worin
- #: für die Verknüpfungsstelle zum Imidstickstoffatom steht, und
die Reste Rⁱ ausgewählt sind unter C₁-C₁₂-Alkyl, bevorzugt C₄-C₈-Alkyl, besonders bevorzugt C₅-C₇-Alkyl. Bei den Resten Rⁱ handelt es sich dann speziell um lineare Alkylreste, die nicht durch Sauerstoffatome unterbrochen sind.

Eine weitere Ausführungsform der Erfindung sind Verbindungen der Formel (1), wobei die Gruppen R unabhängig voneinander ausgewählt sind unter Gruppen der Formeln II.2 bis II.5. Eine bevorzugte Ausführungsform ist die Verwendung von Verbindungen der Formel (1), wobei die Gruppen R unabhängig voneinander ausgewählt sind unter Gruppen der Formel II.2 und x in den Gruppen der Formel II.2 für 2 steht.

Die verschiedenen Reste Rⁱ können jeweils gleiche oder verschiedene Bedeutungen aufweisen. Bevorzugt weisen alle Reste Rⁱ in einer Verbindung der Formel I die gleiche Bedeutung auf.

Die Reste Rⁱ sind jeweils unabhängig voneinander ausgewählt unter linearem oder verzweigtem C₁-C₃₀-Alkyl, welches durch ein oder mehrere nicht benachbarte Sauerstoffatom(e) unterbrochen sein kann. Bevorzugt sind lineare Alkylreste. Bevorzugt ist weiterhin C₃-C₁₈-Alkyl, wie C₄-C₁₂-Alkyl.

In einer bevorzugten Ausführung weisen die zuvor genannten Gruppen R keine Alkylengruppe A auf. In einer weiteren bevorzugten Ausführung weisen die zuvor genannten Gruppen R eine C₁-C₄-Alkylengruppe A auf, die durch 1, 2 oder 3 nicht benachbarte Gruppen, die ausgewählt sind unter -O- und -S-, unterbrochen sein kann.

Als Beispiele für geeignete Gruppen R seien im Einzelnen genannt:

Reste der Formel A: 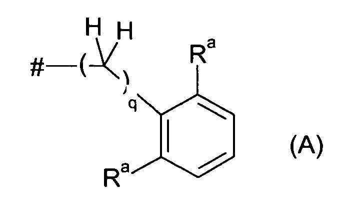 worin # die Verknüpfungsstelle mit dem Imidstickstoffatom des Rylentetracarbonsäurediimids ist, q für eine ganze Zahl 0, 1, 2, 3 oder 4 steht und Ra für C₁-C₃₀-Alkyl steht.

Reste der Formel A umfassen solche, in denen q für 0 steht, wie z. B.
2,6-Di(methyl)phenyl, 2,6-Di(ethyl)phenyl, 2,6-Di(n-propyl)phenyl,
2,6-Di(isopropyl)phenyl, 2,6-Di(n-butyl)phenyl, 2,6-Di(n-pentyl)phenyl,
2,6-Di(n-hexyl)phenyl, 2,6-Di(n-heptyl)phenyl, 2,6-Di(n-octyl)phenyl,
2,6-Di(n-nonyl)phenyl, 2,6-Di(n-decyl)phenyl, 2,6-Di(n-undecy)phenyl,
2,6-Di(n-dodecyl)phenyl, 2,6-Di(n-tridecyl)phenyl, 2,6-Di(n-tetradecyl)phenyl,
2,6-Di(n-pentadecyl)phenyl, 2,6-Di(n-hexadecyl)phenyl, 2,6-Di(n-heptadecyl)phenyl,
2,6-Di(n-octadecyl)phenyl, 2,6-Di(nonadecyl)phenyl, 2,6-Di(eicosyl)phenyl,
2,6-Di(docosanyl)phenyl, 2,6-Di(tricosanyl)phenyl, 2,6-Di(tetracosanyl)phenyl,
2,6-Di(octacosanyl)phenyl;

in denen q für 1 steht, wie z. B.
2,6-Di(methyl)benzyl, 2,6-Di(ethyl)benzyl, 2,6-Di(n-propyl)benzyl,
2,6-Di(isopropyl)benzyl, 2,6-Di(n-butyl)benzyl, 2,6-Di(n-pentyl)benzyl,
2,6-Di(n-hexyl)benzyl, 2,6-Di(n-heptyl)benzyl, 2,6-Di(n-octyl)benzyl,
2,6-Di(n-nonyl)benzyl, 2,6-Di(n-decyl)benzyl, 2,6-Di(n-undecy)benzyl,
2,6-Di(n-dodecyl)benzyl, 2,6-Di(n-tridecyl)benzyl, 2,6-Di(n-tetradecyl)benzyl,
2,6-Di(n-pentadecyl)benzyl, 2,6-Di(n-hexadecyl)benzyl, 2,6-Di(n-heptadecyl)benzyl,
2,6-Di(n-octadecyl)benzyl, 2,6-Di(nonadecyl)benzyl, 2,6-Di(eicosyl)benzyl,
2,6-Di(docosanyl)benzyl, 2,6-Di(tricosanyl)benzyl, 2,6-Di(tetracosanyl)benzyl,
2,6-Di(octacosanyl)benzyl;

in denen q für 2 steht, wie z. B.
2,6-Di(methyl)phenethyl, 2,6-Di(ethyl)phenethyl, 2,6-Di(n-propyl)phenethyl,
2,6-Di(isopropyl)phenethyl, 2,6-Di(n-butyl)phenethyl, 2,6-Di(n-pentyt)phenethyl,
2,6-Di(n-hexyl)phenethyl, 2,6-Di(n-heptyl)phenethyl, 2,6-Di(n-octyl)phenethyl,
2,6-Di(n-nonyl)phenethyl, 2,6-Di(n-decyl)phenethyl, 2,6-Di(n-undecy)phenethyl,
2,6-Di(n-dodecyl)phenethyl, 2,6-Di(n-tridecyl)phenethyl, 2,6-Di(n-tetradecyl)phenethyl,
2,6-Di(n-pentadecyl)phenethyl, 2,6-Di(n-hexadecyl)phenethyl,
2,6-Di(n-heptadecyl)phenethyl, 2,6-Di(n-octadecyl)phenethyl,
2,6-Di(nonadecyl)phenethyl, 2,6-Di(eicosyl)phenethyl, 2,6-Di(docosanyl)phenethyl,
2,6-Di(tricosanyl)phenethyl, 2,6-Di(tetracosanyl)phenethyl,
2,6-Di(octacosanyl)phenethyl;

in denen q für 3 steht, wie z. B.
3-(2,6-Di(methyl)phenyl)propyl, 3-(2,6-Di(ethyl)phenyl)propyl,
3-(2,6-Di(n-propyl)phenyl)propyl, 3-(2,6-Di(isopropyl)phenyl)propyl,
3-(2,6-Di(n-butyl)phenyl)propyl, 3-(2,6-Di(n-pentyl)phenyl)propyl,
3-(2,6-Di(n-hexyl)phenyl)propyl, 3-(2,6-Di(n-heptyl)phenyl)propyl,
3-(2,6-Di(n-octyl)phenyl)propyl, 3-(2,6-Di(n-nonyl)phenyl)propyl,
3-(2,6-Di(n-decyl)phenyl)propyl, 3-(2,6-Di(n-undecy)phenyl)propyl,
3-(2,6-Di(n-dodecyl)phenyl)propyl, 3-(2,6-Di(n-tridecyl)phenyl)propyl,
3-(2,6-Di(n-tetradecyl)phenyl)propyl, 3-(2,6-Di(n-pentadecyl)phenyl)propyl,
3-(2,6-Di(n-hexadecyl)phenyl)propyl, 3-(2,6-Di(n-heptadecyl)phenyl)propyl,
3-(2,6-Di(n-octadecyl)phenyl)propyl, 3-(2,6-Di(nonadecyl)phenyl)propyl,
3-(2,6-Di(eicosyl)phenyl)propyl, 3-(2,6-Di(docosanyl)phenyl)propyl,
3-(2,6-Di(tricosanyl)phenyl)propyl, 3-(2,6-Di(tetracosanyl)phenyl)propyl,
3-(2,6-Di(octacosanyl)phenyl)propyl;

in denen q für 4 steht, wie z. B.
4-(2,6-Di(methyl)phenyl)butyl, 4-(2,6-Di(ethyl)phenyl)butyl,
4-(2,6-Di(n-propyl)phenyl)butyl, 4-(2,6-Di(isopropyl)phenyl)butyl,
4-(2,6-Di(n-butyl)phenyl)butyl, 4-(2,6-Di(n-pentyl)phenyl)butyl,
4-(2,6-Di(n-hexyl)phenyl)butyl, 4-(2,6-Di(n-heptyl)phenyl)butyl,
4-(2,6-Di(n-octyl)phenyl)butyl, 4-(2,6-Di(n-nonyl)phenyl)butyl,
4-(2,6-Di(n-decyl)phenyl)butyl, 4-(2,6-Di(n-undecy)phenyl)butyl,
4-(2,6-Di(n-dodecyl)phenyl)butyl, 4-(2,6-Di(n-tridecyl)phenyl)butyl,
4-(2,6-Di(n-tetradecyl)phenyl)butyl, 4-(2,6-Di(n-pentadecyl)phenyl)butyl,
4-(2,6-Di(n-hexadecyl)phenyl)butyl, 4-(2,6-Di(n-heptadecyl)phenyl)butyl,
4-(2,6-Di(n-octadecyl)phenyl)butyl, 4-(2,6-Di(nonadecyl)phenyl)butyl,
4-(2,6-Di(eicosyl)phenyl)butyl, 4-(2,6-Di(docosanyl)phenyl)butyl,
4-(2,6-Di(tricosanyl)phenyl)butyl, 4-(2,6-Di(tetracosanyl)phenyl)butyl,
4-(2,6-Di(octacosanyl)phenyl)butyl;
des Weiteren Reste der Formel B: 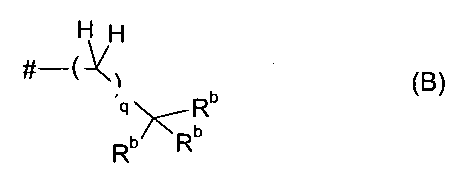 worin # die Verknüpfungsstelle mit dem Imidstickstoffatom des Rylentetracarbonsäurediimids ist, q für eine ganze Zahl 0, 1, 2, 3, 4, 5 oder 6 steht und R^{b} für C₄-C₃₀-Alkyl steht.

Beispiele für geeignete Reste der Formel B umfassen die Formeln B-0.a, B-0.b, B-0.c, B-1.a, B-1.b, B-1.c, B-2.a, B-2.b, B-2.c, B-3.a, B-3.b, B-3.c, B-4.a, B-4.b, B-4.c, B-5.a, B-5.b, B-5.c, B-6.a, B-6.b, B-6.c 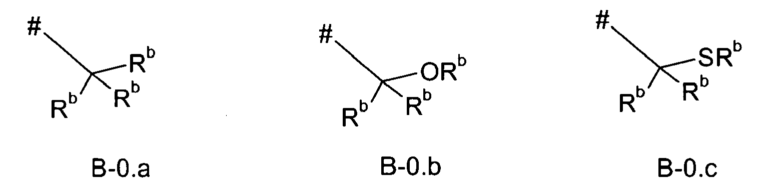 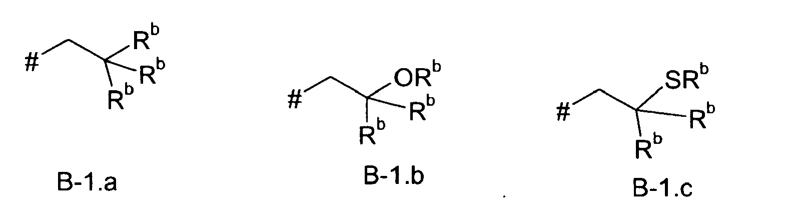 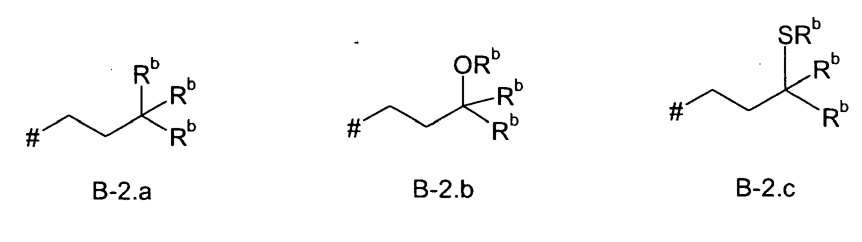 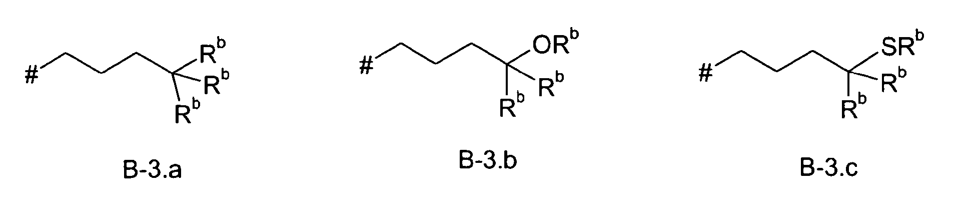 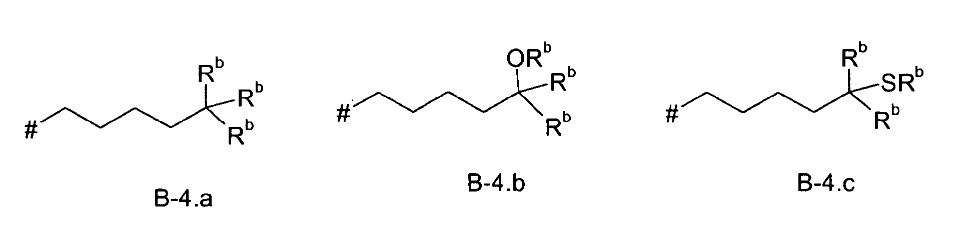 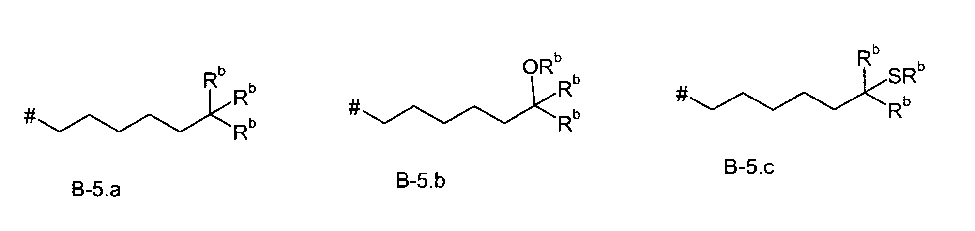 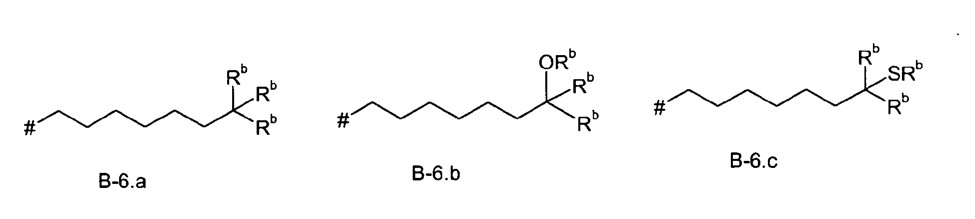 worin # die Verknüpfungsstelle mit dem Imidstickstoffatom des Rylentetracarbonsäurediimids ist, und R^{b} unabhängig voneinander für n-Butyl, n-Pentyl, n-Hexyl, n-Heptyl, n-Octyl, n-Nonyl, n-Decyl, n-Undecyl, n-Dodecyl, n-Tridecyl, n-Tetradecyl, n-Pentadecyl, n-Hexadecyl, n-Heptadecyl, n-Octadecyl, n-Nonadecyl, n-Eicosyl, n-Docosanyl, n-Tricosanyl, n-Tetracosanyl, n-Octacosanyl steht,
des Weiteren Verbindungen der Formel C: 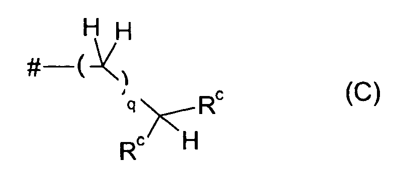 worin # die Verknüpfungsstelle mit dem Imidstickstoffatom des Rylentetracarbonsäurediimids ist, q für eine ganze Zahl 0, 1, 2, 3, 4, 5 oder 6 steht und R^{c} für C₄-C₃₀-Alkyl, C₄-C₃₀-Alkylthio oder C₄-C₃₀-Alkoxy steht.

Reste der Formel H umfassen solche, in denen q für 0 steht, wie z. B.

1-Ethylpropyl, 1-Methylpropyl, 1-Propylbutyl, 1-Ethylbutyl, 1-Methylbutyl, 1-Butylpentyl, 1-Propylpentyl, 1-Ethylpentyl, 1-Methylpentyl, 1-Pentylhexyl, 1-Butylhexyl, 1-Propylhexyl, 1-Ethylhexyl, 1-Methylhexyl, 1-Hexylheptyl, 1-Pentylheptyl, 1-Butylheptyl, 1-Propylheptyl, 1-Ethylheptyl, 1-Methylheptyl, 1-Heptyloctyl, 1-Hexyloctyl, 1-Pentyloctyl, 1-Butyloctyl, 1-Propyloctyl, 1-Ethyloctyl, 1-Methyloctyl, 1-Octylnonyl, 1-Heptylnonyl, 1-Hexylnonyl, 1-Pentylnonyl, 1-Butylnonyl, 1-Propylnonyl, 1-Ethylnonyl, 1-Methylnonyl, 1-Nonyldecyl, 1-Octyldecyl, 1-Heptyldecyl, 1-Hexyldecyl, 1-Pentyldecyl, 1-Butyldecyl, 1-Propyldecyl, 1-Ethyldecyl, 1-Methyldecyl, 1-Decylundecyl, 1-Nonylundecyl, 1-Octylundecyl, 1-Heptylundecyl, 1-Hexylundecyl, 1-Pentylundecyl, 1-Butylundecyl, 1-Propylundecyl, 1-Ethylundecyl, 1-Methylundecyl, 1-Undecyldodecyl, 1-Decyldodecyl, 1-Nonyldodecyl, 1-Octyldodecyl, 1-Heptyldodecyl, 1-Hexyldodecyl, 1-Pentyldodecyl, 1-Butyldodecyl, 1-Propyldodecyl, 1-Ethyldodecyl, 1-Methyldodecyl, 1-Dodecyltridecyl, 1-Undecyltridecyl, 1-Decyltridecyl, 1-Nonyltridecyl, 1-Octyltridecyl, 1-Heptyltridecyl, 1-Hexyltridecyl, 1-Pentyltridecyl, 1-Butyltridecyl, 1-Propyltridecyl, 1-Ethyltridecyl, 1-Methyltridecyl, 1-Tridecyltetrdecyl, 1-Undecyltetradecyl, 1-Decyltetradecyl, 1-Nonyltetradecyl, 1-Octyltetradecyl, 1-Hetyltetradecyl, 1-Hexyltetradecyl, 1-Pentytetradecyl, 1-Butyltetradecyl, 1-Propyltetradecyl, 1-Ethyltetradecyl, 1-Methyltetradecyl, 1-Pentadecylhexadecyl, 1-Tetradecylhexadecyl, 1-Tridecylhexadecyl, 1-Dodecylhexadecyl, 1-Undecylhexadecyl, 1-Decylhexadecyl, 1-Nonylhexadecyl, 1-Octylhexadecyl, 1-Heptylhexadecyl, 1-Hexylhexadecyl, 1-Pentylhexadecyl, 1-Butylhexadecyl, 1-Propylhexadecyl, 1-Ethylhexadecyl, 1-Methylhexadecyl, 1-Hexadecyloctadecyl, 1-Pentadecyloctadecyl, 1-Tetradecyloctadecyl, 1-Tridecyloctadecyl, 1-Dodecyloctadecyl, 1-Undecyloctadecyl, 1-Decyloctadecyl, 1-Nonyloctadecyl, 1-Octylocatedecyl, 1-Heptyloctadecyl, 1-Hexyloctadecyl, 1-Pentyloctadecyl, 1-Butyloctaedecyl, 1-Propyloctadecyl, 1-Ethyloctadecyl, 1-Methyloctadecyl 1-Nonadecyleicosanyl, 1-Octadecyleicosanyl, 1-Heptadecyleicosanyl, 1-Hexadecyleicosanyl, 1-Pentadecyleicosanyl, 1-Tetradecyleicosanyl, 1-Tridecyleicosanyl, 1-Dodecyleicosanyl, 1-Undecyleicosanyl, 1-Decyleicosanyl, 1-Nonyleicosanyl, 1-Octyleicosanyl, 1-Heptyleicosanyl, 1-Hexyleicosanyl, 1-Pentyleicosanyl, 1-Butyleicosanyl, 1-Propyleicosanyl, 1-Ethyleicosanyl, 1-Methyeicosanyl 1-Eicosanyldocosanyl, 1-Nonadecyldocosanyl, 1-Octadecyldocosanyl, 1-Heptadecyldocosanyl, 1-Hexadecyldocosanyl, 1-Pentadecyldocosanyl, 1-Tetradecyldocosanyl, 1-Tridecyldocosanyl, 1-Undecyldocosanyl, 1-Decyldocosanyl, 1-Nonyldocosanyl, 1-Octyldocosanyl, 1-Heptyldocosanyl, 1-Hexyldocosanyl, 1-Pentyldocosanyl, 1-Butyldocosanyl, 1-Propyldocosanyl, 1-Ethyldocosanyl, 1-Methyldocosanyl 1-Tricosanyltetracosanyl, 1-Docosanyltetracosanyl, 1-Nonadecyltetracosanyl, 1-Octadecyltetracosanyl, 1-Heptadecyltetracosanyl, 1-Hexadecyltetracosanyl, 1-Pentadecyltetracosanyl, 1-Pentadecyltetracosanyl, 1-Tetradecyltetracosanyl, 1-Tridecyltetracosanyl, 1-Dodecyltetracosanyl, 1-Undecyltetracosanyl, 1-Decyltetracosanyl, 1-Nonyltetracosanyl, 1-Octyltetracosanyl, 1-Heptyltetracosanyl, 1-Hexyltetracosanyl, 1-Pentyltetracosanyl, 1-Butyltetracosanyl, 1-Propyltetracosanyl, 1-Ethyltetracosanyl, 1-Methyltetracosanyl 1-Heptacosanyloctacosanyl, 1-Hexacosanyloctacosanyl, 1-Pentcosanyloctacosanyl, 1-Tetracosanyloctcosanyl, 1-Tricosanyloctacosanyl, 1-Docosanyloctacosanyl, 1-Nonadecyloctacosanyl, 1-Octadecyloctacosanyl, 1-Heptadecyloctacosanyl, 1-Hexadecyloctacosanyl, 1-Hexadecyloctacosanyl, 1-Pentadecyloctacosanyl, 1-Tetradecyloctacosanyl, 1-Tridecyloctacosanyl, 1-Dodecyloctacosanyl, 1-Undecyloctacosanyl, 1-Decyloctacosanyl, 1-Nonyloctacosanyl, 1-Octyloctacosanyl, 1-Heptyloctacosanyl, 1-Hexyloctacosanyl, 1-Pentyloctacosanyl, 1-Butyloctacosanyl, 1-Propyloctacosanyl, 1-Ethyloctacosanyl, 1-Methyloctacosanyl;

1-Ethyloxypropyl, 1-Methyloxypropyl, 1-Propylbutyl, 1-Ethyloxybutyl, 1-Methyloxybutyl, 1-Butyloxypentyl, 1-Propylpentyl, 1-Ethyloxypentyl, 1-Methyloxypentyl, 1-Pentyloxyhexyl, 1-Butyloxyhexyl, 1-Propylhexyl, 1-Ethyloxyhexyl, 1-Methyloxyhexyl, 1-Hexyloxyheptyl, 1-Pentyloxyheptyl, 1-Butyloxyheptyl, 1-Propylheptyl, 1-Ethyloxyheptyl, 1-Methyloxyheptyl, 1-Heptyloctyl, 1-Hexyloxyoctyl, 1-Pentyloxyoctyl, 1-Butyloxyoctyl, 1-Propyloctyl, 1-Ethyloxyoctyl, 1-Methyloxyoctyl, 1-Octyloxynonyl, 1-Heptylnonyl, 1-Hexyloxynonyl, 1-Pentyloxynonyl, 1-Butyloxynonyl, 1-Propylnonyl, 1-Ethyloxynonyl, 1-Methyloxynonyl, 1-Nonyloxydecyl, 1-Octyloxydecyl, 1-Heptyldecyl, 1-Hexyloxydecyl, 1-Pentyloxydecyl, 1-Butyloxydecyl, 1-Propyldecyl, 1-Ethyloxydecyl, 1-Methyloxydecyl, 1-Decyloxyundecyl, 1-Nonyloxyundecyl, 1-Octyloxyundecyl, 1-Heptylundecyl, 1-Hexyloxyundecyl, 1-Pentyloxyundecyl, 1-Butyloxyundecyl, 1-Propylundecyl, 1-Ethyloxyundecyl, 1-Methyloxyundecyl, 1-Undecyloxydodecyl, 1-Decyloxydodecyl, 1-Nonyloxydodecyl, 1-Octyloxydodecyl, 1-Heptyldodecyl, 1-Hexyloxydodecyl, 1-Pentyloxydodecyl, 1-Butyloxydodecyl, 1-Propyldodecyl, 1-Ethyloxydodecyl, 1-Methyloxydodecyl, 1-Dodecyloxytridecyl, 1-Undecyloxytridecyl, 1-Decyloxytridecyl, 1-Nonyloxytridecyl, 1-Octyloxytridecyl, 1-Heptyltridecyl, 1-Hexyloxytridecyl, 1-Pentyloxytridecyl, 1-Butyloxytridecyl, 1-Propyltridecyl, 1-Ethyloxytridecyl, 1-Methyloxytridecyl, 1-Tridecyloxytetrdecyl, 1-Undecyloxytetradecyl, 1-Decyloxytetradecyl, 1-Nonyloxytetradecyl, 1-Octyloxytetradecyl, 1-Hetyltetradecyl, 1-Hexyloxytetradecyl, 1-Pentytetradecyl, 1-Butyloxytetradecyl, 1-Propyltetradecyl, 1-Ethyloxytetradecyl, 1-Methyloxytetradecyl, 1-Pentadecyloxyhexadecyl, 1-Tetradecyloxyhexadecyl, 1-Tridecyloxyhexadecyl, 1-Dodecyloxyhexadecyl, 1-Undecyloxyhexadecyl, 1-Decyloxyhexadecyl, 1-Nonyloxyhexadecyl, 1-Octyloxyhexadecyl, 1-Heptylhexadecyl, 1-Hexyloxyhexadecyl, 1-Pentyloxyhexadecyl, 1-Butyloxyhexadecyl, 1-Propylhexadecyl, 1-Ethyloxyhexadecyl, 1-Methyloxyhexadecyl, 1-Hexadecyloxyoctadecyl, 1-Pentadecyloxyoctadecyl, 1-Tetradecyloxyoctadecyl, 1-Tridecyloxyoctadecyl, 1-Dodecyloxyoctadecyl, 1-Undecyloxyoctadecyl, 1-Decyloxyoctadecyl, 1-Nonyloxyoctadecyl, 1-Octyloxyocatedecyl, 1-Heptyloctadecyl, 1-Hexyloxyoctadecyl, 1-Pentyloxyoctadecyl, 1-Butyloxyoctaedecyl, 1-Propyloctadecyl, 1-Ethyloxyoctadecyl, 1-Methyloxyoctadecyl, 1-Nonadecyloxyeicosanyl, 1-Octadecyloxyeicosanyl, 1-Heptadecyloxyeicosanyl, 1-Hexadecyloxyeicosanyl, 1-Pentadecyloxyeicosanyl, 1-Tetradecyloxyeicosanyl, 1-Tridecyloxyeicosanyl, 1-Dodecyloxyeicosanyl, 1-Undecyloxyeicosanyl, 1-Decyloxyeicosanyl, 1-Nonyloxyeicosanyl, 1-Octyloxyeicosanyl, 1-Heptyleicosanyl, 1-Hexyloxyeicosanyl, 1-Pentyloxyeicosanyl, 1-Butyloxyeicosanyl, 1-Propyleicosanyl, 1-Ethyloxyeicosanyl, 1-Methyeicosanyl, 1-Eicosanyloxydocosanyl, 1-nonadecyloxydocosanyl, 1-Octadecyloxydocosanyl, 1-Heptadecyloxydocosanyl, 1-Hexadecyloxydocosanyl, 1-Pentadecyloxydocosanyl, 1-Tetradecyloxydocosanyl, 1-Tridecyloxydocosanyl, 1-Undecyloxydocosanyl, 1-Decyloxydocosanyl, 1-Nonyloxydocosanyl, 1-Octyloxydocosanyl, 1-Heptyldocosanyl, 1-Hexyloxydocosanyl, 1-Pentyloxydocosanyl, 1-Butyloxydocosanyl, 1-Propyldocosanyl, 1-Ethyloxydocosanyl, 1-Methyloxydocosanyl, 1-Tricosanyloxytetracosanyl, 1-Docosanyloxytetracosanyl, 1-nonadecyloxytetracosanyl, 1-Octadecyloxytetracosanyl, 1-Heptadecyloxytetracosanyl, 1-Hexadecyloxytetracosanyl, 1-Pentadecyloxytetracosanyl, 1-Pentadecyloxytetracosanyl, 1-Tetradecyloxytetracosanyl, 1-Tridecyloxytetracosanyl, 1-Dodecyloxytetracosanyl, 1-Undecyloxytetracosanyl, 1-Decyloxytetracosanyl, 1-Nonyloxytetracosanyl, 1-Octyloxytetracosanyl, 1-Heptyltetracosanyl, 1-Hexyloxytetracosanyl, 1-Pentyloxytetracosanyl, 1-Butyloxytetracosanyl, 1-Propyltetracosanyl, 1-Ethyloxytetracosanyl, 1-Methyloxytetracosanyl 1-Heptacosanyloxyoctacosanyl, 1-Hexacosanyloxyoctacosanyl, 1-Pentcosanyloxyoctacosanyl, 1-Tetracosanyloxyoctcosanyl, 1-Tricosanytoxyoctacosanyl, 1-Docosanyloxyoctacosanyl, 1-Nonadecyloxyoctacosanyl, 1-Octadecyloxyoctacosanyl, 1-Heptadecyloxyoctacosanyl, 1-Hexadecyloxyoctacosanyl, 1-Hexadecyloxyoctacosanyl, 1-Pentadecyloxyoctacosanyl, 1-Tetradecyloxyoctacosanyl, 1-Tridecyloxyoctacosanyl, 1-Dodecyloxyoctacosanyl, 1-Undecyloxyoctacosanyl, 1-Decyloxyoctacosanyl, 1-Nonyloxyoctacosanyl, 1-Octyloxyoctacosanyl, 1-Heptyloctacosanyl, 1-Hexyloxyoctacosanyl, 1-Pentyloxyoctacosanyl, 1-Butyloxyoctacosanyl, 1-Propyloxyoctacosanyl, 1-Ethyloxyoctacosanyl, 1-Methyloxyoctacosanyl;

1-Ethylthiopropyl, 1-Methylthiopropyl, 1-Propylbutyl, 1-Ethylthiobutyl, 1-Methylthiobutyl, 1-Butylthiopentyl, 1-Propylpentyl, 1-Ethylthiopentyl, 1-Methylthiopentyl, 1-Pentylthiohexyl, 1-Butylthiohexyl, 1-Propylhexyl, 1-Ethylthiohexyl, 1-Methylthiohexyl, 1-Hexylthioheptyl, 1-Pentylthioheptyl, 1-Butylthioheptyl, 1-Propylheptyl, 1-Ethylthioheptyl, 1-Methylthioheptyl, 1-Heptyloctyl, 1-Hexylthiooctyl, 1-Pentylthiooctyl, 1-Butylthiooctyl, 1-Propyloctyl, 1-Ethylthiooctyl, 1-Methylthiooctyl, 1-Octylthiononyl, 1-Heptylnonyl, 1-Hexylthiononyl, 1-Pentylthiononyl, 1-Butylthiononyl, 1-Propylnonyl, 1-Ethylthiononyl, 1-Methylthiononyl, 1-Nonylthiodecyl, 1-Octylthiodecyl, 1-Heptyldecyl, 1-Hexylthiodecyl, 1-Pentylthiodecyl, 1-Butylthiodecyl, 1-Propyldecyl, 1-Ethylthiodecyl, 1-Methylthiodecyl, 1-Decylthioundecyl, 1-Nonylthioundecyl, 1-Octylthioundecyl, 1-Heptylundecyl, 1-Hexylthioundecyl, 1-Pentylthioundecyl, 1-Butylthioundecyl, 1-Propylundecyl, 1-Ethylthioundecyl, 1-Methylthioundecyl, 1-Undecylthiododecyl, 1-Decylthiododecyl, 1-Nonylthiododecyl, 1-Octylthiododecyl, 1-Heptyldodecyl, 1-Hexylthiododecyl, 1-Pentylthiododecyl, 1-Butylthiododecyl, 1-Propyldodecyl, 1-Ethylthiododecyl, 1-Methylthiododecyl, 1-Dodecylthiotridecyl, 1-Undecylthiotridecyl, 1-Decylthiotridecyl, 1-Nonylthiotridecyl, 1-Octylthiotridecyl, 1-Heptyltridecyl, 1-Hexylthiotridecyl, 1-Pentylthiotridecyl, 1-Butylthiotridecyl, 1-Propyltridecyl, 1-Ethylthiotridecyl, 1-Methylthiotridecyl, 1-Tridecylthiotetrdecyl, 1-Undecylthiotetradecyl, 1-Decylthiotetradecyl, 1-Nonylthiotetradecyl, 1-Octylthiotetradecyl, 1-Hetyltetradecyl, 1-Hexylthiotetradecyl, 1-Pentytetradecyl, 1-Butylthiotetradecyl, 1-Propyltetradecyl, 1-Ethylthiotetradecyl, 1-Methylthiotetradecyl, 1-Pentadecylthiohexadecyl, 1-Tetradecylthiohexadecyl, 1-Tridecylthiohexadecyl, 1-Dodecylthiohexadecyl, 1-Undecylthiohexadecyl, 1-Decylthiohexadecyl, 1-Nonylthiohexadecyl, 1-Octylthiohexadecyl, 1-Heptylhexadecyl, 1-Hexylthiohexadecyl, 1-Pentylthiohexadecyl, 1-Butylthiohexadecyl, 1-Propylhexadecyl, 1-Ethylthiohexadecyl, 1-Methylthiohexadecyl, 1-Hexadecylthiooctadecyl, 1-Pentadecylthiooctadecyl, 1-Tetradecylthiooctadecyl, 1-Tridecylthiooctadecyl, 1-Dodecylthiooctadecyl, 1-Undecylthiooctadecyl, 1-Decylthiooctadecyl, 1-Nonylthiooctadecyl, 1-Octylthioocatedecyl, 1-Heptyloctadecyl, 1-Hexylthiooctadecyl, 1-Pentylthiooctadecyl, 1-Butylthiooctaedecyl, 1-Propyloctadecyl, 1-Ethylthiooctadecyl, 1-Methylthiooctadecyl, 1-Nonadecylthioeicosanyl, 1-Octadecylthioeicosanyl, 1-Heptadecylthioeicosanyl, 1-Hexadecylthioeicosanyl, 1-Pentadecylthioeicosanyl, 1-Tetradecylthioeicosanyl, 1-Tridecylthioeicosanyl, 1-Dodecylthioeicosanyl, 1-Undecylthioeicosanyl, 1-Decylthioeicosanyl, 1-Nonylthioeicosanyl, 1-Octylthioeicosanyl, 1-Heptyleicosanyl, 1-Hexylthioeicosanyl, 1-Pentylthioeicosanyl, 1-Butylthioeicosanyl, 1-Propyleicosanyl, 1-Ethylthioeicosanyl, 1-Methyeicosanyl, 1-Eicosanylthiodocosanyl, 1-Nonadecylthiodocosanyl, 1-Octadecylthiodocosanyl, 1-Heptadecylthiodocosanyl, 1-Hexadecylthiodocosanyl, 1-Pentadecylthiodocosanyl, 1-Tetradecylthiodocosanyl, 1-Tridecylthiodocosanyl, 1-Undecylthiodocosanyl, 1-Decylthiodocosanyl, 1-Nonylthiodocosanyl, 1-Octylthiodocosanyl, 1-Heptyldocosanyl, 1-Hexylthiodocosanyl, 1-Pentylthiodocosanyl, 1-Butylthiodocosanyl, 1-Propyldocosanyl, 1-Ethylthiodocosanyl, 1-Methylthiodocosanyl, 1-Tricosanylthiotetracosanyl, 1-Docosanylthiotetracosanyl, 1-Nonadecylthiotetracosanyl, 1-Octadecylthiotetracosanyl, 1-Heptadecylthiotetracosanyl, 1-Hexadecylthiotetracosanyl, 1-Pentadecylthiotetracosanyl, 1-Pentadecylthiotetracosanyl, 1-Tetradecylthiotetracosanyl, 1-Tridecylthiotetracosanyl, 1-Dodecylthiotetracosanyl, 1-Undecylthiotetracosanyl, 1-Decylthiotetracosanyl, 1-Nonylthiotetracosanyl, 1-Octylthiotetracosanyl, 1-Heptyltetracosanyl, 1-Hexylthiotetracosanyl, 1-Pentylthiotetracosanyl, 1-Butylthiotetracosanyl, 1-Propyltetracosanyl, 1-Ethylthiotetracosanyl, 1-Methylthiotetracosanyl 1-Heptacosanylthiooctacosanyl, 1-Hexacosanylthiooctacosanyl, 1-Pentcosanylthiooctacosanyl, 1-Tetracosanylthiooctcosanyl, 1-Tricosanylthiooctacosanyl, 1-Docosanylthiooctacosanyl, 1-Nonadecylthiooctacosanyl, 1-Octadecylthiooctacosanyl, 1-Heptadecylthiooctacosanyl, 1-Hexadecylthiooctacosanyl, 1-Hexadecylthiooctacosanyl, 1-Pentadecylthiooctacosanyl, 1-Tetradecylthiooctacosanyl, 1-Tridecylthiooctacosanyl, 1-Dodecylthiooctacosanyl, 1-Undecylthiooctacosanyl, 1-Decylthiooctacosanyl, 1-Nonylthiooctacosanyl, 1-Octylthiooctacosanyl, 1-Heptyloctacosanyl, 1-Hexylthiooctacosanyl, 1-Pentylthiooctacosanyl, 1-Butylthiooctacosanyl, 1-Propylthiooctacosanyl, 1-Ethylthiooctacosanyl, 1-Methylthiooctacosanyl;
in denen q für 1 steht, wie z. B.
2-Ethylpropyl, 2-Methylpropyl, 2-Propylbutyl, 2-Ethylbutyl, 2-Methylbutyl, 2-Butylpentyl, 2-Propylpentyl, 2-Ethylpentyl, 2-Methylpentyl, 2-Pentylhexyl, 2-Butylhexyl, 2-Propylhexyl, 2-Ethylhexyl, 2-Methylhexyl, 2-Hexylheptyl, 2-Pentylheptyl, 2-Butylheptyl, 2-Propylheptyl, 2-Ethylheptyl, 2-Methylheptyl, 2-Heptyloctyl, 2-Hexyloctyl, 2-Pentyloctyl, 2-Butyloctyl, 2-Propyloctyl, 2-Ethyloctyl, 2-Methyloctyl, 2-Octylnonyl, 2-Heptylnonyl, 2-Hexylnonyl, 2-Pentylnonyl, 2-Butylnonyl, 2-Propylnonyl, 2-Ethylnonyl, 2-Methylnonyl, 2-Nonyldecyl, 2-Octyldecyl, 2-Heptyldecyl, 2-Hexyldecyl, 2-Pentyldecyl, 2-Butyldecyl, 2-Propyldecyl, 2-Ethyldecyl, 2-Methyldecyl, 2-Decylundecyl, 2-Nonylundecyl, 2-Octylundecyl, 2-Heptylundecyl, 2-Hexylundecyl, 2-Pentylundecyl, 2-Butylundecyl, 2-Propylundecyl, 2-Ethylundecyl, 2-Methylundecyl, 2-Undecyldodecyl, 2-Decyldodecyl, 2-Nonyldodecyl, 2-Octyldodecyl, 2-Heptyldodecyl, 2-Hexyldodecyl, 2-Pentyldodecyl, 2-Butyldodecyl, 2-Propyldodecyl, 2-Ethyldodecyl, 2-Methyldodecyl, 2-Dodecyltridecyl, 2-Undecyltridecyl, 2-Decyltridecyl, 2-Nonyltridecyl, 2-Octyltridecyl, 2-Heptyltridecyl, 2-Hexyltridecyl, 2-Pentyltridecyl, 2-Butyltridecyl, 2-Propyltridecyl, 2-Ethyltridecyl, 2-Methyltridecyl, 2-Tridecyltetrdecyl, 2-Undecyltetradecyl, 2-Decyltetradecyl, 2-Nonyltetradecyl, 2-Octyltetradecyl, 2-Hetyltetradecyl, 2-Hexyltetradecyl, 2-Pentytetradecyl, 2-Butyltetradecyl, 2-Propyltetradecyl, 2-Ethyltetradecyl, 2-Methyltetradecyl, 2-Pentadecylhexadecyl, 2-Tetradecylhexadecyl, 2-Tridecylhexadecyl, 2-Dodecylhexadecyl, 2-Undecylhexadecyl, 2-Decylhexadecyl, 2-Nonylhexadecyl, 2-Octylhexadecyl, 2-Heptylhexadecyl, 2-Hexylhexadecyl, 2-Pentylhexadecyl, 2-Butylhexadecyl, 2-Propylhexadecyl, 2-Ethylhexadecyl, 2-Methylhexadecyl, 2-Hexadecyloctadecyl, 2-Pentadecyloctadecyl, 2-Tetradecyloctadecyl, 2-Tridecyloctadecyl, 2-Dodecyloctadecyl, 2-Undecyloctadecyl, 2-Decyloctadecyl, 2-Nonyloctadecyl, 2-Octylocatedecyl, 2-Heptyloctadecyl, 2-Hexyloctadecyl, 2-Pentyloctadecyl, 2-Butyloctaedecyl, 2-Propyloctadecyl, 2-Ethyloctadecyl, 2-Methyloctadecyl 2-Nonadecyleicosanyl, 2-Octadecyleicosanyl, 2-Heptadecyleicosanyl, 2-Hexadecyleicosanyl, 2-Pentadecyleicosanyl, 2-Tetradecyleicosanyl, 2-Tridecyleicosanyl, 2-Dodecyleicosanyl, 2-Undecyleicosanyl, 2-Decyleicosanyl, 2-Nonyleicosanyl, 2-Octyleicosanyl, 2-Heptyleicosanyl, 2-Hexyleicosanyl, 2-Pentyleicosanyl, 2-Butyleicosanyl, 2-Propyleicosanyl, 2-Ethyleicosanyl, 2-Methyeicosanyl 2-Eicosanyldocosanyl, 2-Nonadecyldocosanyl, 2-Octadecyldocosanyl, 2-Heptadecyldocosanyl, 2-Hexadecyldocosanyl, 2-Pentadecyldocosanyl, 2-Tetradecyldocosanyl, 2-Tridecyldocosanyl, 2-Undecyldocosanyl, 2-Decyldocosanyl, 2-Nonyldocosanyl, 2-Octyldocosanyl, 2-Heptyldocosanyl, 2-Hexyldocosanyl, 2-Pentyldocosanyl, 2-Butyldocosanyl, 2-Propyldocosanyl, 2-Ethyldocosanyl, 2-Methyldocosanyl 2-Tricosanyltetracosanyl, 2-Docosanyltetracosanyl, 2-Nonadecyltetracosanyl, 2-Octadecyltetracosanyl, 2-Heptadecyltetracosanyl, 2-Hexadecyltetracosanyl, 2-Pentadecyltetracosanyl, 2-Pentadecyltetracosanyl, 2-Tetradecyltetracosanyl, 2-Tridecyltetracosanyl, 2-Dodecyltetracosanyl, 2-Undecyltetracosanyl, 2-Decyltetracosanyl, 2-Nonyltetracosanyl, 2-Octyltetracosanyl, 2-Heptyltetracosanyl, 2-Hexyltetracosanyl, 2-Pentyltetracosanyl, 2-Butyltetracosanyl, 2-Propyltetracosanyl, 2-Ethyltetracosanyl, 2-Methyltetracosanyl 2-Heptacosanyloctacosanyl, 2-Hexacosanyloctacosanyl, 2-Pentcosanyloctacosanyl, 2-Tetracosanyloctcosanyl, 2-Tricosanyloctacosanyl, 2-Docosanyloctacosanyl, 2-Nonadecyloctacosanyl, 2-Octadecyloctacosanyl, 2-Heptadecyloctacosanyl, 2-Hexadecyloctacosanyl, 2-Hexadecyloctacosanyl, 2-Pentadecyloctacosanyl, 2-Tetradecyloctacosanyl, 2-Tridecyloctacosanyl, 2-Dodecyloctacosanyl, 2-Undecyloctacosanyl, 2-Decyloctacosanyl, 2-Nonyloctacosanyl, 2-Octyloctacosanyl, 2-Heptyloctacosanyl, 2-Hexyloctacosanyl, 2-Pentyloctacosanyl, 2-Butyloctacosanyl, 2-Propyloctacosanyl, 2-Ethyloctacosanyl, 2-Methyloctacosanyl;
in denen q für 2 steht, wie z. B.
3-Ethylpropyl, 3-Methylpropyl, 3-Propylbutyl, 3-Ethylbutyl, 3-Methylbutyl, 3-Butylpentyl, 3-Propylpentyl, 3-Ethylpentyl, 3-Methylpentyl, 3-Pentylhexyl, 3-Butylhexyl, 3-Propylhexyl, 3-Ethylhexyl, 3-Methylhexyl, 3-Hexylheptyl, 3-Pentylheptyl, 3-Butylheptyl, 3-Propylheptyl, 3-Ethylheptyl, 3-Methylheptyl, 3-Heptyloctyl, 3-Hexyloctyl, 3-Pentyloctyl, 3-Butyloctyl, 3-Propyloctyl, 3-Ethyloctyl, 3-Methyloctyl, 3-Octylnonyl, 3-Heptylnonyl, 3-Hexylnonyl, 3-Pentylnonyl, 3-Butylnonyl, 3-Propylnonyl, 3-Ethylnonyl, 3-Methylnonyl, 3-Nonyldecyl, 3-Octyldecyl, 3-Heptyldecyl, 3-Hexyldecyl, 3-Pentyldecyl, 3-Butyldecyl, 3-Propyldecyl, 3-Ethyldecyl, 3-Methyldecyl, 3-Decylundecyl, 3-Nonylundecyl, 3-Octylundecyl, 3-Heptylundecyl, 3-Hexylundecyl, 3-Pentylundecyl, 3-Butylundecyl, 3-Propylundecyl, 3-Ethylundecyl, 3-Methylundecyl, 3-Undecyldodecyl, 3-Decyldodecyl, 3-Nonyldodecyl, 3-Octyldodecyl, 3-Heptyldodecyl, 3-Hexyldodecyl, 3-Pentyldodecyl, 3-Butyldodecyl, 3-Propyldodecyl, 3-Ethyldodecyl, 3-Methyldodecyl, 3-Dodecyltridecyl, 3-Undecyltridecyl, 3-Decyltridecyl, 3-Nonyltridecyl, 3-Octyltridecyl, 3-Heptyltridecyl, 3-Hexyltridecyl, 3-Pentyltridecyl, 3-Butyltridecyl, 3-Propyltridecyl, 3-Ethyltridecyl, 3-Methyltridecyl, 3-Tridecyltetrdecyl, 3-Undecyltetradecyl, 3-Decyltetradecyl, 3-Nonyltetradecyl, 3-Octyltetradecyl, 3-Hetyltetradecyl, 3-Hexyltetradecyl, 3-Pentytetradecyl, 3-Butyltetradecyl, 3-Propyltetradecyl, 3-Ethyltetradecyl, 3-Methyltetradecyl, 3-Pentadecylhexadecyl, 3-Tetradecylhexadecyl, 3-Tridecylhexadecyl, 3-Dodecylhexadecyl, 3-Undecylhexadecyl, 3-Decylhexadecyl, 3-Nonylhexadecyl, 3-Octylhexadecyl, 3-Heptylhexadecyl, 3-Hexylhexadecyl, 3-Pentylhexadecyl, 3-Butylhexadecyl, 3-Propylhexadecyl, 3-Ethylhexadecyl, 3-Methylhexadecyl, 3-Hexadecyloctadecyl, 3-Pentadecyloctadecyl, 3-Tetradecyloctadecyl, 3-Tridecyloctadecyl, 3-Dodecyloctadecyl, 3-Undecyloctadecyl, 3-Decyloctadecyl, 3-Nonyloctadecyl, 3-Octylocatedecyl, 3-Heptyloctadecyl, 3-Hexyloctadecyl, 3-Pentyloctadecyl, 3-Butyloctaedecyl, 3-Propyloctadecyl, 3-Ethyloctadecyl, 3-Methyloctadecyl, 3-Nonadecyleicosanyl, 3-Octadecyleicosanyl, 3-Heptadecyleicosanyl, 3-Hexadecyleicosanyl, 3-Pentadecyleicosanyl, 3-Tetradecyleicosanyl, 3-Tridecyleicosanyl, 3-Dodecyleicosanyl, 3-Undecyleicosanyl, 3-Decyleicosanyl, 3-Nonyleicosanyl, 3-Octyleicosanyl, 3-Heptyleicosanyl, 3-Hexyleicosanyl, 3-Pentyleicosanyl, 3-Butyleicosanyl, 3-Propyleicosanyl, 3-Ethyleicosanyl, 3-Methyeicosanyl, 3-Eicosanyldocosanyl, 3-Nonadecyldocosanyl, 3-Octadecyldocosanyl, 3-Heptadecyldocosanyl, 3-Hexadecyldocosanyl, 3-Pentadecyldocosanyl, 3-Tetradecyldocosanyl, 3-Tridecyldocosanyl, 3-Undecyldocosanyl, 3-Decyldocosanyl, 3-Nonyldocosanyl, 3-Octyldocosanyl, 3-Heptyldocosanyl, 3-Hexyldocosanyl, 3-Pentyldocosanyl, 3-Butyldocosanyl, 3-Propyldocosanyl, 3-Ethyldocosanyl, 3-Methyldocosanyl 3-Tricosanyltetracosanyl, 3-Docosanyltetracosanyl, 3-Nonadecyltetracosanyl, 3-Octadecyltetracosanyl, 3-Heptadecyltetracosanyl, 3-Hexadecyltetracosanyl, 3-Pentadecyltetracosanyl, 3-Pentadecyltetracosanyl, 3-Tetradecyltetracosanyl, 3-Tridecyltetracosanyl, 3-Dodecyltetracosanyl, 3-Undecyltetracosanyl, 3-Decyltetracosanyl, 3-Nonyltetracosanyl, 3-Octyltetracosanyl, 3-Heptyltetracosanyl, 3-Hexyltetracosanyl, 3-Pentyltetracosanyl, 3-Butyltetracosanyl, 3-Propyltetracosanyl, 3-Ethyltetracosanyl, 3-Methyltetracosanyl, 3-Heptacosanyloctacosanyl, 3-Hexacosanyloctacosanyl, 3-Pentcosanyloctacosanyl, 3-Tetracosanyloctcosanyl, 3-Tricosanyloctacosanyl, 3-Docosanyloctacosanyl, 3-Nonadecyloctacosanyl, 3-Octadecyloctacosanyl, 3-Heptadecyloctacosanyl, 3-Hexadecyloctacosanyl, 3-Hexadecyloctacosanyl, 3-Pentadecyloctacosanyl, 3-Tetradecyloctacosanyl, 3-Tridecyloctacosanyl, 3-Dodecyloctacosanyl, 3-Undecyloctacosanyl, 3-Decyloctacosanyl, 3-Nonyloctacosanyl, 3-Octyloctacosanyl, 3-Heptyloctacosanyl, 3-Hexyloctacosanyl, 3-Pentyloctacosanyl, 3-Butyloctacosanyl, 3-Propyloctacosanyl, 3-Ethyloctacosanyl, 3-Methyloctacosanyl,
in denen q für 3 steht, wie
4-Butylpentyl, 4-Propylpentyl, 4-Ethylpentyl, 4-Methylpentyl, 4-Pentylhexyl, 4-Butylhexyl, 4-Propylhexyl, 4-Ethylhexyl, 4-Methylhexyl, 4-Hexylheptyl, 4-Pentylheptyl, 4-Butylheptyl, 4-Propylheptyl, 4-Ethylheptyl, 4-Methylheptyl, 4-Heptyloctyl, 4-Hexyloctyl, 4-Pentyloctyl, 4-Butyloctyl, 4-Propyloctyl, 4-Ethyloctyl, 4-Methyloctyl, 4-Octylnonyl, 4-Heptylnonyl, 4-Hexylnonyl, 4-Pentylnonyl, 4-Butylnonyl, 4-Propylnonyl, 4-Ethylnonyl, 4-Methylnonyl, 4-Nonyldecyl, 4-Octyldecyl, 4-Heptyldecyl, 4-Hexyldecyl, 4-Pentyldecyl, 4-Butyldecyl, 4-Propyldecyl, 4-Ethyldecyl, 4-Methyldecyl, 4-Decylundecyl, 4-Nonylundecyl, 4-Octylundecyl, 4-Heptylundecyl, 4-Hexylundecyl, 4-Pentylundecyl, 4-Butylundecyl, 4-Propylundecyl, 4-Ethylundecyl, 4-Methylundecyl, 4-Undecyldodecyl, 4-Decyldodecyl, 4-Nonyldodecyl, 4-Octyldodecyl, 4-Heptyldodecyl, 4-Hexyldodecyl, 4-Pentyldodecyl, 4-Butyldodecyl, 4-Propyldodecyl, 4-Ethyldodecyl, 4-Methyldodecyl, 4-Dodecyltridecyl, 4-Undecyltridecyl, 4-Decyltridecyl, 4-Nonyltridecyl, 4-Octyltridecyl, 4-Heptyltridecyl, 4-Hexyltridecyl, 4-Pentyltridecyl, 4-Butyltridecyl, 4-Propyltridecyl, 4-Ethyltridecyl, 4-Methyltridecyl, 4-Tridecyltetrdecyl, 4-Undecyltetradecyl, 4-Decyltetradecyl, 4-Nonyltetradecyl, 4-Octyltetradecyl, 4-Hetyltetradecyl, 4-Hexyltetradecyl, 4-Pentytetradecyl, 4-Butyltetradecyl, 4-Propyltetradecyl, 4-Ethyltetradecyl, 4-Methyltetradecyl, 4-Pentadecylhexadecyl, 4-Tetradecylhexadecyl, 4-Tridecylhexadecyl, 4-Dodecylhexadecyl, 4-Undecylhexadecyl, 4-Decylhexadecyl, 4-Nonylhexadecyl, 4-Octylhexadecyl, 4-Heptylhexadecyl, 4-Hexylhexadecyl, 4-Pentylhexadecyl, 4-Butylhexadecyl, 4-Propylhexadecyl, 4-Ethylhexadecyl, 4-Methylhexadecyl, 4-Hexadecyloctadecyl, 4-Pentadecyloctadecyl, 4-Tetradecyloctadecyl, 4-Tridecyloctadecyl, 4-Dodecyloctadecyl, 4-Undecyloctadecyl, 4-Decyloctadecyl, 4-Nonyloctadecyl, 4-Octylocatedecyl, 4-Heptyloctadecyl, 4-Hexyloctadecyl, 4-Pentyloctadecyl, 4-Butyloctaedecyl, 4-Propyloctadecyl, 4-Ethyloctadecyl, 4-Methyloctadecyl 4-Nonadecyleicosanyl, 4-Octadecyleicosanyl, 4-Heptadecyleicosanyl, 4-Hexadecyleicosanyl, 4-Pentadecyleicosanyl, 4-Tetradecyleicosanyl, 4-Tridecyleicosanyl, 4-Dodecyleicosanyl, 4-Undecyleicosanyl, 4-Decyleicosanyl, 4-Nonyleicosanyl, 4-Octyleicosanyl, 4-Heptyleicosanyl, 4-Hexyleicosanyl, 4-Pentyleicosanyl, 4-Butyleicosanyl, 4-Propyleicosanyl, 4-Ethyleicosanyl, 4-Methyeicosanyl, 4-Eicosanyldocosanyl, 4-Nonadecyldocosanyl, 4-Octadecyldocosanyl, 4-Heptadecyldocosanyl, 4-Hexadecyldocosanyl, 4-Pentadecyldocosanyl, 4-Tetradecyldocosanyl, 4-Tridecyldocosanyl, 4-Undecyldocosanyl, 4-Decyldocosanyl, 4-Nonyldocosanyl, 4-Octyldocosanyl, 4-Heptyldocosanyl, 4-Hexyldocosanyl, 4-Pentyldocosanyl, 4-Butyldocosanyl, 4-Propyldocosanyl, 4-Ethyldocosanyl, 4-Methyldocosanyl, 4-Tricosanyltetracosanyl, 4-Docosanyltetracosanyl, 4-Nonadecyltetracosanyl, 4-Octadecyltetracosanyl, 4-Heptadecyltetracosanyl, 4-Hexadecyltetracosanyl, 4-Pentadecyltetracosanyl, 4-Pentadecyltetracosanyl, 4-Tetradecyltetracosanyl, 4-Tridecyltetracosanyl, 4-Dodecyltetracosanyl, 4-Undecyltetracosanyl, 4-Decyltetracosanyl, 4-Nonyltetracosanyl, 4-Octyltetracosanyl, 4-Heptyltetracosanyl, 4-Hexyltetracosanyl, 4-Pentyltetracosanyl, 4-Butyltetracosanyl, 4-Propyltetracosanyl, 4-Ethyltetracosanyl, 4-Methyltetracosanyl 4-Heptacosanyloctacosanyl, 4-Hexacosanyloctacosanyl, 4-Pentcosanyloctacosanyl, 4-Tetracosanyloctcosanyl, 4-Tricosanyloctacosanyl, 4-Docosanyloctacosanyl, 4-Nonadecyloctacosanyl, 4-Octadecyloctacosanyl, 4-Heptadecyloctacosanyl, 4-Hexadecyloctacosanyl, 4-Hexadecyloctacosanyl, 4-Pentadecyloctacosanyl, 4-Tetradecyloctacosanyl, 4-Tridecyloctacosanyl, 4-Dodecyloctacosanyl, 4-Undecyloctacosanyl, 4-Decyloctacosanyl, 4-Nonyloctacosanyl, 4-Octyloctacosanyl, 4-Heptyloctacosanyl, 4-Hexyloctacosanyl, 4-Pentyloctacosanyl, 4-Butyloctacosanyl, 4-Propyloctacosanyl, 4-Ethyloctacosanyl, 4-Methyloctacosanyl,
in denen q für 4 steht, wie
5-Pentylhexyl, 5-Butylhexyl, 5-Propylhexyl, 5-Ethylhexyl, 5-Methylhexyl, 5-Hexylheptyl, 5-Pentylheptyl, 5-Butylheptyl, 5-Propylheptyl, 5-Ethylheptyl, 5-Methylheptyl, 5-Heptyloctyl, 5-Hexyloctyl, 5-Pentyloctyl, 5-Butyloctyl, 5-Propyloctyl, 5-Ethyloctyl, 5-Methyloctyl, 5-Octylnonyl, 5-Heptylnonyl, 5-Hexylnonyl, 5-Pentylnonyl, 5-Butylnonyl, 5-Propylnonyl, 5-Ethylnonyl, 5-Methylnonyl, 5-Nonyldecyl, 5-Octyldecyl, 5-Heptyldecyl, 5-Hexyldecyl, 5-Pentyldecyl, 5-Butyldecyl, 5-Propyldecyl, 5-Ethyldecyl, 5-Methyldecyl, 5-Decylundecyl, 5-Nonylundecyl, 5-Octylundecyl, 5-Heptylundecyl, 5-Hexylundecyl, 5-Pentylundecyl, 5-Butylundecyl, 5-Propylundecyl, 5-Ethylundecyl, 5-Methylundecyl, 5-Undecyldodecyl, 5-Decyldodecyl, 5-Nonyldodecyl, 5-Octyldodecyl, 5-Heptyldodecyl, 5-Hexyldodecyl, 5-Pentyldodecyl, 5-Butyldodecyl, 5-Propyldodecyl, 5-Ethyldodecyl, 5-Methyldodecyl, 5-Dodecyltridecyl, 5-Undecyltridecyl, 5-Decyltridecyl, 5-Nonyltridecyl, 5-Octyltridecyl, 5-Heptyltridecyl, 5-Hexyltridecyl, 5-Pentyltridecyl, 5-Butyltridecyl, 5-Propyltridecyl, 5-Ethyltridecyl, 5-Methyltridecyl, 5-Tridecyltetrdecyl, 5-Undecyltetradecyl, 5-Decyltetradecyl, 5-Nonyltetradecyl, 5-Octyltetradecyl, 5-Hetyltetradecyl, 5-Hexyltetradecyl, 5-Pentytetradecyl, 5-Butyltetradecyl, 5-Propyltetradecyl, 5-Ethyltetradecyl, 5-Methyltetradecyl, 5-Pentadecylhexadecyl, 5-Tetradecylhexadecyl, 5-Tridecylhexadecyl, 5-Dodecylhexadecyl, 5-Undecylhexadecyl, 5-Decylhexadecyl, 5-Nonylhexadecyl, 5-Octylhexadecyl, 5-Heptylhexadecyl, 5-Hexylhexadecyl, 5-Pentylhexadecyl, 5-Butylhexadecyl, 5-Propylhexadecyl, 5-Ethylhexadecyl, 5-Methylhexadecyl, 5-Hexadecyloctadecyl, 5-Pentadecyloctadecyl, 5-Tetradecyloctadecyl, 5-Tridecyloctadecyl, 5-Dodecyloctadecyl, 5-Undecyloctadecyl, 5-Decyloctadecyl, 5-Nonyloctadecyl, 5-Octylocatedecyl, 5-Heptyloctadecyl, 5-Hexyloctadecyl, 5-Pentyloctadecyl, 5-Butyloctaedecyl, 5-Propyloctadecyl, 5-Ethyloctadecyl, 5-Methyloctadecyl 5-Nonadecyleicosanyl, 5-Octadecyleicosanyl, 5-Heptadecyleicosanyl, 5-Hexadecyleicosanyl, 5-Pentadecyleicosanyl, 5-Tetradecyleicosanyl, 5-Tridecyleicosanyl, 5-Dodecyleicosanyl, 5-Undecyleicosanyl, 5-Decyleicosanyl, 5-Nonyleicosanyl, 5-Octyleicosanyl, 5-Heptyleicosanyl, 5-Hexyleicosanyl, 5-Pentyleicosanyl, 5-Butyleicosanyl, 5-Propyleicosanyl, 5-Ethyleicosanyl, 5-Methyeicosanyl, 5-Eicosanyldocosanyl, 5-Nonadecyldocosanyl, 5-Octadecyldocosanyl, 5-Heptadecyldocosanyl, 5-Hexadecyldocosanyl, 5-Pentadecyldocosanyl, 5-Tetradecyldocosanyl, 5-Tridecyldocosanyl, 5-Undecyldocosanyl, 5-Decyldocosanyl, 5-Nonyldocosanyl, 5-Octyldocosanyl, 5-Heptyldocosanyl, 5-Hexyldocosanyl, 5-Pentyldocosanyl, 5-Butyldocosanyl, 5-Propyldocosanyl, 5-Ethyldocosanyl, 5-Methyldocosanyl, 5-Tricosanyltetracosanyl, 5-Docosanyltetracosanyl, 5-Nonadecyltetracosanyl, 5-Octadecyltetracosanyl, 5-Heptadecyltetracosanyl, 5-Hexadecyltetracosanyl, 5-Pentadecyltetracosanyl, 5-Pentadecyltetracosanyl, 5-Tetradecyltetracosanyl, 5-Tridecyltetracosanyl, 5-Dodecyltetracosanyl, 5-Undecyltetracosanyl, 5-Decyltetracosanyl, 5-Nonyltetracosanyl, 5-Octyltetracosanyl, 5-Heptyltetracosanyl, 5-Hexyltetracosanyl, 5-Pentyltetracosanyl, 5-Butyltetracosanyl, 5-Propyltetracosanyl, 5-Ethyltetracosanyl, 5-Methyltetracosanyl 5-Heptacosanyloctacosanyl, 5-Hexacosanyloctacosanyl, 5-Pentcosanyloctacosanyl, 5-Tetracosanyloctcosanyl, 5-Tricosanyloctacosanyl, 5-Docosanyloctacosanyl, 5-Nonadecyloctacosanyl, 5-Octadecyloctacosanyl, 5-Heptadecyloctacosanyl, 5-Hexadecyloctacosanyl, 5-Hexadecyloctacosanyl, 5-Pentadecyloctacosanyl, 5-Tetradecyloctacosanyl, 5-Tridecyloctacosanyl, 5-Dodecyloctacosanyl, 5-Undecyloctacosanyl, 5-Decyloctacosanyl, 5-Nonyloctacosanyl, 5-Octyloctacosanyl, 5-Heptyloctacosanyl, 5-Hexyloctacosanyl, 5-Pentyloctacosanyl, 5-Butyloctacosanyl, 5-Propyloctacosanyl, 5-Ethyloctacosanyl, 5-Methyloctacosanyl
in denen q für 5 steht, wie z. B.
6-Hexylheptyl, 6-Pentylheptyl, 6-Butylheptyl, 6-Propylheptyl, 6-Ethylheptyl, 6-Methylheptyl, 6-Heptyloctyl, 6-Hexyloctyl, 6-Pentyloctyl, 6-Butyloctyl, 6-Propyloctyl, 6-Ethyloctyl, 6-Methyloctyl, 6-Octylnonyl, 6-Heptylnonyl, 6-Hexylnonyl, 6-Pentylnonyl, 6-Butylnonyl, 6-Propylnonyl, 6-Ethylnonyl, 6-Methylnonyl, 6-Nonyldecyl, 6-Octyldecyl, 6-Heptyldecyl, 6-Hexyldecyl, 6-Pentyldecyl, 6-Butyldecyl, 6-Propyldecyl, 6-Ethyldecyl, 6-Methyldecyl, 6-Decylundecyl, 6-Nonylundecyl, 6-Octylundecyl, 6-Heptylundecyl, 6-Hexylundecyl, 6-Pentylundecyl, 6-Butylundecyl, 6-Propylundecyl, 6-Ethylundecyl, 6-Methylundecyl, 6-Undecyldodecyl, 6-Decyldodecyl, 6-Nonyldodecyl, 6-Octyldodecyl, 6-Heptyldodecyl, 6-Hexyldodecyl, 6-Pentyldodecyl, 6-Butyldodecyl, 6-Propyldodecyl, 6-Ethyldodecyl, 6-Methyldodecyl, 6-Dodecyltridecyl, 6-Undecyltridecyl, 6-Decyltridecyl, 6-Nonyltridecyl, 6-Octyltridecyl, 6-Heptyltridecyl, 6-Hexyltridecyl, 6-Pentyltridecyl, 6-Butyltridecyl, 6-Propyltridecyl, 6-Ethyltridecyl, 6-Methyltridecyl, 6-Tridecyltetrdecyl, 6-Undecyltetradecyl, 6-Decyltetradecyl, 6-Nonyltetradecyl, 6-Octyltetradecyl, 6-Hetyltetradecyl, 6-Hexyltetradecyl, 6-Pentytetradecyl, 6-Butyltetradecyl, 6-Propyltetradecyl, 6-Ethyltetradecyl, 6-Methyltetradecyl, 6-Pentadecylhexadecyl, 6-Tetradecylhexadecyl, 6-Tridecylhexadecyl, 6-Dodecylhexadecyl, 6-Undecylhexadecyl, 6-Decylhexadecyl, 6-Nonylhexadecyl, 6-Octylhexadecyl, 6-Heptylhexadecyl, 6-Hexylhexadecyl, 6-Pentylhexadecyl, 6-Butylhexadecyl, 6-Propylhexadecyl, 6-Ethylhexadecyl, 6-Methylhexadecyl, 6-Hexadecyloctadecyl, 6-Pentadecyloctadecyl, 6-Tetradecyloctadecyl, 6-Tridecyloctadecyl, 6-Dodecyloctadecyl, 6-Undecyloctadecyl, 6-Decyloctadecyl, 6-Nonyloctadecyl, 6-Octylocatedecyl, 6-Heptyloctadecyl, 6-Hexyloctadecyl, 6-Pentyloctadecyl, 6-Butyloctaedecyl, 6-Propyloctadecyl, 6-Ethyloctadecyl, 6-Methyloctadecyl 6-Nonadecyleicosanyl, 6-Octadecyleicosanyl, 6-Heptadecyleicosanyl, 6-Hexadecyleicosanyl, 6-Pentadecyleicosanyl, 6-Tetradecyleicosanyl, 6-Tridecyleicosanyl, 6-Dodecyleicosanyl, 6-Undecyleicosanyl, 6-Decyleicosanyl, 6-Nonyleicosanyl, 6-Octyleicosanyl, 6-Heptyleicosanyl, 6-Hexyleicosanyl, 6-Pentyleicosanyl, 6-Butyleicosanyl, 6-Propyleicosanyl, 6-Ethyleicosanyl, 6-Methyeicosanyl, 6-Eicosanyldocosanyl, 6-Nonadecyldocosanyl, 6-Octadecyldocosanyl, 6-Heptadecyldocosanyl, 6-Hexadecyldocosanyl, 6-Pentadecyldocosanyl, 6-Tetradecyldocosanyl, 6-Tridecyldocosanyl, 6-Undecyldocosanyl, 6-Decyldocosanyl, 6-Nonyldocosanyl, 6-Octyldocosanyl, 6-Heptyldocosanyl, 6-Hexyldocosanyl, 6-Pentyldocosanyl, 6-Butyldocosanyl, 6-Propyldocosanyl, 6-Ethyldocosanyl, 6-Methyldocosanyl, 6-Tricosanyltetracosanyl, 6-Docosanyltetracosanyl, 6-Nonadecyltetracosanyl, 6-Octadecyltetracosanyl, 6-Heptadecyltetracosanyl, 6-Hexadecyltetracosanyl, 6-Pentadecyltetracosanyl, 6-Pentadecyltetracosanyl, 6-Tetradecyltetracosanyl, 6-Tridecyltetracosanyl, 6-Dodecyltetracosanyl, 6-Undecyltetracosanyl, 6-Decyltetracosanyl, 6-Nonyltetracosanyl, 6-Octyltetracosanyl, 6-Heptyltetracosanyl, 6-Hexyltetracosanyl, 6-Pentyltetracosanyl, 6-Butyltetracosanyl, 6-Propyltetracosanyl, 6-Ethyltetracosanyl, 6-Methyltetracosanyl 6-Heptacosanyloctacosanyl, 6-Hexacosanyloctacosanyl, 6-Pentcosanyloctacosanyl, 6-Tetracosanyloctcosanyl, 6-Tricosanyloctacosanyl, 6-Docosanyloctacosanyl, 6-Nonadecyloctacosanyl, 6-Octadecyloctacosanyl, 6-Heptadecyloctacosanyl, 6-Hexadecyloctacosanyl, 6-Hexadecyloctacosanyl, 6-Pentadecyloctacosanyl, 6-Tetradecyloctacosanyl, 6-Tridecyloctacosanyl, 6-Dodecyloctacosanyl, 6-Undecyloctacosanyl, 6-Decyloctacosanyl, 6-Nonyloctacosanyl, 6-Octyloctacosanyl, 6-Heptyloctacosanyl, 6-Hexyloctacosanyl, 6-Pentyloctacosanyl, 6-Butyloctacosanyl, 6-Propyloctacosanyl, 6-Ethyloctacosanyl, 6-Methyloctacosanyl,
in denen q für 6 steht, wie z. B.
7-Heptyloctyl, 7-Hexyloctyl, 7-Pentyloctyl, 7-Butyloctyl, 7-Propyloctyl, 7-Ethyloctyl, 7-Methyloctyl, 7-Octylnonyl, 7-Heptylnonyl, 7-Hexylnonyl, 7-Pentylnonyl, 7-Butylnonyl, 7-Propylnonyl, 7-Ethylnonyl, 7-Methylnonyl, 7-Nonyldecyl, 7-Octyldecyl, 7-Heptyldecyl, 7-Hexyldecyl, 7-Pentyldecyl, 7-Butyldecyl, 7-Propyldecyl, 7-Ethyldecyl, 7-Methyldecyl, 7-Decylundecyl, 7-Nonylundecyl, 7-Octylundecyl, 7-Heptylundecyl, 7-Hexylundecyl, 7-Pentylundecyl, 7-Butylundecyl, 7-Propylundecyl, 7-Ethylundecyl, 7-Methylundecyl, 7-Undecyldodecyl, 7-Decyldodecyl, 7-Nonyldodecyl, 7-Octyldodecyl, 7-Heptyldodecyl, 7-Hexyldodecyl, 7-Pentyldodecyl, 7-Butyldodecyl, 7-Propyldodecyl, 7-Ethyldodecyl, 7-Methyldodecyl, 7-Dodecyltridecyl, 7-Undecyltridecyl, 7-Decyltridecyl, 7-Nonyltridecyl, 7-Octyltridecyl, 7-Heptyltridecyl, 7-Hexyltridecyl, 7-Pentyltridecyl, 7-Butyltridecyl, 7-Propyttridecyl, 7-Ethyltridecyl, 7-Methyltridecyl, 7-Tridecyltetrdecyl, 7-Undecyltetradecyl, 7-Decyltetradecyl, 7-Nonyltetradecyl, 7-Octyltetradecyl, 7-Hetyltetradecyl, 7-Hexyltetradecyl, 7-Pentytetradecyl, 7-Butyltetradecyl, 7-Propyltetradecyl, 7-Ethyltetradecyl, 7-Methyltetradecyl, 7-Pentadecylhexadecyl, 7-Tetradecylhexadecyl, 7-Tridecylhexadecyl, 7-Dodecylhexadecyl, 7-Undecylhexadecyl, 7-Decylhexadecyl, 7-Nonylhexadecyl, 7-Octylhexadecyl, 7-Heptylhexadecyl, 7-Hexylhexadecyl, 7-Pentylhexadecyl, 7-Butylhexadecyl, 7-Propylhexadecyl, 7-Ethylhexadecyl, 7-Methylhexadecyl, 7-Hexadecyloctadecyl, 7-Pentadecyloctadecyl, 7-Tetradecyloctadecyl, 7-Tridecyloctadecyl, 7-Dodecyloctadecyl, 7-Undecyloctadecyl, 7-Decyloctadecyl, 7-Nonyloctadecyl, 7-Octylocatedecyl, 7-Heptyloctadecyl, 7-Hexyloctadecyl, 7-Pentyloctadecyl, 7-Butyloctaedecyl, 7-Propyloctadecyl, 7-Ethyloctadecyl, 7-Methyloctadecyl 7-Nonadecyleicosanyl, 7-Octadecyleicosanyl, 7-Heptadecyleicosanyl, 7-Hexadecyleicosanyl, 7-Pentadecyleicosanyl, 7-Tetradecyleicosanyl, 7-Tridecyleicosanyl, 7-Dodecyleicosanyl, 7-Undecyleicosanyl, 7-Decyleicosanyl, 7-Nonyleicosanyl, 7-Octyleicosanyl, 7-Heptyleicosanyl, 7-Hexyleicosanyl, 7-Pentyleicosanyl, 7-Butyleicosanyl, 7-Propyleicosanyl, 7-Ethyleicosanyl, 7-Methyleicosanyl, 7-Eicosanyldocosanyl, 7-Nonadecyldocosanyl, 7-Octadecyldocosanyl, 7-Heptadecyldocosanyl, 7-Hexadecyldocosanyl, 7-Pentadecyldocosanyl, 7-Tetradecyldocosanyl, 7-Tridecyldocosanyl, 7-Undecyldocosanyl, 7-Decyldocosanyl, 7-Nonyldocosanyl, 7-Octyldocosanyl, 7-Heptyldocosanyl, 7-Hexyldocosanyl, 7-Pentyldocosanyl, 7-Butyldocosanyl, 7-Propyldocosanyl, 7-Ethyldocosanyl, 7-Methyldocosanyl 7-Tricosanyltetracosanyl, 7-Docosanyltetracosanyl, 7-Nonadecyltetracosanyl, 7-Octadecyltetracosanyl, 7-Heptadecyltetracosanyl, 7-Hexadecyltetracosanyl, 7-Pentadecyltetracosanyl, 7-Pentadecyltetracosanyl, 7-Tetradecyltetracosanyl, 7-Tridecyltetracosanyl, 7-Dodecyltetracosanyl, 7-Undecyltetracosanyl, 7-Decyltetracosanyl, 7-Nonyltetracosanyl, 7-Octyltetracosanyl, 7-Heptyltetracosanyl, 7-Hexyltetracosanyl, 7-Pentyltetracosanyl, 7-Butyltetracosanyl, 7-Propyltetracosanyl, 7-Ethyltetracosanyl, 7-Methyltetracosanyl 7-Heptacosanyloctacosanyl, 7-Hexacosanyloctacosanyl, 7-Pentcosanyloctacosanyl, 7-Tetracosanyloctcosanyl, 7-Tricosanyloctacosanyl, 7-Docosanyloctacosanyl, 7-Nonadecyloctacosanyl, 7-Octadecyloctacosanyl, 7-Heptadecyloctacosanyl, 7-Hexadecyloctacosanyl, 7-Hexadecyloctacosanyl, 7-Pentadecyloctacosanyl, 7-Tetradecyloctacosanyl, 7-Tridecyloctacosanyl, 7-Dodecyloctacosanyl, 7-Undecyloctacosanyl, 7-Decyloctacosanyl, 7-Nonyloctacosanyl, 7-Octyloctacosanyl, 7-Heptyloctacosanyl, 7-Hexyloctacosanyl, 7-Pentyloctacosanyl, 7-Butyloctacosanyl, 7-Propyloctacosanyl, 7-Ethyloctacosanyl, 7-Methyloctacosanyl.

Die efindungsgemäßen Dibenzorylentetracarbonsäuredümide I können unsubstituiert sein oder an den in Formel I angegebene Positionen des Rylengerüsts durch Reste R' substituiert sein, die ausgewählt sind unter (Aryloxy-, Arylthio-, Hetaryloxy- oder Hetarylthio.

Die Dibenzopentarylentetracarbonsäurediimide la können dementsprechend bis zu 4 und die Dibenzoquaterrylentetracarbonsäurediimide Ib bis zu 2 Substituenten R' im Rylengerüst tragen.

Durch den Einsatz eines substituierten und eines unsubstituierten peri-(Dioxaborolan-2-yl)perylendicarbonsäureimids II bei der Suzuki-Kupplung können auch zweifach substituierte Dibenzopentarylentetracarbonsäurediimide gezielt hergestellt werden.

Dibenzopentarylentetracarbonsäurediimide la mit 2 unterschiedlichen Restepaaren R' sind analog durch den Einsatz zweier unterschiedlich substituierter peri-(Dioxaborolan-2-yl)perylendicarbonsäureimide II erhältlich.

Bei den bevorzugten (Het)Aryloxy- und -thioresten R' handelt es sich insbesondere um Phenoxy-, Thiophenoxy-, 2-Naphthoxy-, 2-Naphthylthio-, 2-, 3- und 4-Pyridyloxy-, 2-, 3-und 4-Pyridylthio-, 2-, 4- und 5-Pyrimidyloxy- und 2-, 4- und 5-Pyrimidylthioreste, wobei die Phenoxy- und Thiophenoxyreste besonders bevorzugt und die Phenoxyreste ganz besonders bevorzugt sind.

Die (Het)Aryloxy- und -thioreste R' können ihrerseits durch die in Anspruch 1, 2 und 3 genannten Reste substituiert sein.

Die besonders bevorzugten (Thio)Phenoxyreste R' können dabei einfach in ortho-, meta- oder vorzugsweise para-Position substituiert sein. Sie können auch zwei-, drei-, vier- oder fünffach substituiert sein, wobei alle denkbaren Substitutionsmuster möglich sind.

Besonders bevorzugte Reste R' sind (Thio)Phenoxyreste, die in para-Position durch einen tert.-Alkylrest, wie tert.-Butyl oder insbesondere einen tert.-Alkylrest der Formel 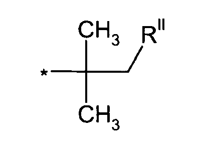 substituiert sind,
worin* für die Bindungsstelle zum Benzolring steht und
R^{II} folgende Bedeutung hat:
Wasserstoff;
C₁-C₈-Alkyl, dessen Kohlenstoffkette durch eine oder mehrere Gruppierungen -O-, -S-, -NR¹-, -CO- und/oder -SO₂- unterbrochen sein kann und das ein- oder mehrfach durch C₁-C₆-Alkoxy und/oder einen über eine Stickstoffatom gebundenen, 5- bis 7-gliedrigen heterocyclischen Rest, der weitere Heteroatome enthalten und aromatisch sein kann, substituiert sein kann;
C₅-C₈-Cycloalkyl, dessen Kohlenstoffgerüst durch eine oder mehrere Gruppierungen -O-, -S-, -NR¹-, -CO- und/oder -SO₂- unterbrochen sein kann und das ein- oder mehrfach durch C₁-C₆-Alkyl substituiert sein kann,
wobei R¹ für Wasserstoff oder C₁-C₆-Alkyl steht.

In einer bevorzugten Ausführung steht R" für Wasserstoff. In einer weiteren bevorzugten Ausführung steht R" für C₁,-C₆-Alkyl, das 1 oder 2 nicht benachbarte tertiäre oder quartäre Kohlenstoffatome aufweist. Besonders bevorzugte Reste R' sind dann Phenoxyreste und Thiophenoxyreste, die in para-Position durch 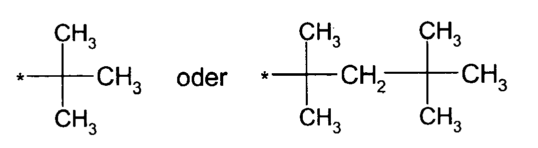 substituiert sind.

Beispiele für ganz besonders bevorzugte para-substituierte (Thio)Phenoxyreste R' sind im Einzelnen:
4-(1,1-Dimethylpropyl)phenoxy, 4-(1,1-Dimethylbutyl)phenoxy, 4-(1,1-Dimethylpentyl)phenoxy, 4-(1,1,3,3-Tetramethylbutyl)phenoxy, 4-(2-Cyclopentyl-1,1-dimethylethyl)phenoxy, 4-(2-Cyclohexyl-1,1-dimethylethyl)phenoxy, 4-(2-Cycloheptyl-1,1-dimethylethyl)phenoxy und 4-[1,1-Dimethyl-2-(4-morpholinyl)ethyl]phenoxy;
4-(1,1-Dimethylpropyl)thiophenoxy, 4-(1,1-Dimethylbutyl)thiophenoxy, 4-(1,1-Dimethylpentyl)thiophenoxy, 4-(1,1,3,3-Tetramethylbutyl)thiophenoxy, 4-(2-Cyclopentyl-1,1-dimethylethyl)thiophenoxy, 4-(2-Cyclohexyl-1,1-dimethylethyl)thiophenoxy, 4-(2-Cycloheptyl-1,1-dimethylethyl)thiophenoxy und 4-[1,1-Dimethyl-2-(4-morpholinyl)ethyl]thiophenoxy.

Weiterhin besonders bevorzugte Reste R' sind ortho,ortho'-disubstituierte (Thio)Phenoxyreste der Formel

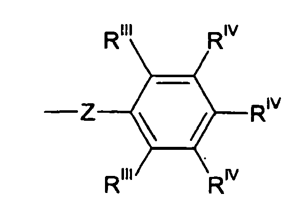

Die Reste R^{III} in den beiden ortho-Positionen können gleich oder verschieden sein, bevorzugt sind sie jedoch gleich.

Die (Thio)Phenoxyreste R' können auch in einer, zweien oder allen drei weiteren Ringpositionen durch gleiche oder ungleiche, von Wasserstoff verschiedene Reste R^{IV} substituiert sein.

Vorzugsweise sind die (Thio)Phenoxyreste R' nur in ortho- und ortho'-Position oder zusätzlich in para-Position substituiert.

Insbesondere haben die Variablen in der oben genannten Formel folgende Bedeutung:
- Z: -O- oder -S-, vorzugsweise -O-;
- R^{III}: gleiche oder verschiedene Reste:
(i) C₁-C₁₈-Alkyl, dessen Kohlenstoffkette durch eine oder mehrere Gruppierungen -O-, -S-, -NR¹- und/oder -CO- unterbrochen sein kann und das ein- oder mehrfach durch C₁-C₁₂-Alkoxy, Hydroxy und/oder Halogen substituiert sein kann, wobei höchstens einer der Reste R"' in 1-Position ein tertiäres Kohlenstoffatom aufweisen kann;
(ii) C₃-C₈-Cycloalkyl, das ein- oder mehrfach durch C₁-C₈-Alkyl und/oder C₁-C₁₂-Alkoxy substituiert sein kann, wobei höchstens einer der Reste R"' in 1-Position ein tertiäres Kohlenstoffatom aufweisen kann;
(iii) Aryl oder Hetaryl, das jeweils ein- oder mehrfach durch C₁-C₈-Alkyl, C₁-C₁₂-Alkoxy, Hydroxy und/oder Halogen substituiert sein kann;
(iv) ein Rest -U-Aryl, der ein- oder mehrfach durch die vorstehenden, als Substituenten für die Arylreste (iii) genannten Reste substituiert sein kann, wobei U eine Gruppierung -O-, -S- oder -NR¹- bedeutet;
(v) C₁-C₁₂-Alkoxy, Hydroxy, Halogen oder Cyano;
- R^{IV}: gleiche oder verschiedene Reste:
Wasserstoff;
einer der für R"' genannten Reste (i), (ii), (iii), (iv) und (v), vorzugsweise C₄-C₁₈-Alkylreste, die in der 1-Position ein tertiäres Kohlenstoffatom enthalten können;
- R¹: Wasserstoff oder C₁-C₆-Alkyl.

Als Rest R^{III} bevorzugt sind dabei Alkyl-, Cycloalkyl- und Phenylreste, vor allem Alkylreste mit sekundärem oder primärem Kohlenstoffatom in 1-Position sowie Methyl und Cycloalkylreste mit sekundärem Kohlenstoffatom in 1-Position, wobei die Alkyl- und Cycloalkylreste mit sekundärem Kohlenstoffatom in 1-Position besonders hervorzuheben sind.

Als Beispiele für diese ganz besonders bevorzugte (Thio)Phenoxyreste R' seien im Einzelnen genannt:
2,6-Dimethylphenoxy, 2,6-Diethylphenoxy, 2,6-Diisopropylphenoxy, 2,6-Di(2-butyl)phenoxy, 2,6-Di(n-butyl)phenoxy, 2,6-Di(2-hexyl)phenoxy, 2,6-Di(n-hexyl)phenoxy, 2,6-Di(2-dodecyl)phenoxy, 2,6-Di(n-dodecyl)phenoxy, 2,6-Dicyclohexylphenoxy, 2,6-Diphenylphenoxy, 2,6-Dimethyl-4-(n-butyl)phenoxy, 2,6-Diethyl-4-(n-butyl)phenoxy, 2,6-Diisopropyl-4-(n-butyl)phenoxy, 2,6-Di(2-butyl)-4-(n-butyl)phenoxy, 2,4,6-Tri(n-butyl)phenoxy, 2,6-Di(2-hexyl)-4-(n-butyl)phenoxy, 2,6-Di(n-hexyl)-4-(n-butyl)phenoxy, 2,6-Di(2-dodecyl)-4-(n-butyl)phenoxy, 2,6-Di(n-dodecyl}-4-(n-butyl)phenoxy, 2,6-Dicyclohexyl-4-(n-butyl)phenoxy, 2,6-Diphenyl-4-(n-butyl)phenoxy, 2,6-Dimethyl-4-(n-nonyl)phenoxy, 2,6-Diethyl-4-(n-nonyl)phenoxy, 2,6-DÜsopropyl-4-(n-nonyl)phenoxy, 2,6-Di(2-butyl)-4-(n-nonyl)phenoxy, 2,6-Di(2-butyl)-4-(n-nonyl)phenoxy, 2,6-Di(2-hexyl)-4-(n-nonyl)phenoxy, 2,6-Di(n-hexyl)-4-(n-nonyl)phenoxy, 2,6-Di(2-dodecyl)-4-(n-nonyl)-phenoxy, 2,6-Di(n-dodecyl)-4-(n-nonyl)phenoxy, 2,6-Dicyclohexyl-4-(n-nonyl)phenoxy, 2,6-Diphenyl-4-(n-nonyl)phenoxy, 2,6-Dimethyl-4-(n-octadecyl)phenoxy, 2,6-Diethyl-4-(n-octadecyl)phenoxy, 2,6-DÜsopropyl-4-(n-octadecyl)phenoxy, 2,6-Di(2-butyl)-4-(n-octadecyl)phenoxy, 2,6-Di(2-butyl)-4-(n-octadecyl)phenoxy, 2,6-Di(2-hexyl)-4-(n-octa-decyl)phenoxy, 2,6-Di(n-hexyl)-4-(n-octadecyl)phenoxy, 2,6-Di(2-dodecyl)-4-(n-octa-decyl)phenoxy, 2,6-Di(n-dodecyl)-4-(n-octa-decyl)phenoxy, 2,6-Dicyclohexyl-4-(n-octa-decyl)phenoxy,2,6-Dimethyl-4-(tert.-butyl)phenoxy, 2,6-Diethyl-4-(tert.-butyl)phenoxy, 2,6-Diisopropyl-4-(tert.-butyl)phenoxy, 2,6-Di(2-butyl)-4-(tert.-butyl)phenoxy, 2,6-Di-(n-butyl)-4-(tert.-butyl)phenoxy, 2,6-Di(2-hexyl)-4-(tert.-butyl)phenoxy, 2,6-Di(n-hexyl)-4-(tert.-butyl)phenoxy, 2,6-Di(2-dodecyl)-4-(tert.-butyl)phenoxy, 2,6-Di(n-dodecyl)-4-(tert.-butyl)phenoxy, 2,6-Dicyclohexyl-4-(tert.-butyl)phenoxy, 2,6-Diphenyl-4-(tert.-butyl)-phenoxy, 2,6-Dimethyl-4-(tert.-octyl)phenoxy, 2,6-Diethyl-4-(tert.-octyl)phenoxy, 2,6-Diisopropyl-4-(tert.-octyl)phenoxy, 2,6-Di(2-butyl)-4-(tert.-octyl)phenoxy, 2,6-Di-(n-butyl)-4-(tert.-octyl)phenoxy, 2,6-Di(2-hexyl)-4-(tert.-octyl)phenoxy, 2,6-Di(n-hexyl)-4-(tert.-octyl)phenoxy, 2,6-Di(2-dodecyl)-4-(tert.-octyl)phenoxy, 2,6-Di(n-dodecyl)-4-(tert.-octyl)phenoxy, 2,6-Dicyclohexyl-4-(tert.-octyl)phenoxy und 2,6-Diphenyl-4-(tert.-octyl)phenoxy;
2,6-Dimethylthiophenoxy, 2,6-Diethylthiophenoxy, 2,6-Diisopropylthiophenoxy, 2,6-Di(2-butyl)thiophenoxy, 2,6-Di(n-butyl)thiophenxoy, 2,6-Di(2-hexyl)thiophenoxy, 2,6-Di(n-hexyl)thiophenoxy, 2,6-Di(2-dodecyl)thiophenoxy, 2,6-Di(n-dodecyl)thiophenoxy, 2,6-Dicyclohexylthiophenoxy, 2,6-Diphenylthiophenoxy, 2,6-Dimethyl-4-(n-butyl)thio-phenoxy, 2,6-Diethyl-4-(n-butyl)thiophenoxy, 2,6-Diisopropyl-4-(n-butyl)thiophenoxy, 2,6-Di(2-butyl)-4-(n-butyl)-thiophenoxy, 2,4,6-Tri(n-butyl)thiophenoxy, 2,6-Di(2-hexyl)-4-(n-butyl)thiophenoxy, 2,6-Di(n-hexyl)-4-(n-butyl)thiophenoxy, 2,6-Di(2-dodecyl)-4-(n-butyl)thiophenoxy, 2,6-Di(n-dodecyl)-4-(n-butyl)thiophenoxy, 2,6-Dicyclohexyl-4-(n-butyl)thiophenoxy, 2,6-Diphenyl-4-(n-butyl)thiophenoxy, 2,6-Di-methyl-4-(n-nonyl)thio-phenoxy, 2,6-Diethyl-4-(n-nonyl)thiophenoxy, 2,6-Diisopropyl-4-(n-nonyl)thiophenoxy, 2,6-Di(2-butyl)-4-(n-nonyl)thiophenoxy, 2,6-Di(2-butyl)-4-(n-nonyl)thiophenoxy, 2,6-Di(2-hexyl)-4-(n-nonyl)thiophenoxy, 2,6-Di(n-hexyl)-4-(n-nonyl)thiophenoxy, 2,6-Di(2-dodecyl)-4-(n-nonyl)thiophenoxy, 2,6-Di(n-dodecyl)-4-(n-nonyl)thiophenoxy, 2,6-Dicyclohexyl-4-(n-nonyl)thiophenoxy, 2,6-Diphenyl-4-(n-nonyl)thiophenoxy, 2,6-(Dimethyl)-4-(n-octadecyl)thiophenoxy, 2,6-(Diethyl)-4-(n-octadecyl)thiophenoxy, 2,6-Diisopropyl-4-(n-octadecyl)thiophenoxy, 2,6-Di(2-butyl)-4-(n-octadecyl)thiophenoxy, 2,6-Di(2-butyl)-4-(n-octa-decyl)thiophenoxy, 2,6-Di(2-hexyl)-4-(n-octadecyl)thiophen-oxy, 2,6-Di(n-hexyl)-4-(n-octadecyl)thiophenoxy, 2,6-Di(2-dodecyl)-4-(n-octadecyl)thio-phenoxy, 2,6-Di(n-do-decyl)-4-(n-octadecyl)thiophenoxy, 2,6-Dicyclohexyl-4-(n-octa-decyl)thiophenoxy, 2,6-Dimethyl-4-(tert.-butyl)thiophenoxy, 2,6-Diethyl-4-(tert.-butyl)-thiophenoxy, 2,6-Diiso-propyl-4-(tert.-butyl)thiophenoxy, 2,6-Di(2-butyl)-4-(tert.-butyl)-thiophenoxy, 2,6-Di-(n-butyl)-4-(tert.-butyl)thiophenoxy, 2,6-Di(2-hexyl)-4-(tert.-butyl)-thiophenoxy, 2,6-Di(n-hexyl)-4-(tert.-butyl)thiophenoxy, 2,6-Di(2-dodecyl)-4-(tert.-butyl)-thiophenoxy, 2,6-Di(n-dodecyl)-4-(tert.-butyl)thiophenoxy, 2,6-Dicyclohexyl-4-(tert.-butyl)thiophenoxy, 2,6-Diphenyl-4-(tert.-butyl)thiophenoxy, 2,6-Dimethyl-4-(tert.-octyl)-thiophenoxy, 2,6-Diethyl-4-(tert.-octyl)thiophenoxy, 2,6-Diisopropyl-4-(tert.-octyl)thio-phenoxy, 2,6-Di(2-butyl)-4-(tert.-octyl)thiophenoxy, 2,6-Di-(n-butyl)-4-(tert.-octyl)thio-phenoxy, 2,6-Di(2-hexyl)-4-(tert.-octyl)thiophenoxy, 2,6-Di(n-hexyl)-4-(tert.-octyl)thio-phenoxy, 2,6-Di(2-dodecyl)-4-(tert.-octyl)thiophenoxy, 2,6-Di(n-dodecyl)-4-(tert.-octyl)-thiophenoxy, 2,6-Dicyclohexyl-4-(tert.-octyl)thiophenoxy und 2,6-Diphenyl-4-(tert.-octyl)thiophenoxy.

Als Beispiele für die in den Formeln genannten Reste R, R', R", R^{III}, R^{IV}, R¹ bis R⁴ sowie deren Substituenten seien im Einzelnen genannt:
Methyl, Ethyl, Propyl, Isopropyl, Butyl, Isobutyl, tert.-Butyl, Pentyl, Isopentyl, Neopentyl, tert.-Pentyl, Hexyl, 2-Methylpentyl, Heptyl, 1-Ethylpentyl, Octyl, 2-Ethylhexyl, Isooctyl, Nonyl, Isononyl, Decyl, Isodecyl, Undecyl, Dodecyl, Tridecyl, Isotridecyl, Tetradecyl, Pentadecyl, Hexadecyl, Heptadecyl, Octadecyl, Nonadecyl und Eicosyl (die obigen Bezeichnungen Isooctyl, Isononyl, Isodecyl und Isotridecyl sind Trivialbezeichnungen und stammen von den nach der Oxosynthese erhaltenen Alkoholen);
2-Methoxyethyl, 2-Ethoxyethyl, 2-Propoxyethyl, 2-Isopropoxyethyl, 2-Butoxyethyl, 2-und 3-Methoxypropyl, 2- und 3-Ethoxypropyl, 2- und 3-Propoxypropyl, 2- und 3-Butoxypropyl, 2- und 4-Methoxybutyl, 2- und 4-Ethoxybutyl, 2- und 4-Propoxybutyl, 3,6-Di-oxaheptyl, 3,6-Dioxaoctyl, 4,8-Dioxanonyl, 3,7-Dioxaoctyl, 3,7-Dioxanonyl, 4,7-Dioxa-octyl, 4,7-Dioxanonyl, 2- und 4-Butoxybutyl, 4,8-Dioxadecyl,
3,6,9-Trioxadecyl, 3,6,9-Trioxaundecyl, 3,6,9-Trioxadodecyl, 3,6,9,12-Tetraoxatridecyl und 3,6,9,12-Tetraoxatetradecyl;
2-Methylthioethyl, 2-Ethylthioethyl, 2-Propylthioethyl, 2-Isopropylthioethyl, 2-Butylthioethyl, 2- und 3-Methylthiopropyl, 2- und 3-Ethylthiopropyl, 2- und 3-Propylthiopropyl, 2-und 3-Butylthiopropyl, 2- und 4-Methylthiobutyl, 2- und 4-Ethylthiobutyl, 2- und 4-Pro-pylthiobutyl, 3,6-Dithiaheptyl, 3,6-Dithiaoctyl, 4,8-Dithianonyl, 3,7-Dithiaoctyl, 3,7-Di-thianonyl, 2- und 4-Butylthiobutyl, 4,8-Dithiadecyl, 3,6,9-Trithiadecyl, 3,6,9-Trithiaundecyl, 3,6,9-Trithiadodecyl, 3,6,9,12-Tetrathiatridecyl und 3,6,9,12-Tetrathiatetradecyl;
2-Monomethyl- und 2-Monoethylaminoethyl, 2-Dimethylaminoethyl, 2- und 3- Dimethylaminopropyl, 3-Monoisopropylaminopropyl, 2- und 4-Monopropylaminobutyl, 2- und 4-Dimethylaminobutyl, 6-Methyl-3,6-diazaheptyl, 3,6-Dimethyl-3,6-diazaheptyl, 3,6-Di-azaoctyl, 3,6-Dimethyl-3,6-diazaoctyl, 9-Methyl-3,6,9-triazadecyl, 3,6,9-Trimethyl-3,6,9-triazadecyl, 3,6,9-Triazaundecyl, 3,6,9-Trimethyl-3,6,9-triazaundecyl, 12-Methyl-3,6,9,12-tetraazatridecyl und 3,6,9,12-Tetramethyl-3,6,9,12-tetra-azatridecyl;
(1-Ethylethyliden)aminoethylen, (1-Ethylethyliden)aminopropylen, (1-Ethylethyliden)aminobutylen, (1-Ethylethyliden)aminodecylen und (1-Ethylethyliden)aminododecylen;
Propan-2-on-1-yl, Butan-3-on-1-yl, Butan-3-on-2-yl und 2-Ethylpentan-3-on-1-yl;
2-Methylsulfoxidoethyl, 2-Ethylsulfoxidoethyl, 2-Propylsulfoxidoethyl, 2-Isopropylsulfoxidoethyl, 2-Butylsulfoxidoethyl, 2- und 3-Methylsulfoxidopropyl, 2- und 3-Ethylsulfoxidopropyl, 2- und 3-Propylsulfoxidopropyl, 2- und 3-Butylsulfoxidopropyl, 2- und 4-Methylsulfoxidobutyl, 2- und 4-Ethylsulfoxidobutyl, 2- und 4-Propylsulfoxidobutyl und 4-Butylsulfoxidobutyl;
2-Methylsulfonylethyl, 2-Ethylsulfonylethyl, 2-Propylsulfonylethyl, 2-Isopropylsulfonylethyl, 2-Butylsulfonylethyl, 2- und 3-Methylsulfonylpropyl, 2- und 3-Ethylsulfonylpropyl, 2- und 3-Propylsulfonylpropyl, 2- und 3-Butylsulfonylproypl, 2- und 4-Methylsulfonylbutyl, 2- und 4-Ethylsulfonylbutyl, 2- und 4-Propylsulfonylbutyl und 4-Butylsulfonylbutyl;
Carboxymethyl, 2-Carboxyethyl, 3-Carboxypropyl, 4-Carboxybutyl, 5-Carboxypentyl, 6-Carboxyhexyl, 8-Carboxyoctyl, 10-Carboxydecyl, 12-Carboxydodecyl und 14-Carboxytetradecyl;
Sulfomethyl, 2-Sulfoethyl, 3-Sulfopropyl, 4-Sulfobutyl, 5-Sulfopentyl, 6-Sulfohexyl, 8-Sulfooctyl, 10-Sulfodecyl, 12-Sulfododecyl und 14-Sulfotetradecyl;
2-Hydroxyethyl, 2- und 3-Hydroxypropyl, 1-Hydroxyprop-2-yl, 3- und 4-Hydroxybutyl, 1-Hydroxybut-2-yl und 8-Hydroxy-4-oxaoctyl;
2-Cyanoethyl, 3-Cyanopropyl, 3- und 4-Cyanobutyl, 2-Methyl-3-ethyl-3-cyanopropyl, 7-Cyano-7-ethylheptyl und 4-Methyl-7-methyl-7-cyanoheptyl;
2-Chlorethyl, 2- und 3-Chlorpropyl, 2-, 3- und 4-Chlorbutyl, 2-Bromethyl, 2- und 3-Brompropyl und 2-, 3- und 4-Brombutyl;
2-Nitroethyl, 2- und 3-Nitropropyl und 2-, 3- und 4-Nitrobutyl;
Methoxy, Ethoxy, Propoxy, Isopropoxy, Butoxy, Isobutoxy, sec.-Butoxy, tert.-Butoxy, Pentoxy, Isopentoxy, Neopentoxy, tert.-Pentoxy und Hexoxy;
Methylthio, Ethylthio, Propylthio, Isopropylthio, Butylthio, Isobutylthio, sec.-Butylthio, tert.-Butylthio, Pentylthio, Isopentylthio, Neopentylthio, tert.-Pentylthio und Hexylthio;
Ethinyl, 1- und 2-Propinyl, 1-, 2- und 3-Butinyl, 1-, 2-, 3- und 4-Pentinyl, 1-, 2-, 3-, 4-und 5-Hexinyl, 1-, 2-, 3-, 4-, 5-, 6-, 7-, 8- und 9-Decinyl, 1-, 2-, 3-, 4-, 5-, 6-, 7-, 8-, 9-, 10- und 11-Dodecinyl und 1-, 2-, 3-, 4-, 5-, 6-, 7-, 8-, 9-, 10-, 11-, 12-, 13-, 14-, 15-, 16-und 17-Octadecinyl;
Ethenyl, 1- und 2-Propenyl, 1-, 2- und 3-Butenyl, 1-, 2-, 3- und 4-Pentenyl, 1-, 2-, 3-, 4-und 5-Hexenyl, 1-, 2-, 3-, 4-, 5-, 6-, 7-, 8- und 9-Decenyl, 1-, 2-, 3-, 4-, 5-, 6-, 7-, 8-, 9-, 10- und 11-Dodecenyl und 1-, 2-, 3-, 4-, 5-, 6-, 7-, 8-, 9-, 10-, 11-, 12-, 13-, 14-, 15-, 16-und 17-Octadecenyl;
Methylamino, Ethylamino, Propylamino, Isopryplamino, Butylamino, Isobutylamino, Pentylamino, Hexylamino, Dimethylamino, Methylethylamino, Diethylamino, Dipropylamino, Diisopropylamino, Dibutylamino, Diisobutylamino, Dipentylamino, Dihexylamino, Dicyclopentylamino, Dicyclohexylamino, Dicycloheptylamino, Diphenylamino und Dibenzylamino;
Formylamino, Acetylamino, Propionylamino und Benzoylamino;
Carbamoyl, Methylaminocarbonyl, Ethylaminocarbonyl, Propylaminocarbonyl, Butylaminocarbonyl, Pentylaminocarbonyl, Hexylaminocarbonyl, Heptylaminocarbonyl, Octylaminocarbonyl, Nonylaminocarbonyl, Decylaminocarbonyl und Phenylaminocarbonyl;
Aminosulfonyl, N,N-Dimethylaminosulfonyl, N,N-Diethylaminosulfonyl, N-Methyl-N-ethylaminosulfonyl, N-Methyl-N-dodecylaminosulfonyl, N-Dodecylaminosulfonyl, (N,N-Dimethylamino)ethylaminosulfonyl, N,N-(Propoxyethyl)dodecylaminosulfonyl, N,N-Diphenylaminosulfonyl, N,N-(4-tert.-Butylphenyl)octadecylaminosulfonyl und N,N-Bis(4-Chlorphenyl)aminosulfonyl;
Methoxycarbonyl, Ethoxycarbonyl, Propoxycarbonyl, Isopropoxycarbonyl, Hexoxycarbonyl, Dodecyloxycarbonyl, Octadecyloxycarbonyl, Phenoxycarbonyl, (4-tert.-Butylphenoxy)carbonyl und (4-Chlorphenoxy)carbonyl;
Methoxysulfonyl, Ethoxysulfonyl, Propoxysulfonyl, Isopropoxysulfonyl, Butoxysulfonyl, Isobutoxysulfonyl, tert.-Butoxysulfonyl, Hexoxysulfonyl, Dodecyloxysulfonyl, Octadecyloxysulfonyl, Phenoxysulfonyl, 1- und 2-Naphthyloxysulfonyl, (4-tert.-Butylphenoxy)-sulfonyl und (4-Chlorphenoxy)sulfonyl;
Chlor, Brom und Iod;
Phenylazo, 2-Napthylazo, 2-Pyridylazo und 2-Pyrimidylazo;
Cyclopropyl, Cyclobutyl, Cyclopentyl, 2- und 3-Methylcyclopentyl, 2- und 3-Ethylcyclopentyl, Cyclohexyl, 2-, 3- und 4-Methylcyclohexyl, 2-, 3- und 4-Ethylcyclohexyl, 3- und 4-Propylcyclohexyl, 3- und 4-Isopropylcyclohexyl, 3- und 4-Butylcyclohexyl, 3- und 4-sec.-Butylcyclohexyl, 3- und 4-tert.-Butylcyclohexyl, Cycloheptyl, 2-, 3- und 4-Methylcycloheptyl, 2-, 3- und 4-Ethylcycloheptyl, 3- und 4-Propylcycloheptyl, 3- und 4-Isopropylcycloheptyl, 3- und 4-Butylcycloheptyl, 3- und 4-sec.-Butylcycloheptyl, 3-und 4-tert.-Butylcycloheptyl, Cyclooctyl, 2-, 3-, 4- und 5-Methylcyclooctyl, 2-, 3-, 4- und 5-Ethylcyclooctyl und 3-, 4- und 5-Propylcyclooctyl; 3- und 4-Hydroxycyclohexyl, 3- und 4-Nitrocyclohexyl und 3- und 4-Chlorcyclohexyl;
1-, 2- und 3-Cyclopentenyl, 1-, 2-, 3- und 4-Cyclohexenyl, 1-, 2- und 3-Cycloheptenyl und 1-, 2-, 3- und 4-Cyclooctenyl;
2-Dioxanyl, 1-Morpholinyl, 1-Thiomorpholinyl, 2- und 3-Tetrahydrofuryl, 1-, 2- und 3-Pyrrolidinyl, 1-Piperazyl, 1-Diketopiperazyl und 1-, 2-, 3- und 4-Piperidyl;
Phenyl, 2-Naphthyl, 2- und 3-Pyrryl, 2-, 3- und 4-Pyridyl, 2-, 4- und 5-Pyrimidyl, 3-, 4-und 5-Pyrazolyl, 2-, 4- und 5-Imidazolyl, 2-, 4- und 5-Thiazolyl, 3-(1,2,4-Triazyl), 2-(1,3,5-Triazyl), 6-Chinaldyl, 3-, 5-, 6- und 8-Chinolinyl, 2-Benzoxazolyl, 2-Benzothiazolyl, 5-Benzothiadiazolyl, 2- und 5-Benzimidazolyl und 1- und 5-Isochinolyl;
1-, 2-, 3-, 4-, 5-, 6- und 7-Indolyl, 1-, 2-, 3-, 4-, 5-, 6- und 7-Isoindolyl, 5-(4-Methylisoindolyl), 5-(4-Phenylisoindolyl), 1-, 2-, 4-, 6-, 7- und 8-(1,2,3,4-Tetrahydroisochinolinyl), 3-(5-Phenyl)-(1,2,3,4-tetrahydroisochinolinyl), 5-(3-Dodecyl-(1,2,3,4-tetrahydroisochinolinyl), 1-, 2-, 3-, 4-, 5-, 6-, 7- und 8-(1,2,3,4-Tetrahydrochinolinyl) und 2-, 3-, 4-, 5-, 6-, 7- und 8-Chromanyl, 2-, 4- und 7-Chinolinyl, 2-(4-Phenylchinolinyl) und 2-(5-Ethylchinolinyl);
2-, 3- und 4-Methylphenyl, 2,4-, 3,5- und 2,6-Dimethylphenyl, 2,4,6-Trimethylphenyl, 2-, 3- und 4-Ethylphenyl, 2,4-, 3,5- und 2,6-Diethylphenyl, 2,4,6-Triethylphenyl, 2-, 3- und 4-Propylphenyl, 2,4-, 3,5- und 2,6-Dipropylphenyl, 2,4,6-Tripropylphenyl, 2-, 3- und 4-Isopropylphenyl, 2,4-, 3,5- und 2,6-Diisopropylphenyl, 2,4,6-Triisopropylphenyl, 2-, 3-und 4-Butylphenyl, 2,4-, 3,5- und 2,6-Dibutylphenyl, 2,4,6-Tributylphenyl, 2-, 3- und 4-Isobutylphenyl, 2,4-, 3,5- und 2,6-Diisobutylphenyl, 2,4,6-Triisobutylphenyl, 2-, 3- und 4-sec.-Butylphenyl, 2,4-, 3,5- und 2,6-Di-sec.-butylphenyl und 2,4,6-Tri-sec.-butylphenyl; 2-, 3- und 4-Methoxyphenyl, 2,4-, 3,5- und 2,6-Dimethoxyphenyl, 2,4,6-Trimethoxyphenyl, 2-, 3- und 4-Ethoxyphenyl, 2,4-, 3,5- und 2,6-Diethoxyphenyl, 2,4,6-Triethoxyphenyl, 2-, 3- und 4-Propoxyphenyl, 2,4-, 3,5- und 2,6-Dipropoxyphenyl, 2-, 3- und 4-Isopropoxyphenyl, 2,4- und 2,6-Diisopropoxyphenyl und 2-, 3- und 4-Butoxyphenyl; 2-, 3- und 4-Chlorphenyl und 2,4-, 3,5- und 2,6-Dichlorphenyl; 2-, 3-und 4-Hydroxyphenyl und 2,4-, 3,5- und 2,6-Dihydroxyphenyl; 2-, 3- und 4-Cyanophenyl; 3- und 4-Carboxyphenyl; 3- und 4-Carboxamidophenyl, 3- und 4-N-Methylcarboxamido-phenyl und 3- und 4-N-Ethylcarboxamidophenyl; 3- und 4-Acetylaminophenyl, 3- und 4-Propionylaminophenyl und 3- und 4-Buturylaminophenyl; 3- und 4-N-Phenylamino-phenyl, 3- und
   4-N-(o-Tolyl)aminophenyl, 3- und 4-N-(m-Tolyl)aminophenyl und 3- und 4-(p-Tolyl)aminophenyl; 3- und 4-(2-Pyridyl)aminophenyl, 3- und 4-(3-Pyridyl)aminophenyl, 3- und 4-(4-Pyridyl)aminophenyl, 3- und 4-(2-Pyrimidyl)aminophenyl und 4-(4-Pyrimidyl)aminophenyl;
4-Phenylazophenyl, 4-(1-Naphthylazo)phenyl, 4-(2-Naphthylazo)phenyl, 4-(4-Naphthylazo)phenyl,4-(2-Pyriylazo)phenyl, 4-(3-Pyridylazo)phenyl, 4-(4-Pyridylazo)phenyl, 4-(2-Pyrimidylazo)phenyl, 4-(4-Pyrimidylazo)phenyl und 4-(5-Pyrimidylazo)phenyl;
Phenoxy, Phenylthio, 2-Naphthoxy, 2-Naphthylthio, 2-, 3- und 4-Pyridyloxy, 2-, 3- und 4-Pyridylthio, 2-, 4- und 5-Pyrimidyloxy und 2-, 4- und 5-Pyrimidylthio.

Die Dibenzorylentetracarbonsäurediimide I können vorteilhaft nach den erfindungsgemäßen Verfahren durch Suzuki-Kupplung der entsprechenden peri-(Dioxaborolan-2-yl)rylendicarbonsäureimide II 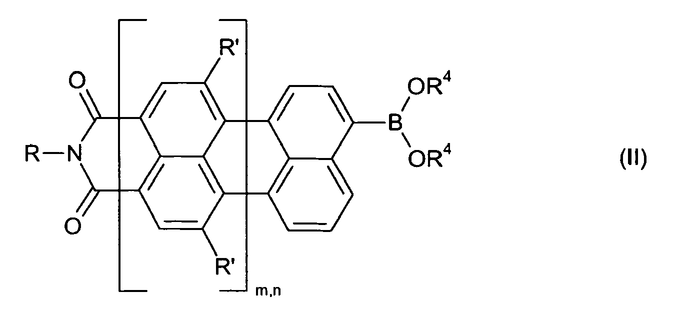 mit 5,11-Dibromtetracen (III) (Schritt a)), erste Cyclodehydrierung des gebildeten Tetracen-5,11-bis(rylendicarbonsäureimids) IV (Schritt b)) und weitere Cyclodehydrierung des gebildeten Bisrylenderivat V (Schritt c)) hergestellt werden.

Die Herstellung der Dibenzorylentetracarbonsäurediimide Ib mit unsymmetrisch aufgebautem Ringgerüst unterscheidet sich dabei lediglich dadurch von der Herstellung der symmetrischen Dibenzorylentetracarbonsäurediimide la, dass die Suzuki-Kupplung in Schritt a) zweistufig durch aufeinanderfolgende Umsetzung von 5,11-Dibromtetracen III all mit den beiden peri-(Dioxaborolan-2-yl)rylendicarbonsäureimiden IIb1 und IIb2 erfolgt.

Die zweistufige Verfahrensvariante in Schritt a) ist natürlich analog auch für Dibenzorylentetracarbonsäuredümide la mit symmetrisch aufgebautem Ringgerüst, die voneinander abweichende Reste R und/oder R' enthalten, anzuwenden.

Die Suzuki-Kupplung in Schritt a) wird in Gegenwart eines organischen Lösungsmittels, gewünschtenfalls im Gemisch im Wasser, sowie eines Übergangsmetallkatalysators und einer Base durchgeführt.

Die hierbei als Rylenedukt eingesetzten peri-(Dioxaborolan-2-yl)rylendicarbonsäureimide 11 sind wie 5,11-Dibromtetracen (III) bekannt und z. B. in der WO-A-05/70894 sowie der älteren deutschen Patentanmeldung 102005018241.0 beschrieben.

Das Molverhältnis von II zu III beträgt in der Regel 1,7 : 1 bis 2,3 : 1, bevorzugt 1,9 : 1 bis 2,1 : 1. Bei Einsatz zweier verschiedener Dioxaborolanylrylenderivate II liegt das Molverhältnis von II zu III jeweils üblicherweise bei 0,7 : 1 bis 1,3 : 1, vorzugsweise bei 0,9 : 1 bis 1,1 : 1.

Als Lösungsmittel eignen sich für Schritt a) alle Lösungsmittel, in denen sich die Dioxaborolanylrylenderivate II und Dibromtetracen III bei Reaktionstemperatur vollständig und die verwendeten Katalysatoren und Basen zumindest partiell lösen, so dass weitgehend homogene Reaktionsbedingungen vorliegen. Es können sowohl unpolar-aprotische als auch polar-aprotische Lösungsmittel eingesetzt werden, wobei die unpolar-aprotischen Lösungsmittel bevorzugt sind.

Beispiele für bevorzugte unpolar-aprotische Lösungsmittel sind bei > 100 °C siedende Lösungsmittel aus den folgenden Gruppen: Aliphaten (insbesondere C₈-C₁₈-Alkane), unsubstituierte, alkylsubstituierte und kondensierte Cycloaliphaten (insbesondere unsubstituierte C₇-C₁₀-Cycloalkane, C₆-C₈-Cycloalkane, die durch ein bis drei C₁-C₆-Alkylgruppen substituiert sind, polycyclische gesättigte Kohlenwasserstoffe mit 10 bis 18 C-Atomen), alkyl- und cycloalkylsubstituierte Aromaten (insbesondere Benzol, das durch ein bis drei C₁-C₆-Alkylgruppen oder einen C₅-C₈-Cycloalkylrest substituiert ist) und kondensierte Aromaten, die alkylsubstituiert und/oder teilhydriert sein können (insbesondere Naphthalin, das durch ein bis vier C₁-C₆-Alkylgruppen substituiert ist) sowie Mischungen dieser Lösungsmittel.

Als Beispiele für besonders bevorzugte Lösungsmittel seien im Einzelnen genannt: Octan, Isooctan, Nonan, Isononan, Decan, Isodecan, Undecan, Dodecan, Hexadecan und Octadecan; Cycloheptan, Cyclooctan, Methylcyclohexan, Dimethylcyclohexan, Trimethylcyclohexan, Ethylcyclohexan, Diethylcyclohexan, Propylcyclohexan, Isopropylcyclohexan, Dipropylcyclohexan, Butylcyclohexan, tert.-Butylcyclohexan, Methylcycloheptan und Methylcyclooctan; Toluol, o-, m- und p-Xylol, 1,3,5-Trimethylbenzol (Mesitylen), 1,2,4- und 1,2,3-Trimethylbenzol, Ethylbenzol, Propylbenzol, Isopropylbenzol, Butylbenzol, Isobutylbenzol, tert.-Butylbenzol und Cyclohexylbenzol; Naphthalin, Decahydronaphthalin (Dekalin), 1- und 2-Methylnaphthalin und 1- und 2-Ethylnaphthalin; Kombinationen aus den zuvor genannten Lösungsmitteln, wie sie aus den hochsiedenden, teil- oder durchhydrierten Fraktionen thermischer und katalytischer Crackprozesse bei der Rohöl- oder Naphthaverarbeitung gewonnen werden können, z. B. Gemische vom Exxsol^{®} Typ und Alkylbenzolgemische vom Solvesso^{®} Typ.

Ganz besonders bevorzugte Lösungsmittel sind Xylol (alle Isomeren), Mesitylen und vor allem Toluol.

Beispiele für geeignete polar-aprotische Lösungsmittel sind N,N-disubstituierte aliphatische Carbonsäureamide (insbesondere N,N-Di-Ci-C₁-alkyl-C₁-C₄-carbonsäureamide), stickstoffhaltige Heterocyclen und aprotische Ether (insbesondere cyclische Ether und Di-C₁-C₆-alkylether von monomeren und oligomeren C₂-C₃-Alkylenglykolen, die bis zu 6 Alkylenoxideinheiten enthalten können, vor allem Diethylenglykoldi-C₁-C₄-alkylether).

Als Beispiele für besonders geeignete Lösungsmittel aus dieser Gruppe seien im Einzelnen genannt: N,N-Dimethylformamid, N,N-Diethylformamid, N,N-Dimethylacetamid und N,N-Dimethylbutyramid; N-Methyl-2-pyrrolidon, Chinolin, Isochinolin, Chinaldin, Pyrimidin, N-Methylpiperidin und Pyridin; Tetrahydrofuran, Dioxan, Diethylenglykoldimethylether und Diethylenglykoldiethylether.

Die Lösungsmittelmenge liegt üblicherweise bei 10 bis 1000 ml, bevorzugt bei 50 bis 500 ml und besonders bevorzugt bei 75 bis 250 ml je g II.

Vorzugsweise setzt man Wasser, gegebenenfalls zusammen mit einem protischen organischen Lösungsmittel, insbesondere einem aliphatischen Alkohol, insbesondere einem C₁-C₅-Alkohol, wie Ethanol, als zusätzliches Lösungsmittel ein. In diesem Fall werden in der Regel 10 bis 1000 ml, insbesondere 15 bis 500 ml und vor allem 20 bis 250 ml Wasser und gegebenenfalls protisches organisches Lösungsmittel je I aprotisches (vorzugsweise unpolar-aprotisches) Lösungsmittel verwendet.

Als Übergangsmetallkatalysatoren eignen sich insbesondere Palladiumkomplexe, wie Tetrakis(triphenylphosphin)palladium(0), Tetrakis(tris-o-tolylphosphin)palladium(0), [1,2-Bis(diphenylphosphino)ethan]palladium(II)chlorid, [1,1'-Bis(diphenylphosphino)-ferrocen]palladium(II)chlorid, Bis(triethylphosphin)palladium(II)chlorid, Bis(tricyclohexylphosphin)palladium(II)acetat, (2,2'-Bipyridyl)palladium(II)chlorid, Bis(triphenylphosphin)palladium(II)chlorid, Tris(dibenzylidenaceton)dipalladium(0), 1,5-Cyclooctadienpalladium(II)chlorid, Bis(acetonitril)palladium(II)chlorid und Bis(benzonitril)palladium(II)chlorid, wobei Tris(dibenzylidenaceton)dipalladium(0), [1,1'-Bis(diphenylphosphino)ferrocen]palladium(II)chlorid und Tetrakis(triphenylphosphin)palladium(0) bevorzugt sind.

Üblicherweise wird der Übergangsmetallkatalysator in einer Menge von 1 bis 20 mol-%, vor allem 1,5 bis 5 mol-%, bezogen auf II, eingesetzt.

Die gleichzeitige Anwesenheit freier Ligandmoleküle, z. B. von Tris(tert.-butyl)-phosphin, Triphenylphosphin, Tris(o-tolyl)phosphin, 1,1'-(2,2'-Diphenylphosphino)-binaphthalin
oder insbesondere Bis(2-diphenylphosphino)phenylether, kann in manchen Fällen von Vorteil sein. Geeignete Mengen liegen bei 80 bis 500 mol-%, bevorzugt 100 bis
300 mol-%, bezogen auf den Übergangsmetallkatalysator.

Als Base kommen vorzugsweise die Alkalimetallsalze, vorzugsweise die Natrium- und insbesondere die Kaliumsalze, schwacher organischer und vor allem schwacher anorganischer Säuren, wie Formiate, Acetate, Proprionate, Hydrogencarbonate und bevorzugt Carbonate, wie Natriumacetat, Kaliumacetat, Natriumhydrogencarbonat, Natriumcarbonat, Kaliumhydrogencarbonat und Kaliumcarbonat, zum Einsatz. Besonders bevorzugte Basen sind die Carbonate, wobei Natriumcarbonat und vor allem Kaliumcarbonat ganz besonders bevorzugt sind.

In der Regel liegt die Basenmenge bei 1 bis 100 mol, insbesondere 5 bis 50 mol und vor allem 10 bis 30 mol je mol II.

Wenn eine zweistufige Kupplung mit zwei verschiedenen Dioxaborolanylrylenderivaten II durchgeführt wird, empfiehlt es sich, das Produkt des ersten Kupplungsschrittes zusammen mit dem Übergangsmetallkatalysator zu isolieren und die Base stufenweise zuzugeben.

Die Reaktionstemperatur beträgt im Allgemeinen 20 bis 180 °C, bevorzugt 40 bis 150 °C und besonders bevorzugt 60 bis 120 °C. Wird in Schritt a) Wasser eingesetzt, so empfiehlt es sich, die Umsetzung nicht bei Temperaturen über 100 °C vorzunehmen, da ansonsten unter Druck gearbeitet werden müsste.

Es empfiehlt sich unter Schutzgas zu arbeiten.

Die Reaktion ist üblicherweise in 0,5 bis 48 h, insbesondere in 3 bis 24 h und vor allem in 5 bis 20 h beendet.

Verfahrenstechnisch geht man in Schritt a) zweckmäßigerweise wie folgt vor:
Man legt Dioxaborolanylrylenderivat II und Dibromtetracen III sowie Lösungsmittel vor, gibt Übergangsmetallkatalysator und die vorzugsweise in Wasser gelöste Base zu und erhitzt die Mischung 0,5 bis 48 h unter Schutzgas auf die gewünschte Reaktionstemperatur. Nach Abkühlen auf Raumtemperatur trennt man die organische Phase aus dem Reaktionsgemisch ab und destilliert das Lösungsmittel unter vermindertem Druck ab.
Die Reinheit des so hergestellten Tetracen-5,11-bis(rylendicarbonsäureimids) IV reicht im Allgemeinen für die Weiterverarbeitung aus. Gegebenenfalls kann das Rohprodukt durch Waschen mit Wasser und gewünschtenfalls einem geeigneten organischen Lösungsmittel, insbesondere einem chlorierten aliphatischen oder aromatischen Kohlenwasserstoff, oder durch Säulenchromatographie an Kieselgel mit Dichlormethan als Eluens weiter aufgereinigt werden.

Schritt b) der erfindungsgemäßen Herstellungsverfahren, die Cyclodehydrierung des Tetracen-5,1 1-bis(rylendicarbonsäureimids) IV zum Bisrylendrivat V, wird unter wasserfreien Bedingungen in Gegenwart eines inerten organischen Lösungsmittels und einer Lewis-Säure vorgenommen.

Als Lösungsmittel eignen sich dabei grundsätzlich alle unter den Reaktionsbedingungen inerten organischen Lösungsmittel, wobei polar-aprotische Lösungsmittel besonders geeignet sind.

Bevorzugte Lösungsmittel sind halogenierte und nitrierte aliphatische oder aromatische Kohlenwasserstoffe. Neben den aromatischen Kohlenwasserstoffen, wie Chlorbenzol, Di- und Trichlorbenzol und Nitrobenzol, sind insbesondere die entsprechenden aliphatischen Kohlenwasserstoffe, vor allem die Methane und Ethane, hervorzuheben. Als Beispiele für die besonders bevorzugten Lösungsmittel seien im Einzelnen genannt: Dichlor-, Trichlor-, Tribrom-, Tetrachlor- und Tetrabrommethan, Nitromethan, 1,2-Dichlor-, 1,1-Dibrom- und 1,2-Dibromethan, 1,1,1-Trichlor-, 1,1,2-Trichlor-, 1,1,1-Tribrom-und 1,1,2-Tribromethan und 1,1,1,2-Tetrachlor-, 1,1,2,2-Tetrachlor-, 1,1,1,2-Tetrabrom- und 1,1,2,2-Tetrabromethan, wobei Dichlormethan (Methylenchlorid), Trichlormethan (Chloroform) und Nitromethan ganz besonders bevorzugt sind.

Die Lösungsmittelmenge beträgt in der Regel 10 bis 100 ml, insbesondere 20 bis 40 ml je g IV.

Als Lewis-Säuren sind vor allem Eisen(III)halogenide, wie Eisen(III)chlorid und Eisen(III)bromid, und Aluminiumtrihalogenide, wie Aluminiumtrichlorid, geeignet.

Üblicherweise werden 2 bis 10 mol, vorzugsweise 6 bis 8 mol, Lewis-Säure je mol IV eingesetzt.

Die Cyclodehydrierung in Schritt b) wird in der Regel bei Temperaturen um Raumtemperatur durchgeführt, d. h., ein Erhitzen des Reaktionsgemischs ist im Allgemeinen nicht erforderlich.

Es ist zweckmäßig, die Reaktion unter Lichtausschluss durchzuführen.

Die Reaktionszeiten liegen üblicherweise bei etwa 0,5 bis 5 h, insbesondere bei 1 bis 2 h.

Verfahrenstechnisch geht man in Schritt b) zweckmäßigerweise wie folgt vor:
Man legt Tetracenderivat IV und Lösungsmittel vor, gibt die vorzugsweise in weiterem Lösungsmittel gelöste Lewis-Säure zu und rührt die Mischung unter Lichtausschluss 1 bis 10 h bei Raumtemperatur. Anschließend gibt man das Reaktionsgemisch in einen niederen aliphatischen Alkohol, wie Methanol, und isoliert das ausgefällte Produkt durch Abfiltrieren und Trocknen.

Die Reinheit des so hergestellten Bisrylenderivats V reicht in der Regel für den zweiten Cyclodehydrierungsschritt c) aus. Gegebenenfalls kann man das Rohprodukt durch Säulenchromatographie an Kieselgel mit Dichlormethan als Eluens weiter aufreinigen.

Die zweite Cyclodehydrierung zum Dibenzorylentetracarbonsäuredümid I wird in einem Hydroxy- und Aminofunktionen aufweisenden und eine im Wesentlichen ungelöste Base enthaltenden organischen Reaktionsmedium durchgeführt.

Als organisches Reaktionsmedium sind dabei vor allem Aminoalkohole geeignet, die 2 bis 20, vorzugsweise 2 bis 10 C-Atome aufweisen. Die Kohlenstoffkette dieser Alkohole kann durch Sauerstoffatome in Etherfunktion unterbrochen sein. Beispiele für besonders geeignete Lösungsmittel sind Ethanolamin, Triethanolamin und Diethanolamin, wobei Ethanolamin bevorzugt ist. Es ist auch möglich, Mischungen von Alkoholen und Aminen zu verwenden, die jeweils einen Siedepunkt von mindestens 70 °C haben und bei der Reaktionstemperatur flüssig sind.

Üblicherweise werden 1,5 bis 150 ml, bevorzugt 5 bis 50 ml Reaktionsmedium je g Bisrylenderivat V eingesetzt.

Um das Bisrylenderivat V in Lösung zu überführen, kann es zweckmäßig sein, geringe Mengen eines polar-aprotischen Lösungsmittels, insbesondere eines N,N-disubstituierten aliphatischen Carbonsäureamids, wie Dimethylformamid, zuzusetzen.

Geeignete Mengen an diesem zusätzlichen Lösungsmittel liegen zweckmäßigerweise bei etwa 1 bis 10 ml, insbesondere 3 bis 5 ml Lösungsmittel je g Bisrylenderivat V.

Als im Reaktionsmedium im Wesentlichen ungelöste Basen eignen sich die in Schritt a) eingesetzten Alkalimetallbasen, wobei auch hier die Alkalimetallcarbonate, insbesondere Natriumcarbonat und vor allem Kaliumcarbonat, bevorzugt sind.

In der Regel beträgt die Basenmenge 1 bis 10 mol, bevorzugt 2 bis 5 mol je mol Bisrylenderivat V.

Die Reaktionstemperatur liegt im Allgemeinen bei 40 bis 200 °C, insbesondere bei 80 bis 160 °C.

Die Reaktion ist üblicherweise in 0,5 bis 24 h, vorzugsweise in 1 bis 12 h beendet.

Verfahrenstechnisch geht man in Schritt c) zweckmäßigerweise wie folgt vor:
Man rührt eine Mischung von Bisrylenderivat V, Lösungsmittel und Base 0,5 bis 24 h bei der gewünschten Reaktionstemperatur und fällt das Produkt nach Abkühlen auf Raumtemperatur durch Zugabe von Wasser oder eines Alkohols, wie Ethanol, aus dem Reaktionsgemisch aus, filtriert ab und wäscht mit Wasser, Alkoholen, wie Methanol, und/oder Dichlormethan.

Die Reinigung der erhaltenen Dibenzorylentetracarbonsäurediimide I kann wie folgt vorgenommen werden:
Katalysatorrückstände können durch eine schnelle Filtration über Kieselgel unter Waschen mit einem halogenierten aliphatischen Kohlenwasserstoff, wie Dichlormethan, entfernt werden. Rückstände nichtumgesetzter Edukte auf Perylen- oder Naphthalinbasis können durch Säulenchromatographie an Kieselgel mit Dichlormethan oder Mischungen von Dichlormethan und unpolaren Lösungsmitteln, wie Petrolether, als Eluens oder durch Extraktion mit Chlorbenzol entfernt werden.

Die erfindungsgemäßen Dibenzorylentetracarbonsäurediimide I zeigen starke Absorption im nahen Infrarotbereich bei Wellenlängen von 800 bis 1100 nm und ergänzen damit den mit Hilfe der bisher bekannten Rylenverbindungen zugänglichen Spektralbereich auf vorteilhafte Weise. Das Absorptionsmaximum der verschiedenen Dibenzorylentetracarbonsäurediimide I liegt etwa in folgenden Bereichen: Dibenzopentarylenderivate la: 1000 bis 1100 nm; Dibenzoquaterrylenderivate Ib: 900 bis 950 nm; Dibenzoterrylenderivate la: 800 bis 850 nm. Die Dibenzorylentetracarbonsäurediimide I können daher besonders vorteilhaft in Kombination mit den bei 940 bis 980 bzw. 1064 nm emittierenden kommerziellen Lasern zum Schweißbehandeln von Kunststoffteilen eingesetzt werden.

Die erfindungsgemäßen Dibenzorylentetracarbonsäurediimide I eignen sich für eine Reihe weiterer Anwendungen, wie die generelle Einfärbung von organischen und anorganischen Materialien, z. B. von Lacken, Druckfarben und Kunststoffen, zur Herstellung im nahinfraroten Bereich des elektromagnetischen Spektrums absorbierender wässriger Polymerisatdispersionen, zur Erzeugung für das menschliche Auge nicht sichtbarer, Infrarotlicht absorbierender Markierungen und Beschriftungen, als Infrarotabsorber für das Wärmemanagement und als Aktivkomponenten in der Photovoltaik.

Die Verbindungen der Formel (I) eignen sich besonders vorteilhaft für einen Einsatz in der organischen Photovoltaik (OPV). Prinzipiell eignen sich diese Verbindungen dabei für einen Einsatz in Farbstoff-sensibilisierten Solarzellen. Bevorzugt ist jedoch ihr Einsatz in Solarzellen, die durch eine Diffusion von angeregten Zuständen (Excitonendiffusion) gekennzeichnet sind. Dabei zeichnet sich eines oder beide der eingesetzten Halbleitermaterialien durch eine Diffusion von angeregten Zuständen aus. Geeignet ist auch die Kombination wenigstens eines Halbleitermaterials, das durch eine Diffusion von angeregten Zuständen gekennzeichnet ist, mit Polymeren, die eine Leitung der angeregten Zustände entlang der Polymerkette zulassen. Derartige Solarzellen werden im Sinne der Erfindung als excitonische Solarzellen bezeichnet. Die Direktumwandlung von Solarenergie in elektrische Energie in Solarzellen beruht auf dem inneren Photoeffekt eines Halbleitermaterials, d. h. der Erzeugung von Elektron-Loch-Paaren durch Absorption von Photonen und der Trennung der negativen und positiven Ladungsträger an einem p-n-Übergang oder einem Schottky-Kontakt. Ein Exciton kann z. B. entstehen, wenn ein Photon in einen Halbleiter eindringt und ein Elektron zum Übergang aus dem Valenzband in das Leitungsband anregt. Um Strom zu erzeugen, muss der durch die absorbierten Photonen erzeugte angeregte Zustand jedoch einen p-n-Übergang erreichen, um ein Loch und ein Elektron zu erzeugen, welches dann zur Anode und Kathode fließt. Die so erzeugte Photospannung kann in einem äußeren Stromkreis einen Photostrom bewirken, durch den die Solarzelle ihre Leistung abgibt. Von dem Halbleiter können dabei nur solche Photonen absorbiert werden, die eine Energie aufweisen, die größer als seine Bandlücke ist. Die Größe der Halbleiterbandlücke bestimmt also den Anteil des Sonnenlichts, der in elektrische Energie umgewandelt werden kann. Die beschriebenen excitonischen Solarzellen bestehen normalerweise aus zwei absorbierenden Materialien mit unterschiedlichen Bandlücken, um die Sonnenenergie möglichst effektiv zu nutzen. Die meisten organischen Halbleiter haben Excitonen-Diffusionslängen von bis zu 10 nm. Hier besteht weiterhin ein Bedarf an organischen Halbleitern, über die der angeregte Zustand über möglichst große Distanzen weitergeleitet werden kann. Überraschenderweise wurde nun gefunden, dass sich die zuvor beschriebenen Verbindungen der allgemeinen Formel I besonders vorteilhaft für einen Einsatz in excitonischen Solarzellen eignen.

Geeignete organische Solarzellen sind in der Regel schichtförmig aufgebaut und umfassen in der Regel zumindest die folgenden Schichten: Anode, photoaktive Schicht und Kathode. Diese Schichten befinden sich in der Regel auf einem dafür üblichen Substrat. Der Aufbau organischer Solarzellen ist z. B. in US 2005/0098726 A1 und US 2005/0224905 A1 beschrieben, worauf hier in vollem Umfang Bezug genommen wird.

Geeignete Substrate sind z. B. oxidische Materialien (wie Glas, Quarz, Keramik, SiO₂, etc.), Polymere (z. B. Polyvinylchlorid, Polyolefine, wie Polyethylen und Polypropylen, Polyester, Fluorpolymere, Polyamide, Polyurethane, Polyalkyl(meth)acrylate, Polystyrol und Mischungen und Komposite davon) und Kombinationen davon.

Als Elektroden (Kathode, Anode) eignen sich prinzipiell Metalle (vorzugsweise der Gruppen 8, 9, 10 oder 11 des Periodensystems, z. B. Pt, Au, Ag, Cu, Al, In, Mg, Ca), Halbleiter (z. B. dotiertes Si, dotiertes Ge, Indium-Zinn-Oxid (ITO), Gallium-Indium-Zinn-Oxid (GITO), Zink-Indium-Zinn-Oxid (ZITO), etc.), Metalllegierungen (z. B. auf Basis Pt, Au, Ag, Cu, etc., speziell Mg/Ag-Legierungen), Halbleiterlegierungen, etc. Bevorzugt wird als Anode ein gegenüber einfallendem Licht im Wesentlichen transparentes Material eingesetzt. Dazu zählt z. B. ITO, dotiertes ITO, ZnO, TiO₂ Ag, Au, Pt. Bevorzugt wird als Kathode ein das einfallende Licht im Wesentlichen reflektierendes Material eingesetzt. Dazu zählen z. B. Metallfilme, z. B. aus Al, Ag, Au, In, Mg, Mg/A1, Ca, etc.

Die photoaktive Schicht ihrerseits umfasst wenigstens eine oder besteht aus wenigstens einer Schicht, die als organisches Halbleitermaterial wenigsten eine Verbindung enthält, die ausgewählt ist unter Verbindungen der Formeln I und II, wie zuvor definiert. In einer Ausführung umfasst die photoaktive Schicht wenigstens ein organisches Akzeptormaterial. Zusätzlich zu der photoaktiven Schicht kann es eine oder mehrere weitere Schichten geben, z. B. eine Schicht mit elektronenleitenden Eigenschaften (ETL, electron transport layer) und eine Schicht, die ein löcherleitendes Material (hole transport layer, HTL) enthält, die nicht absorbieren müssen, Excitonen und Löcher blockierende Schichten (z. B. excition blocking layers, EBL), die nicht absorbieren sollen, Multiplikatorschichten (multiplication layers). Geeignete Excitonen und Löcher blockierende Schichten sind z. B. in US 6,451,415 beschrieben.

Geeignete Excitonenblockerschichten sind z. B. Bathocuproine (BCP), 4,4',4"-Tris(N-(3-methylphenyl)-N-phenylamino)triphenylamin (m-MTDATA) oder Polyethylendioxithiophen (PEDOT), wie in US 7,026,041 beschrieben.

Die erfindungsgemäßen excitonischen Solarzellen basieren auf photoaktiven Donor-Akzeptor-Heteroübergängen. Wird wenigstens eine Verbindung der Formel (I) als HTM eingesetzt, muss das entsprechende ETM so gewählt werden, dass nach Anregung der Verbindungen ein schneller Elektronenübergang auf das ETM stattfindet. Geeignete ETM sind z. B. C60 und andere Fullerene, Perylen-3,4:9,10-bis(dicarboximide) PTCDI, etc. Wird wenigstens eine Verbindung der Formel (I) als ETM eingesetzt, muss das komplementäre HTM so gewählt werden, dass nach Anregung der Verbindung ein schneller Löcherübertrag auf das HTM stattfindet. Der Heteroübergang kann flach ausgeführt werden (vgl. Two layer organic photovoltaic cell, C. W. Tang, Appl. Phys. Lett., 48 (2), 183-185 (1986) oder N. Karl, A. Bauer, J. Holzäpfel, J. Marktanner, M. Möbus, F. Stölzle, Mol. Cryst. Liq. Cryst., 252, 243-258 (1994).) oder als Volumen-Heteroübergang (bulk heterojunction bzw. interpenetrierenes Donor-akzeptor-Netzwerk, vgl. z. B. C. J. Brabec, N. S. Sariciftci, J. C. Hummelen, Adv. Funct. Mater., 11 (1), 15 (2001).) realisiert werden. Die photoaktive Schicht auf Basis eines Heteroübergangs zwischen wenigstens einer Verbindung der Formel (I) und einem HTL oder ETL kann in Solarzellen mit MiM-, pin-, pn-, Mip- oder Min-Aufbau zum Einsatz kommen (M=Metall, p=p-dotierter organischer oder anorganischer Halbleiter, n=n-dotierter organischer oder anorganischer Halbleiter, i=intrinsisch leitfähiges System organischer Schichten, vgl. z. B. J. Drechsel et al., Org. Eletron., 5 (4), 175 (2004) oder Maennig et al., Appl. Phys. A 79, 1-14 (2004)). Sie kann auch in Tandemzellen, wie von P. Peumnas, A. Yakimov, S. R. Forrest in J. Appl. Phys, 93 (7), 3693-3723 (2003) (vgl. Patente US 04461922, US 06198091 und US 06198092) beschrieben, verwendet werden. Sie kann auch in Tandemzellen aus zwei oder mehreren aufeinandergestapelten MiM-, pin-, Mip- oder Min-Dioden (vgl. Patentanmeldung DE 103 13 232.5) verwendet werden (J. Drechsel et al., Thin Solid Films, 451452, 515-517 (2004)).

Dünne Schichten der Verbindungen und aller anderer Schichten können durch Aufdampfen im Vakuum oder in Inertgasatmosphäre, durch Laserablation oder durch lösungs- oder dispersionsprozessierbare Verfahren wie Spin-Coating, Rakeln, Gießverfahren, Aufsprühen, Tauchbeschichtung oder Drucken (z. B. Inkjet, Flexo, Offset, gravure; Tiefdruck, Nanoimprint) hergestellt werden. Die Schichtdicken der M-, n-, i- und p-Schichten betragen typischerweise 10 bis 1000 nm, bevorzugt 10 bis 400 nm.

Als Substrat werden z. B. Glas, Metall- oder Polymerfolien verwendet, die in der Regel mit einer transparenten, leitfähigen Schicht (wie z. B. SnO₂:F, SnO₂:In, ZnO:Al, Carbon-Nanotubes, dünne Metallschichten) beschichtet sind.

Neben den Verbindungen der allgemeinen Formel (I) eignen sich die folgenden Halbleitermaterialien für einen Einsatz in der organischen Photovoltaik:

Acene, wie Anthracen, Tetracen, Pentace, die jeweils unsubstituiert oder substituiert sein können. Substituierte Acene umfassen vorzugsweise wenigstens einen Substituenten, der ausgewählt ist unter elektronenschiebenden Substituenten (z. B. Alkyl, Alkoxy, Ester, Carboxylat oder Thioalkoxy), elektronenziehenden Substituenten (z. B. Halogen, Nitro oder Cyano) und Kombinationen davon. Dazu zählen 2,9-Dialkylpentacene und 2,10-Dialkylpentacene, 2,10-Dialkoxypentacene, 1,4,8,11-Tetraalkoxypentacene und Rubren (5,6,11,12-Tetraphenylnaphthacen). Geeignete substituierte Pentacene sind in US 2003/0100779 und US 6,864,396 beschrieben, worauf hier Bezug genommen wird. Ein bevorzugtes Acen ist Rubren (5,6,11,12-Tetraphenylnaphthacen);

Phthalocyanine, beispielsweise Phthalocyanine, die wenigstens einen Halogensubstituenten tragen, wie Hexadecachlorophthalocyanine und Hexadecafluorophthalocyanine, metallfreie oder zweiwertige Metalle oder Metallatom-haltige Gruppen enthaltende Phthalocyanine, insbesondere die des Titanyloxy, Vanadyloxy, Eisens, Kupfers, Zinks, etc. Geeignete Phthalocyanine sind insbesondere Kupferphthalocyanin, Zinkphthalocyanin, metallfreies Phthalocyanin, Hexadecachlorokupferphthalocyanin, Hexadecachlorozinkphthalocyanin, metallfreies Hexadecachlorophathlocyanin, Hexadecafluorokupferphthalocyanin, Hexadecafluorophthalocyanin oder metallfreies Hexadefluorophthalocyanin;

Porphyrine, wie z. B. 5,10,15,20-Tetra(3-pyridyl)porphyrin (TpyP);

Flüssigkristalline (LC-) Materialien, beispielsweise Coronene, wie Hexabenzocoronen (HBC-PhC12), Coronendiimide, oder Triphenylene, wie 2,3,6,7,10,11-Hexahexylthiotriphenylen (HTT6), 2,3,6,7,10,11-Hexakis-(4-n-nonylphenyl)-triphenylen (PTP9) oder 2,3,6,7,10,11-Hexakis-(undecyloxy)-triphenyten (HAT11). Besonders bevorzugt sind flüssigkristalline Materialien, die diskotisch sind;

Thiophene, Oligothiophene und substituierte Derivate davon. Geeignete Oligothiophene sind Quaterthiophene, Quinquethiophene, Sexithiophene,

α,ω-Di(C₁₋C₈)-alkyloligothiophenes, wie α,ω-Dihexylquaterthiophene, α,ω-Dihexylquinquethiophene und α,ω-Dihexylsexithiophene, Poly(alkylthiophene), wie Poly(3-hexylthiophen), Bis(dithienothiophene), Anthradithiophene und Dialkylanthradithiophene, wie Dihexylanthradithiophen, Phenylene-Thiophen- (P-T-) Oligomere und Derivate davon, speziell α,ω-Alkyl-substituierte Phenylen-Thiophen-Oligomere;

Geeignet sind weiterhin Verbindungen des Typs α,α'-Bis(2,2-dicyanovinyl)-quinquethiophen (DCV5T), (3-(4-Octylphenyl)-2,2'-bithiophen) (PTOPT), Poly(3-(4'-(1,4,7-trioxaoctyl)phenyl)thiophen (PEOPT), (Poly(3-(2'-methoxy-5'-octylphenyl)thiophen)) (POMeOPT), Poly(3-octylthiophen) (P30T), Poly(pyridopyrazinvinylen)-Polythiophen-Blends, wie EHH-PpyPz, Copolymere PTPTB, BBL, F8BT, PFMO, siehe Brabec C., Adv. Mater., 2996, 18, 2884, (PCPDTBT) Poly[2,6-(4,4-bis-(2-ethylhexyl)-4H-cyclopenta[2,1-b;3,4-b']-dithiophen)-4,7-(2,1,3-benzothiadiazol);

Paraphenylenvinylen und Paraphenylenvinylen enthaltende Oligomere oder Polymere wie z. B. Polyparaphenylenvinylen, MEH-PPV (Poly(2-methoxy-5-(2'-ethylhexyloxy)-1,4-phenylenvinylen, MDMO-PPV (Poly(2-methoxy-5-(3'7'-dimethyloctyloxy)-1,4-phenylenvinylen)), PPV, CN-PPV (mit verschiedenen Alkoxy-Derivaten);

Phenylenethinylen/Phenylenvinylen-Hybridpolymere (PPE-PPV);

Polyfluorene und alternierende Polyfluoren-Copolymere wie z. B. mit 4,7-Dithien-2'-yl-2,1,3-benzothiadiazol, geeignet sind weiterhin Poly(9,9'-dioctylfluoren-co-benzothio-diazol) (F₈BT), Poly(9,9'-dibctylfluoren-co-bis-N,N'-(4-butyl-phenyl)-bis-N,N'-phenyl-1,4-phenylendiamin (PFB);

Polycarbazole, d. h. Carbazol enthaltende Oligomere und Polymere, wie (2,7) und (3,6).

Polyaniline, d. h. Anilin enthaltende Oligomere und Polymere, wie (2,7) und (3,6).

Triarylamine, Polytriarylamine, Polycyclopentadiene, Polypyrrole, Polyfurane, Polysilole, Polyphosphole, TPD, CBP, Spiro-MeOTAD.

Fullerene, speziell C60 und seine Derivate wie PCBM (= [6,6]-Phenyl-C₆₁-buttersäuremethylester). In derartigen Zellen ist das Fullerenderivat ein Lochleiter.

Kupfer(I)-iodid, Kupfer(I)-thiocyanat.

p-n-Mischmaterialien, d. h. Donor und Akzeptor in einem Material, Polymer, Blockpolymer, Polymere mit C60s, C60-Azofarben, Triaden carotenoid-porphyrin-quinode LC Donor/Akzeptor-Systeme wie von Kelly in S. Adv. Mater. 2006, 18, 1754, beschrieben.

Alle zuvor genannten Halbleitermaterialien können auch dotiert sein. Beispiele von Dotierstoffen für p-Halbleiter: 2,3,5,6-Tetrafluoro-7,7,8,8-tetracyanochinodimethan (F₄-TCNQ), etc.

Die erfindungsgemäßen (neuen) Verbindungen (I) eignen sich auch besonders vorteilhaft als organische Halbleiter. Sie fungieren dabei in der Regel als n-Halbleiter. Werden die erfindungsgemäß eingesetzten Verbindungen der Formel (I) mit anderen Halbleitern kombiniert und ergibt sich aus der Lage der Energieniveaus, dass die anderen Halbleiter als n-Halbleiter fungieren, so können die Verbindungen (I) auch ausnahmsweise als p-Halbleiter fungieren. Dies ist z. B. bei der Kombination mit cyanosubstituierten Perylentetracarboximiden der Fall. Die Verbindungen der Formel (I) zeichnen sich durch ihre Luftstabilität aus. Weiterhin verfügen sie über eine hohe Ladungstransportmobilität und haben ein hohes on/off-Verhältnis. Sie eignen sich in besonders vorteilhafter Weise für organische Feldeffekttransistoren. Die erfindungsgemäßen Verbindungen eignen sich vorteilhaft zur Herstellung von integrierten Schaltkreisen (ICs), für die bislang übliche n-Kanal MOSFET (metal oxide semiconductor field-effect transistor (MOSFET) zum Einsatz kommen. Dabei handelt es sich dann um CMOS-analoge Halbleiterbausteine, z. B. für Mikroprozessoren, Mikrocontroller, statische RAM, und andere digitale Logikbausteine. Zur Herstellung von Halbleitermaterialien können die erfindungsgemäßen Verfahren nach einem der folgenden Verfahren weiterverarbeitet werden: Drucken (Offset, Flexo, Gravur, Screen, Inkjet, Elektrofotografie), Verdampfen, Lasertransfer, Fotolithografie, Dropcasting. Sie eignen sich insbesondere für einen Einsatz in Displays (speziell großflächigen und/oder flexiblen Displays) und RFID-Tags.

Die erfindungsgemäßen Verbindungen eignen sich weiterhin besonders vorteilhaft zur Datenspeicherung in Dioden, speziell in OLEDs, in der Photovoltaik, als UV-Absorber, als optischer Aufheller, als unsichtbares Label und als Fluoreszenzlabel für Biomoleküle, wie Proteine, DNA, Zucker und Kombinationen davon.

Die erfindungsgemäßen Verbindungen eignen sich weiterhin besonders vorteilhaft als Fluoreszenzfarbstoff in einem auf Fluoreszenzkonversion beruhenden Display; in einem lichtsammelnden Kunststoffteil, welches gegebenenfalls mit einer Solarzelle kombiniert ist; als Pigmentfarbstoff in elektrophoretischen Displays; als Fluoreszenzfarbstoff in einer auf Chemolumineszenz basierenden Anwendung (z. B. in glow sticks).

Die erfindungsgemäßen Verbindungen eignen sich weiterhin besonders vorteilhaft als Fluoreszenzfarbstoff in einem auf Fluoreszenzkonversion beruhenden Display. Derartige Displays umfassen im Algemeinen ein transparentes Substrat, einen auf dem Substrat befindlichen Fluoreszenzfarbstoff und eine Strahlungsquelle. Übliche Strahlungsquellen senden blaues (color-by-blue) oder UV-Licht (color-by-uv) aus. Die Farbstoffe absorbieren entweder das blaue oder das UV-Licht und werden als Grünemitter eingesetzt. In diesen Displays wird z. B. das rote Licht erzeugt, indem der Rotemitter durch einen blaues oder UV-Licht absorbierenden Grünemitter angeregt wird. Geeignete color-by-blue-Displays sind z. B. in der WO 98/28946 beschrieben. Geeignete color-by-uv-Displays werden z. B. von W. A. Crossland, I. D. Sprigle und A. B. Davey in Photoluminescent LCDs (PL-LCD) using phosphors Cambridge University and Screen Technology Ltd., Cambridge, UK, beschrieben.

Die erfindungsgemäßen Verbindungen eignen sich weiterhin besonders als Fluoreszenzemitter in OLEDs, in denen sie entweder durch Elektrolumineszenz oder durch einen entsprechenden Phosphoreszenzemitter über Förster Energietransfer (FRET) angeregt werden.

Die erfindungsgemäßen Verbindungen eignen sich weiterhin besonders in Displays, welche basierend auf einem elektrophoretischen Effekt über geladene Pigmentfarbstoffe Farben an- und ausschalten. Derartige elektrophoretische Displays sind z. B. in der US 2004/0130776 beschrieben.

Die erfindungsgemäßen Verbindungen eignen sich weiterhin besonders für den Einsatz in einem lichtsammelnden Kunststoffteil, welches großflächig Licht absorbiert und an seinen Kanten nach mehrfacher Brechung das Licht emittiert (so genannte LISAs). Derartige LISAs können an den Kanten Solarzellen wie z. B. Siliciumsolarzellen oder organische Solarzellen aufweisen, die das konzentrierte Licht in elektrische Energie umwandeln. Eine Kombination lichtsammelnder Kunststoffe mit Solarzellen ist z. B. in der US 4,110,123 beschrieben.

Die erfindungsgemäßen Verbindungen eignen sich weiterhin besonders in Chemolumineszenzanwendungen. Dazu zählen so genannte "Glow Sticks". Zu deren Herstellung kann wenigstens eine Verbindung der Formel (I) z. B. in einem Alkylphthalat gelöst werden. Eine Anregung der Chemolumineszenz kann durch Mischung eines Oxalsäureesters mit Wasserstoffperoxid erfolgen, z. B. nachdem diese beiden zunächst separaten Komponenten durch Zerbrechen eines Glases gemischt werden. Die resultierende Reaktionsenergie führt zur Anregung und Fluoreszenz der Farbstoffe. Derartige Glow Sticks können als Notlicht, z. B. beim Angeln, in Seenotrettungswesten oder anderen Sicherheitsanwendungen eingesetzt werden.

Gegenstand der Erfindung sind weiterhin organische Feldeffekttransistoren, umfassend ein Substrat mit wenigstens einer Gate-Struktur, einer Source-Elektrode und einer Drain-Elektrode und wenigstens einer Verbindung der Formel I, wie zuvor definiert, als n-Halbleiter. Gegenstand der Erfindung sind weiterhin Substrate mit einer Vielzahl von organischen Feldeffekttransistoren, wobei zumindest ein Teil der Feldeffekttransistoren wenigstens eine Verbindung der Formel I, wie zuvor definiert, als n-Halbleiter enthält. Gegenstand der Erfindung sind auch Halbleiterbausteine, die wenigstes ein solches Substrat umfassen.

Eine spezielle Ausführungsform ist ein Substrat mit einem Muster (Topographie) von organischen Feldeffekttransistoren, wobei jeder Transistor
- einen auf dem Substrat befindlichen organischen Halbleiter;
- eine Gate-Struktur zur Steuerung der Leitfähigkeit des leitenden Kanals; und
- leitfähige Source- und Drain-Elektroden an den beiden Enden des Kanals
enthält, wobei der organische Halbleiter aus wenigstens einer Verbindung der Formel (I) besteht oder eine Verbindung der Formel (I) umfasst. Des Weiteren umfasst der organische Feldeffekttransistor in der Regel ein Dielektrikum.

Eine weitere spezielle Ausführungsform ist ein Substrat mit einem Muster von organischen Feldeffekttransistoren, wobei jeder Transistor einen integrierten Schaltkreis bildet oder Teil eines integrierten Schaltkreises ist und wobei zumindest ein Teil der Transistoren wenigstens eine Verbindung der Formel (I) umfasst.

Als Substrate eignen sich prinzipiell die dafür bekannten Materialien. Geeignete Substrate umfassen z. B. Metalle (vorzugsweise Metalle der Gruppen 8, 9, 10 oder 11 des Periodensystems, wie Au, Ag, Cu), oxidische Materialien (wie Glas, Quarz, Keramiken, SiO₂), Halbleiter (z. B. gedoptes Si, gedoptes Ge), Metalllegierungen (z. B. auf Basis von Au, Ag, Cu, etc.), Halbleiterlegierungen, Polymere (z. B. Polyvinylchlorid, Polyolefine, wie Polyethylen und Polypropylen, Polyester, Fluoropolymere, Polyamide, Polyimide, Polyurethane, Polyalkyl(meth)acrylate, Polystyrol und Mischungen und Komposite davon), anorganische Feststoffe (z. B. Ammoniumchlorid), Papier und Kombinationen davon. Die Substrate können flexibel oder unflexibel solid, mit gekrümmter oder planarer Geometrie sein, abhängig von der gewünschten Anwendung.

Ein typisches Substrat für Halbleiterbausteine umfasst eine Matrix (z. B. eine Quarz-oder Polymermatrix) und, optional, eine dielektrische Deckschicht.

Geeignete Dielektrika sind SiO₂, Polystyrol, Poly-a-methylstyrol, Polyolefine (wie Polypropylen, Polyethylen, Polyisobuten) Polyvinylcarbazol, fluorierte Polymere (z. B. Cytop, CYMM) Cyanopullane, Polyvinylphenol, Poly-p-xylol, Polyvinylchlorid oder thermisch oder durch Luftfeuchtigkeit vernetzbare Polymere. Spezielle Dielektrika sind "self assembled nanodielectrics", d. h. Polymere, welche aus SiCl-Funktionalitäten enthaltenden Monomeren wie z. B. Cl₃SiOSiCl₃, Cl₃Si-(CH₂)₆-SiCl₃ , Cl₃Si-(CH₂)₁₂-SiCl₃ erhalten und/oder welche durch Luftfeuchtigkeit oder durch Zugabe von Wasser in Verdünnung mit Lösungsmitteln vernetzt werden (siehe z. B. Faccietti Adv. Mat. 2005, 17, 1705-1725). An Stelle von Wasser können auch hydroxylgruppenhaltige Polymere wie Polyvinylphenol oder Polyvinylalkohol oder Copolymere aus Vinylphenol und Styrol als Vernetzungskomponenten dienen. Es kann auch wenigstens ein weiteres Polymer während des Vernetzungsvorgangs zugegen sein, wie z. B. Polystyrol, welches dann mitvernetzt wird (siehe Facietti, US-Patentanmeldung 2006/0202195).

Das Substrat kann zusätzlich Elektroden aufweisen, wie Gate-, Drain- und Source-Elektroden von OFETs, die normalerweise auf dem Substrat lokalisiert sind (z. B. abgeschieden auf oder eingebettet in eine nichtleitende Schicht auf dem Dielektrikum). Das Substrat kann zusätzlich leitfähige Gate-Elektroden der OFETs enthalten, die üblicherweise unterhalb der dielektrischen Deckschicht (d. h. dem Gate-Dielektrikum) angeordnet sind.

Nach einer speziellen Ausführung befindet sich eine Isolatorschicht (gate insulating layer) auf wenigstes einem Teil der Substratoberfläche. Die Isolatorschicht umfasst wenigstens einen Isolator, der vorzugsweise ausgewählt ist unter anorganischen Isolatoren, wie SiO₂, SiN, etc., ferroelektrischen Isolatoren, wie Al₂O₃, Ta₂O₅ La₂O₅, TiO₂, Y₂O₃, etc., organischen Isolatoren, wie Polyimiden, Benzocyclobuten (BCB), Polyvinylalkoholen, Polyacrylaten, etc. und Kombinationen davon.

Geeignete Materialien für Source- und Drain-Elektroden sind prinzipiell elektrisch leitfähige Materialien. Dazu zählen Metalle, vorzugsweise Metalle der Gruppen 8, 9, 10 oder 11 des Periodensystems, wie Pd, Au, Ag, Cu, Al, Ni, Cr, etc. Geeignet sind weiterhin leitfähige Polymere, wie PEDOT (=Poly(3,4-ethylenedioxythiophene); PSS (= Poly(styrolsulfonat), Polyanilin, oberflächenmodifiziertes Gold, etc. Bevorzugte elektrisch leitfähige Materialien haben einen spezifischen Widerstand von weniger als 10 ⁻³, vorzugsweise weniger als 10 ⁻⁴, insbesondere weniger als 10 ⁻⁶ oder 10 ⁻⁷ Ohm x Meter.

Nach einer speziellen Ausführung befinden sich Drain- und Source-Elektroden zumindest teilweise auf dem organischen Halbleitermaterial. Selbstverständlich kann das Substrat weitere Komponenten umfassen, wie sie üblicherweise in Halbleitermaterialien oder ICs eingesetzt werden, wie Isolatoren, Widerstände, Kondensatoren, Leiterbahnen, etc.

Die Elektroden können nach üblichen Verfahren, wie Verdampfen, lithographische Verfahren oder einen anderen Strukturierungsprozess aufgebracht werden.

Die Halbleitermaterialien können auch mit geeigneten Hilfsmitteln (Polymere, Tenside) in disperser Phase durch Verdrucken verarbeitet werden.

In einer ersten bevorzugten Ausführungsform erfolgt die Abscheidung wenigstens einer Verbindung der allgemeinen Formel I (und gegebenenfalls weiterer Halbleitermaterialien) durch ein Gasphasenabscheidungsverfahren (Physical Vapor Deposition PVD). PVD-Verfahren werden unter Hochvakuumbedingungen durchgeführt und umfassen die folgenden Schritte: Verdampfen, Transport, Abscheidung. Überraschenderweise wurde gefunden, dass sich die Verbindungen der allgemeinen Formel I besonders vorteilhaft für einen Einsatz in einem PVD-Verfahren eignen, da sie sich im Wesentlichen nicht zersetzen und/oder unerwünschte Nebenprodukte bilden. Das abgeschiedene Material wird in hoher Reinheit erhalten. In einer speziellen Ausführung wird das abgeschiedene Material in Form von Kristallen erhalten oder enthält einen hohen kristallinen Anteil. Allgemein wird zur PVD wenigstens eine Verbindung der allgemeinen Formel I auf eine Temperatur oberhalb ihrer Verdampfungstemperatur erhitzt und durch Abkühlen unterhalb der Kristallisationstemperatur auf einem Substrat abgeschieden. Die Temperatur des Substrats bei der Abscheidung liegt vorzugsweise in einem Bereich von etwa 20 bis 250 _{°}C, besonders bevorzugt 50 bis 200 _{°}C. Überraschenderweise wurde gefunden, dass erhöhte Substrattemperaturen bei der Abscheidung der Verbindungen der Formel I vorteilhafte Auswirkungen auf die Eigenschaften der erzielten Halbleiterelemente haben können.

Die erhaltenen Halbleiterschichten weisen im Allgemeinen eine Dicke auf, die für einen ohmschen Kontakt zwischen Source- und Drain-Elektrode ausreicht. Die Abscheidung kann unter einer Inertatmosphäre, z. B. unter Stickstoff, Argon oder Helium, erfolgen.

Die Abscheidung erfolgt üblicherweise bei Umgebungsdruck oder unter reduziertem Druck. Ein geeigneter Druckbereich beträgt etwa 10⁻⁷ bis 1,5 bar.

Vorzugsweise wird die Verbindung der Formel (I) auf dem Substrat in einer Dicke von 10 bis 1000 nm, besonders bevorzugt 15 bis 250 nm, abgeschieden. In einer speziellen Ausführung wird die Verbindung der Formel 1 zumindest teilweise in kristalliner Form abgeschieden. Hierfür eignet sich speziell das zuvor beschriebene PVD-Verfahren. Weiterhin ist es möglich, zuvor hergestellte organische Halbleiterkristalle einzusetzen. Geeignete Verfahren zur Gewinnung von derartigen Kristallen werden von R. A. Laudise et al. in "Physical Vapor Growth of Organic Semi-Conductors", Journal of Crystal Growth 187 (1998), Seiten 449-454, und in "Physical Vapor Growth of Centimeter-sized Crystals of α-Hexathiophene", Journal of Cystal Growth 1982 (1997), Seiten 416-427, beschrieben, worauf hier Bezug genommen wird.

Die Verbindungen der allgemeinen Formel (I) können auch besonders vorteilhaft aus Lösung prozessiert werden. In einer zweiten bevorzugten Ausführungsform erfolgt die Abscheidung wenigstens einer Verbindung der allgemeinen Formel (I) (und gegebenenfalls weiterer Halbleitermaterialien) daher durch eine Rotationsbeschichtung (spin coating). Die Verbindungen der Formel (I) sollten sich auch zur Herstellung von Halbleiterelementen, speziell OFETs oder auf der Basis von OFETs, durch ein Druckverfahren eignen. Es können dafür übliche Druckprozesse (Inkjet, Flexo, Offset, gravure; Tiefdruck, Nanoprint) verwendet werden. Bevorzugte Lösungsmittel für den Einsatz der Verbindungen der Formel (I) in einem Druckverfahren sind aromatische Lösungsmittel wie Toluol, Xylol, etc. Man kann zu diesen "Halbleitertinten" verdickend wirkende Substanzen, wie Polymere zusetzen, z. B. Polystyrol, etc. Dabei verwendet man als Dielektrikum die zuvor genannten Verbindungen.

In einer bevorzugten Ausführungsform handelt es sich bei dem erfindungsgemäßen Feldeffekttransistor um einen Dünnschichttransistor (thin film transistor, TFT). Gemäß einem üblichen Aufbau verfügt ein Dünnschichttransistor über eine auf dem Substrat befindliche Gate-Elektrode, eine auf dieser und dem Substrat befindliche Gate-Isolierschicht, eine auf der Gate-Isolierschicht befindliche Halbleiterschicht, eine ohmsche Kontaktschicht auf der Halbleiterschicht sowie über eine Source-Elektrode und eine Drain-Elektrode auf der ohmschen Kontaktschicht.

In einer bevorzugten Ausführungsform wird die Oberfläche des Substrats vor der Abscheidung wenigstens einer Verbindung der allgemeinen Formel (I) (und gegebenenfalls wenigstens eines weiteren Halbleitermaterials) einer Modifizierung unterzogen. Diese Modifizierung dient der Bildung von die Halbleitermaterialien bindenden Bereichen und/oder von Bereichen, auf denen keine Halbleitermaterialien abgeschieden werden können. Bevorzugt wird die Oberfläche des Substrats mit wenigstens einer Verbindung (C1) modifiziert, die geeignet ist, an die Oberfläche des Substrats sowie die Verbindungen der Formel (I) zu binden. In einer geeigneten Ausführungsform wird ein Teil der Oberfläche oder die komplette Oberfläche des Substrats mit wenigstens einer Verbindung (C1) beschichtet, um eine verbesserte Abscheidung wenigstens einer Verbindung der allgemeinen Formel (I) (und gegebenenfalls weiterer halbleitender Verbindungen) zu ermöglichen. Eine weitere Ausführungsform umfasst die Abscheidung eines Musters von Verbindungen der allgemeinen Formel (C1) auf dem Substrat nach einem entsprechenden Herstellungsverfahren. Dazu zählen die dafür bekannten Maskenprozesse sowie so genannte "Patterning"-Verfahren, wie sie z. B. in der US 11/353934 beschrieben sind, worauf hier in vollem Umfang Bezug genommen wird.

Geeignete Verbindungen der Formel (C1) sind zu einer bindenden Wechselwirkung sowohl mit dem Substrat als auch mit wenigstens einer Halbleiterverbindung der allgemeinen Formel I befähigt. Der Begriff "bindende Wechselwirkung" umfasst die Bildung einer chemischen Bindung (kovalenten Bindung), ionischen Bindung, koordinativen Wechselwirkung, Van der Waals-Wechselwirkungen, z. B. Dipol-Dipol-Wechselwirkungen) etc. und Kombinationen davon. Geeignete Verbindungen der allgemeinen Formel (C1) sind:
- Silane, Phosphonsäuren, Carbonsäuren, Hydroxamsäuren, wie Alkyltrichlorsilane, z. B. n-(Octadecyl)trichlorsilan; Verbindungen mit Trialkoxysilan-Gruppen, z. B. Alkyltrialkoxysilane, wie n-Octadecyltrimethoxysilan, n-Octadecyltriethoxysilan, n-Octadecyltri-(n-propyl)oxysilan, n-Octadecyltri-(isöpropyl)oxysilan; Trialkoxyaminoalkylsilane, wie Triethoxyaminopropylsilan und N[(3-triethoxysilyl)-propyl]-ethylendiamin; Trialkoxyalkyl-3-glycidylethersilane, wie Triethoxypropyl-3-glycidylethersilan; Trialkoxyallylsilane wie Allyltrimethoxysilan; Trialkoxy-(isocyanatoalkyl)silane; Trialkoxysilyl(meth)acryloxyalkane und Trialkoxysilyl-(meth)acrylamidoalkane, wie 1-Triethoxysilyl-3-acryloxypropan.
- Amine, Phosphine und Schwefel enthaltende Verbindungen, speziell Thiole.

Bevorzugt ist die Verbindung (C1) ausgewählt unter Alkyltrialkoxysilanen, speziell n-Octadecyltrimethoxysilan, n-Octadecyltriethoxysilan; Hexaalkyldisilazanen, und speziell Hexamethyldisilazane (HMDS); C₈-C₃₀-Alkylthiolen, speziell Hexadecanthiol; Mercaptocarbonsäuren und Mercaptosulfonsäuren speziell Mercaptoessigsäure, 3-Mercaptopropionsäure, Mercaptobernsteinsäure, 3-Mercapto-1-propansulfonsäure und den Alkalimetall- und Ammoniumsalzen davon.

Es sind auch verschiedene Halbleiterarchitekturen mit den erfindungsgemäßen Halbleitern denkbar wie z. B. Top Contact, Top Gate, Bottom Contact, Bottom Gate, oder aber ein vertikaler Aufbau wie z. B. ein VOFET (Vertical organic field effect transistor) wie z. B. in US 2004/0046182 beschrieben.

Die Schichtdicken betragen bei Halbleitern z. B. 10 nm bis 5 µm, beim Dielektrikum 50 nm bis 10 µm, die Elektroden können z. B. 20 nm bis 1 µm dick sein. Die OFETs können auch zu anderen Bauteilen wie Ringoszillatoren oder Inverter kombiniert werden.

Ein weiterer Aspekt der Erfindung ist die Bereitstellung elektronischer Bauteile, die mehrere Halbleiterkomponenten umfassen, wobei es sich um n- und/oder p-Halbleiter handeln kann. Beispiele solcher Bauteile sind Feldeffekttransistoren (FETs), bipolare Flächentransistoren (bipolar junction transistors, BJTs), Tunneldioden, Wechselrichter, lichtemittierende Bauteile, biologische und chemische Detektoren oder Sensoren, temperaturabhängige Detektoren, Photodetektoren wie Polarisations-sensitive Photodetektoren, Gatter, AND-, NAND-, NOT-, OR-, TOR-, und NOR-Gatter, Inverter, Register, Schalter, Zeitbausteine, statische oder dynamische Speicher und andere dynamische oder sequentielle logische oder andere digitale Bauteile einschließlich programmierbarer Schaltungen.

### Beispiele

Das in den Beispielen als Edukt III eingesetzte 5,11-Dibromtetracen wurde wie folgt hergestellt:
Eine Lösung von 708 mg (4 mmol) N-Bromsuccinimid in 40 ml Dimethylformamid wurde zu einer auf 60 °C erwärmten Lösung von 456 mg (2 mmol) Tetracen in 200 ml Chloroform gegeben. Die resultierende Lösung wurde 5 h bei 60 °C gerührt und anschließend auf Raumtemperatur abgekühlt. Nach Verdampfen des Chloroforms im Vakuum und Zugabe von Wasser wurde der gebildete Niederschlag abfiltriert, getrocknet und aus Toluol umkristallisiert. Es wurden 710 mg 5,11-Dibromtetracen (III) in Form eines orangefarbenen Feststoffs erhalten, was einer Ausbeute von 92 % entspricht.

### Beispiel 1: N,N'-Bis(2,6-diisopropylphenyl)-9,10;21,22-dibenzopentarylen-3,4;15,16-tetracarbonsäurediimid la1

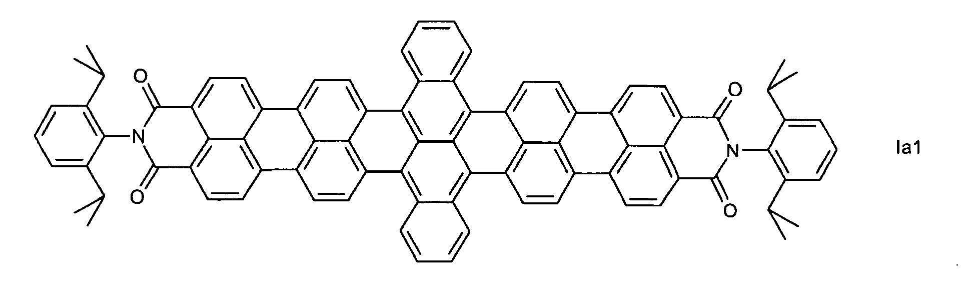

a) Herstellung von 5,11-Bis[N-(2,6-diisopropylphenyl)-3,4-dicarbonsäureimidperylen-9-yl)tetracen IVa1
   Unter Schutzgas wurden einer Lösung von 193 mg (0,5 mmol) 5,11-Dibromtetracen und 608 mg (1 mmol) N-(2,6-diisopropylphenyl}-9-(4,4,5,5-tetramethyl-1,3,2-dioxaboran-2-yl)perylen-3,4-dicarbonsäureimid in 30 ml Toluol und 2 ml Ethanol 20 mg (13 µmol) Tris(dibenzylidenaceton)dipalladium, 40 mg (80 µmol) Bis(2-diphenylphosphino)phenylether und 2 ml einer 2M Lösung von Kaliumcarbonat (4 mmol) in Wasser zugesetzt. Die Mischung wurde 15 h auf 95 °C erhitzt.
   Nach Abkühlen auf Raumtemperatur wurde die organische Phase mehrfach mit Wasser gewaschen und über Magnesiumsulfat getrocknet. Das nach Entfernen der organischen Lösungsmittel im Vakuum erhaltene Rohprodukt wurde einer Säulenchromatographie an Kieselgel mit Dichlormethan als Eluens unterzogen.
   Es wurden 424 mg IVa1 in Form eines tiefroten Feststoffs erhalten, was einer Ausbeute von 71 % entspricht.
   Analytische Daten:
   R_{f}-Wert (CH₂Cl₂) = 0,5;
   Absorption: (CHCl₃): λₘₐₓ (ε) = 524 (97100), 355 (8500) nm (l/mol cm).
b) Cyclodehydrierung
   Einer Lösung von 120 mg (0,1 mmol) IVa1 in 10 ml trockenem Dichlormethan wurde
   eine Lösung von 260 mg (2 mmol) wasserfreiem Eisen(III)chlorid in 3 ml Nitromethan langsam zugesetzt. Die Mischung wurde dann 4 h bei Raumtemperatur unter Lichtausschluss gerührt.
   Der nach Fällen des Reaktionsgemischs auf 50 ml Methanol gebildete Niederschlag wurde abfiltriert, im Vakuum getrocknet und dann in 3 ml Ethanolamin in Gegenwart von 2 g Kaliumcarbonat 2 h auf 120 °C erwärmt.
   Das nach Abkühlen auf Raumtemperatur durch Zugabe von Wasser ausgefällte Rohprodukt wurde abfiltriert, mit Methanol und Dichlormethan gewaschen und dann durch Extraktion mit Chlorbenzol gereinigt.
   Es wurden 68 mg la1 in Form eines violetten Feststoffs erhalten, was einer Ausbeute von 58 % entspricht.
   Analytische Daten:
   Absorption (Chlorbenzol): λₘₐₓ = 1018, 542, 318 nm.

### Beispiel 2: N,N'-Bis(2,6-diisopropylphenyl)-1,6,13,18-tetra[(4-tert.-octyl)phenoxy]-9,10;21,22-dibenzopentarylen-3,4;15,16-tetracarbonsäurediimid la2

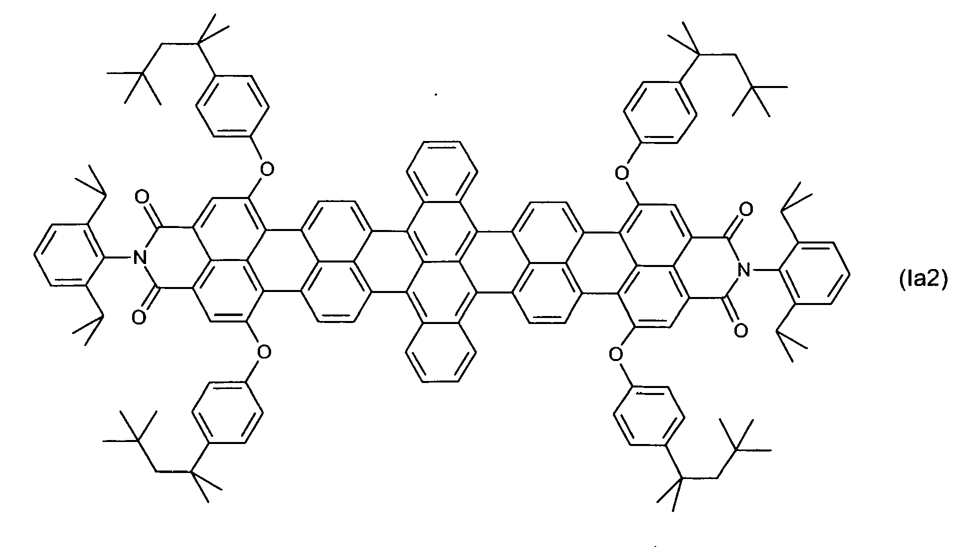

a) 5,11-Bis[N-(2,6-diisopropylphenyl)-1,6-bis((4-tert.-octyl)phenoxy)-3,4-dicarbonsäureimidperylen-9-yl]tetracen IVa2
   Unter Schutzgas wurden 610 mg (0,6 mmol) N-(2,6-diisopropyl-phenyl)-1,6-bis((4-tert.-octyl)phenxoy)-9-(4,4,5,5-tetramethyl-1,3,2-dioxaboran-2-yl)perylen-3,4-dicarbonsäureimid und 116 mg (0,3 mmol) 5,11-Dibromtetracen wurden unter Erwärmen auf 80 °C in 40 ml Toluol und 4 ml Ethanol gelöst. Anschließend wurden 16 mg (12 µmol) Tris(dibenzylidenaceton)dipalladium, 30 mg (60 µmol) Bis(2-diphenylphosphino)phenylether und 2,5 ml einer 2M Lösung von Kaliumcarbonat (0,1 mmol) in Wasser zugegeben. Die Mischung wurde 15 h auf 85 °C erhitzt.
   Nach Abkühlen auf Raumtemperatur wurde die organische Phase mehrfach mit Wasser gewaschen und über Magnesiumsulfat getrocknet. Das nach Entfernen der organischen Lösungsmittel im Vakuum erhaltene Rohprodukt wurde einer Säulenchromatographie an Kieselgel mit Toluol als Eluens unterzogen.
   Es wurden 354 mg IVa2 in Form eines tiefroten Feststoffs erhalten, was einer Ausbeute von 59 % entspricht.
   Analytische Daten:
   Rr-Wert (Toluol) = 0,62;
   Absorption (CHCl₃): λₘₐₓ (ε) = 530 (94860), 420 (16960) nm (I / mol cm).
b) Cyclodehydrierung
   Einer Lösung von 200 mg (0,1 mmol) IVa2 in 10 ml trockenem Dichlormethan wurde
   eine Lösung von 260 mg (2 mmol) wasserfreiem Eisen(III)chlorid in 3 ml Nitromethan langsam zugesetzt. Die Mischung wurde dann 3 h bei Raumtemperatur unter Lichtausschluss gerührt.
   Der nach Fällen des Reaktionsgemischs auf 50 ml Methanol gebildete Niederschlag wurde abfiltriert, im Vakuum getrocknet, dann in 1 ml Dimethylformamid gelöst und zusammen mit 3 ml Ethanolamin und 2 g Kaliumcarbonat 2 h auf 120 °C erwärmt.
   Das nach Abkühlen auf Raumtemperatur durch Zugabe von Wasser ausgefällte Rohprodukt wurde abfiltriert, getrocknet und anschließend durch Säulenchromatographie an Kieselgel mit einer 1:1-Mischung von Petrolether und Dichlormethan gereinigt.
   Es wurden 105 mg la2 in Form eines violetten Feststoffs erhalten, was einer Ausbeute von 53 % entspricht.
   Analytische Daten:
   Rr-Wert (Toluol) = 0,63;
   Absorption (CH₂Cl₂): λₘₐₓ (ε) = 1037 (149500), 567 (46700), 321 nm (84500) nm
      (l / mol cm);
   Absorption (PMMA): λₘₐₓ = 571, 1054 nm.

### Beispiel 3: N,N'-Bis-(N-(1-heptyloctyl)-9,10:21,22-dibenzopentarylen-3,4:15,16-bis(dicarboximide)

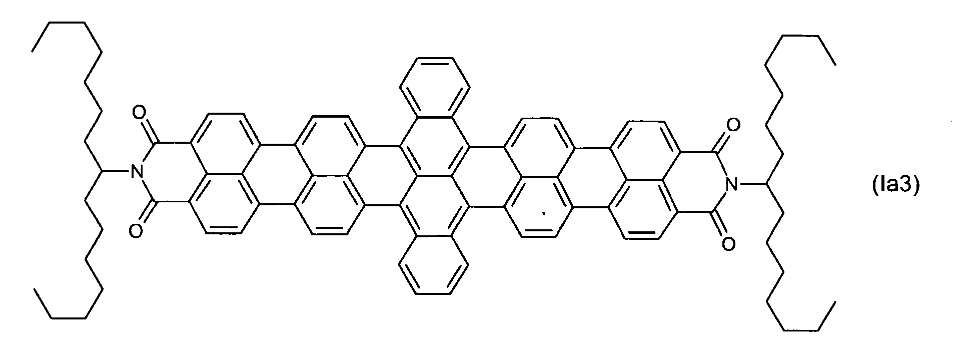

a) Herstellung von N-(1-Heptyloctyl)-9-bromperylen-3,4-dicarbonsäureimid IVa3
   Unter Schutzgas wurde eine Mischung von 3 g (7,48 mmol) 9-Bromperylen-3,4-dicarbonsäureanhydrid und 8 g (35 mmol) 1-Heptyloctylamin in 150 ml N-Methylpyrrolidon und 3 ml Essigsäure 20 h auf 135 °C erhitzt.
   Nach Abkühlen auf Raumtemperatur wurde das Reaktionsgemisch auf 500 mL Wasser gefällt. Der Niederschlag wurde abfiltriert, mit Wasser und Methanol gewaschen und im Vakuum getrocknet. Das erhaltene Rohprodukt wurde durch Säulenchromatographie an Kieselgel mit Dichlormethan als Eluens gereinigt. Es wurden 2,9 g (63 %) der Titelverbindung in Form eines roten Feststoffs mit einem Schmelzpunkt von 163 °C erhalten.
   Analytische Daten:
   R_{f}-Wert (Toluol) = 0,66;
   ¹H-NMR (500 MHz, CD₂Cl₂, 25 °C): δ = 8,63 (d, 1H, J=8,2 Hz), 8,61 (d, 1H, J=8,2 Hz), 8,45 (d, 1H, J=8,2 Hz), 8,42 (d, 1H, J=8,2 Hz), 8,36 (d, 1H, J=7,6 Hz), 8,27 (d, 1H, J=8,2 Hz), 8,20 (d, 1H, J=8,2 Hz), 7,87 (d, 1H, J=8,2 Hz), 7,70 (t, 1H, J=7,6 Hz), 5,17-5,05 (m, 1H), 2,23-2,16 (m, 2H), 1,89- 1,81 (m, 2H), 1,27-1,20 (m, 20 H), 0,82-0,77 (m, 6H).
   ¹³C-NMR (Spinecho, 125 MHz, CD₂Cl₂, 25 °C): δ = 165,16, 164,12, 136,16, 132,73, 132,01, 131,26, 129,90, 129,73, 129,47, 128,93, 128,20, 126,14, 126,08,124,38, 123,67, 122,06, 121,31, 120.75,120,48, 54,69, 32,65, 32,15, 29,90, 29,59, 27,40, 22,98, 14,53.
   IR (KBr) v = 2924, 2853, 2365, 1697, 1653, 1594, 1572, 1450, 1460, 1408, 1355, 1292, 1244, 1172, 1136, 1109, 840, 810, 754 cm⁻¹;
   UV-Vis (CHCl₃) λₘₐₓ (ε): 514 (51000), 489 nm (52000 M⁻¹ cm⁻¹);
   Fluoreszenz (CHCl₃) λₘₐₓ: 571, 544 nm;
   MS (FD): [M⁺] berechnet für C₃₇H₄₀BrN₂O₂, 610,64; gefunden, 611,1 (100%).
b) Herstellung von N-(1-Heptyloctyl)-9-(4,4,5,5-tetramethyl-1,3,2-dioxaborolan-2-yl)perylen-3,4-dicarbonsäureimid 111a3
   Eine Mischung aus 1,2 g (2 mmol) N-(1-Heptyloctyl)-9-bromperylen-3,4-dicarbonsäureimid IVa3, 558 mg (2,5 mmol) Bis(pinacolato)diboron, 44 mg (0,1 mmol) PdCl₂(dppf) (dppf = Ph₂PC₅H₄FeC₅H₄PPh₂) und 588 mg (5,3 mmol) Kaliumacetat in 20 mL Dioxan wurden unterArgonatmosphäre 16 h bei 70 °C gerührt. Danach ließ man die Reaktionsmischung auf Raumtemperatur abkühlen.
   Nach dem Abkühlen auf Raumtemperatur wurde das Reaktionsgemisch mit Dichlormethan extrahiert und zweimal mit Wasser gewaschen. Die organische Phase wurde abgetrennt und über MgSO₄ getrocknet. Das erhaltene Rohprodukt wurde durch Säulenchromatographie an Kieselgel mit Dichlormethan als Eluens gereinigt. Man erhielt 1,0 g (78 %) IIIa3 in Form eines roten Feststoffs mit dem Schmelzpunkt von 213 °C.
   Analytische Daten:
   R_{f}-Wert (Toluol) = 0,26;
   ¹H-NMR (300 MHz, THF-d₈, 25 °C): δ = 8,87 (d, 1H, J=7,7 Hz), 8,55-8,47 (m, 6H), 8,15 (d, 1H, J=7,7 Hz), 7,59 (t, 1H, J=7,7 Hz), 5,27-5,17 (m, 1H), 2,40-2,28 (m, 2H), 1,84-1,77 (m, 2H), 1,44 (s, 12H), 1,34-1,24 (m, 20H), 0,85- 0,81 (t, 6H, J=6,8 Hz); ¹³C-NMR (75 MHz, THF-d₈, 25 °C): δ= 165,24, 164,33, 139,04, 137,83, 137,28, 132,81, 132,20, 131,45, 130,64, 130,02, 128,60, 127,81, 127,40, 124,38, 123,34, 123,25, 122,04, 121,90, 121,28;
   IR (KBr) v =: 2925, 2854, 2362, 2337, 1691, 1653, 1592, 1507, 1461, 1416, 1376, 1332, 1272, 1246, 1209, 1142, 1113, 1068, 966, 858, 811, 754, 674 cm⁻¹;
   UV-Vis (CHCl₃) λₘₐₓ (ε): 514 (47000), 489 nm (45000 M⁻¹ cm⁻¹);
   Fluoreszenz (CHCl₃) λₘₐₓ: 577, 546 nm;
   MS (FD): [M⁺] berechnet für C₄₃H₅₂BN₂O₄, 657,71; gefunden, 657,9 (100%).
c) Herstellung von 5,11-Bis[N-(1-heptyloctyl)-3,4-dicarbonsäureimidperylen-9-yl)tetracen IIa3
   Unter Schutzgas wurde zu 96 mg (0,25 mmol) 5,11-Dibromtetracen und 360 mg
   (0,5 mmol) N-(1-Heptyloctyl)-9-(4,4,5,5-tetramethyl-1,3,2-dioxaborolan-2-yl)perylen-3,4-dicarboximid eine Mischung aus 25 mL Toluol und 1,5 mL Ethanol gegeben. Man rührte 20 min bei 80 °C und gab danach 15 mg Tris(di-benzylidenaceton)dipalladium, 30 mg Bis(2-diphenylphosphinophenyl)ether und 1,5 mL einer 2M Lösung von K₂CO₃ in Wasser zu. Man rührte das Reaktionsgemisch 15 h bei 85 °C unter Schutzgasatmosphäre. Nach Abkühlen auf Raumtemperatur wurde die organische Phase mehrmals mit Wasser gewaschen und mit Toluol extrahiert. Das nach Entfernen der organischen Lösungsmittel im Vakuum erhaltene Rohprodukt wurde einer Säulenchromatographie an Kieselgel mit Dichlormethan als Eluens unterzogen. Man erhielt 260 mg (81 %) der Titelverbindung in Form eines tiefroten Feststoffs erhalten.
   Analytische Daten:
   R_{f}-Wert (Toluol) = 0,32;
   UV-Vis (CHCl₃) λₘₐₓ (rel. Int.): 521 (1,0), 489 (0,7);
   MS (FD): [M+], berechnet für C₉₂H₉₀N₂O₄ 1287,75; gefunden, 1288,9 (100%), M⁺.
d) Cyclodehydrierung
   Zu einer Lösung von 100 mg (0,078 mmol) 5,11-Bis-(N-(1-heptyloctyl)-perylendicarboximid-9-yl)tetracen IIa3 in 10 mL trockenem Dichlormethan gab man langsam unter Schutzatmosphäre eine Lösung von 200 mg (1,2 mmol) wasserfreiem Eisen(III)-chlorid in trockenem Nitromethan (1,5 mL). Die Mischung wurde dann 12 h bei Raumtemperatur unter Lichtausschluss gerührt und anschließend auf 50 mL Methanol gegossen. Der Niederschlag wurde abfiltriert, mit Methanol gewaschen und im Vakuum bei Raumtemperatur getrocknet.
   Man löste den trockenen Niederschlag in 1 mL trocknem Dimethylformamid und gab 2 g wasserfreies Kaliumcarbonat und 3 mL Ethanolamin zu. Man rührte das Reaktionsgemisch 4 h bei 120 °C. Nach dem Abkühlen auf Raumtemperatur gab man 25 ml Wasser zu dem Reaktionsgemisch. Der gebildete Niederschlag wurde abfiltriert, mit Wasser, Methanol und Dichlormethan gewaschen und im Vakuum getrocknet. Die Reinigung des rohen Produkts durch Säulenchromatographie an Kielsegel mit Dichlormethan als Eluens ergab 51 mg (51%) der Titelverbindung.
   Analytik:
   R_{f} = 0.72 (Dichloromethan);
   UV-Vis (CH₂Cl₂) λₘₐₓ (rel. Int.): 1001 (1,0), 539 (0,34).
   MS (FD): [M+], berechnet für C₉₂H₈₆N₂O₄ 1283,72; gefunden, 1284,4 (100%), M⁺.

## Patentansprüche

1. Dibenzorylentetracarbonsäurediimide der allgemeinen Formel I 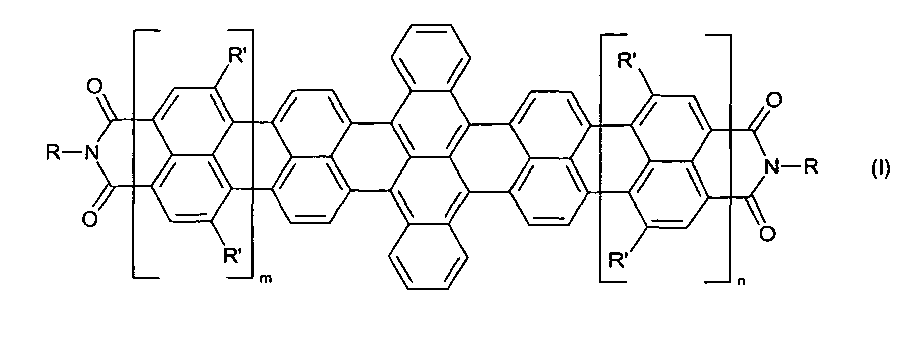 in der die Variablen folgende Bedeutung haben:
R' gleiche oder verschiedene Reste:
Wasserstoff;
Aryloxy, Arylthio, Hetaryloxy oder Hetarylthio, an das jeweils weitere gesättigte oder ungesättigte 5- bis 7-gliedrige Ringe, deren Kohlenstoffgerüst durch eine oder mehrere Gruppierungen -O-, -S-, -NR¹-, -N=CR¹-, -CO-, -SO- und/oder -SO₂- unterbrochen sein kann, anelliert sein können, wobei das gesamte Ringsystem ein- oder mehrfach durch die Reste (i), (ii), (iii), (iv) und/oder (v) substituiert sein kann:
(i) C₁-C₃₀-Alkyl, dessen Kohlenstoffkette durch eine oder mehrere Gruppierungen -O-, -S-, -NR¹-, -N=CR¹-, -C≡C-, -CR¹=CR¹-, -CO-, -SO- und/oder -SO₂- unterbrochen sein kann und das ein- oder mehrfach substituiert sein kann durch: C₁-C₁₂-Alkoxy, C₁-C₆-Alkylthio, -C≡CR¹, -CR¹=CR¹₂, Hydroxy, Mercapto, Halogen, Cyano, Nitro, -NR²R³, -NR²COR³, -CONR²R³, -SO₂NR²R³, -COOR², -SO₃R², Aryl und/oder gesättigtes oder ungesättigtes C₄-C₇-Cycloalkyl, dessen Kohlenstoffgerüst durch eine oder mehrere Gruppierungen -O-, -S-, -NR¹-, -N=CR¹-, -CR¹=CR¹-, -CO-, -SO- und/oder -SO₂- unterbrochen sein kann, wobei die Aryl- und Cycloalkylreste jeweils ein- oder mehrfach durch C₁-C₁₈-Alkyl und/oder die vorstehenden, als Substituenten für Alkyl genannten Reste substituiert sein können;
(ii) C₃-Ca-Cycloalkyl, dessen Kohlenstoffgerüst durch eine oder mehrere Gruppierungen -O-, -S-, -NR¹-, -N=CR¹-, -CR¹=CR¹-, -CO-, -SO- und/oder -SO₂- unterbrochen sein kann und an das weitere gesättigte oder ungesättigte 5- bis 7-gliedrige Ringe, deren Kohlenstoffgerüst durch eine oder mehrere Gruppierungen -O-, -S-, -NR¹-, -N=CR¹-, -CR¹=CR¹-, -CO-, -SO- und/oder -SO₂- unterbrochen sein kann, anelliert sein können, wobei das gesamte Ringsystem ein- oder mehrfach substituiert sein kann durch: C₁-C₁₈-Alkyl, C₁-C₁₂-Alkoxy, C₁-C₆-Alkylthio, -C≡CR¹, -CR¹=CR¹², Hydroxy, Mercapto, Halogen, Cyano, Nitro, -NR²R³, -NR²COR³, -CONR²R³, -SO₂NR²R³, -COOR² und/oder -SO₃R²;
(iii) Aryl oder Hetaryl, an das weitere gesättigte oder ungesättigte 5- bis 7-gliedrige Ringe, deren Kohlenstoffgerüst durch eine oder mehrere Gruppierungen -O-, -S-, -NR¹-, -N=CR¹-, -CR¹=CR¹-, -CO-, -SO- und/oder -SO₂- unterbrochen sein kann, anelliert sein können, wobei das gesamte Ringsystem ein- oder mehrfach substituiert sein kann durch: C₁-C₁₈-Alkyl, C₁-C₁₂-Alkoxy, C₁-C₆-Alkylthio, -C≡CR¹, -CR¹=CR², Hydroxy, Mercapto, Halogen, Cyano, Nitro, -NR²R³, -NR²COR³, -CONR²R³, -SO₂NR²R³, -COOR², -SO₃R², Aryl und/oder Hetaryl, das jeweils ein- oder mehrfach durch C₁-C₁₈-Alkyl, C₁-C₁₂-Alkoxy, Hydroxy, Mercapto, Halogen, Cyano, Nitro, -NR²R³, -NR²COR³, -CONR²R³, -SO₂NR²R³, -COOR² und/oder -SO₃R² substituiert sein kann;
(iv) ein Rest -U-Aryl, der ein- oder mehrfach durch die vorstehenden, als Substituenten für die Arylreste (iii) genannten Reste substituiert sein kann, wobei U eine Gruppierung -O-, -S-, -NR¹-, -CO-, -SO- oder -SO₂- bedeutet;
(v) C₁-C₁₂-Alkoxy, C₁-C₆-Alkylthio, -C≡CR¹, -CR¹=CR², Hydroxy, Mercapto, Halogen, Cyano, Nitro, -NR²R³, -NR²COR³, -CONR²R³, -SO₂NR²R³, -COOR² oder -SO₃R²;
R gleiche oder verschiedene Reste:
Wasserstoff;
C₁-C₃₀-Alkyl, dessen Kohlenstoffkette durch eine oder mehrere Gruppierungen -O-, -S-, -NR¹-, -N=CR¹-, -C≡C-, -CR¹=CR¹-, -CO-, -SO- und/oder -SO₂- unterbrochen sein kann und das ein- oder mehrfach durch die als Substituenten für die Reste R' genannten Reste (ii), (iii), (iv) und/oder (v) substituiert sein kann;
C₃-C₈-Cy_{d}oalkyl, an das weitere gesättigte oder ungesättigte 5- bis 7-gliedrige Ringe, deren Kohlenstoffgerüst durch eine oder mehrere Gruppierungen -O-, -S-, -NR¹-, -N=CR¹-, -CR¹=CR¹⁻, -CO-, -SO- und/oder -SO₂- unterbrochen sein kann, anelliert sein können, wobei das gesamte Ringsystem ein- oder mehrfach durch die als Substituenten für die Reste R' genannten Reste (i), (ii), (iii), (iv) und/oder (v) substituiert sein kann;
Aryl oder Hetaryl, an das jeweils weitere gesättigte oder ungesättigte 5-bis 7-gliedrige Ringe, deren Kohlenstoffgerüst durch eine oder mehrere Gruppierungen -O-, -S-, -NR¹-, -N=CR¹-, -CR¹=CR¹-, -CO-, -SO- und/oder -SO₂- unterbrochen sein kann, anelliert sein können, wobei das gesamte Ringsystem ein- oder mehrfach substituiert sein kann durch die als Sub- stituenten für die Reste R' genannten Reste (i), (ii), (iii), (iv), (v) und/oder Aryl- und/oder Hetarylazo, das jeweils ein- oder mehrfach durch C₁-C₁₀-Alkyl, C₁-C₆-Alkoxy und/oder Cyano substituiert sein kann;
R¹ Wasserstoff oder C₁-C₁₈-Alkyl, wobei die Reste R¹ gleich oder verschieden sein können, wenn sie mehrfach auftreten;
R², R³ unabhängig voneinander:
Wasserstoff;
C₁-C₁₈-Alkyl, dessen Kohlenstoffkette durch eine oder mehrere Gruppierungen -O-, -S-, -CO-, -SO- und/oder -SO₂- unterbrochen sein kann und das ein- oder mehrfach durch C₁-C₁₂-Alkoxy, C₁-C₆-Alkylthio, Hydroxy, Mercapto, Halogen, Cyano, Nitro, Aryl und/oder -COOR¹ substituiert sein kann;
Aryl oder Hetaryl, an das jeweils weitere gesättigte oder ungesättigte 5-bis 7-gliedrige Ringe, deren Kohlenstoffgerüst durch eine oder mehrere Gruppierungen -O-, -S-, -CO- und/oder -SO₂- unterbrochen sein kann, anelliert sein können, wobei das gesamte Ringsystem ein- oder mehrfach durch C₁-C₁₂-Alkyl und/oder die vorstehenden, als Substituenten für Alkyl genannten Reste substituiert sein kann;
C₃-C₈-Cycloalkyl, an das weitere gesättigte oder ungesättigte 5- bis 7-gliedrige Ringe, deren Kohlenstoffgerüst durch eine oder mehrere Gruppierungen -O-, -S-, -NR¹-, -N=CR¹-, -CR¹=CR¹-, -CO-, -SO- und/oder -SO₂- unterbrochen sein kann, anelliert sein können, wobei das gesamte Ringsystem ein- oder mehrfach durch C₁-C₁₂-Alkyl und/oder die vorstehenden, als Substituenten für Alkyl genannten Reste substituiert sein kann;
m, n unabhängig voneinander 0 oder 1.

2. Dibenzorylentetracarbonsäurediimide der allgemeinen Formel I nach Anspruch 1, in der die Variablen folgende Bedeutung haben:
R' gleiche oder verschiedene Reste:
Wasserstoff;
Phenoxy oder Thiophenoxy, das jeweils ein- oder mehrfach durch gleiche oder verschiedene Reste (i), (ii), (iii), (iv) und/oder (v) substituiert sein kann:
(i) C₁-C₃₀-Alkyl, dessen Kohlenstoffkette durch eine oder mehrere Gruppierungen -O-, -S-, -NR¹-, -C≡C-, -CR¹=CR¹- und/oder -CO- und/oder -SO₂- unterbrochen sein kann und das ein- oder mehrfach substituiert sein kann durch: C₁-C₁₂-Alkoxy, Hydroxy, Halogen, Cyano und/oder Aryl, das ein- oder mehrfach durch C₁-C₁₈-Alkyl und/oder C₁-C₁-Alkoxy substituiert sein kann;
(ii) C₃-C₈-Cycloalkyl, dessen Kohlenstoffgerüst durch eine oder mehrere Gruppierungen -O-, -S-, -NR¹-, -CR¹=CR¹- und/oder -CO- unterbrochen sein kann und das ein- oder mehrfach durch C₁-C₁₈-Alkyl, C₁-C₁₂-Alkoxy und/oder C₁-C₆-Alkylthio substituiert sein kann;
(iii) Aryl oder Hetaryl, an das jeweils weitere 5- bis 7-gliedrige gesättigte oder ungesättigte Ringe, deren Kohlenstoffgerüst durch eine oder mehrere Gruppierungen -O-, -S-, -NR¹-, -N=CR¹-, -CR¹=CR¹-, -CO-, -SO- und/oder -SO₂- unterbrochen sein kann, anelliert sein können, wobei das gesamte Ringsystem ein- oder mehrfach substituiert sein kann durch: C₁-C₁₈-Alkyl, C₁-C₁₂-Alkoxy, -C≡CR¹, -CR¹=CR¹², Hydroxy, Halogen, Cyano, -NR²R³, -NR²COR³, -CONR²R³, -SO₂NR²R³, -COOR², -SO₃R², Aryl und/oder Hetaryl, das jeweils ein- oder mehrfach durch C₁-C₁₈-Alkyl, C₁-C₁₈-Alkoxy und/oder Cyano substituiert sein kann;
(iv) ein Rest -U-Aryl, der ein- oder mehrfach durch die vorstehenden, als Substituenten für die Arylreste (iii) genannten Reste substituiert sein kann, wobei U eine Gruppierung -O-, -S-, -NR¹-, -CO-, -SO- oder -SO₂- bedeutet;
(v) C₁-C₁₂-Alkoxy, C₁-C₆-Alkylthio, -C≡CR¹, -CR¹=CR¹², Hydroxy, Mercapto, Halogen, Cyano, Nitro, -NR²R³, -NR²COR³, -CONR²R³, -SO₂NR²R³, -COLOR² oder -SO₃R²;
R gleiche oder verschiedene Reste:
C₁-C₃₀-Alkyl, dessen Kohlenstoffkette durch eine oder mehrere Gruppierungen -O- und/oder -CO- unterbrochen sein kann und das ein- oder mehrfach substituiert sein kann durch: C₁-C₆-Alkoxy, Cyano und/oder Aryl, das ein- oder mehrfach durch C₁-C₁₈-Alkyl oder C₁-C₆-Alkoxy substituiert sein kann;
C₅-C₈-Cy_{d}oalkyl, das ein- oder mehrfach durch C₁-C₆-Alkyl substituiert sein kann;
Phenyl, Naphthyl, Pyridyl oder Pyrimidyl, das jeweils ein- oder mehrfach substituiert sein kann durch: C₁-C₁₈-Alkyl, C₁-C₆-Alkoxy, Halogen, Cyano, Nitro, -CONR²R³, -SO₂NR²R³ und/oder Phenyl- und/oder Naphthylazo, das jeweils ein- oder mehrfach durch C₁₀-C₁₀-Alkyl, C₁-C₆-Alkoxy und/oder Cyano substituiert sein kann;
R¹ Wasserstoff oder C₁-C₆-Alkyl;
R², R³ unabhängig voneinander:
Wasserstoff;
C₁-C₁₈-Alkyl, das ein- oder mehrfach durch C₁-C₆-Alkoxy, Hydroxy, Halogen und/oder Cyano substituiert sein kann; Aryl oder Hetaryl, das jeweils ein- oder mehrfach durch C₁-C₆-Alkyl und/oder die vorstehenden, als Substituenten für Alkyl genannten Reste substituiert sein kann;
m, n 0 oder 1.

3. Dibenzorylentetracarbonsäurediimide der allgemeinen Formel I nach Anspruch 1, in der die Variablen folgende Bedeutung haben:
R' gleiche Reste:
Wasserstoff;
Phenoxy, das ein- oder mehrfach durch gleiche oder verschiedene Reste (i), (ii), (iii). (iv) und/oder (v) substituiert sein kann:
(i) C₁-C₁₈-Alkyl, dessen Kohlenstoffkette durch eine oder mehrere Gruppierungen -O-, -S-, -NR¹- und/oder -CO- unterbrochen sein kann und das ein- oder mehrfach durch C₁-C₁₂-Alkoxy, Hydroxy und/oder Halogen substituiert sein kann;
(ii) C₃-C₈-Cycloalkyl, das ein- oder mehrfach durch C₁-C₁₈-Alkyl und/oder C₁-C₁₂-Alkoxy substituiert sein kann;
(iii) Aryl oder Hetaryl, das ein- oder mehrfach durch C₁-C₁₈-Alkyl, C₁-C₁₂- Alkoxy, Hydroxy und/oder Halogen substituiert sein kann;
(iv) ein Rest -U-Aryl, der ein- oder mehrfach durch die vorstehenden, als Substituenten für die Arylreste (iii) genannten Reste substituiert sein kann, wobei U eine Gruppierung -O-, -S- oder -NR¹⁻ bedeutet;
(v) C₁-C₁₂-Alkoxy, Hydroxy, Halogen oder Cyano;
R gleiche Reste:
C₁-C₃₀-Alkyl, dessen Kohlenstoffkette durch eine oder mehrere Gruppierungen -O- und/oder -CO- unterbrochen sein kann und das ein- oder mehrfach substituiert sein kann durch: C₁-C₆-Alkoxy, Cyano und/oder Aryl, das ein- oder mehrfach durch C₁-C₁₈-Alkyl oder C₁-Cₛ-Alkoxy substituiert sein kann;
C₅-C₈-Cycloalkyl, das ein- oder mehrfach durch C₁-C₆-Alkyl substituiert sein kann;
Phenyl, Naphthyl, Pyridyl oder Pyrimidyl, das jeweils ein- oder mehrfach substituiert sein kann durch: C₁-C₁₈-Alkyl, C₁-C₆-Alkoxy, Halogen, Cyano, Nitro, -CONR²R³, -S0₂NR²R³ und/oder Phenyl- und/oder Naphthylazo, das jeweils ein- oder mehrfach durch C₁-C₁₀-Alkyl, C₁-C₆-Alkoxy und/oder Cyano substituiert sein kann;
R¹ Wasserstoff oder C₁-C₆,-Alkyl;
R², R³ unabhängig voneinander:
Wasserstoff;
C₁-C₁₈-Alkyl, das ein- oder mehrfach durch C₁-C₆-Alkoxy, Hydroxy, Halogen und/oder Cyano substituiert sein kann;
Aryl oder Hetaryl, das jeweils ein- oder mehrfach durch C₁-C₆-Alkyl und/oder die vorstehenden, als Substituenten für Alkyl genannten Reste substituiert sein kann;
m, n 1.

4. Dibenzorylentetracarbonsäurediimide nach einem der vorhergehenden Ansprüche, wobei die Reste R unabhängig voneinander ausgewählt sind unter Gruppen der Formeln II.1 bis II.5:
#-(A)ₚ-C(Ri)ₓ (II.1) 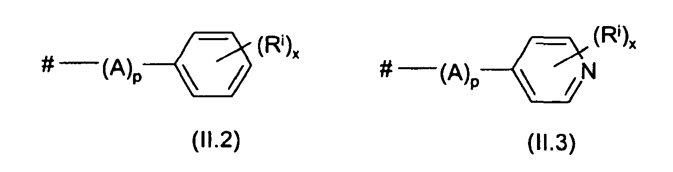 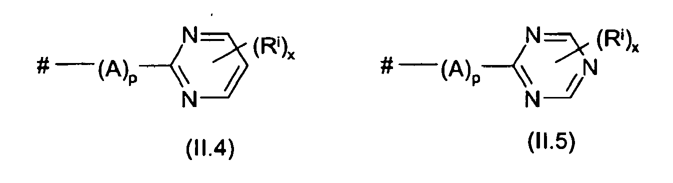
worin
# für die Verknüpfungsstelle zum Imidstickstoffatom steht,
p für 0 oder 1 steht,
x in den Verbindungen der Formel II.1 für 2 oder 3 steht, in den Verbindungen der Formeln II.2, II3 und II.4 für 1, 2 oder 3 steht und in den Verbindungen der Formel II.5 für 1 oder 2 steht,
A soweit vorhanden, für eine C₁-C₁₀-Alkylengruppe steht, die durch eine oder mehrere nicht benachbarte Gruppen, die ausgewählt sind unter -O- und -S-, unterbrochen sein kann,
wobei für den Fall, dass in den Verbindungen der Formel II.1 x für 2 steht, das Kohlenstoffatom, das die Reste Rⁱ trägt, zusätzlich ein H-Atom trägt,
die Reste Rⁱ jeweils unabhängig voneinander ausgewählt sind unter C₁-C₃₀-Alkyl, welche durch ein oder mehrere nicht benachbarte Sauerstoffatom(e) unterbrochen sein können, wobei in den Verbindungen der Formel II.1 wenigstens einer der Reste Rⁱ auch für C₁-C₃₀-Alkyloxy oder C₁-C₃₀-Alkylthio stehen kann.

5. Dibenzorylentetracarbonsäurediimide nach einem der Ansprüche 1 bis 4, wobei die Reste R' beide für Wasserstoff stehen.

6. Dibenzorylentetracarbonsäurediimide nach einem der Ansprüche 1 bis 6, wobei die Reste R' ausgewählt sind unter Resten der Formel 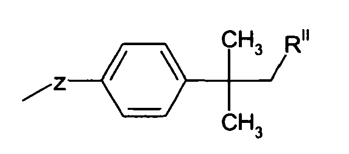 worin
Z für O oder S steht, und
R^{II} ausgewählt ist unter Wasserstoff, C₁-C₈-Alkyl, dessen Kohlenstoffkette durch eine oder mehrere Gruppierungen -O-, -S-, -NR¹⁻, -C(=O)- und/oder -SO₂- unterbrochen sein kann und das ein- oder mehrfach durch C₁-C₆-Alkoxy und/oder einen über eine Stickstoffatom gebundenen, 5- bis 7-gliedrigen heterocyclischen Rest, der weitere Heteroatome enthalten und aromatisch sein kann, substituiert sein kann, und
C₅-C₈-Cycloalkyl, dessen Kohlenstoffgerüst durch eine oder mehrere Grup- pierungen -O-, -S-, -NR¹-, -C(=O)-und/oder-SO2- unterbrochen sein kann und das ein- oder mehrfach durch C₁-C₆-Alkyl substituiert sein kann, wobei R¹ für Wasserstoff oder C₁-C₆-Alkyl steht.

7. Verfahren zur Herstellung von Dibenzorylentetracarbonsäurediimiden der allgemeinen Formel Ia 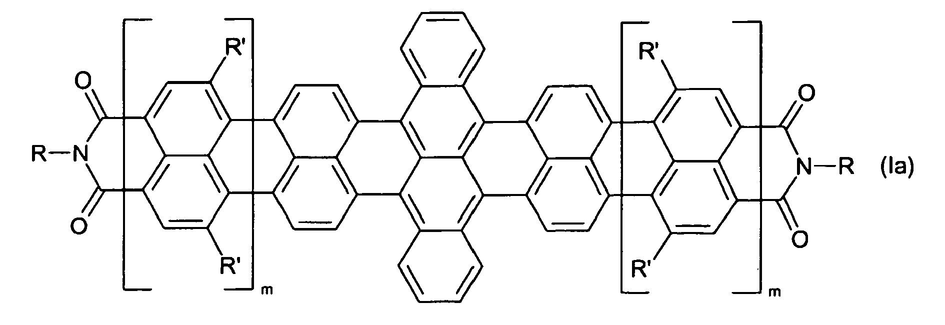 in der R, R' und m die in Anspruch 1 angegebene Bedeutung haben, **dadurch gekennzeichnet, dass** man
a) ein peri-(Dioxaborolan-2-yl)rylendicarbonsäureimid der allgemeinen Formel IIa 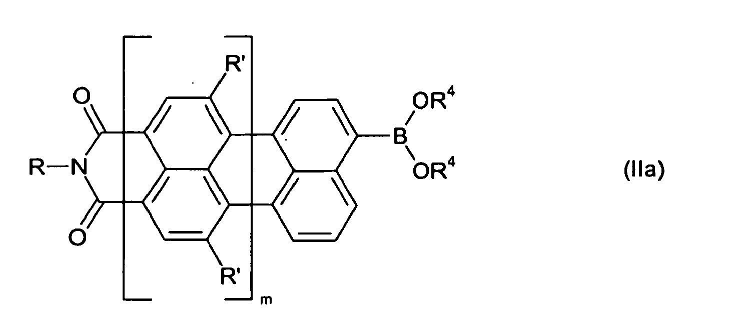 worin R⁴ gleich oder verschieden sind und unabhängig voneinander für Wasserstoff, C₁-C₃₀-Alkyl, C₅-C₈-Cycloalkyl, Aryl oder Heteroaryl stehen, und wobei zwei am selben Boratom gebundene Reste OR⁴ gemeinsam auch für -OCH₂CH₂O- stehen können, worin 1, 2, 3 oder 4 Wasserstoffatome auch durch C₁-C₃₀-Alkyl-, C₅-C₈-Cycloalkyl-, Aryl- oder Heteroarylgruppen ersetzt sein können,
in Gegenwart eines organischen Lösungsmittels, gewünschtenfalls im Gemisch mit Wasser, sowie eines Übergangsmetallkatalysators und einer Base einer Suzuki-Kupplungsreaktion mit 5,11-Dibromtetracen der Formel III. 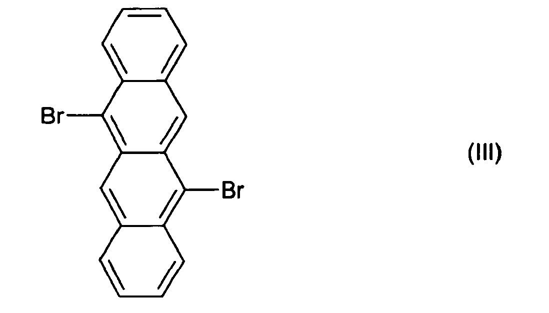 unterzieht,
b) das in Schritt a) gebildete Tetracen-5,1 1-bis(rylendicarbonsäureimid) der allgemeinen Formel IVa 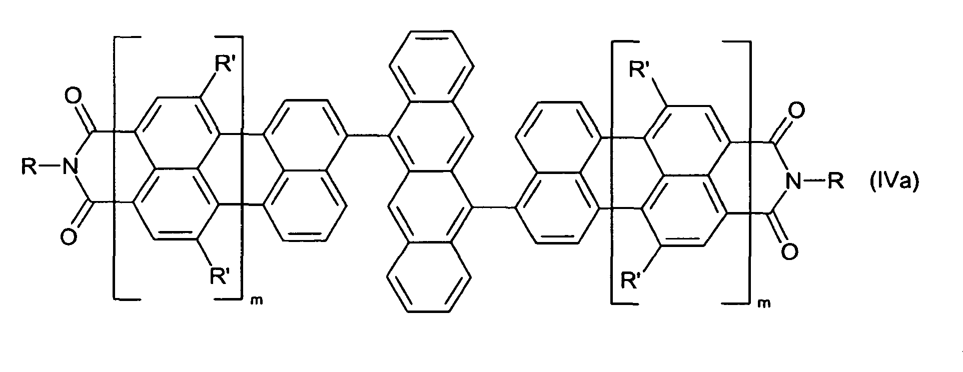 einer ersten Cyclodehydrierung in Gegenwart eines inerten organischen Lösungsmittels und einer Lewis-Säure unterzieht und
c) das in Schritt b) gebildete Bisrylenderivat der allgemeinen Formel Va 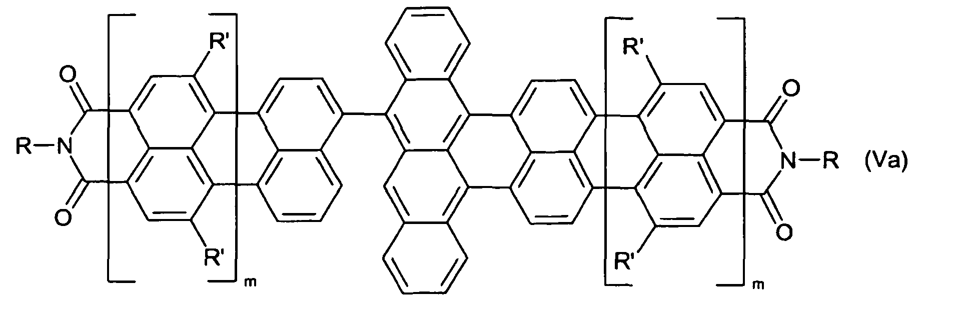 in einem Hydroxy- und Aminofunktionen aufweisenden und eine im Wesentlichen ungelöste Base enthaltenden organischen Reaktionsmedium weiter zum Dibenzorylentetracarbonsäurediimid la cyclodehydriert.

8. Verfahren zur Herstellung von Dibenzorylentetracarbonsäurediimiden der allgemeinen Formel Ib 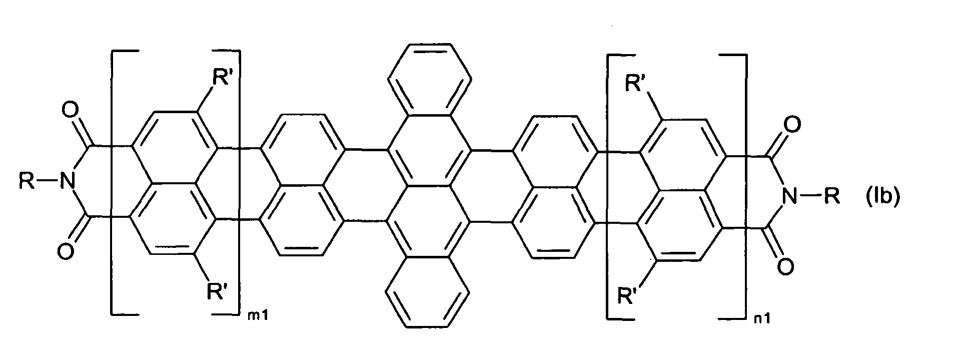 in der R und R' die in Anspruch 1 angegebene Bedeutung haben, m1 und n1 voneinander verschieden sind und 0 oder 1 bedeuten, **dadurch gekennzeichnet, dass** man
a1) ein peri-(Dioxaborolan-2-yl)rylendicarbonsäureimid der allgemeinen Formel IIb1 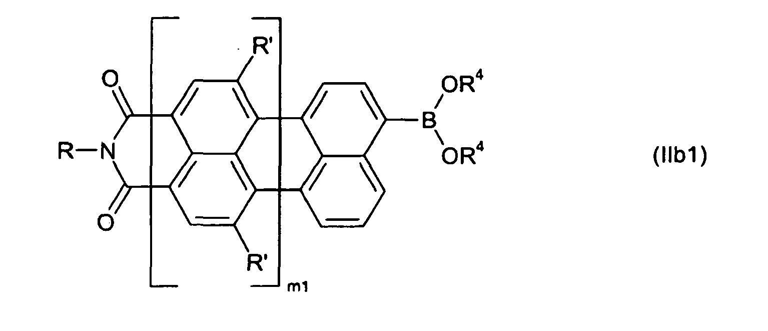 worin R⁴ gleich oder verschieden sind und unabhängig voneinander für Wasserstoff, C,-C₃₀-Alkyl, C₅-C₈-Cycloalkyl, Aryl oder Heteroaryl stehen, und wobei zwei am selben Boratom gebundene Reste OR⁴ gemeinsam auch für -OCH₂CH₂O- stehen können, worin 1, 2, 3 oder 4 Wasserstoffatome auch durch C₁-C₃₀-Alkyl-, C₅-C₈-Cycloalkyl-, Aryl- oder Heteroarylgruppen ersetzt sein können,
einer ersten Suzuki-Kupplungsreaktion mit 5,11-Dibromtetracen (III) und
a2) das in Schritt a1) gebildete 5-Bromtetracen-11-rylendicarbonsäureimid der
allgemeinen Formal III' 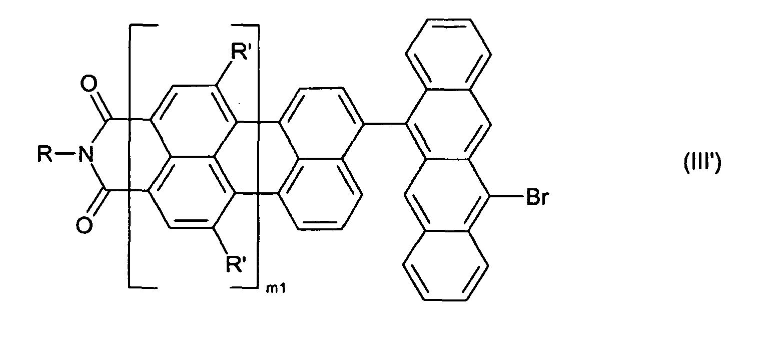 einer zweiten Suzuki-Kupplungsreaktion mit einem peri-(Dioxaborolan-2-yl)rylendicarbonsäureimid der allgemeinen Formel IIb2 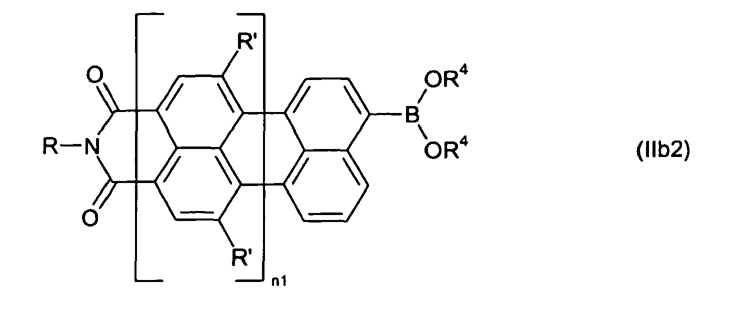 in Gegenwart eines organischen Lösungsmittels, gewünschtenfalls im Gemisch mit Wasser, sowie eines Übergangsmetallkatalysators und einer Base unterzieht,
b) das in Schritt a2) gebildete Tetracen-5,11-bis(rylendicarbonsäureimid) der
allgemeinen Formel IVb 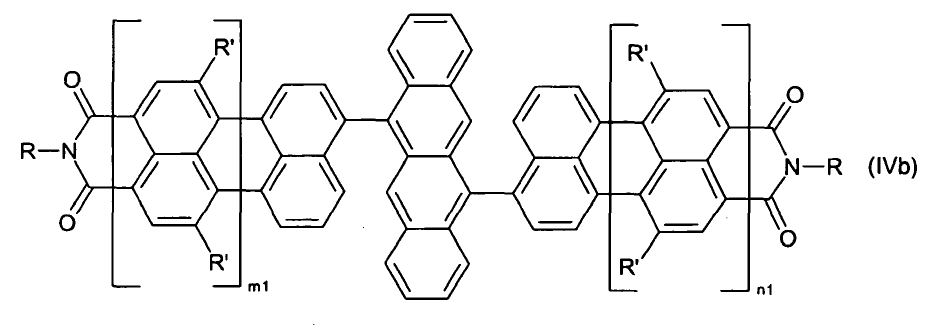 einer ersten Cyclodehydrierung in Gegenwart eines inerten organischen Lösungsmittels und einer Lewis-Säure unterzieht und
c) das in Schritt b) gebildete Bisrylenderivat der allgemeinen Formel Vb 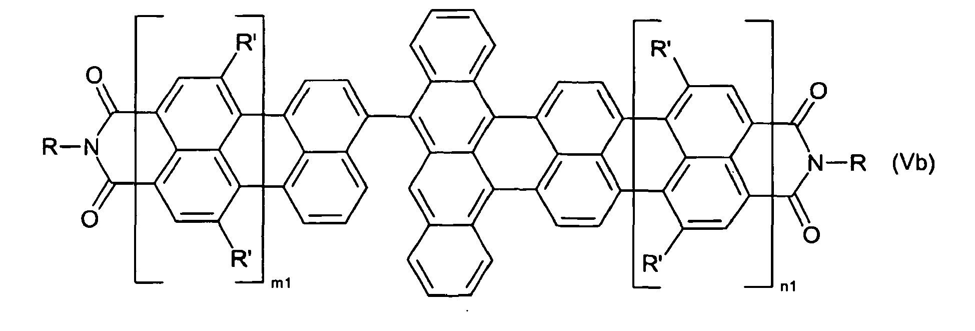 in einem Hydroxy- und Aminofunktionen aufweisenden und eine im Wesentlichen ungelöste Base enthaltenden organischen Reaktionsmedium weiter zum Dibenzorylentetracarbonsäurediimid Ib cyclodehydriert.

9. Tetracen-5,11-bis(rylendicarbonsäureimide) der allgemeinen Formel IV 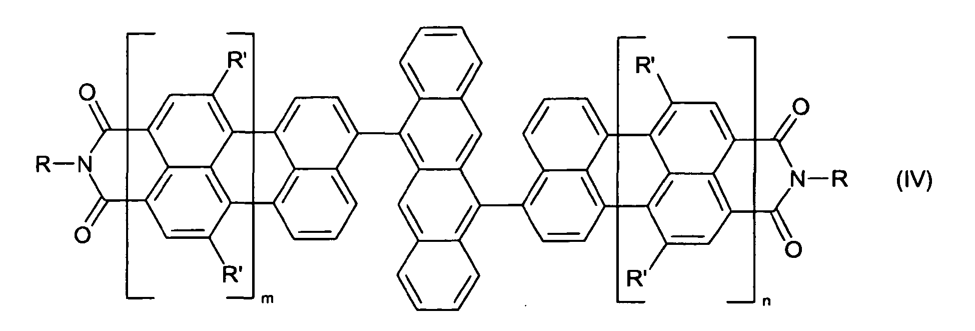 in der die Variablen folgende Bedeutung haben:
R' gleiche oder verschiedene Reste:
Wasserstoff;
Aryloxy, Arylthio, Hetaryloxy oder Hetarylthio, an das jeweils weitere gesättigte oder ungesättigte 5- bis 7-gliedrige Ringe, deren Kohlenstoffgerüst durch eine oder mehrere Gruppierungen -O-, -S-, -NR¹-, -N=CR¹-, -CO-, -SO- und/oder -SO₂- unterbrochen sein kann, anelliert sein können, wobei das gesamte Ringsystem ein- oder mehrfach durch die Reste (i), (ii), (iii), (iv) und/oder (v) substituiert sein kann:
(i) C₁-C₃₀-Alkyl, dessen Kohlenstoffkette durch eine oder mehrere Gruppierungen -O-, -S-, -NR₁-, -N=CR¹-, -C≡C-, -CR¹=CR¹-, -CO-, -SO- und/oder -SO₂- unterbrochen sein kann und das ein- oder mehrfach substituiert sein kann durch: C₁-C₁₂-Alkoxy, C₁-C₆-Alkylthio, -C≡CR¹, -CR¹=CR¹₂, Hydroxy, Mercapto, Halogen, Cyano, Nitro, -NR²R³, -NR²COR³, -CONR²R³, -SO₂NR²R³, -COOR², -SO₃R², Aryl und/oder gesättigtes oder ungesättigtes C₄-C₇-Cycloalkyl, dessen Kohlenstoffgerüst durch eine oder mehrere Gruppierungen -O-, -S-, -NR¹-, -N=CR¹-, -CR¹=CR¹-, -CO-, -SO- und/oder -SO₂- unterbrochen sein kann, wobei die Aryl- und Cycloalkylreste jeweils ein- oder mehrfach durch C₁-C₁₈-Alkyl und/oder die vorstehenden, als Substituenten für Alkyl genannten Reste substituiert sein können;
(ii) C₃-C₈-Cycloalkyl, dessen Kohlenstoffgerüst durch eine oder mehrere Gruppierungen -O-, -S-, -NR¹-, -N=CR¹-, -CR¹=CR¹-, -CO-, -SO- und/oder -SO₂- unterbrochen sein kann und an das weitere gesättigte oder unge- sättigte 5- bis 7-gliedrige Ringe, deren Kohlenstoffgerüst durch eine oder mehrere Gruppierungen -O-, -S-, -NR¹-, -N=CR¹-, -CR¹=CR¹-, -CO-, -SO- und/oder -SO₂- unterbrochen sein kann, anelliert sein können, wobei das gesamte Ringsystem ein- oder mehrfach substituiert sein kann durch: C₁-C₁₈-Alkyl, C₁-C₁₂-Alkoxy, C₁-C₆-Alkylthio, -C≡CR¹, -CR¹=CR¹₂, Hydroxy, Mercapto, Halogen, Cyano, Nitro, -NR²R³, -NR²COR³, -CONR²R³, -SO₂NR²R³, -COOR² und/oder -SO₃R²;
(iii) Aryl oder Hetaryl, an das weitere gesättigte oder ungesättigte 5- bis 7-gliedrige Ringe, deren Kohlenstoffgerüst durch eine oder mehrere Gruppierungen -O-, -S-, -NR¹-, -N=CR¹-, -CR¹=CR¹-, -CO-, -SO- und/oder -SO₂- unterbrochen sein kann, anelliert sein können, wobei das gesamte Ringsystem ein- oder mehrfach substituiert sein kann durch: C₁-C₁₈-Alkyl, C₁-C₁₂-Alkoxy, C₁-C₆-Alkylthio, -C≡CR¹, -CR¹=CR¹₂, Hydroxy, Mercapto, Halogen, Cyano, Nitro, -NR²R³, -NR²COR³, -CONR²R3, -SO₂NR²R³, -COOR², -SO₃R², Aryl und/oder Hetaryl, das jeweils ein- oder mehrfach durch C₁-C₁₈-Alkyl, C₁-C₁₂-Alkoxy, Hydroxy, Mercapto, Halogen, Cyano, Nitro, -NR²R³, -NR²COR³, -CONR²R³, -SO₂NR²R³, -COOR² und/oder -SO₃R² substituiert sein kann;
(iv) ein Rest -U-Aryl, der ein- oder mehrfach durch die vorstehenden, als Substituenten für die Arylreste (iii) genannten Reste substituiert sein kann, wobei U eine Gruppierung -O-, -S-, -NR¹-, -CO-, -SO- oder -SO₂- bedeutet;
(v) C₁-C₁₂-Alkoxy, C₁-C₆-Alkylthio, -C≡CR¹, -CR¹=CR¹₂, Hydroxy, Mercapto, Halogen, Cyano, Nitro, -NR²R³, -NR²COR³, -CONR²R³, -SO₂NR²R³, -COOR² oder -SO₃R²;
R gleiche oder verschiedene Reste:
Wasserstoff;
C₁-C₃₀-Alkyl, dessen Kohlenstoffkette durch eine oder mehrere Gruppierungen -O-, -S-, -NR¹-, -N=CR¹-, -C≡C-, -CR¹=CR¹-, -CO-, -SO- und/oder -SO₂- unterbrochen sein kann und das ein- oder mehrfach durch die als Substituenten für die Reste R' genannten Reste (ii), (iii), (iv) und/oder (v) substituiert sein kann;
C₃-C₈-Cycloalkyl, an das weitere gesättigte oder ungesättigte 5- bis 7-gliedrige Ringe, deren Kohlenstoffgerüst durch eine oder mehrere Gruppierungen -O-, -S-, -NR¹-, -N=CR¹-, -CR¹=CR¹-, -CO-, -SO- und/oder -SO₂- unterbrochen sein kann, anelliert sein können, wobei das gesamte Ringsystem ein- oder mehrfach durch die als Substituenten für die Reste R' genannten Reste (i), (ii), (iii), (iv) und/oder (v) substituiert sein kann;
Aryl oder Hetaryl, an das jeweils weitere gesättigte oder ungesättigte 5-bis 7-gliedrige Ringe, deren Kohlenstoffgerüst durch eine oder mehrere Gruppierungen -O-, -S-, -NR¹-, -N=CR¹-, -CR¹=CR¹-, -CO-, -SO- und/oder -SO₂- unterbrochen sein kann, anelliert sein können, wobei das gesamte
R¹ Ringsystem ein- oder mehrfach substituiert sein kann durch die als Substituenten für die Reste R' genannten Reste (i), (ii), (iii), (iv), (v) und/oder Aryl- und/oder Hetarylazo, das jeweils ein- oder mehrfach durch C₁-C₁₀-Alkyl, C₁-C₆-Alkoxy und/oder Cyano substituiert sein kann; Wasserstoff oder C₁-C₁₈-Alkyl, wobei die Reste R¹ gleich oder verschieden sein können, wenn sie mehrfach auftreten;
R², R³ unabhängig voneinander:
Wasserstoff;
C₁-C₁₈-Alkyl, dessen Kohlenstoffkette durch eine oder mehrere Gruppierungen -O-, -S-, -CO-, -SO- und/oder -SO₂- unterbrochen sein kann und das ein- oder mehrfach durch C₁-C₁₂-Alkoxy, C₁-C₆-Alkylthio, Hydroxy, Mercapto, Halogen, Cyano, Nitro, Aryl, und/oder -COOR¹ substituiert sein kann;
Aryl oder Hetaryl, an das jeweils weitere gesättigte oder ungesättigte 5-bis 7-gliedrige Ringe, deren Kohlenstoffgerüst durch eine oder mehrere Gruppierungen -O-, -S-, -CO- und/oder -SO₂- unterbrochen sein kann, anelliert sein können, wobei das gesamte Ringsystem ein- oder mehrfach durch C₁-C₁₂-Alkyl und/oder die vorstehenden, als Substituenten für Alkyl genannten Reste substituiert sein kann;
C₃-C₈-Cycloalkyl, an das weitere gesättigte oder ungesättigte 5- bis 7-gliedrige Ringe, deren Kohlenstoffgerüst durch eine oder mehrere Gruppierungen -O-, -S-, -NR¹-, -NR^{1a}, -N=CR¹-, -CR¹=CR¹-, -CO-, -SO- und/oder -SO₂- unterbrochen sein kann, anelliert sein können, wobei das gesamte Ringsystem ein- oder mehrfach durch C₁-C₁₂-Alkyl und/oder die vorstehenden, als Substituenten für Alkyl genannten Reste substituiert sein kann;
m, n unabhängig voneinander 0 oder 1.

10. Bisrylenderivate der allgemeinen Formel V 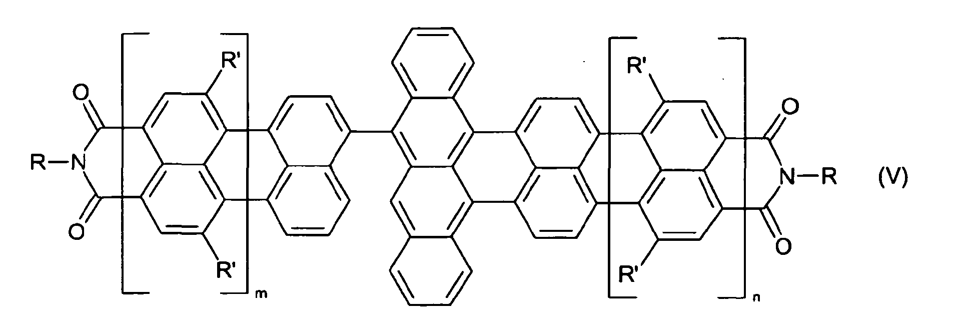 in der die Variablen folgende Bedeutung haben:
R' gleiche oder verschiedene Reste:
Wasserstoff;
Aryloxy, Arylthio, Hetaryloxy oder Hetarylthio, an das jeweils weitere gesättigte oder ungesättigte 5- bis 7-gliedrige Ringe, deren Kohlenstoffgerüst durch eine oder mehrere Gruppierungen -O-, -S-, -NR¹-, -N=CR¹-, -CO-, -SO- und/oder -SO₂- unterbrochen sein kann, anelliert sein können, wobei das gesamte Ringsystem ein- oder mehrfach durch die Reste (i), (ii), (iii), (iv) und/oder (v) substituiert sein kann:
(i) C₁-C₃₀-Alkyl, dessen Kohlenstoffkette durch eine oder mehrere Gruppierungen -O-, -S-, -NR¹-, -N=CR¹-, -C≡C-, -CR¹=CR¹-, -CO-, -SO- und/oder -SO₂- unterbrochen sein kann und das ein- oder mehrfach substituiert sein kann durch: C₁-C₁₂-Alkoxy, C₁-C₆-Alkylthio, -C≡CR¹, -CR¹=CR¹₂, Hydroxy, Mercapto, Halogen, Cyano, Nitro, -NR²R³, -NR²COR³, -CONR²R³, -SO₂NR²R³, -COOR², -SO₃R², Aryl und/oder gesättigtes oder ungesättigtes C₄-C₇-Cycloalkyl, dessen Kohlenstoffgerüst durch eine oder mehrere Gruppierungen -O-, -S-, -NR¹-, -N=CR¹-, -CR¹=CR¹-, -CO-, -SO- und/oder -SO₂- unterbrochen sein kann, wobei die Aryl- und Cycloalkylreste jeweils ein- oder mehrfach durch C₁-C₁₈-Alkyl und/oder die vorstehenden, als Substituenten für Alkyl genannten Reste substituiert sein können;
(ii) C₃-C₈-Cycloalkyl, dessen Kohlenstoffgerüst durch eine oder mehrere Gruppierungen -O-, -S-, -NR¹-, -N=CR¹-, -CR¹=CR¹-, -CO-, -SO- und/oder -SO₂- unterbrochen sein kann und an das weitere gesättigte oder ungesättigte 5- bis 7-gliedrige Ringe, deren Kohlenstoffgerüst durch eine oder mehrere Gruppierungen -O-, -S-, -NR¹-, -N=CR¹-, -CR¹=CR¹-, -CO-, -SO- und/oder -SO₂- unterbrochen sein kann, anelliert sein können, wobei das gesamte Ringsystem ein- oder mehrfach substituiert sein kann durch: C₁-C₁₈-Alkyl, C₁-C₁₂-Alkoxy, C₁-C₆-Alkylthio, -C≡CR¹, -CR¹=CR¹₂, Hydroxy, Mercapto, Halogen, Cyano, Nitro, -NR²R³, -NR²COR³, -CONR²R³, -SO₂NR²R³, -COOR² und/oder -SO₃R²;
(iii) Aryl oder Hetaryl, an das weitere gesättigte oder ungesättigte 5- bis 7-gliedrige Ringe, deren Kohlenstoffgerüst durch eine oder mehrere Gruppierungen -O-, -S-, -NR¹-, -N=CR¹-, -CR¹=CR¹-, -CO-, -SO- und/oder -SO₂- unterbrochen sein kann, anelliert sein können, wobei das gesamte Ringsystem ein- oder mehrfach substituiert sein kann durch: C₁-C₁₈-Alkyl, C₁-C₁₂-Alkoxy, C₁-C₆-Alkylthio, -C≡CR¹, -CR¹=CR¹₂, Hydroxy, Mercapto, Halogen, Cyano, Nitro, -NR²R^{3,} -NR²COR³, -CONR²R³, -SO₂NR²R³, -COOR², -SO₃R², Aryl und/oder Hetaryl, das jeweils ein- oder mehrfach durch C₁-C₁₈-Alkyl, C₁-C₁₂-Alkoxy, Hydroxy, Mercapto, Halogen, Cyano, Nitro, -NR²R³, -NR²COR³, -CONR²R³, -SO₂NR²R³, -COOR² und/oder -SO₃R² substituiert sein kann;
(iv) ein Rest -U-Aryl, der ein- oder mehrfach durch die vorstehenden, als Substituenten für die Arylreste (iii) genannten Reste substituiert sein kann, wobei U eine Gruppierung -O-, -S-, -NR¹-, -CO-, -SO- oder -SO₂- bedeutet;
(v) C₁-C₁₂-Alkoxy, C₁-C₆-Alkylthio, -C≡CR¹, -CR¹=CR¹₂, Hydroxy, Mercapto, Halogen, Cyano, Nitro, -NR²R³, -NR²COR³, -CONR²R³, -SO₂NR²R³, -COOR² oder -SO₃R²;
R gleiche oder verschiedene Reste:
Wasserstoff;
C₁-C₃₀-Alkyl, dessen Kohlenstoffkette durch eine oder mehrere Gruppierungen -O-, -S-, -NR¹-, -N=CR¹-, -C≡C-, -CR¹=CR¹-, -CO-, -SO- und/oder -SO₂- unterbrochen sein kann und das ein- oder mehrfach durch die als Substituenten für die Reste R' genannten Reste (ii), (iii), (iv) und/oder (v) substituiert sein kann;
C₃-C₈-Cycloalkyl, an das weitere gesättigte oder ungesättigte 5- bis 7-gliedrige Ringe, deren Kohlenstoffgerüst durch eine oder mehrere Gruppierungen -O-, -S-, -NR¹-, -N=CR¹-, -CR¹=CR¹-, -CO-, -SO- und/oder -SO₂- unterbrochen sein kann, anelliert sein können, wobei das gesamte Ringsystem ein- oder mehrfach durch die als Substituenten für die Reste R' genannten Reste (i), (ii), (iii), (iv) und/oder (v) substituiert sein kann;
Aryl oder Hetaryl, an das jeweils weitere gesättigte oder ungesättigte 5-bis 7-gliedrige Ringe, deren Kohlenstoffgerüst durch eine oder mehrere Gruppierungen -O-, -S-, -NR¹-, -N=CR¹-, -CR¹=CR¹-, -CO-, -SO- und/oder -SO₂- unterbrochen sein kann, anelliert sein können, wobei das gesamte Ringsystem ein- oder mehrfach substituiert sein kann durch die als Substituenten für die Reste R' genannten Reste (i), (ii), (iii), (iv), (v) und/oder Aryl- und/oder Hetarylazo, das jeweils ein- oder mehrfach durch C₁-C₁₀-Alkyl, C₁-C₆-Alkoxy und/oder Cyano substituiert sein kann;
R¹ Wasserstoff oder C₁-C₁₈-Alkyl, wobei die Reste R¹ gleich oder verschieden sein können, wenn sie mehrfach auftreten;
R², R³ unabhängig voneinander:
Wasserstoff;
C₁-C₁₈-Alkyl, dessen Kohlenstoffkette durch eine oder mehrere Gruppierungen -O-, -S-, -CO-, -SO- und/oder -SO₂- unterbrochen sein kann und das ein- oder mehrfach durch C₁-C₁₂-Alkoxy, C₁-C₆-Alkylthio, Hydroxy, Mercapto, Halogen, Cyano, Nitro, Aryl und/oder -COOR¹ substituiert sein kann;
Aryl oder Hetaryl, an das jeweils weitere gesättigte oder ungesättigte 5-bis 7-gliedrige Ringe, deren Kohlenstoffgerüst durch eine oder mehrere Gruppierungen -O-, -S-, -CO- und/oder -SO₂- unterbrochen sein kann, anelliert sein können, wobei das gesamte Ringsystem ein- oder mehrfach durch C₁-C₁₂Alkyl und/oder die vorstehenden, als Substituenten für Alkyl genannten Reste substituiert sein kann;
C₃-C₈-Cycloalkyl, an das weitere gesättigte oder ungesättigte 5- bis 7-gliedrige Ringe, deren Kohlenstoffgerüst durch eine oder mehrere Gruppierungen -O-, -S-, -NR¹-, -NR^{1a}, -N=CR¹-, -CR¹=CR¹-, -CO-, -SO- und/oder -SO₂- unterbrochen sein kann, anelliert sein können, wobei das gesamte Ringsystem ein- oder mehrfach durch C₁-C₁₂-Alkyl und/oder die vorstehenden, als Substituenten für Alkyl genannten Reste substituiert sein kann;
m, n unabhängig voneinander 0 oder 1.

11. Verwendung von Dibenzorylentetracarbonsäurediimiden gemäß den Ansprüchen 1 bis 6 als IR-Laserstrahlen absorbierende Materialien beim Schweißbehandeln von Kunststoffteilen.

12. Verwendung von Dibenzorylentetracarbonsäurediimiden gemäß den Ansprüchen 1 bis 6 als Infrarotabsorber für das Wärmemanagement.

13. Verwendung von Dibenzorylentetracarbonsäurediimiden gemäß den Ansprüchen 1 bis 6 als Aktivkomponenten in der Photovoltaik.

14. Verwendung von Dibenzorylentetracarbonsäurediimiden, wie in einem der Ansprüchen 1 bis 6 definiert, in der organischen Photovoltaik, insbesondere als Halbleiter in excitonischen Solarzellen.

15. Organischer Feldeffekttransistor, umfassend ein Substrat mit wenigstens einer Gate-Struktur, einer Source-Elektrode und einer Drain-Etektrode und wenigstens einer Verbindung der Formel I, wie in einem der Ansprüche 1 bis 6 definiert, als n-Halbleiter.

16. Substrat mit einer Vielzahl von organischen Feldeffekttransistoren, wobei zumindest ein Teil der Feldeffekttransistoren wenigstens einer Verbindung der Formel I, wie in einem der Ansprüche 1 bis 6 definiert, als n-Halbleiter enthält.

17. Verwendung wenigstens einer Verbindung der allgemeinen Formel I, wie in einem der Ansprüche 1 bis 6 definiert, für optische Label, zur unsichtbaren Markierung von Produkten, als Fluoreszenzfarbstoffe, als Fluoreszenzlabel für Biomoleküle und als Pigmente.

18. Verwendung wenigstens einer Verbindung der allgemeinen Formel I, wie in einem der Ansprüche 1 bis 6 definiert, als Fluoreszenzfarbstoff in einem auf Fluoreszenzkonversion beruhenden Display; in einem lichtsammelnden Kunststoffteil, welches gegebenenfalls mit einer Solarzelle kombiniert ist; als Pigmentfarbstoff in elektrophoretischen Displays; als Fluoreszenzfarbstoff in einer auf Chemolumineszenz basierenden Anwendung.

## Claims

1. A dibenzorylenetetracarboximide of the general formula I 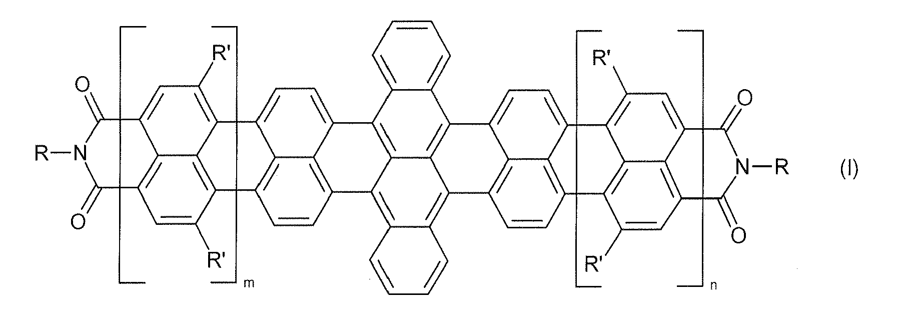 in which the variables are each defined as follows:
R' are identical or different radicals:
hydrogen;
aryloxy, arylthio, hetaryloxy or hetarylthio, to each of which may be fused further saturated or unsaturated 5- to 7-membered rings whose carbon skeleton may be interrupted by one or more -O-, -S-, -NR¹- , -N=CR¹-, -CO-, -SO- and/or -SO₂-moieties, where the entire ring system may be mono- or polysubstituted by the radicals (i), (ii), (iii), (iv) and/or (v):
(i) C₁-C₃₀-alkyl whose carbon chain may be interrupted by one or more -O-, -S-, -NR¹-, -N=CR¹-, -C≡C-, -CR¹=CR¹-, -CO-, -SO- and/or -SO₂- moieties and which may be mono- or polysubstituted by: C₁-C₁₂-alkoxy, C₁-C₆-alkylthio, -C≡CR¹,- CR¹=CR¹₂, hydroxyl, mercapto, halogen, cyano, nitro, -NR²R³, -NR²COR³, -CONR²R³, -SO₂NR²R³, -COOR², -SO₃R², aryl and/or saturated or unsaturated C₄-C₇-cycloalkyl whose carbon skeleton may be interrupted by one or more -O-, -S-, -NR¹-, -N=CR¹-, -CR¹=CR¹-, -CO-, -SO- and/or -SO₂- moieties, where the aryl and cycloalkyl radicals may each be mono- or polysubstituted by C₁-C₁₈-alkyl and/or the above radicals specified as substituents for alkyl;
(ii) C₃-C₈-cycloalkyl whose carbon skeleton may be interrupted by one or more -O-, -S-, -NR¹-, -N=CR¹-, -CR¹=CR¹- -CO-, -SO- and/or -SO₂- moieties and to which may be fused further saturated or unsaturated 5- to 7-membered rings whose carbon skeleton may be interrupted by one or more -O-, -S-, -NR¹-,-N=CR¹-, -CR¹=CR¹-, -CO-, -SO- and/or -SO₂- moieties, where the entire ring system may be mono- or polysubstituted by: C₁-C₁₈-alkyl, C₁-C₁₂-alkoxy, C₁-C₆-alkylthio, -C≡CR¹, -CR¹≡CR¹₂, hydroxyl, mercapto, halogen, cyano, nitro, -NR²R³, -NR²COR³, -CONR²R³, -SO₂NR²R³, -COOR² and/or -SO₃R²;
(iii) aryl or hetaryl, to which may be fused further saturated or unsaturated 5- to 7-membered rings whose carbon skeleton may be interrupted by one or more -O-, -S-, -NR¹-, -N=CR¹-, -CR¹=CR¹-, -CO-, -SO- and/or -SO₂- moieties, where the entire ring system may be mono- or polysubstituted by: C₁-C₁₈-alkyl, C₁-C₁₂-alkoxy, C₁-C₆-alkylthio, -C≡CR¹, -CR¹=CR¹2, hydroxyl, mercapto, halogen, cyano, nitro, -NR²R³, -NR²COR³, -CONR²R³, -SO₂NR²R³, -COOR², -SO₃R², aryl and/or hetaryl, each of which may be mono- or polysubstituted by C₁-C₁₈-alkyl, C₁-C₁₂-alkoxy, hydroxyl, mercapto, halogen, cyano, nitro, -NR²R³, -NR²COR³, -CONR²R³, -SO₂NR²R³, -COOR² and/or -SO₃R²;
(iv) a -U-aryl radical which may be mono- or polysubstituted by the above radicals specified as substituents for the aryl radicals (iii), where U is an -O-, -S-, -NR¹-, -CO-, -SO- or -SO₂- moiety;
(v) C₁-C₁₂-alkoxy, C₁-C₆-alkylthio, -C≡CR¹, -CR¹=CR¹₂, hydroxyl, mercapto, halogen, cyano, nitro, -NR²R³, -NR²COR³, -CONR²R³, -SO₂NR²R³, -COOR² or -SO₃R²;
R are identical or different radicals:
hydrogen; C₁-C₃₀-alkyl whose carbon chain may be interrupted by one or more -O-, -S-, -NR¹- -N=CR¹-, -C≡C-, -CR¹=CR¹- -CO-, -SO-and/or -SO₂- moieties and which may be mono- or polysubstituted by the (ii), (iii), (iv) and/or (v) radicals specified as substituents for the R' radicals;
C₃-C₈-cycloalkyl, to which may be fused further saturated or unsaturated 5- to 7-membered rings whose carbon skeleton may be interrupted by one or more -O-, -S-, -NR¹- -N=CR¹-, -CR¹=CR¹-, -CO-, -SO- and/or -SO₂- moieties, where the entire ring system may be mono- or polysubstituted by the (i), (ii), (iii), (iv) and/or (v) radicals specified as substituents for the R' radicals;
aryl or hetaryl, to each of which may be fused further saturated or unsaturated 5- to 7-membered rings whose carbon skeleton may be interrupted by one or more -O-, -S-, -NR¹- -N=CR¹-, -CR¹=CR¹-, -CO-, -SO- and/or -SO₂- moieties, where the entire ring system may be mono- or polysubstituted by the (i), (ii), (iii), (iv), (v) radicals specified as substituents for the R' radicals, and/or aryl- and/or hetarylazo, each of which may be mono- or polysubstituted by C₁-C₁₀-alkyl, C₁-C₆-alkoxy and/or cyano;
R¹ is hydrogen or C₁-C₁₈-alkyl, where the R¹ radicals may be the same or different when they occur more than once;
R², R³ are each independently:
hydrogen;
C₁-C₁₈-alkyl whose carbon chain may be interrupted by one or more -O-, -S-, -CO-, -SO- and/or -SO₂- moieties and which may be mono- or polysubstituted by C₁-C₁₂-alkoxy, C₁-C₆-alkylthio, hydroxyl, mercapto, halogen, cyano, nitro, aryl and/or -COOR¹;
aryl or hetaryl, to each of which may be fused further saturated or unsaturated 5- to 7-membered rings whose carbon skeleton may be interrupted by one or more -O-, -S-, -CO- and/or -SO₂-moieties, where the entire ring system may be mono- or polysubstituted by C₁-C₁₂-alkyl and/or the above radicals specified as substituents for alkyl;
C₃-C₈-cycloalkyl to which may be fused further saturated or unsaturated 5- to 7-membered rings whose carbon skeleton may be interrupted by one or more -O-, -S-, -NR¹- -N=CR¹-, -CR¹=CR¹-, -CO-, -SO- and/or -SO₂- moieties, where the entire ring system may be mono- or polysubstituted by C₁-C₁₂-alkyl and/or the above radicals specified as substituents for alkyl;
m, n are each independently 0 or 1.

2. A dibenzorylenetetracarboximide of the general formula I according to claim 1, in which the variables are each defined as follows:
R' are identical or different radicals:
hydrogen;
phenoxy or thiophenoxy, each of which may be mono- or polysubstituted by identical or different radicals (i), (ii), (iii), (iv) and/or (v):
(i) C₁-C₃₀-alkyl whose carbon chain may be interrupted by one or more -O-, -S-, -NR¹-, -C≡C-, -CR¹=CR¹- and/or -CO- and/or -SO₂- moieties and which may be mono- or polysubstituted by: C₁-C₁₂-alkoxy, hydroxyl, halogen, cyano, and/or aryl which may be mono- or polysubstituted by C₁-C₁₈-alkyl or C₁-C₆-alkoxy;
(ii) C₃-C₈-cydoalkyl whose carbon skeleton may be interrupted by one or more -O-, -S-, -NR¹-, -CR¹=CR¹- and/or -CO-moieties and which may be mono- or polysubstituted by: C₁-C₁₈-alkyl, C₁-C₁₂-alkoxy and/or C₁-C₆-alkylthio;
(iii) aryl or hetaryl, to each of which may be fused further saturated or unsaturated 5- to 7-membered rings whose carbon skeleton may be interrupted by one or more -O-, -S-, -NR¹-, -N=CR¹-, -CR¹=CR¹-, -CO-, -SO- and/or -SO₂- moieties, where the entire ring system may be mono- or polysubstituted by: C₁-C₁₈-alkyl, C₁-C₁₂-alkoxy, -C≡CR¹, -CR¹=CR¹₂, hydroxyl, halogen, cyano, -NR²R³, -NR²COR³, -CONR²R³, -SO₂NR²R³, -COOR², -SO₃R², aryl and/or hetaryl, each of which may be mono- or polysubstituted by C₁-C₁₈-alkyl, C₁-C₁₈-alkoxy and/or cyano;
(iv) a -U-aryl radical which may be mono- or polysubstituted by the above radicals specified as substituents for the aryl radicals (iii), where U is an -O-, -S-, -NR¹-, -CO-, -SO- or -SO₂- moiety;
(v) C₁-C₁₂-alkoxy, C₁-C₆-alkylthio, -C≡CR¹, -CR¹=CR¹₂, hydroxyl, mercapto, halogen, cyano, nitro, -NR²R³, -NR²COR³, -CONR²R³, -SO₂NR²R³, -COOR² or -SO₃R²;
R are identical or different radicals:
C₁-C₃₀-alkyl whose carbon chain may be interrupted by one or more -O- and/or -CO- moieties and which may be mono- or polysubstituted by: C₁-C₆-alkoxy, cyano and/or aryl which may be substituted by C₁-C₁₈-alkyl or C₁-C₆-alkoxy;
C₅-C₈-cycloalkyl which may be mono- or polysubstituted by C₁-C₆-alkyl;
phenyl, naphthyl, pyridyl or pyrimidyl, each of which may be mono- or polysubstituted by: C₁-C₁₈-alkyl, C₁-C₆-alkoxy, halogen, cyano, nitro, -CONR²R³, -SO₂NR²R³ and/or phenyl- and/or naphthylazo, each of which may be mono- or polysubstituted by C₁-C₁₀-alkyl, C₁-C₆-alkoxy and/or cyano;
R¹ is hydrogen or C₁-C₆-alkyl;
R2, R³ are each independently:
hydrogen;
C₁-C₁₈-alkyl which may be mono- or polysubstituted by C₁-C₆-alkoxy, hydroxyl, halogen and/or cyano;
aryl or hetaryl, each of which may be mono- or polysubstituted by C₁-C₆-alkyl and/or the above radicals specified as substituents for alkyl;
m, n are each 0 or 1.

3. A dibenzorylenetetracarboximide of the general formula I according to claim 1, in which the variables are each defined as follows:
R' are identical radicals:
hydrogen;
phenoxy which may be mono- or polysubstituted by identical or different radicals (i), (ii), (iii), (iv) and/or (v):
(i) C₁-C₁₈-alkyl whose carbon chain may be interrupted by one or more -O-, -S-, -NR¹- and/or -CO- moieties and which may be mono- or polysubstituted by: C₁-C₁₂-alkoxy, hydroxyl and/or halogen;
(ii) C₃-C₈-cydoalkyl which may be mono- or polysubstituted by C₁-C₁₈-alkyl and/or C₁-C₁₂-alkoxy;
(iii) aryl or hetaryl which may be mono- or polysubstituted by C₁-C₁₈-alkyl, C₁-C₁₂-alkoxy, hydroxyl and/or halogen;
(iv) a -U-aryl radical which may be mono- or polysubstituted by the above radicals specified as substituents for the aryl radicals (iii), where U is an -O-, -S- or -NR¹- moiety;
R (v) C₁-C₁₂-alkoxy, hydroxyl, halogen or cyano; are identical radicals:
C₁-C₃₀-alkyl whose carbon chain may be interrupted by one or more -O- and/or -CO- moieties and which may be mono- or polysubstituted by: C₁-C₆-alkoxy, cyano and/or aryl which may be mono- or polysubstituted by C₁-C₁₈-alkyl or C₁-C₆-alkoxy;
C₅-C₈-cycloalkyl which may be mono- or polysubstituted by C₁-C₆-alkyl;
phenyl, naphthyl, pyridyl or pyrimidyl, each of which may be mono- or polysubstituted by: C₁-C₁₈-alkyl, C₁-C₆-alkoxy, halogen, cyano, nitro, -CONR²R³, -SO₂NR²R³ and/or phenyl- and/or naphthylazo, each of which may be mono- or polysubstituted by C₁-C₁₀-alkyl, C₁-C₆-alkoxy and/or cyano;
R¹ is hydrogen or C₁-C₆-alkyl;
R², R³ are each independently: hydrogen;
C₁-C₁₈-alkyl which may be mono- or polysubstituted by C₁-C₆-alkoxy, hydroxyl, halogen and/or cyano;
aryl or hetaryl, each of which may be mono- or polysubstituted by C₁-C₆-alkyl and/or the above radicals specified as substituents for alkyl;
n, m are each 1.

4. A dibenzorylenetetracarboximide according to any of the preceding claims, where the R radicals are each independently selected from groups of the formulae II.1 to II.5:
#-(A)ₚ₋C(Rⁱ)ₓ (II.1) 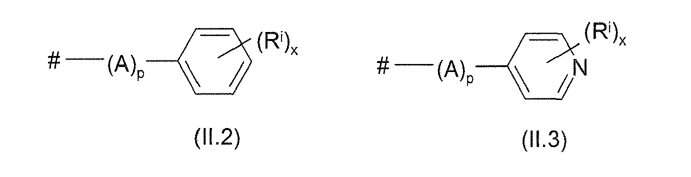 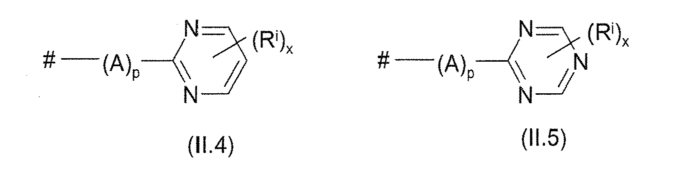
in which
# is the site of attachment to the imide nitrogen atom,
p is 0 or 1,
x is 2 or 3 in the compounds of the formula II.1, is 1, 2 or 3 in the compounds of the formulae II.2, II.3 and II.4, and is 1 or 2 in the compounds of the formula II.5,
A, where present, is a C₁-C₁₀-alkylene group which may be interrupted by one or more nonadjacent groups selected from -O- and -S-, where, in the case that x is 2 in the compounds of the formula II.1, the carbon atom which bears the Rⁱ radicals additionally bears a hydrogen atom,
the Rⁱ radicals are each independently selected from C₁-C₃₀₋alkyl which may be interrupted by one or more nonadjacent oxygen atom(s), where at least one of the Rⁱ radicals in the compounds of the formula II.1 may also be C₁-C₃₀-alkyloxy or C₁-C₃₀-alkylthio.

5. A dibenzorylenetetracarboximide according to any of claims 1 to 4, where the R' radicals are both hydrogen.

6. A dibenzorylenetetracarboximide according to any of claims 1 to 4, where the R' radicals are selected from radicals of the formula 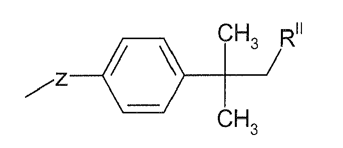 in which
Z is O or S, and
R" is selected from hydrogen, C₁-C₈-alkyl whose carbon chain may be interrupted by one or more -O-, -S-, -NR¹-, -C(=O)- and/or -SO₂- moieties and which may be mono- or polysubstituted by C₁-C₆-alkoxy and/or a 5- to 7-membered heterocyclic radical which is bonded via a nitrogen atom and may comprise further heteroatoms and be aromatic, and C₅-C₈-cycloalkyl whose carbon skeleton may be interrupted by one or more -O-, -S-, -NR¹-, -C(=O)- and/or -SO₂- moieties and which may be mono- or polysubstituted by C₁-C₆-alkyl, where R¹ is hydrogen or C₁-C₆-alkyl.

7. A process for preparing dibenzorylenetetracarboximides of the general
formula la 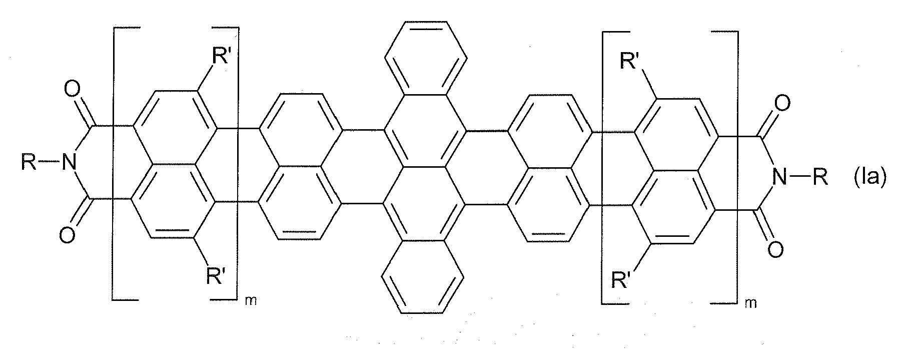 in which R, R' and m are each as defined in claim 1, which comprises
a) subjecting a peri-(dioxaborolan-2-yl)rylenedicarboximide of the general formula IIa 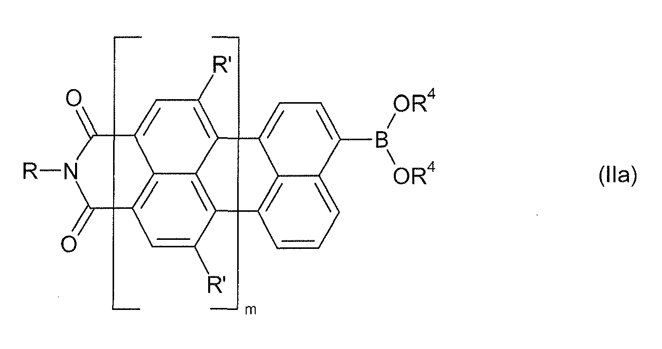 in which R⁴ are the same or different and are each independently
hydrogen, C₁-C₃₀-alkyl, C₅-C₈-cydoalkyl, aryl or heteroaryl, and where two OR⁴ radicals bonded to the same boron atom together may also be -OCH₂CH₂O-, in which 1, 2, 3 or 4 hydrogen atoms may also be replaced by C₁-C₃₀-alkyl, C₅-C₈-cycloalkyl, aryl or heteroaryl groups,
in the presence of an organic solvent, if desired in a mixture with water, and of a transition metal catalyst and of a base, to a Suzuki coupling reaction with 5,11-dibromotetracene of the formula III 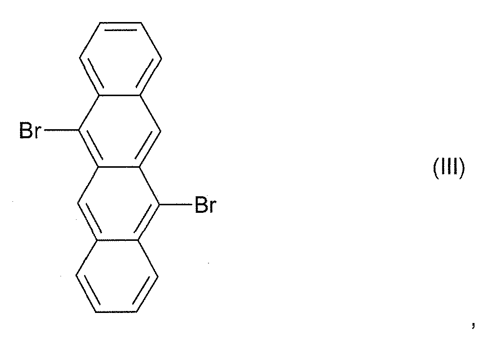
b) subjecting the tetracene-5,11-bis(rylenedicarboximide) of the general formula IVa formed in step a) 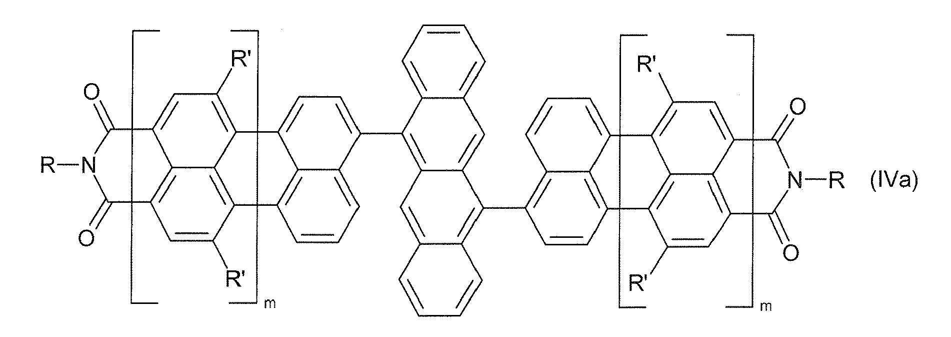 to a first cyclodehydrogenation in the presence of an inert organic solvent and of a Lewis acid and
c) cyclodehydrogenating the bisrylene derivative of the general formula Va formed in step b) 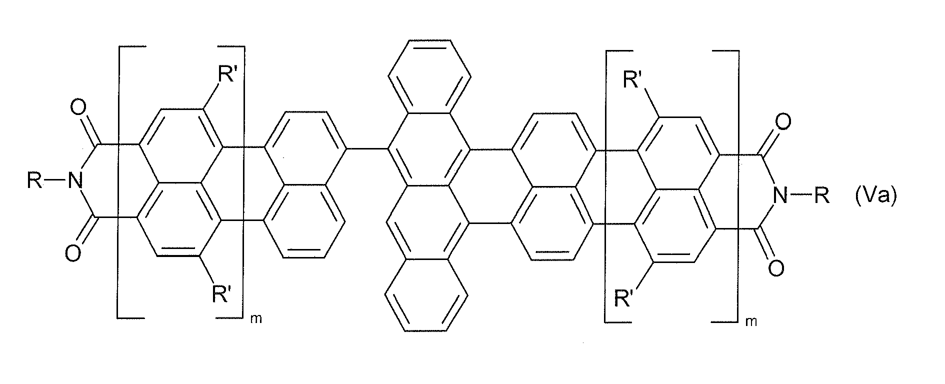 in an organic reaction medium which has hydroxyl and amino functions and comprises an essentially undissolved base further to give the dibenzorylenetetracarboximide la.

8. A process for preparing dibenzorylenetetracarboximides of the general formula lb 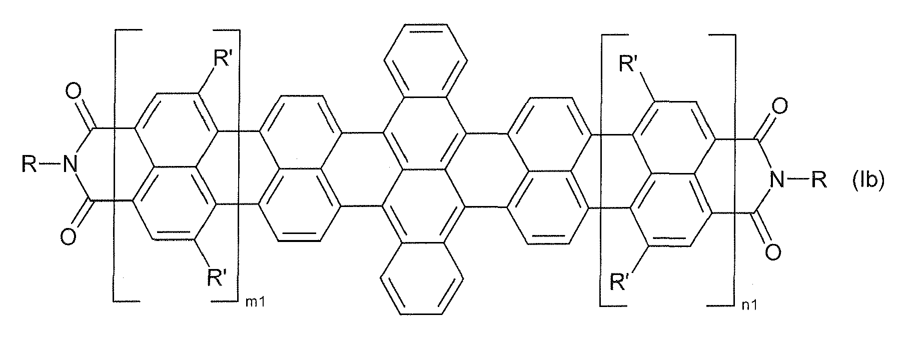 in which R and R' are each as defined in claim 1, m1 and n1 are different from one another and are each 0 or 1, which comprises
a1) subjecting a peri-(dioxaborolan-2-yl)rylenedicarboximide of the
general formula IIb1 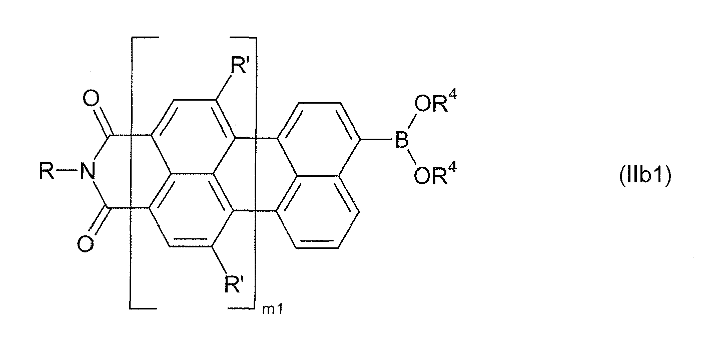 in which R⁴ are the same or different and are each independently
hydrogen, C₁-C₃₀-alkyl, C₅-C₈-cydoalkyl, aryl or heteroaryl, and where two OR⁴ radicals bonded to the same boron atom together may also be -OCH₂CH_{2O}-, in which 1, 2, 3 or 4 hydrogen atoms may also be replaced by C₁-C₃₀-alkyl, C₅-C₈-cycloalkyl, aryl or heteroaryl groups,
to a first Suzuki coupling reaction with 5,11-dibromotetracene (III) and
a2) subjecting the 5-bromotetracene-11-dicarboximide of the general
formula III' formed in step a1) 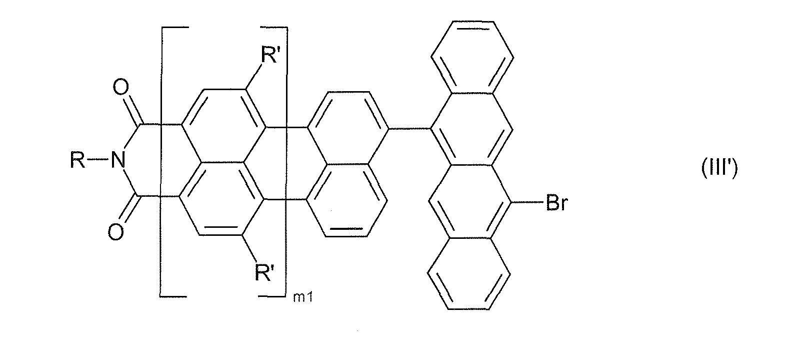 to a second Suzuki coupling reaction with a peri-(dioxaborolan-2-yl)rylenedicarboximide of the general formula IIb2 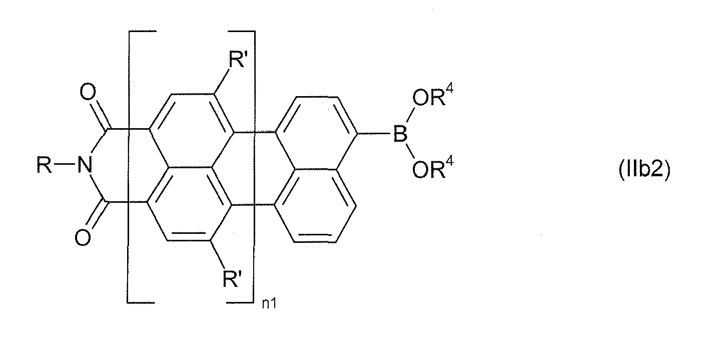 in the presence of an organic solvent, if desired in a mixture with water, and of a transition metal catalyst and of a base,
b) subjecting the tetracene-5,11-bis(rylenedicarboximide) of the general formula IVb formed in step a2) 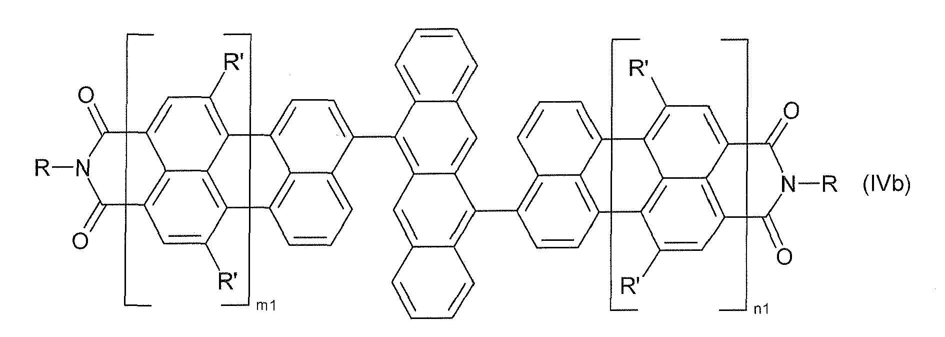 to a first cyclodehydrogenation in the presence of an inert organic solvent and of a Lewis acid and
c) cyclodehydrogenating the bisrylene derivative of the general formula Vb formed in step b) 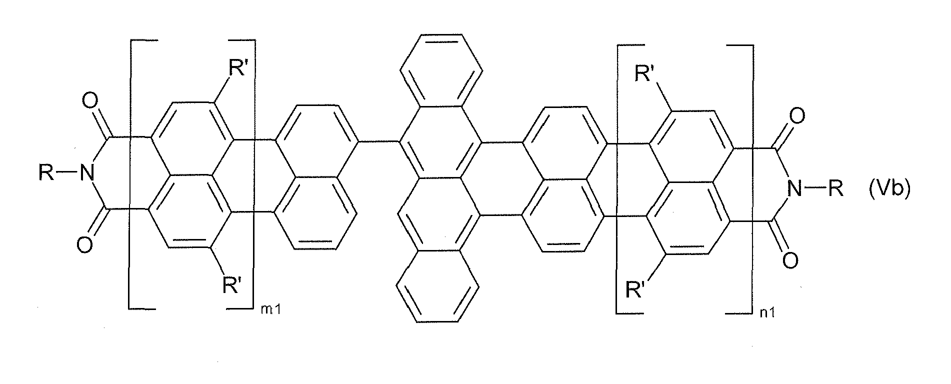 in an organic reaction medium which has hydroxyl and amino functions and comprises an essentially undissolved base further to give the dibenzorylenetetracarboximide Ib.

9. A tetracene-5,11-bis(rylenedicarboximide) of the general formula IV 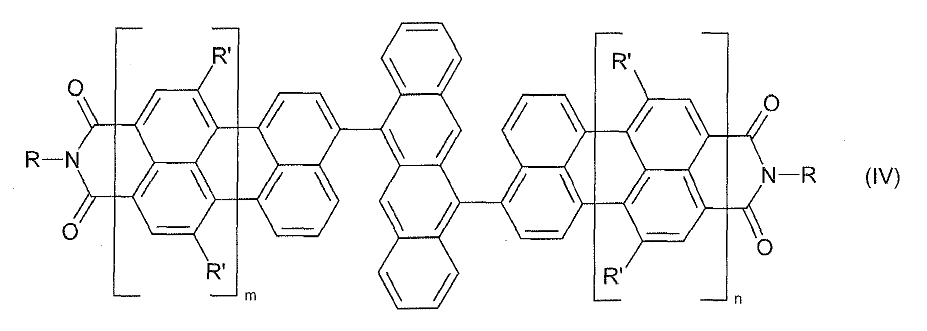 in which the variables are each defined as follows:
R' are identical or different radicals:
hydrogen;
aryloxy, arylthio, hetaryloxy or hetarylthio, to each of which may be fused further saturated or unsaturated 5- to 7-membered rings whose carbon skeleton may be interrupted by one or more -O-, -S-, -NR¹- -N=CR¹-, -CO-, -SO- and/or -SO₂-moieties, where the entire ring system may be mono- or polysubstituted by the radicals (i), (ii), (iii), (iv) and/or (v):
(i) C₁-C₃₀-alkyl whose carbon chain may be interrupted by one or more -O-, -S-, -NR¹-, -N=CR¹-, -C≡C-, -CR¹=CR¹, -CO-, -SO- and/or -SO₂- moieties and which may be mono- or polysubstituted by: C₁-C₁₂-alkoxy, C₁-C₆-alkylthio, -C≡CR¹, -CR¹=CR¹₂, hydroxyl, mercapto, halogen, cyano, nitro, -NR²R³, -NR²COR³, -CONR²R³, -SO₂NR²R³, -COOR², -SO₃R², aryl and/or saturated or unsaturated C₄-C₇-cydoalkyl whose carbon skeleton may be interrupted by one or more -O-, -S-, -NR¹-, -N=CR¹-, -CR¹=CR¹-, -CO-, -SO- and/or -SO₂- moieties, where the aryl and cycloalkyl radicals may each be mono- or polysubstituted by C₁-C₁₈-alkyl and/or the above radicals specified as substituents for alkyl;
(ii) C₃-C₈-cycloalkyl whose carbon skeleton may be interrupted by one or more -O-, -S-, -NR¹-, -N=CR¹-, -CR¹=CR¹-, -CO-, -SO- and/or -SO₂- moieties and to which may be fused further saturated or unsaturated 5- to 7-membered rings whose carbon skeleton may be interrupted by one or more -O-, -S-, -NR¹-, - N=CR¹-, -CR¹=CR¹, -CO-, -SO- and/or -SO₂- moieties, where the entire ring system may be mono- or polysubstituted by: C₁-C₁₈-alkyl, C₁-C₁₂-alkoxy, C₁-C₆-alkylthio, -C≡CR¹, -CR¹=CR¹₂, hydroxyl, mercapto, halogen, cyano, nitro, -NR²R³, -NR²COR³, -CONR²R³, -SO₂NR²R³, -COOR² and/or -SO₃R²;
(iii) aryl or hetaryl, to which may be fused further saturated or unsaturated 5- to 7-membered rings whose carbon skeleton may be interrupted by one or more -O-, -S-, -NR¹-, -N=CR¹-, -CR¹=CR¹-, -CO-, -SO- and/or -SO₂- moieties, where the entire ring system may be mono- or polysubstituted by: C₁-C₁₈-alkyl, C₁-C₁₂-alkoxy, C₁-C₆-alkylthio, -C≡CR¹, -CR¹=CR¹₂, hydroxyl, mercapto, halogen, cyano, nitro, -NR²R³, -NR²COR³, -CONR²R³, -S0₂NR²R³, -COOR², -SO₃R², aryl and/or hetaryl, each of which may be mono- or polysubstituted by C₁-C₁₈-alkyl, C₁-C₁₂-alkoxy, hydroxyl, mercapto, halogen, cyano, nitro, -NR²R³, -NR²COR³, -CONR²R³, -SO₂NR²R³, -COOR² and/or -SO₃R²_{;}
(iv) a -U-aryl radical which may be mono- or polysubstituted by the above radicals specified as substituents for the aryl radicals (iii), where U is an -O-, -S-, -NR¹-, -CO-, -SO- or -SO₂- moiety;
(v) C₁-C₁₂-alkoxy, C₁-C₆-alkylthio, -C≡CR¹, -CR¹=CR¹₂, hydroxyl, mercapto, halogen, cyano, nitro, -NR²R³, -NR²COR³, -CONR²R³, -SO₂NR²R³, -COOR² or -SO₃R²,
R are identical or different radicals:
hydrogen;
C₁-C₃₀-alkyl whose carbon chain may be interrupted by one or more -O-, -S-, -NR¹-, -N=CR¹-, -C≡C-, -CR¹=CR¹-, -CO-, -SO-and/or -SO₂- moieties and which may be mono- or polysubstituted by the (ii), (iii), (iv) and/or (v) radicals specified as substituents for the R' radicals;
C₃-C₈-cycloalkyl, to which may be fused further saturated or unsaturated 5- to 7-membered rings whose carbon skeleton may be interrupted by one or more -O-, -S-, -NR¹-, -N=CR¹-, -CR¹=CR¹-, -CO-, -SO- and/or -SO₂- moieties, where the entire ring system may be mono- or polysubstituted by the (i), (ii), (iii), (iv) and/or (v) radicals specified as substituents for the R' radicals;
aryl or hetaryl, to which may be fused further saturated or unsaturated 5- to 7-membered rings whose carbon skeleton may be interrupted by one or more -O-, -S-, -NR¹-, -N=CR¹-, -CR¹=CR¹-, -CO-, -SO- and/or -SO₂- moieties, where the entire ring system may be mono- or polysubstituted by the (i), (ii), (iii), (iv), (v) radicals specified as substituents for the R' radicals; and/or aryl- and/or hetarylazo, each of which may be mono- or polysubstituted by C₁-C₁₀-alkyl, C₁-C₆-alkoxy and/or cyano;
R¹ is hydrogen or C₁-C,₈-alkyl, where the R₁ radicals may be the same or different when they occur more than once;
R², R³ are each independently:
hydrogen;
C₁-C₁₈-alkyl whose carbon chain may be interrupted by one or more -O-, -S-, -CO-, -SO- and/or -SO₂- moieties and which may be mono- or polysubstituted by C₁-C₁₂-alkoxy, C₁-C₆-alkylthio, hydroxyl, mercapto, halogen, cyano, nitro, aryl and/or -COOR¹;
aryl or hetaryl, to each of which may be fused further saturated or unsaturated 5- to 7-membered rings whose carbon skeleton may be interrupted by one or more -O-, -S-, -CO- and/or -SO₂-moieties, where the entire ring system may be mono- or polysubstituted by C₁-C₁₂-alkyl and/or the above radicals specified as substituents for alkyl;
C₃-C₈-cycloalkyl to which may be fused further saturated or unsaturated 5- to 7-membered rings whose carbon skeleton may be interrupted by one or more -O-, -S-, -NR¹-, -NR^{1a}, -N=CR¹-, -CR¹=CR¹- -CO-, -SO- and/or -SO₂- moieties, where the entire ring system may be mono- or polysubstituted by C₁-C₁₂-alkyl and/or the above radicals specified as substituents for alkyl;
m, n are each independently 0 or 1.

10. A bisrylene derivative of the general formula V 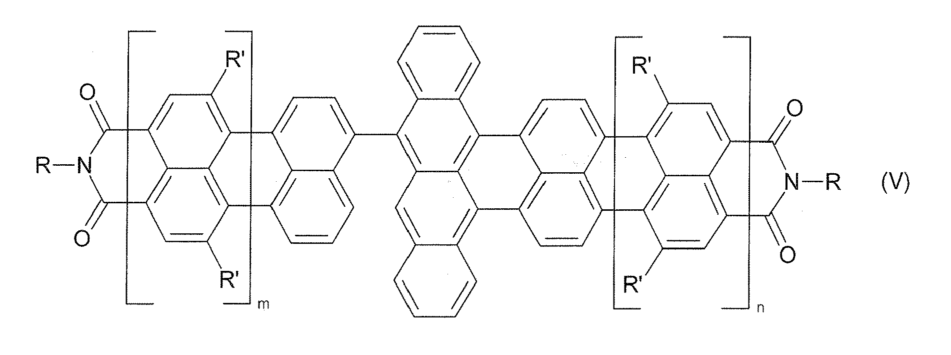 in which the variables are each defined as follows:
R' are identical or different radicals:
hydrogen;
aryloxy, arylthio, hetaryloxy or hetarylthio, to each of which may be fused further saturated or unsaturated 5- to 7-membered rings whose carbon skeleton may be interrupted by one or more -O-, -S-, -NR¹-, -N=CR¹-, -CO-, -SO- and/or -SO₂-moieties, where the entire ring system may be mono- or polysubstituted by the radicals (i), (ii), (iii), (iv) and/or (v):
(i) C₁-C₃₀-alkyl whose carbon chain may be interrupted by one or more -O-, -S-, -NR¹- -N=CR¹- -C≡C-, -CR¹=CR¹-, -CO-, -SO- and/or -SO₂- moieties and which may be mono- or polysubstituted by: C₁-C₁₂-alkoxy, C₁-C₆-alkylthio, -C≡CR¹, -CR¹=CR¹₂, hydroxyl, mercapto, halogen, cyano, nitro, -NR²R³, -NR²COR³, -CONR²R³, -SO₂NR²R³, -COOR², -SO₃R², aryl and/or saturated or unsaturated C₄-C₇-cycloalkyl whose carbon skeleton may be interrupted by one or more -O-, -S-, -NR¹-, -N=CR¹-, -CR¹=CR¹-, -CO-, -SO- and/or -SO₂- moieties, where the aryl and cycloalkyl radicals may each be mono- or polysubstituted by C₁-C₁₈-alkyl and/or the above radicals specified as substituents for alkyl;
(ii) C₃-C₈-cycloalkyl whose carbon skeleton may be interrupted by one or more -O-, -S-, -NR¹- -N=CR¹-, -CR¹=CR¹-, -CO-, -SO- and/or -SO₂- moieties and to which may be fused further saturated or unsaturated 5- to 7-membered rings whose carbon skeleton may be interrupted by one or more -O-, -S-, -NR¹-, - N=CR¹-, -CR¹=CR¹-, -CO-, -SO- and/or -SO₂- moieties, where the entire ring system may be mono- or polysubstituted by: C₁-C₁₈-alkyl, C₁-C₁₂-alkoxy, C₁-C₆-alkylthio, -C≡CR¹, -CR¹=CR¹₂, hydroxyl, mercapto, halogen, cyano, nitro, -NR²R³, -NR²COR³, -CONR²R³, -SO₂NR²R³, -COOR² and/or -SO₃R²;
(iii) aryl or hetaryl, to which may be fused further saturated or unsaturated 5- to 7-membered rings whose carbon skeleton may be interrupted by one or more -O-, -S-, -NR¹-, -N=CR¹-, -CR¹=CR¹-, -CO-, -SO- and/or -SO₂- moieties, where the entire ring system may be mono- or polysubstituted by: C₁-C₁₈-alkyl, C₁-C₁₂-alkoxy, C₁-C₆-alkylthio, -C≡CR¹, -CR¹=GR¹₂, hydroxyl, mercapto, halogen, cyano, nitro, -NR²R³, -NR²COR³, -CONR²R³, -SO₂NR²R³, -COOR², -SO₃R², aryl and/or hetaryl, each of which may be mono- or polysubstituted by C₁-C₁₈-alkyl, C₁-C₁₂-alkoxy, hydroxyl, mercapto, halogen, cyano, nitro, -NR²R³, -NR²COR³, -CONR²R³, -SO₂NR²R³, -COOR² and/or -SO₃R²_{;}
(iv) a -U-aryl radical which may be mono- or polysubstituted by the above radicals specified as substituents for the aryl radicals (iii), where U is an -O-, -S-, -NR¹-, -CO-, -SO- or -SO₂- moiety;
(v) C₁-C₁₂-alkoxy, C₁-C₆-alkylthio, -C≡CR¹, -CR¹=CR¹₂, hydroxyl, mercapto, halogen, cyano, nitro, -NR²R³, -NR²COR³, -CONR²R³, -SO₂NR²R³, -COOR² or -SO₃R²,
R are identical or different radicals:
hydrogen;
C₁-C₃₀-alkyl whose carbon chain may be interrupted by one or more -O-, -S-, -NR¹-, -N=CR¹-, -C≡C-, -CR¹=CR¹-, -CO-, -SO- and/or -SO₂- moieties and which may be mono- or polysubstituted by the (ii), (iii), (iv) and/or (v) radicals specified as substituents for the R' radicals;
C₃-C₈-cycloalkyl, to which may be fused further saturated or unsaturated 5- to 7-membered rings whose carbon skeleton may be interrupted by one or more -O-, -S-, -NR¹-, -N=CR¹-, -CR¹=CR¹-, -CO-, -SO- and/or -SO₂- moieties, where the entire ring system may be mono- or polysubstituted by the (i), (ii), (iii), (iv) and/or (v) radicals specified as substituents for the R' radicals;
aryl or hetaryl, to each of which may be fused further saturated or unsaturated 5- to 7-membered rings whose carbon skeleton may be interrupted by one or more -O-, -S-, -NR¹-, -N=CR¹-, -CR¹=CR¹, -CO-, -SO- and/or -SO₂- moieties, where the entire ring system may be mono- or polysubstituted by the (i), (ii), (iii), (iv), (v) radicals specified as substituents for the R' radicals; and/or aryl- and/or hetarylazo, each of which may be mono- or polysubstituted by C₁-C₁₀-alkyl, C₁-C₆-alkoxy and/or cyano;
R¹ is hydrogen or C₁-C₁₈-alkyl, where the R¹ radicals may be the same or different when they occur more than once;
R², R³ are each independently:
hydrogen;
C₁-C₁₈-alkyl whose carbon chain may be interrupted by one or more -O-, -S-, -CO-, -SO- and/or -SO₂- moieties and which may be mono- or polysubstituted by C₁-C₁₂-alkoxy, C₁-C₆-alkylthio, hydroxyl, mercapto, halogen, cyano, nitro, aryl and/or -COOR¹; aryl or hetaryl, to each of which may be fused further saturated or unsaturated 5- to 7-membered rings whose carbon skeleton may be interrupted by one or more -O-, -S-, -CO- and/or -SO₂-moieties, where the entire ring system may be mono- or polysubstituted by C₁-C₁₂-alkyl and/or the above radicals specified as substituents for alkyl;
C₃-C₈-cydoalkyl to which may be fused further saturated or unsaturated 5- to 7-membered rings whose carbon skeleton may be interrupted by one or more -O-, -S-, -NR¹-, -NR^{1a}, -N=CR¹-, -CR¹=CR¹-, -CO-, -SO- and/or -SO₂- moieties, where the entire ring system may be mono- or polysubstituted by C₁-C₁₂-alkyl and/or the above radicals specified as substituents for alkyl;
m, n are each independently 0 or 1.

11. The use of dibenzorylenetetracarboximides according to claims 1 to 6 as materials which absorb IR laser beams in the fusion treatment of plastics parts.

12. The use of dibenzorylenetetracarboximides according to claims 1 to 6 as infrared absorbers for heat management.

13. The use of dibenzorylenetetracarboximides according to claims 1 to 6 as active components in photovoltaics.

14. The use of dibenzorylenetetracarboximides as defined in any of claims 1 to 6 in organic photovoltaics, especially as a semiconductor in excitonic solar cells.

15. An organic field-effect transistor comprising a substrate comprising at least one gate structure, a source electrode and a drain electrode and at least one compound of the formula I as defined in any of claims 1 to 6 as an n-semiconductor.

16. A substrate comprising a multitude of organic field-effect transistors, wherein at least some of the field-effect transistors comprise at least one compound of the formula I as defined in any of claims 1 to 6 as an n-semiconductor.

17. The use of at least one compound of the general formula I as defined in any of claims 1 to 6 for optical labels, for invisible marking of products, as fluorescent dyes, as a fluorescent label for biomolecules and as pigments.

18. The use of at least one compound of the general formula I as defined in any of claims 1 to 6 as a fluorescent dye in a display based on fluorescence conversion; in a light-collecting plastics part which is optionally combined with a solar cell; as a pigment dye in electrophoretic displays; as a fluorescent dye in an application based on chemoluminescence.

## Revendications

1. Diimides de l'acide dibenzorylènetétracarboxylique de formule générale I 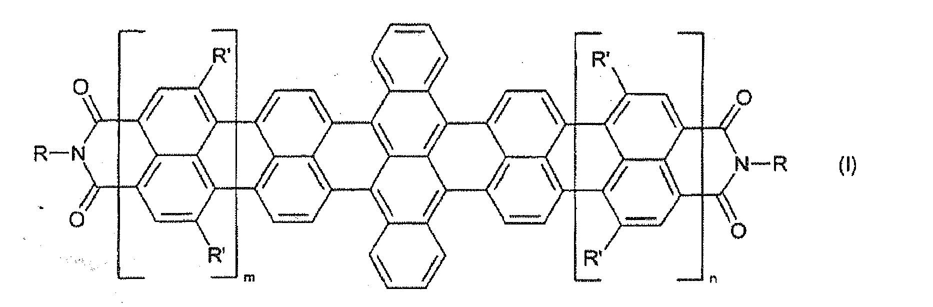 dans laquelle les variables ont la signification suivante :
R' des radicaux identiques ou différents :
hydrogène ;
aryloxy, arylthio, hétaryloxy ou hétarylthio, sur lequel à chaque fois d'autres cycles saturés ou insaturés de 5 à 7 chaînons, dont la structure carbonée peut être interrompue par un ou plusieurs groupements -O-, -S-, -NR¹-, -N=CR¹-, -CO-, -SO-et/ou -SO₂-, peuvent être condensés, l'ensemble du système cyclique pouvant être monosubstitué ou polysubstitué par les radicaux (i), (ii), (iii), (iv) et/ou (v) :
(i) C₁-C₃₀-alkyle, dont la chaîne carbonée peut être interrompue par un ou plusieurs groupements -O-, -S-, -NR¹-, -N=CR¹-, -C≡C-, -CR¹=CR¹-, -CO-, -SO- et/ou -SO₂- et qui peut être monosubstitué ou polysubstitué par : C₁-C₁₂-alcoxy, C₁-C₆-alkylthio, -C≡CR¹, -CR¹=CR¹₂, hydroxy, mercapto, halogène, cyano, nitro, -NR²R³, -NR²COR³, -CONR²R³, -SO₂NR²R³, -COOR², -SO₃R², aryle et/ou C₄-C₇-cycloalkyle saturé ou insaturé, dont la structure carbonée peut être interrompue par un ou plusieurs groupements -O-, -S-, -NR¹-, -N=CR¹, -CR¹=CR¹-, -CO-, -SO- et/ou -SO2-, les radicaux aryle et cycloalkyle pouvant à chaque fois être monosubstitués ou polysubstitués par C₁-C₁₈-alkyle et/ou les radicaux ci-dessus mentionnés comme substituants pour alkyle ;
(ii) C₃-C₈-cycloalkyle, dont la structure carbonée peut être interrompue par un ou plusieurs groupements -O- , -S- , -NR¹- , -N=CR¹-, -CR¹=CR¹-, -CO-, -SO- et/ou -SO₂- et sur lequel d'autres cycles saturés ou insaturés de 5 à 7 chaînons, dont la structure carbonée peut être interrompue par un ou plusieurs groupements -O-, -S-, -NR¹-, -N=CR¹-, -CR¹=CR¹-, -CO-, -SO- et/ou -SO₂-, peuvent être condensés, l'ensemble du système cyclique pouvant être monosubstitué ou polysubstitué par : C₁-C₁₈-alkyle, C₁-C₁₂-alcoxy, C₁-C₆-alkylthio, -C≡CR¹, -CR¹=CR¹₂, hydroxy, mercapto, halogène, cyano, nitro, -NR²R³, -NR²COR³, -CONR²R³, -SO²NR²R³, -COOR² et/ou -SO₃R² ;
(iii) aryle ou hétaryle, sur lequel d'autres cycles saturés ou insaturés de 5 à 7 chaînons, dont la structure carbonée peut être interrompue par un ou plusieurs groupements -O- , -S- , -NR¹- , -N=CR¹- , -CR¹=CR¹-, -CO-, -SO- et/ou -SO₂-, peuvent être condensés, l'ensemble du système cyclique pouvant être monosubstitué ou polysubstitué par : C₁-C₁₈-alkyle, C₁-C₁₂-alcoxy, C₁-C₆-alkylthio, -C≡CR¹, -CR¹=CR¹₂, hydroxy, mercapto, halogène, cyano, nitro, -NR²R³, -NR²COR³, -CONR²R³, -SO₂NR²R³, -COOR², -SO₃R² aryle et/ou hétaryle, qui peut à chaque fois être monosubstitué ou polysubstitué par C₁-C₁₈-alkyle, C₁-C₁₂-alcoxy, hydroxy, mercapto, halogène, cyano, nitro, -NR²R³, -NR²COR³ -CONR²R³, -SO₂NR²R³, -COOR² et/ou -SO₃R² ;
(iv) un radical -U-aryle, qui peut être monosubstitué ou polysubstitué par les radicaux ci-dessus, mentionnés comme substituants pour les radicaux aryle (iii), où U signifie un groupement -O-, -S-, -NR¹-, -CO-, -SO- ou -SO₂- ;
(v) C₁-C₁₂-alcoxy, C₁-C₆-alkylthio, -C≡CR¹, -CR¹=CR¹₂, hydroxy, mercapto, halogène, cyano, nitro, -NR²R³, -NR²COR³, -CONR²R³, -SO₂NR²R³, -COOR² ou -SO₃R² ;
R des radicaux identiques ou différents :
hydrogène ;
C₁-C₃₀-alkyle, dont la chaîne carbonée peut être interrompue par un ou plusieurs groupements -O-, -S-, -NR¹-, -N=CR¹-, -C≡C-, -CR¹=CR¹ -, -CO-, -SO- et/ou -SO₂₋ et qui peut être monosubstitué ou polysubstitué par les radicaux (ii), (iii), (iv) et/ou (v) mentionnés comme substituants pour les radicaux R' ;
C₃-C₈-cycloalkyle, sur lequel d'autres cycles saturés ou insaturés, de 5 à 7 chaînons, dont la structure carbonée peut être interrompue par un ou plusieurs groupements -O-, -S-, -NR¹-, -N=CR¹-, -CR¹=CR¹-, -CO-, -SO- et/ou -SO₂-, peuvent être condensés, l'ensemble du système cyclique pouvant être monosubstitué ou polysubstitué par les radicaux (i), (ii), (iii), (iv) et/ou (v) mentionnés comme substituants pour les radicaux R' ;
aryle ou hétaryle, sur lequel à chaque fois d'autres cycles saturés ou insaturés, de 5 à 7 chaînons, dont la structure carbonée peut être interrompue par un ou plusieurs groupements -O-, -S-, -NR¹-, -N=CR¹ -, -CR¹ =CR¹-, -CO-, -SO- et/ou -SO₂-, peuvent être condensés, l'ensemble du système cyclique pouvant être monosubstitué ou polysubstitué par les radicaux (i), (ii), (iii), (iv), (v) mentionnés comme substituants pour les radicaux R' et/ou arylazo et/ou hétarylazo, qui peut être à chaque fois monosubstitué ou polysubstitué par C₁-C₁₀-alkyle, C₁-C₆-alcoxy et/ou cyano ;
R¹ hydrogène ou C₁-C₁₈-alkyle, où les radicaux R¹ peuvent être identiques ou différents, lorsqu'ils existent plusieurs fois ;
R², R³ indépendamment l'un de l'autre :
hydrogène ;
C₁-C₁₈-alkyle, dont la chaîne carbonée peut être interrompue par un ou plusieurs groupements -O-, -S-, -CO-, -SO- et/ou -SO₂- et qui peut être monosubstitué ou polysubstitué par C₁-C₁₂-alcoxy, C₁-C₆-alkylthio, hydroxy, mercapto, halogène, cyano, nitro, aryle et/ou -COOR¹ ;
aryle ou hétaryle, sur lequel à chaque fois d'autres cycles saturés ou insaturés, de 5 à 7 chaînons, dont la structure carbonée peut être interrompue par un ou plusieurs groupements -O-, -S-, -CO- et/ou -SO₂-, peuvent être condensés, l'ensemble du système cyclique pouvant être monosubstitué ou polysubstitué par C₁-C₁₂-alkyle et/ou les radicaux ci-dessus, mentionnés comme substituants pour alkyle ;
C₃-C₈-cycloalkyle, sur lequel d'autres cycles saturés ou insaturés, de 5 à 7 chaînons, dont la structure carbonée peut être interrompue par un ou plusieurs groupements -O-, -S-, -NR¹-, -N=CR¹-, -CR¹=CR¹-, -CO-, -SO- et/ou -SO₂-,peuvent être condensés, l'ensemble du système cyclique pouvant être monosubstitué ou polysubstitué par C₁-C₁₂-alkyle et/ou les radicaux ci-dessus, mentionnés comme substituants pour alkyle ;
m, n indépendamment l'un de l'autre, 0 ou 1.

2. Diimides de l'acide dibenzorylènetétracarboxylique de formule générale I selon la revendication 1, dans laquelle les variables ont la signification suivante :
R' des radicaux identiques ou différents : hydrogène ;
phénoxy ou thiophénoxy, qui peut être à chaque fois monosubstitué ou polysubstitué par des radicaux (i), (ii), (iii), (iv) et/ou (v) identiques ou différents :
(i) C₁-C₃₀-alkyle, dont la chaîne carbonée peut être interrompue par un ou plusieurs groupements -O- , -S-, -NR¹-, -C≡C- ,-CR¹=CR¹-et/ou -CO- et/ou -SO₂- et qui peut être monosubstitué ou polysubstitué par : C₁-C₁₂-alcoxy, hydroxy, halogène, cyano et/ou aryle, qui peut être monosubstitué ou polysubstitué par C₁-C₁₈-alkyle et/ou C₁-C₆-alcoxy ;
(ii) C₃-C₈-cycloalkyle, dont la structure carbonée peut être interrompue par un ou plusieurs groupements -O-, -S-, -NR¹-, -CR¹=CR¹- et/ou -CO- et qui peut être monosubstitué ou polysubstitué par C₁-C₁₈-alkyle, C₁-C₁₂-alcoxy et/ou C₁-C₆-alkylthio ;
(iii) aryle ou hétaryle, sur lequel à chaque fois d'autres cycles saturés ou insaturés de 5 à 7 chaînons, dont la structure carbonée peut être interrompue par un ou plusieurs groupements -O-, -S-, -NR¹-, -N=CR¹, -CR¹=CR¹-, -CO-, -SO- et/ou -SO₂-, peuvent être condensés, l'ensemble du système cyclique pouvant être monosubstitué ou polysubstitué par : C₁-C₁₈-alkyle, C₁-C₁₂-alcoxy, -C≡CR¹, -CR¹=CR¹₂, hydroxy, halogène, cyano, -NR²R³ , -NR²COR³, -CONR²R³, -SO₂NR²R³, -COOR², -SO₃R², aryle et/ou hétaryle, qui peut à chaque fois être monosubstitué ou polysubstitué par C₁-C₁₈-alkyle, C₁-C₁₈-alcoxy et/ou cyano ;
(iv) un radical -U-aryle, qui peut être monosubstitué ou polysubstitué par les radicaux ci-dessus, mentionnés comme substituants pour les radicaux aryle (iii), où U signifie un groupement -O-, -S-, -NR¹-, -CO-, -SO- ou -SO₂- ;
(v) C₁-C₁₂-alcoxy, C₁-C₆-alkylthio, -C≡CR¹, -CR¹=CR¹₂, hydroxy, mercapto, halogène, cyano, nitro, -NR²R³, -NR²COR³, -CONR²R³, -SO₂NR²R³, -COOR² ou -SO₃R² ;
R des radicaux identiques ou différents : C₁-C₃₀-alkyle, dont la chaîne carbonée peut être interrompue par un ou plusieurs groupements -O- et/ou -CO- et qui peut être monosubstitué ou polysubstitué par : C₁-C₆-alcoxy, cyano et/ou aryle, qui peut être monosubstitué ou polysubstitué par C₁-C₁₈-alkyle ou C₁-C₆-alcoxy ; C₅-C₈-cycloalkyle, qui peut être monosubstitué ou polysubstitué par C₁-C₆-alkyle ;
phényle, naphtyle, pyridyle ou pyrimidyle, qui peut être à chaque fois monosubstitué ou polysubstitué par : C₁-C₁₈-alkyle, C₁-C₆-alcoxy, halogène, cyano, nitro, -CONR²R³, -SO₂NR²R³ et/ou phénylazo et/ou naphtylazo, qui peut à chaque fois être monosubstitué ou polysubstitué par C₁-C₁₀-alkyle, C₁-C₆-alcoxy et/ou cyano ;
R¹ hydrogène ou C₁-C₆-alkyle ;
R², R³ indépendamment l'un de l'autre :
hydrogène
C₁-C₁₈-alkyle, qui peut être monosubstitué ou polysubstitué par C₁-C₆-alcoxy, hydroxy, halogène et/ou cyano ;
aryle ou hétaryle, qui peut être à chaque fois monosubstitué ou polysubstitué par C₁-C₆-alkyle et/ou les radicaux ci-dessus, mentionnés comme substituants pour alkyle ; m, n 0 ou 1.

3. Diimides de l'acide dibenzorylènetétracarboxylique de formule générale I selon la revendication 1, dans laquelle les variables ont la signification suivants :
R' des radicaux identiques :
hydrogène ;
phénoxy, qui peut être monosubstitué ou polysubstitué par des radicaux (i), (ii), (iii), (iv) et/ou (v) identiques ou différents :
(i) C₁-C₁₈-alkyle, dont la chaîne carbonée peut être interrompue par un ou plusieurs groupements -O-, -S-, -NR¹- et/ou -CO- et qui peut être monosubstitué ou polysubstitué par C₁-C₁₂-alcoxy, hydroxy et/ou halogène ;
(ii) C₃-C₈-cycloalkyle, qui peut être monosubstitué ou polysubstitué par C₁-C₁₈-alkyle et/ou C₁-C₁₂-alcoxy ;
(iii) aryle ou hétaryle, qui peut être monosubstitué ou polysubstitué par C₁-C₁₈-alkyle, C₁-C₁₂-alcoxy, hydroxy et/ou halogène ;
(iv) un radical -U-aryle, qui peut être monosubstitué ou polysubstitué par les radicaux ci-dessus, mentionnés comme substituants pour les radicaux aryle (iii), où U signifie un groupement -O-, -S- ou -NR¹- ;
(v) C₁-C₁₂-alcoxy, hydroxy, halogène ou cyano ;
R des radicaux identiques :
C₁-C₃₀-alkyle, dont la chaîne carbonée peut être interrompue par un ou plusieurs groupements -O- et/ou -CO- et qui peut être monosubstitué ou polysubstitué par : C₁-C₆-alcoxy, cyano et/ou aryle, qui peut être monosubstitué ou polysubstitué par C₁-C₁₈-alkyle ou C₁-C₆-alcoxy ; C₅-C₈-cycloalkyle, qui peut être monosubstitué ou polysubstitué par C₁-C₆-alkyle ;
phényle, naphtyle, pyridyle ou pyrimidyle, qui peut être à chaque fois monosubstitué ou polysubstitué par : C₁-C₁₈-alkyle, C₁-C₆-alcoxy, halogène, cyano, nitro, -CONR²R³, -SO₂NR²R³ et/ou phénylazo et/ou naphtylazo, qui peut à chaque fois être monosubstitué ou polysubstitué par C₁-C₁₀-alkyle, C₁-C₆-alcoxy et/ou cyano ;
R¹ hydrogène ou C₁-C₆-alkyle ;
R², R³ indépendamment l'un de l'astre
hydrogène ;
C₁-C₁₈-alkyle, qui peut être monosubstitué ou polysubstitué par C₁-C₆-alcoxy, hydroxy, halogène et/ou cyano ;
aryle ou hétaryle, qui peut être à chaque fois monosubstitué ou polysubstitué par C₁-C₆-alkyle et/ou les radicaux ci-dessus, mentionnés comme substituants pour alkyle ;
m, n 1.

4. Diimides de l'acide dibenzorylènetétracarboxylique selon l'une quelconque des revendications précédentes, où les radicaux R sont choisis indépendamment l'un de l'autre parmi les groupes des formules II.1 à II.5 :
#-(A)ₚ-C(Rⁱ)ₓ (II.1) 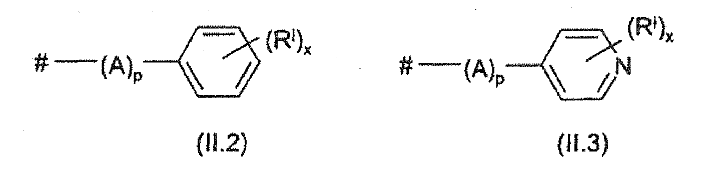 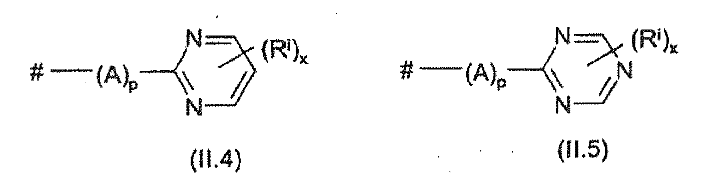
où
# signifie le site de liaison à l'atome d'azote de la fonction imide,
p vaut 0 ou 1,
x dans les composés de formule II.1, vaut 2 ou 3, dans les composés des formules II.2, II.3 et II.4, vaut 1, 2 ou 3 et dans les composés de formule II.5, vaut 1 ou 2,
A pour autant qu'il soit présent, représente un groupe C₁-C₁₀-alkylène, qui peut être interrompu par un ou plusieurs groupes non adjacents, qui sont choisis parmi -O- et -S-,
- où, pour le cas où dans les composés de formule II.1, x vaut 2, l'atome de carbone qui porte les radicaux Rⁱ, porte en plus un atome H,
- les radicaux Rⁱ sont choisis, à chaque fois indépendamment l'un de l'autre, parmi C₁-C₃₀-alkyle, qui peuvent être interrompus par un ou plusieurs atomes d'oxygène non adjacents, où, dans les composés de formule II.1, au moins un des radicaux Rⁱ peut également représenter C₁-C₃₀-alkyloxy ou C₁-C₃₀-alkylthio.

5. Diimides de l'acide dibenzorylènetétracarboxylique selon l'une quelconque des revendications 1 à 4, où les radicaux R' représentent tous les deux hydrogène.

6. Diimides de l'acide dibenzorylènetétracarboxylique selon l'une quelconque des revendications 1 à 4, où les radicaux R' sont choisis parmi les radicaux de formule 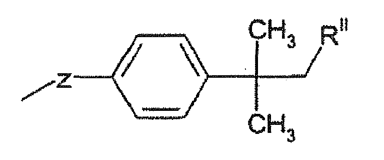 où
Z représente O ou S, et
R^{II} est choisi parmi l'hydrogène,
C₁-C₈-alkyle, dont la chaîne carbonée peut être interrompue par un ou plusieurs groupements -O-, -S-, -NR¹-, -C(=O)- et/ou -SO₂- et qui peut être monosubstitué ou polysubstitué par C₁-C₆-alcoxy et/ou un radical hétérocyclique de 5 à 7 chaînons, lié via un atome d'azote, qui peut contenir d'autres hétéroatomes et qui peut être aromatique, et
C₅-C₈-cycloalkyle, dont la structure carbonée peut être interrompue par un ou plusieurs groupements -O-, -S-, -NR¹-, -C(=O)- et/ou -SO₂- et qui peut être monosubstitué ou polysubstitué par C₁-C₆-alkyle, où R¹ représente hydrogène ou C₁-C₆-alkyle.

7. Procédé pour la préparation de diimides de l'acide dibenzorylènetétracarboxylique de formule générale Ia 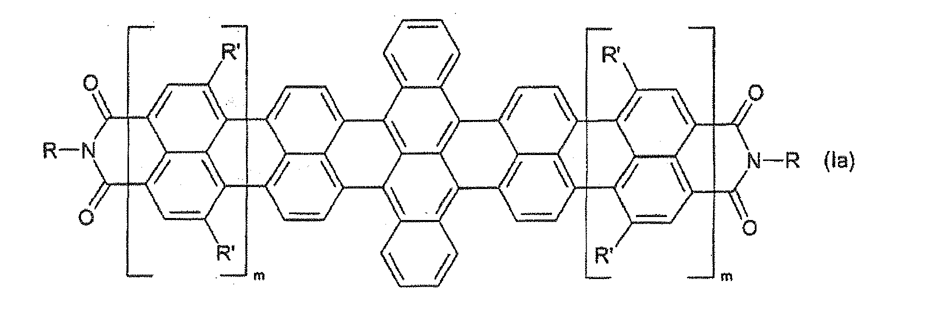 dans laquelle R, R' et m présentent la signification indiquée dans la revendication 1, **caractérisé en ce qu'**on
a) soumet un imide de l'acide péri-(dioxaborolan-2-yl)rylènedicarboxylique de formule générale IIa 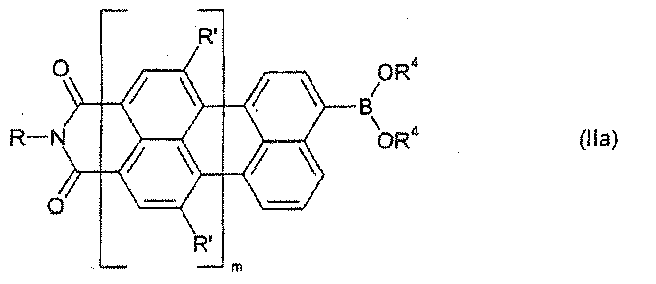 dans laquelle R⁴ sont identiques ou différents et représentent, indépendamment l'un de l'autre, hydrogène, C₁-C₃₀-alkyle, C₅-C₈-cycloalkyle, aryle ou hétéroaryle, et où deux radicaux OR⁴ liés au même atome de bore peuvent également représenter, ensemble, -OCH₂CH₂O-, où 1, 2, 3 ou 4 atomes d'hydrogène peuvent également être remplacés par des groupes C₁-C₃₀-alkyle, C₅-C₈-cycloalkyle, aryle ou hétéroaryle,
en présence d'un solvant organique, si souhaité en mélange avec de l'eau, ainsi que d'un catalyseur à base d'un métal de transition et d'une base, à une réaction de couplage de Suzuki avec du 5,11-dibromotétracène de formule III 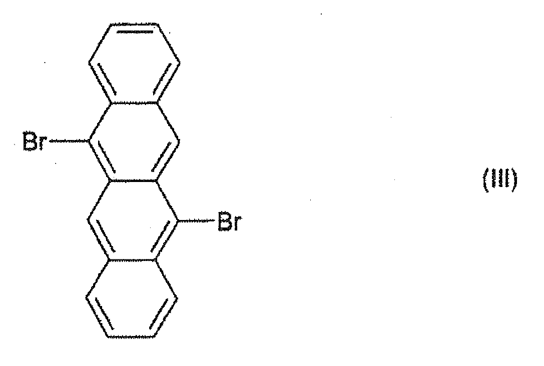
b) soumet le tétracène-5,11-bis(imide de l'acide rylènedicarboxylique) de formule générale IVa formé dans l'étape a) 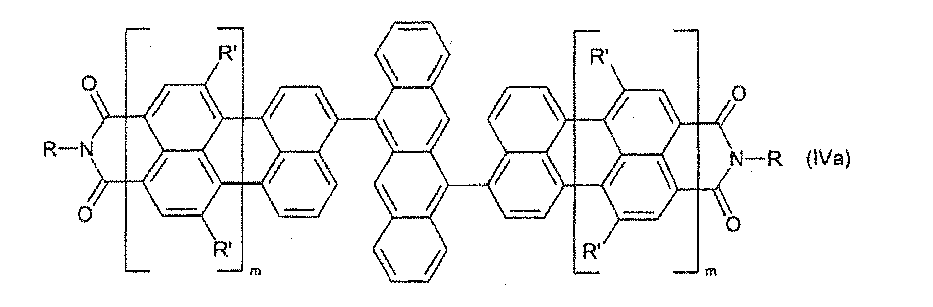 à une première cyclodéshydrogénation en présence d'un solvant organique inerte et d'un acide de Lewis et
c) cyclodéshydrogène davantage le dérivé de bisrylène de formule générale Va formé dans l'étape b) 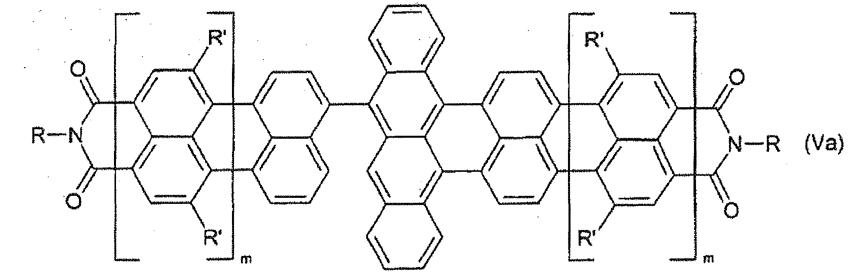 dans un milieu réactionnel organique présentant des fonctions hydroxy et amino et contenant une base essentiellement non dissoute en diimide de l'acide dibenzorylènetétracarboxylique Ia.

8. Procédé pour la préparation de diimides de l'acide dibenzorylènetétracarboxylique de formule générale Ib 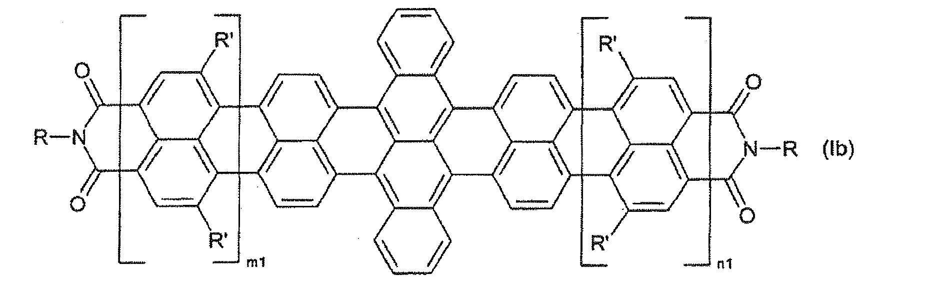 dans laquelle R et R' présentent la signification indiquée dans la revendication 1, m1 et n1 sont différents l'un de l'autre et valent 0 ou 1, **caractérisé en ce qu'**on
a1) soumet un imide de l'acide péri-(dioxaborolan-2-yl)rylènedicarboxylique de formule générale IIb1 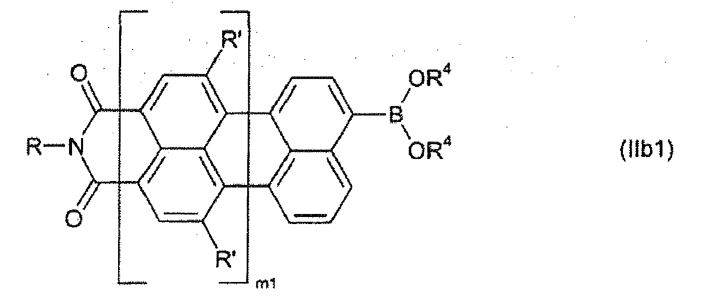 dans laquelle R⁴ sont identiques ou différents et représentent, indépendamment l'un de l'autre, hydrogène, C₁-C₃₀-alkyle, C₅-C₈-cycloalkyle, aryle ou hétéroaryle, et où deux radicaux OR⁴ liés au même atome de bore peuvent également représenter, ensemble, -OCH₂CH₂O-, où 1, 2, 3 ou 4 atomes d'hydrogène peuvent également être remplacés par des groupes C₁-C₃₀-alkyle, C₅-C₈-cycloalkyle, aryle ou hétéroaryle,
à une première réaction de couplage de Suzuki avec du 5,11-dibromotétracène (III) et
a2) soumet l'imide de l'acide 5-bromotétracène-11-rylènedicarboxylique de formule générale III' formé dans l'étape a1) 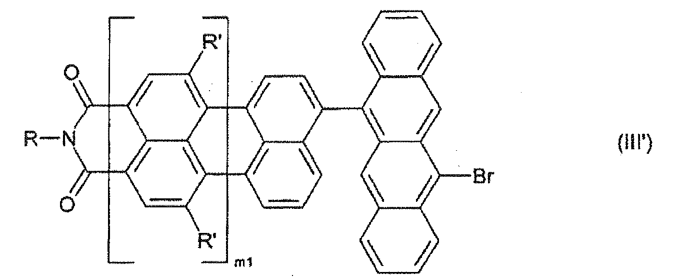 à une deuxième réaction de couplage de Suzuki avec un imide de l'acide péri-(dioxaborolan-2-yl)rylènedicarboxylique de formule générale IIb2 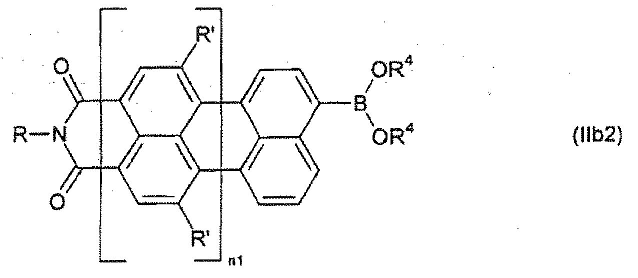 en présence d'un solvant organique, si souhaité en mélange avec de l'eau, ainsi que d'un catalyseur à base d'un métal de transition et d'une base,
b) soumet le tétracène-5,11-bis(imide de l'acide rylènedicarboxylique) de formule générale IVb formé dans l'étape a2) 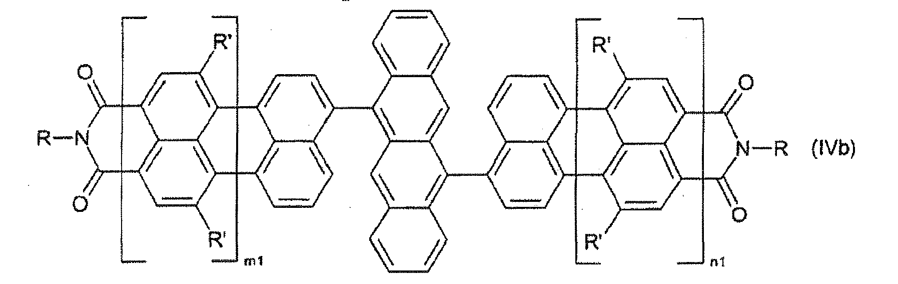 à une première cyclodéshydrogénation en présence d'un solvant organique inerte et d'un acide de Lewis et
c) cyclodéshydrogène davantage le dérivé bisrylène de formule générale Vb formé dans l'étape b) 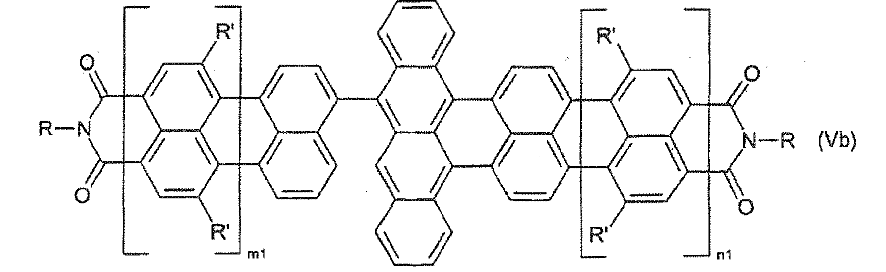 dans un milieu réactionnel organique présentant des fonctions hydroxy et amino et contenant une base essentiellement non dissoute en diimide de l'acide dibenzorylènetétracarboxylique Ib.

9. Tétracène-5,11-bis(imides de l'acide rylènedicarboxylique) de formule générale IV 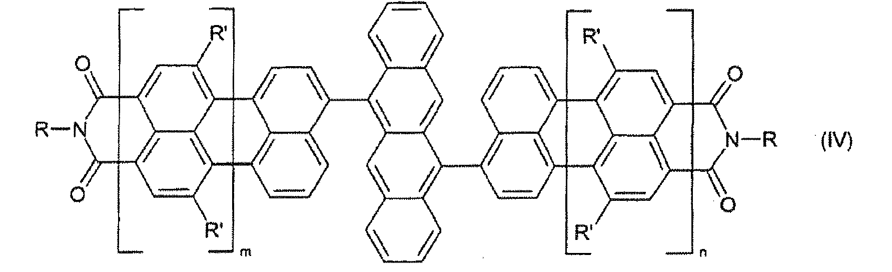 dans laquelle les variables ont la signification suivants :
R' des radicaux identiques ou différents :
hydrogène ;
aryloxy, arylthio, hétaryloxy ou hétarylthio, sur lequel à chaque fois d'autres cycles saturés ou insaturés de 5 à 7 chaînons, dont la structure carbonée peut être interrompue par un ou plusieurs groupements -O-, -S-, -NR¹-, -N=CR¹-, -CO-, -SO-et/ou -SO₂-, peuvent être condensés, l'ensemble du système cyclique pouvant être monosubstitué ou polysubstitué par les radicaux (i), (ii), (iii), (iv) et/ou (v) :
(i) C₁-C₃₀-alkyle, dont la chaîne carbonée peut être interrompue par un ou plusieurs groupements -O-, -S-, -NR¹-, -N=CR¹-, -C=C-, -CR¹=CR¹-, -CO-, -SO- et/ou -SO₂- et qui peut être monosubstitué ou polysubstitué par : C₁-C₁₂-alcoxy, C₁-C₆-alkylthio, -C≡CR¹, -CR¹=CR¹₂, hydroxy, mercapto, halogène, cyano, nitro, -NR²R³, -NR²COR³, -CONR²R³, -SO₂NR²R³ -COOR², -SO₃R², aryle et/ou C₄-C₇-cycloalkyle saturé ou insaturé, dont la structure carbonée peut être interrompue par un ou plusieurs groupements -O-, -S-, -NR¹-, -N=CR¹, -CR¹=CR¹-, -CO-, -SO- et/ou -SO₂-, les radicaux aryle et cycloalkyle pouvant à chaque fois être monosubstitués ou polysubstitués par C₁-C₁₈-alkyle et/ou les radicaux ci-dessus mentionnés comme substituants pour alkyle ;
(ii) C₃-C₈-cycloalkyle, dont la structure carbonée peut être interrompue par un ou plusieurs groupements -O-, -S-, -NR¹-, -N=CR¹-, -CR¹=CR¹-, -CO-, -SO- et/ou -SO₂- et sur lequel d'autres cycles saturés ou insaturés de 5 à 7 chaînons, dont la structure carbonée peut être interrompue par un ou plusieurs groupements -O-, -S-, -NR¹-, -N=CR¹-, -CR¹=CR¹-, -CO-, -SO- et/ou -SO₂-, peuvent être condensés, l'ensemble du système cyclique pouvant être monosubstitué ou polysubstitué par : C₁-C₁₈-alkyle, C₁-C₁₂-alcoxy, C₁-C₆-alkylthio, -C≡CR¹, -CR¹=CR¹₂, hydroxy, mercapto, halogène, cyano, nitro, -NR²R³, -NR²COR³, -CONR²R³, -SO₂NR²R³, -COOR² et/ou -SO₃R²;
(iii) aryle ou hétaryle, sur lequel d'autres cycles saturés ou insaturés de 5 à 7 chaînons, dont la structure carbonée peut être interrompue par un ou plusieurs groupements -O-, -S-, -NR¹-, -N=CR¹-, -CR¹=CR¹-, -CO-, -SO- et/ou -SO₂-, peuvent être condensés, l'ensemble du système cyclique pouvant être monosubstitué ou polysubstitué par : C₁-C₁₈-alkyle, C₁-C₁₂-alcoxy, C₁-C₆-alkylthio, -C≡CR¹, -CR¹=CR¹₂, hydroxy, mercapto, halogène, cyano, nitro, -NR²R³, -NR²COR³, -CONR²R³, -SO₂NR²R³, -COOR², -SO₃R², aryle et/ou hétaryle, qui peut à chaque fois être monosubstitué ou polysubstitué par C₁-C₁₈-alkyle, C₁-C₁₂-alcoxy, hydroxy, mercapto, halogène, cyano, nitro, -NR²R³, -NR²COR³, -CONR²R³, -SO₂NR²R³, -COOR² et/ou -SO₃R²;
(iv) un radical -U-aryle, qui peut être monosubstitué ou polysubstitué par les radicaux ci-dessus, mentionnés comme substituants pour les radicaux aryle (iii), où U signifie un groupement -O-, -S-, -NR¹-, -CO-, -SO- ou -SO₂- ;
(v) C₁-C₁₂-alcoxy, C₁-C₆-alkylthio, -C≡CR¹, -CR¹=CR¹₂, hydroxy, mercapto, halogène, cyano, nitro, -NR²R³, -NR²COR³, -CONR²R³, -SO₂NR²R³, -COOR² ou -SO₃R² ;
R des radicaux identiques ou différents :
hydrogène ;
C₁-C₃₀-alkyle, dont la chaîne carbonée peut être interrompue par un ou plusieurs groupements -O-, -S-, -NR¹-, -N=CR¹-, -C≡C-, -CR¹=CR¹-, -CO-, -SO- et/ou -SO₂- et qui peut être monosubstitué ou polysubstitué par les radicaux (ii), (iii), (iv) et/ou (v) mentionnés comme substituants pour les radicaux R' ;
C₃-C₈-cycloalkyle, sur lequel d'autres cycles saturés ou insaturés, de 5 à 7 chaînons, dont la structure carbonée peut être interrompue par un ou plusieurs groupements -O-, -S-, -NR¹-, -N=CR¹-, -CR¹=CR¹-, -CO-, -SO- et/ou -SO₂-, peuvent être condensés, l'ensemble du système cyclique pouvant être monosubstitué ou polysubstitué par les radicaux (i), (ii), (iii), (iv) et/ou (v) mentionnés comme substituants pour les radicaux R' ;
aryle ou hétaryle, sur lequel à chaque fois d'autres cycles saturés ou insaturés, de 5 à 7 chaînons, dont la structure carbonée peut être interrompue par un ou plusieurs groupements -O-, -S-, -NR¹-, -N=CR¹-, -CR¹=CR¹-, -CO-, -SO- et/ou -SO₂-, peuvent être condensés, l'ensemble du système cyclique pouvant être monosubstitué ou polysubstitué par les radicaux (i), (ii), (iii), (iv), (v) mentionnés comme substituants pour les radicaux R' et/ou arylazo et/ou hétarylazo, qui peut être à chaque fois monosubstitué ou polysubstitué par C₁-C₁₀-alkyle, C₁-C₆-alcoxy et/ou cyano ;
R¹ hydrogène ou C₁-C₁₈-alkyle, où les radicaux R¹ peuvent être identiques ou différents, lorsqu'ils existent plusieurs fois ;
R², R³ indépendamment l'un de l'astre
hydrogène ;
C₁-C₁₈-alkyle, dont la chaîne carbonée peut être interrompue par un ou plusieurs groupements -O-, -S-, -CO-, -SO- et/ou -SO₂- et qui peut être monosubstitué ou polysubstitué par C₁-C₁₂-alcoxy, C₁-C₆-alkylthio, hydroxy, mercapto, halogène, cyano, nitro, aryle et/ou -COOR¹;
aryle ou hétaryle, sur lequel à chaque fois d'autres cycles saturés ou insaturés, de 5 à 7 chaînons, dont la structure carbonée peut être interrompue par un ou plusieurs groupements -O-, -S-, -CO- et/ou -SO₂-, peuvent être condensés, l'ensemble du système cyclique pouvant être monosubstitué ou polysubstitué par C₁-C₁₂-alkyle et/ou les radicaux ci-dessus, mentionnés comme substituants pour alkyle ;
C₃-C₈-cycloalkyle, sur lequel d'autres cycles saturés ou insaturés, de 5 à 7 chaînons, dont la structure carbonée peut être interrompue par un ou plusieurs groupements -O-, -S-, -NR¹- -NR^{1a}, -N=CR¹-, -CR¹=CR¹-, -CO-, -SO- et/ou -SO₂-, peuvent être condensês, l'ensemble du système cyclique pouvant être monosubstitué ou polysubstitué par C₁-C₁₂-alkyle et/ou les radicaux ci-dessus, mentionnés comme substituants pour alkyle ;
m, n indépendamment l'un de l'autre, 0 ou 1.

10. Dérivés de bisrylène de formule générale V 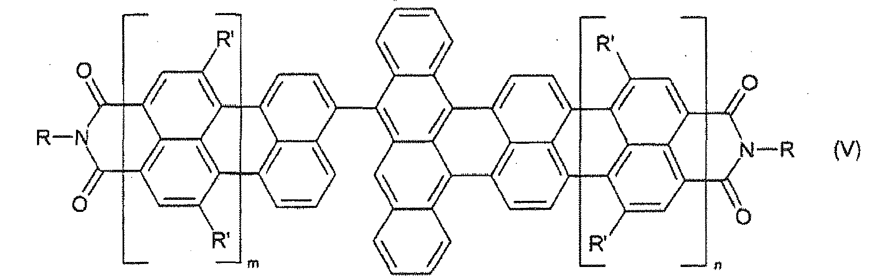 dans laquelle les variables ont la signification suivante :
R' des radicaux identiques ou différents : signifie hydrogène ;
aryloxy, arylthio, hétaryloxy ou hétarylthio, sur lequel à chaque fois d'autres cycles saturés ou insaturés de 5 à 7 chaînons, dont la structure carbonée peut être interrompue par un ou plusieurs groupements -O-, -S-, -NR¹-, -N=CR¹-, -CO-, -SO-et/ou -SO₂-, peuvent être condensés, l'ensemble du système cyclique pouvant être monosubstitué ou polysubstitué par les radicaux (i), (ii), (iii), (iv) et/ou (v) :
(i) C₁-C₃₀-alkyle, dont la chaîne carbonée peut être interrompue par un ou plusieurs groupements -O-, -S-, -NR¹-, -N=CR¹-, -C≡C-, -CR¹=CR¹-, -CO-, -SO- et/ou -SO₂- et qui peut être monosubstitué ou polysubstitué par : C₁-C₁₂-alcoxy, C₁-C₆-alkylthio, -C≡CR¹, -CR¹=CR¹₂, hydroxy, mercapto, halogène, cyano, nitro, -NR²R³, -NR²COR³, -CONR²R³, -SO₂NR²R³, -COOR², -SO₃R², aryle et/ou C₄-C₇-cycloalkyle saturé ou insaturé, dont la structure carbonée peut être interrompue par un ou plusieurs groupements -O-, -S-, -NR¹-, -N=CR¹, -CR¹=CR¹-, -CO-, -SO- et/ou -SO₂-, les radicaux aryle et cycloalkyle pouvant à chaque fois être monosubstitués ou polysubstitués par C₁-C₁₈-alkyle et/ou les radicaux ci-dessus mentionnés comme substituants pour alkyle ;
(ii) C₃-C₈-cycloalkyle, dont la structure carbonée peut être interrompue par un ou plusieurs groupements -O- , -S-, -NR¹-, -N=CR¹-, -CR¹=CR¹-, -CO-, -SO- et/ou -SO₂- et sur lequel d'autres cycles saturés ou insaturés de 5 à 7 chaînons, dont la structure carbonée peut être interrompue par un ou plusieurs groupements -O-, -S-, -NR¹-, -N=CR¹-, -CR¹=CR¹-, -CO-, -SO- et/ou -SO₂-, peuvent être condensés, l'ensemble du système cyclique pouvant être monosubstitué ou polysubstitué par : C₁-C₁₈-alkyle, C₁-C₁₂-alcoxy, C₁-C₆-alkylthio, -C≡CR¹, -CR¹=CR¹₂, hydroxy, mercapto, halogène, cyano, nitro, -NR²R³, NR²COR³, -CONR²R³, -SO₂NR²R³, -COOR² et/ou -SO₃R² ;
(iii) aryle ou hétaryle, sur lequel d'autres cycles saturés ou insaturés de 5 à 7 chaînons, dont la structure carbonée peut être interrompue par un ou plusieurs groupements -O-, -S-, -NR¹-, -N=CR¹-, -CR¹=CR¹-, -CO-, -SO- et/ou -SO₂-, peuvent être condensés, l'ensemble du système cyclique pouvant être monosubstitué ou polysubstitué par : C₁-C₁₈-alkyle, C₁-C₁₂-alcoxy, C₁-C₆-alkylthio, -C≡CR¹, -CR¹=CR¹₂, hydroxy, mercapto, halogène, cyano, nitro, -NR²R³, -NR²COR³, -CONR²R³, -SO₂NR²R³, -COOR², -SO₃R², aryle et/ou hétaryle, qui peut à chaque fois être monosubstitué ou polysubstitué par C₁-C₁₈-alkyle, C₁-C₁₂-alcoxy, hydroxy, mercapto, halogène, cyano, nitro, -NR²R³, -NR²COR³, -CONR²R³, -SO₂NR²R³, -COOR² et/ou -SO₃R² ;
(iv) un radical -U-aryle, qui peut être monosubstitué ou polysubstitué par les radicaux ci-dessus, mentionnés comme substituants pour les radicaux aryle (iii), où U signifie un groupement -O-, -S-, -NR¹-, -CO-, -SO- ou -SO₂- ;
(v) C₁-C₁₂-alcoxy, C₁-C₆-alkylthio, -C≡CR¹, -CR¹=CR¹₂, hydroxy, mercapto, halogène, cyano, nitro, -NR²R³, -NR²COR³, -CONR²R³, -SO₂NR²R³, -COOR² ou -SO₃R²;
R des radicaux identiques ou différents :
hydrogène ;
C₁-C₃₀-alkyle, dont la chaîne carbonée peut être interrompue par un ou plusieurs groupements -O-, -S-, -NR¹-, -N=CR¹-, -C≡C-, -CR¹=CR¹-, -CO-, -SO-et/ou -SO₂- et qui peut être monosubstitué ou polysubstitué par les radicaux (ii), (iii), (iv) et/ou (v) mentionnés comme substituants pour les radicaux R' ;
C₃-C₈-cycloalkyle, sur lequel d'autres cycles saturés ou insaturés, de 5 à 7 chaînons, dont la structure carbonée peut être interrompue par un ou plusieurs groupements -O-, -S-, -NR¹-, -N=CR¹-, -CR¹=CR¹-, -CO-, -SO- et/ou -SO₂-, peuvent être condensés, l'ensemble du système cyclique pouvant être monosubstitué ou polysubstitué par les radicaux (i), (ii), (iii), (iv) et/ou (v) mentionnés comme substituants pour les radicaux R'
aryle ou hétaryle, sur lequel à chaque fois d'autres cycles saturés ou insaturés, de 5 à 7 chaînons, dont la structure carbonée peut être interrompue par un ou plusieurs groupements -O-, -S-, -NR¹-, -N=CR¹-, -CR¹=CR¹-, -CO-, -SO- et/ou -SO₂-, peuvent être condensés, l'ensemble du système cyclique pouvant être monosubstitué ou polysubstitué par les radicaux (i), (ii), (iii), (iv), (v) mentionnés comme substituants pour les radicaux R' et/ou arylazo et/ou hétarylazo, qui peut être à chaque fois monosubstitué ou polysubstitué par C₁-C₁₀-alkyle, C₁-C₆-alcoxy et/ou cyano ;
R¹ hydrogène ou C₁-C₁₈-alkyle, où les radicaux R¹ peuvent être identiques ou différents, lorsqu'ils existent plusieurs fois ;
R², R³ indépendamment l'un de l'autre :
hydrogène ;
C₁-C₁₈-alkyle, dont la chaîne carbonée peut être interrompue par un ou plusieurs groupements -O-, -S-, -CO-, -SO- et/ou -SO₂- et qui peut être monosubstitué ou polysubstitué par C₁-C₁₂-alcoxy, C₁-C₆-alkylthio, hydroxy, mercapto, halogène, cyano, nitro, aryle te/ou -COOR¹ ;
aryle ou hétaryle, sur lequel à chaque fois d'autres cycles saturés ou insaturés, de 5 à 7 chaînons, dont la structure carbonée peut être interrompue par un ou plusieurs groupements -O-, -S-, -CO- et/ou -SO₂-, peuvent être condensés, l'ensemble du système cyclique pouvant être monosubstitué ou polysubstitué par C₁-C₁₂-alkyle et/ou les radicaux ci-dessus, mentionnés comme substituants pour alkyle ;
C₃-C₈-cycloalkyle, sur lequel d'autres cycles saturés ou insaturés, de 5 à 7 chaînons, dont la structure carbonée peut être interrompue par un ou plusieurs groupements -O-, -S-, -NR¹-, -NR^{1a}, -N=CR¹-, -CR₁=CR¹-, -CO-, -SO- et/ou -SO₂-, peuvent être condensés, l'ensemble du système cyclique pouvant être monosubstitué ou polysubstitué par
C₁-C₁₂-alkyle et/ou les radicaux ci-dessus, mentionnés comme substituants pour alkyle ;
m, n indépendamment l'un de l'autre, 0 ou 1.

11. Utilisation de diimides de l'acide dibenzorylènetétracarboxylique selon les revendications 1 à 6 comme matériaux absorbant les rayons laser IR lors du traitement par soudure de pièces en matériau synthétique.

12. Utilisation de diimides de l'acide dibenzorylènetétracarboxylique selon les revendications 1 à 6 comme absorbants des infrarouges pour la gestion de la chaleur.

13. Utilisation de diimides de l'acide dibenzorylènetétracarboxylique selon les revendications 1 à 6 comme composants actifs dans le domaine photovoltaïque.

14. Utilisation de diimides de l'acide dibenzorylènetétracarboxylique, tels que définis dans l'une quelconque des revendications 1 à 6, dans le domaine de la photovoltaïque organique, en particulier comme semi-conducteur dans les cellules solaires excitoniques.

15. Transistor à effet de champ, organique, comprenant un substrat présentant au moins une structure de grille, une électrode de source et une électrode de drain et au moins un composé de formule I, tel que défini dans l'une quelconque des revendications 1 à 6, comme semi-conducteur n.

16. Substrat présentant une multitude de transistors à effet de champ, organiques, au moins une partie des transistors à effet de champ contenant au moins un composé de formule I, tel que défini dans l'une quelconque des revendications 1 à 6, comme semi-conducteur n.

17. Utilisation d'au moins un composé de formule générale I, tel que défini dans l'une quelconque des revendications 1 à 6, pour le marquage optique destiné au marquage invisible de produits, comme colorants de fluorescence, comme marqueur de fluorescence pour des biomolécules et comme pigments.

18. Utilisation d'au moins un composé de formule générale I, tel que défini dans l'une quelconque des revendications 1 à 6, comme colorant de fluorescence dans un affichage basé sur une conversion de fluorescence ; dans une pièce en matériau synthétique collectrice de lumière, qui est le cas échéant combinée avec une cellule solaire ; comme colorant pigmentaire dans les affichages électrophorétiques ; comme colorant de fluorescence dans une application basée sur la chimiluminescence.
